(19) Europäisches Patentamt
European Patent Office
Office européen des brevets

(11) EP 1 498 490 A1

(12) EUROPEAN PATENT APPLICATION
published in accordance with Art. 158(3) EPC

(43) Date of publication:
**19.01.2005 Bulletin 2005/03**

(21) Application number: **03723096.8**

(22) Date of filing: **09.04.2003**

(51) Int Cl.[7]: **C12P 21/08**, C12N 15/09, C12N 5/10, C07K 16/18 // (A61K39/395, A61P9:00, 29:00, 31:04, 31:12, 31:14, 35:00, 37:02, 37:04, 37:08, 43:00, G01N33:15, 33:50, C12P21:08), C12R1:91, C12N5:10, C12R1:91

(86) International application number:
**PCT/JP2003/004502**

(87) International publication number:
**WO 2003/085118 (16.10.2003 Gazette 2003/42)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **09.04.2002 JP 2002106820**
**31.01.2003 JP 2003024685**

(71) Applicant: **KYOWA HAKKO KOGYO CO., LTD.**
**Chiyoda-ku, Tokyo 100-8185 (JP)**

(72) Inventors:
- **Satoh, Mitsuo, Tokyo Research Laboratories Machida-shi, Tokyo 194-8533 (JP)**
- **Kamachi, Reiko, Tokyo Research Laboratories Machida-shi, Tokyo 194-8533 (JP)**
- **Kanda, Yutaka, Tokyo Research Laboratories Machida-shi, Tokyo 194-8533 (JP)**
- **Mori, Katsuhiro, Tokyo Research Laboratories Machida-shi, Tokyo 194-8533 (JP)**
- **Yamano, Kazuya, Tokyo Research Laboratories Machida-shi, Tokyo 194-8533 (JP)**
- **Kinoshita, Satoko, Pharmaceutical Research Instit. Nagaizumi-c., Sunto-g.,Shizuoka 411-8731 (JP)**
- **Iida, Shigeru, Tokyo Research Laboratories Machida-shi, Tokyo 194-8533 (JP)**

(74) Representative: **VOSSIUS & PARTNER**
**Siebertstrasse 4**
**81675 München (DE)**

Remarks:
A request for correction (Rule 88 EPC) of Fig. 35 and 36 has been filed.

# (54) PROCESS FOR PRODUCING ANTIBODY COMPOSITION

(57) A process for producing an antibody composition using a cell, which comprises using a cell resistant to a lectin which recognizes a sugar chain in which 1-position of fucose is bound to 6-position of *N*-acetylglucosamine in the reducing end through α-bond in a complex *N*-glycoside-linked sugar chain, and a cell used for the process.

EP 1 498 490 A1

## Description

TECHNICAL FIELD

The present invention relates to a process for producing an antibody composition, and a cell used for producing the antibody composition.

BACKGROUND ART

Regarding the sugar chain of an antibody, Boyd *et al.* have examined effects of a sugar chain on antibody-dependent cell-mediated cytotoxic activity (hereinafter referred to as "ADCC activity") and complement-dependent cytotoxic activity (hereinafter referred to as "CDC activity") by treating a human complementarity determining region (hereinafter referred to as "CDR")-grafted antibody CAMPATH-1H (human IgG1 subclass) produced by a Chinese hamster ovary cell (CHO cell) or a mouse myeloma-derived NS0 cell with various sugar hydrolyzing enzymes, and reported that elimination of the non-reducing end sialic acid did not have influence upon both activities, but the CDC activity alone was affected by further removal of galactose residue and about 50% of the activity was decreased, and that complete removal of the sugar chain caused disappearance of both activities [*Molecular Immunol*., 32, 1311 (1995)]. Also, Lifely *et al*. have analyzed the sugar chain bound to a human CDR-grafted antibody CAMPATH-1H (human IgG1 subclass) which was produced by CHO cell, NS0 cell or rat myeloma Y0 cell, measured its ADCC activity, and reported that the CAMPATH-1H derived from Y0 cell showed the highest ADCC activity, suggesting that *N*-acetylglucosamine (hereinafter sometimes referred to as "GlcNAc") at the bisecting position is important for the activity [*Glycobiology,* 5, 813 (1995); WO 99/54342].

Furthermore, addition-modification of fucose to *N*-acetylglucosamine in the reducing end in the *N*-glycoside-linked sugar chain of an antibody changes the ADCC activity of the antibody greatly (WO00/61739). These reports indicate that the structure of the sugar chain plays an important role in the effector functions of human antibodies of IgGl subclass.

In general, most of the humanized antibodies of which application to medicaments is in consideration are prepared using genetic recombination techniques and produced using animal cells, such as Chinese hamster ovary tissue-derived CHO cell, as the host cell. But as described above, since the sugar chain structure plays a remarkably important role in the effector function of antibodies and differences are observed in the sugar chain structure of glycoproteins expressed by host cells, development of a host cell which can be used for the production of an antibody having higher effector function is desired.

As a method for adjusting an enzyme relating to the modification of a sugar chain in a host cell and modifying the sugar chain structure of a produced glycoprotein, application of an inhibitor against an enzyme relating to the modification of a sugar chain has been attempted.

An inhibitor against an enzyme relating to the modification of a sugar chain includes tunicamycin which selectively inhibits formation of GlcNAc-P-P-Dol which is the first step of the formation of a core oligosaccharide which is a precursor of an *N*-glycoside-linked sugar chain, castanospermin and *N*-methyl-1-deoxynojirimycin which are inhibitors of glycosidase I, bromocondulitol which is an inhibitor of glycosidase II, 1-deoxynojirimycin and 1,4-dioxy-1,4-imino-D-mannitol which are inhibitors of mannosidase I, swainsonine which is an inhibitor of mannosidase II and the like. As an inhibitor specific for a glycosyltransferase, deoxy derivatives of substrates against *N*-acetylglucosamine transferase V (GnTV) and the like are being developed [*Glycobiology Series 2 - Destiny of Sugar Chain in Cell* (Kodansha Scientific), edited by Katsutaka Nagai, Senichiro Hakomori and Akira Kobata (1993)]. Also, it is known that 1-deoxynojirimycin inhibits synthesis of a complex type sugar chain and increases the ratio of high mannose type and hybrid type sugar chains. Actually, it has been reported that sugar chain structure of IgG was changed and properties such as antigen binding activity and antibody-dependent cell-mediated cytotoxic activity were changed when the inhibitors such as castanospermin, *N*-methyl-1-deoxynojirimycin, swainsonine and tunicamycin were added to a medium [*Molecular Immunol.,* 26, 1113 (1989)]. However, since these inhibitors have weak specificity and it is difficult to sufficiently inhibit the target enzyme, it is difficult to surely control the sugar chain structure of an produced antibody.

Furthermore, modification of a sugar chain structure of a produced glycoprotein has been attempted by introducing a gene encoding an enzyme relating to the modification of a sugar chain. As examples, it has been reported that 1) a protein in which a number of sialic acid is added to the non-reducing end of the sugar chain can be produced by introducing rat β-galactoside-α-2,6-sialyltransferase into CHO cell [*J. Biol. Chem.,* 261, 13848 (1989)], 2) an H antigen (Fucα1-2Galβ1-) in which fucose (hereinafter also referred to as "Fuc") was added to the non-reducing end of the sugar chain was expressed by introducing human β-galactoside-2-α-fucosyltransferase into mouse L cell [*Science,* 252, 1668 (1991)], and 3) when an antibody is produced by using CHO cell into which β-1,4-*N*-acetylglucosamine transferase III (GnTIII) is introduced, an antibody having a higher ratio of addition of the bisecting-positioned *N*-acetylglucosamine of *N*-glycoside-linked sugar chain can be produced [*Glycobiology,* 5, 813 (1995): WO 99/54342].

When an antibody was expressed by using CHO cell into which GnTIII was introduced, the antibody had ADCC activity 16 times higher than the antibody expressed by using the parent cell line. However, since it has been reported that excess expression of GnTIII or β-1,4-*N*-acetylglucosamine transferase V (GnTV) shows toxicity for CHO cell, it is not suitable for the production of therapeutic antibodies.

It has been reported that a glycoprotein having changed sugar chain structure can be produced when a mutant in which the activity of a gene encoding an enzyme relating to the modification of a sugar chain is used as a host cell. Specifically, it is known that an antibody having a high mannose type sugar chain structure can be produced by using a CHO cell mutant cell line deficient in the activity of *N*-acetylglucosamine transferase I (GnTI) [*J. Immuno*., 160, 3393 (1998)]. Also, expression of an antibody having a sugar chain structure in which sialic acid is not added to the non-reducing end side in the sugar chain and expression of an antibody having no addition of galactose, using CMP-sialic acid transporter- and UDP-galactose transporter-deficient cell lines, have been reported [*J. Immuno.,* 160, 3393 (1998)]. However, no success has been reported in expressing an antibody having an improved effector function suitable for application to medicaments. In addition, since a mutation is introduced at random by a mutagen treatment in these cell lines, they are not appropriate as cell lines used in the production of pharmaceutical preparations.

Thus, in order to modify the sugar chain structure of the glycoprotein to be produced, control of the activity of the enzyme relating to the modification of a sugar chain in the host cell has been attempted, but actually, the structures of sugar chains are various and complex, and solution of the physiological roles of sugar chains would be insufficient, so that trial and error are repeated. Particularly, although it has been revealed little by little that the effector function of antibodies is greatly influenced by the sugar chain structure, a truly important sugar chain structure has not been specified yet, a host cell capable of producing an antibody molecule which has been modified so as to have a suitable sugar chain structure has not been obtained.

DISCLOSURE OF THE INVENTION

The present invention relates to the following (1) to (81).

(1) A process for producing an antibody composition using a cell, which comprises using a cell resistant to a lectin which recognizes a sugar chain in which 1-position of fucose is bound to 6-position of *N*-acetylglucosamine in the reducing end through α-bond in a complex *N*-glycoside-linked sugar chain.

(2) The process according to (1), wherein the cell is a cell in which the activity of a protein selected from the group consisting of the following (a), (b) and (c) is decreased or deleted:

(a) an enzyme protein relating to synthesis of an intracellular sugar nucleotide, GDP-fucose;
(b) an enzyme protein relating to modification of a sugar chain in which 1-position of fucose is bound to 6-position of *N*-acetylglucosamine in the reducing end through α-bond in a complex *N*-glycoside-linked sugar chain;
(c) a protein relating to transport of an intracellular sugar nucleotide, GDP-fucose, to the Golgi body.

(3) The process according to (2), wherein the enzyme protein relating to modification of a sugar chain in which 1-position of fucose is bound to 6-position of *N*-acetylglucosamine in the reducing end through α-bond in a complex *N*-glycoside-linked sugar chain is α1,6-fucosyltransferase.

(4) The process according to (2) or (3), wherein the activity of the protein is decreased or deleted by a technique selected from the group consisting of (a) to (d):

(a) a gene disruption technique which comprises targeting a gene encoding the protein;
(b) a technique for introducing a dominant negative mutant of a gene encoding the protein;
(c) a technique for introducing mutation into the protein;
(d) a technique for suppressing transcription and/or translation of a gene encoding the protein.

(5) The process according to (4), wherein the technique for suppressing transcription and/or translation of a gene encoding the protein is an RNAi method.

(6) The process according to any one of (3) to (5), wherein the α1,6-fucosyltransferase is a protein encoded by a DNA selected from the group consisting of (a) to (h) or a protein selected from the group consisting of the following (i) to (n):

(a) a DNA comprising the nucleotide sequence represented by SEQ ID NO:79;
(b) a DNA comprising the nucleotide sequence represented by SEQ ID NO:80;
(c) a DNA comprising the nucleotide sequence represented by SEQ ID NO:81;
(d) a DNA comprising the nucleotide sequence represented by SEQ ID NO:82;

(e) a DNA which hybridizes with a DNA comprising the nucleotide sequence represented by SEQ ID NO:79 under stringent conditions and encodes a protein having an α1,6-fucosyltransferase activity;
(f) a DNA which hybridizes with a DNA comprising the nucleotide sequence represented by SEQ ID NO:80 under stringent conditions and encodes a protein having an α1,6-fucosyltransferase activity;
(g) a DNA which hybridizes with a DNA comprising the nucleotide sequence represented by SEQ ID NO:81 under stringent conditions and encodes a protein having an α1,6-fucosyltransferase activity;
(h) a DNA which hybridizes with a DNA comprising the nucleotide sequence represented by SEQ ID NO:82 under stringent conditions and encodes a protein having an α1,6-fucosyltransferase activity;
(i) a protein comprising the amino acid sequence represented by SEQ ID NO:91;
(j) a protein comprising the amino acid sequence represented by SEQ ID NO:92;
(k) a protein which comprises an amino acid sequence in which at least one amino acid is deleted, substituted, inserted and/or added in the amino acid sequence represented by SEQ ID NO:91 and has an α1,6-fucosyltransferase activity;
(l) a protein which comprises an amino acid sequence in which at least one amino acid is deleted, substituted, inserted and/or added in the amino acid sequence represented by SEQ ID NO:92 and has an α1,6-fucosyltransferase activity;
(m) a protein which comprises an amino acid sequence having a homology of at least 80% with the amino acid sequence represented by SEQ ID NO:91 and has an α1,6-fucosyltransferase activity;
(n) a protein which comprises an amino acid sequence having a homology of at least 80% with the amino acid sequence represented by SEQ ID NO:92 and has an α1,6-fucosyltransferase activity.

(7) The process according to (5) or (6), wherein the RNAi method is a method in which a double-stranded RNA comprising an RNA selected from the group consisting of (a) to (d) and its complementary RNA and being capable of decreasing the amount of mRNA of α1,6-fucosyltransferase is introduced into or expressed in the cell:

(a) an RNA corresponding to a DNA comprising a nucleotide sequence of continuous 10 to 30 nucleotides in the nucleotide sequence represented by SEQ ID NO:79;
(b) an RNA corresponding to a DNA comprising a nucleotide sequence of continuous 10 to 30 nucleotides in the nucleotide sequence represented by SEQ ID NO:80;
(c) an RNA corresponding to a DNA comprising a nucleotide sequence of continuous 10 to 30 nucleotides in the nucleotide sequence represented by SEQ ID NO:81;
(d) an RNA corresponding to a DNA comprising a nucleotide sequence of continuous 10 to 30 nucleotides in the nucleotide sequence represented by SEQ ID NO:82.

(8) The process according to (7), wherein the double-stranded RNA comprising an RNA selected from the group consisting of (a) to (d) and its complementary RNA is introduced into or expressed in the cell to thereby decrease the amount of mRNA of α1,6-fucosyltransferase and provide the cell with the resistance to a lectin which recognizes a sugar chain in which 1-position of fucose is bound to 6-position of *N*-acetylglucosamine in the reducing end through α-bond in a complex *N*-glycoside-linked sugar chain:

(a) an RNA comprising the nucleotide sequence represented by SEQ ID NO:83;
(b) an RNA comprising the nucleotide sequence represented by SEQ ID NO:84;
(c) an RNA which comprises a nucleotide sequence in which one or a few nucleotides are deleted or added in the nucleotide sequence represented by SEQ ID NO:83 and has substantially the same RNAi activity as the RNA represented by SEQ ID NO:83;
(d) an RNA which comprises a nucleotide sequence in which one or a few nucleotides are deleted or added in the nucleotide sequence represented by SEQ ID NO:84 and has substantially the same RNAi activity as the RNA represented by SEQ ID NO:84.

(9) The process according to (7) or (8), wherein the double-stranded RNA is introduced into the cell by using a vector into which a DNA corresponding to the RNA according to (7) or (8) and its complementary DNA are inserted.
(10) The process according to any one of (1) to (9), wherein the cell is resistant to at least one lectin selected from the group consisting of the following (a) to (d):

(a) a *Lens culinaris* lectin;
(b) a *Pisum sativum* lectin;
(c) a *Vicia faba* lectin;
(d) an *Aleuria aurantia* lectin.

(11) The process according to any one of (1) to (10), wherein the cell is a cell into which a gene encoding an antibody molecule is introduced.
(12) The process according to (11), wherein the antibody molecule is selected from the group consisting of the following (a) to (d):

(a) a human antibody;
(b) a humanized antibody;
(c) an antibody fragment comprising the Fc region of (a) or (b);
(d) a fusion protein comprising the Fc region of (a) or (b).

(13) The process according to (11) or (12), wherein the antibody molecule belongs to an IgG class.
(14) The process according to any one of (1) to (13), wherein the antibody composition has a higher antibody-dependent cell-mediated cytotoxic activity than an antibody composition produced by its parent cell.
(15) The process according to (14), wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by its parent cell.
(16) The process according to (15), wherein the ratio of a sugar chain in which fucose is not bound to *N*-acetylglucosamine in the reducing end through α-bond is 20% or more of total complex *N*-glycoside-linked sugar chains bound to the Fc region in the antibody composition.
(17) The process according to (15) or (16), wherein the sugar chain in which fucose is not bound is a sugar chain in which 1-position of fucose is not bound to 6-position of *N*-acetylglucosamine in the reducing end in the complex *N*-glycoside-linked sugar chain through α-bond.
(18) The process according to any one of (1) to (17), wherein the cell is selected from the group consisting of a yeast, an animal cell, an insect cell and a plant cell.
(19) The process according to any one of (1) to (18), wherein the cell is a mouse myeloma cell.
(20) The process according to (19), wherein the mouse myeloma cell is NS0 cell or SP2/0-Ag14 cell.
(21) The process according to any one of (1) to (18), wherein the cell is selected from the group consisting of the following (a) to (g):

(a) a CHO cell derived from a Chinese hamster ovary tissue;
(b) a rat myeloma cell line YB2/3HL.P2.G11.16Ag.20 cell;
(c) a BHK cell derived from a Syrian hamster kidney tissue;
(d) a hybridoma cell which produces an antibody;
(e) a human leukemic cell line Namalwa cell;
(f) an embryonic stem cell;
(g) a fertilized egg cell.

(22) A cell into which a gene encoding an antibody composition is introduced, which is resistant to a lectin which recognizes a sugar chain in which 1-position of fucose is bound to 6-position of *N*-acetylglucosamine in the reducing end through α-bond in a complex *N*-glycoside-linked sugar chain.
(23) The cell according to (22), wherein the activity of a protein selected from the group consisting of the following (a), (b) and (c) is more decreased or deleted than its parent cell:

(a) an enzyme protein relating to synthesis of an intracellular sugar nucleotide, GDP-fucose;
(b) an enzyme protein relating to modification of a sugar chain in which 1-position of fucose is bound to 6-position of *N*-acetylglucosamine in the reducing end through α-bond in a complex *N*-glycoside-linked sugar chain;
(c) a protein relating to transport of an intracellular sugar nucleotide, GDP-fucose, to the Golgi body.

(24) The cell according to (23), wherein the enzyme protein relating to modification of a sugar chain in which 1-position of fucose is bound to 6-position of *N*-acetylglucosamine in the reducing end through α-bond in a complex *N*-glycoside-linked sugar chain is α1,6-fucosyltransferase.
(25) The cell according to (23) or (24), wherein the activity of the protein is decreased or deleted by a technique selected from the group consisting of (a) to (d):

(a) a gene disruption technique which comprises targeting a gene encoding the protein;
(b) a technique for introducing a dominant negative mutant of a gene encoding the protein;
(c) a technique for introducing mutation into the protein;

(d) a technique for suppressing transcription and/or translation of a gene encoding the protein.

(26) The cell according to (25), wherein the technique for suppressing transcription and/or translation of a gene encoding the protein is an RNAi method.
(27) The cell according to any one of (24) to (26), wherein the α1,6-fucosyltransferase is a protein encoded by a DNA selected from the group consisting of (a) to (h) or a protein selected from the group consisting of the following (i) to (n):

(a) a DNA comprising the nucleotide sequence represented by SEQ ID NO:79;
(b) a DNA comprising the nucleotide sequence represented by SEQ ID NO:80;
(c) a DNA comprising the nucleotide sequence represented by SEQ ID NO:81;
(d) a DNA comprising the nucleotide sequence represented by SEQ ID NO:82;
(e) a DNA which hybridizes with a DNA comprising the nucleotide sequence represented by SEQ ID NO:79 under stringent conditions and encodes a protein having an α1,6-fucosyltransferase activity;
(f) a DNA which hybridizes with a DNA comprising the nucleotide sequence represented by SEQ ID NO:80 under stringent conditions and encodes a protein having an α1,6-fucosyltransferase activity;
(g) a DNA which hybridizes with a DNA comprising the nucleotide sequence represented by SEQ ID NO:81 under stringent conditions and encodes a protein having an α1,6-fucosyltransferase activity;
(h) a DNA which hybridizes with a DNA comprising the nucleotide sequence represented by SEQ ID NO:82 under stringent conditions and encodes a protein having an α1,6-fucosyltransferase activity;
(i) a protein comprising the amino acid sequence represented by SEQ ID NO:91;
(j) a protein comprising the amino acid sequence represented by SEQ ID NO:92;
(k) a protein which comprises an amino acid sequence in which at least one amino acid is deleted, substituted, inserted and/or added in the amino acid sequence represented by SEQ ID NO:91 and has an α1,6-fucosyltransferase activity;
(l) a protein which comprises an amino acid sequence in which at least one amino acid is deleted, substituted, inserted and/or added in the amino acid sequence represented by SEQ ID NO:92 and has an α1,6-fucosyltransferase activity;
(m) a protein which comprises an amino acid sequence having a homology of at least 80% with the amino acid sequence represented by SEQ ID NO:91 and has an α1,6-fucosyltransferase activity;
(n) a protein which comprises an amino acid sequence having a homology of at least 80% with the amino acid sequence represented by SEQ ID NO:92 and has an α1,6-fucosyltransferase activity.

(28) The cell according to (26) or (27), wherein the RNAi method is a method in which a double-stranded RNA comprising an RNA selected from the group consisting of (a) to (d) and its complementary RNA and being capable of decreasing the amount of mRNA of α1,6-fucosyltransferase is introduced into or expressed in the cell:

(a) an RNA corresponding to a DNA comprising a nucleotide sequence of continuous 10 to 30 nucleotides in the nucleotide sequence represented by SEQ ID NO:79;
(b) an RNA corresponding to a DNA comprising a nucleotide sequence of continuous 10 to 30 nucleotides in the nucleotide sequence represented by SEQ ID NO:80;
(c) an RNA corresponding to a DNA comprising a nucleotide sequence of continuous 10 to 30 nucleotides in the nucleotide sequence represented by SEQ ID NO:81;
(d) an RNA corresponding to a DNA comprising a nucleotide sequence of continuous 10 to 30 nucleotides in the nucleotide sequence represented by SEQ ID NO:82.

(29) The cell according to (28), wherein the double-stranded RNA comprising an RNA selected from the group consisting of (a) to (d) and its complementary RNA is introduced into or expressed in the cell to thereby decrease the amount of mRNA of α1,6-fucosyltransferase and provide the cell with the resistance to a lectin which recognizes a sugar chain in which 1-position of fucose is bound to 6-position of *N*-acetylglucosamine in the reducing end through α-bond in a complex *N*-glycoside-linked sugar chain:

(a) an RNA comprising the nucleotide sequence represented by SEQ ID NO:83;
(b) an RNA comprising the nucleotide sequence represented by SEQ ID NO:84;
(c) an RNA which comprises a nucleotide sequence in which one or a few nucleotides are deleted or added in the nucleotide sequence represented by SEQ ID NO:83 and has substantially the same RNAi activity as the RNA represented by SEQ ID NO:83;
(d) an RNA which comprises a nucleotide sequence in which one or a few nucleotides are deleted or added

in the nucleotide sequence represented by SEQ ID NO:84 and has substantially the same RNAi activity as the RNA represented by SEQ ID NO:84.

(30) The process according to (28) or (29), wherein the double-stranded RNA is introduced into the cell by using a vector into which a DNA corresponding to the RNA according to (28) or (29) and its complementary DNA are inserted.
(31) The cell according to any one of (22) to (30), wherein the cell is resistant to at least one lectin selected from the group consisting of the following (a) to (d):

(a) a *Lens culinaris* lectin;
(b) a *Pisum sativum* lectin;
(c) a *Vicia faba* lectin;
(d) an *Aleuria aurantia* lectin.

(32) The cell according to any one of (22) to (31), wherein the antibody molecule is selected from the group consisting of the following (a), (b), (c) and (d):

(a) a human antibody;
(b) a humanized antibody;
(c) an antibody fragment comprising the Fc region of (a) or (b);
(d) a fusion protein comprising the Fc region of (a) or (b).

(33) The cell according to any one of (22) to (32), wherein the antibody molecule belongs to an IgG class.
(34) The cell according to any one of (22) to (33), wherein the antibody composition has a higher antibody-dependent cell-mediated cytotoxic activity than an antibody composition produced by its parent cell.
(35) The cell according to (34), wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by its parent cell.
(36) The cell according to (35), wherein the ratio of a sugar chain in which fucose is not bound to *N*-acetylglucosamine in the reducing end through α-bond is 20% or more of total complex *N*-glycoside-linked sugar chains bound to the Fc region in the antibody composition.
(37) The cell according to (35), wherein the sugar chain in which fucose is not bound is a sugar chain in which 1-position of fucose is not bound to 6-position of *N*-acetylglucosamine in the reducing end in the complex *N*-glycoside-linked sugar chain through α-bond.
(38) The cell according to any one of (22) to (37), wherein the cell is selected from the group consisting of a yeast, an animal cell, an insect cell and a plant cell.
(39) The cell according to any one of (22) to (37), wherein the cell is a mouse myeloma cell.
(40) The cell according to (39), wherein the mouse myeloma cell is NS0 cell or SP2/0-Ag14 cell.
(41) The cell according to any one of (22) to (38), wherein the cell is selected from the group consisting of the following (a) to (g):

(a) a CHO cell derived from a Chinese hamster ovary tissue;
(b) a rat myeloma cell line YB2/3HL.P2.G11.16Ag.20 cell;
(c) a BHK cell derived from a Syrian hamster kidney tissue;
(d) a hybridoma cell which produces an antibody;
(e) a human leukemic cell line Namalwa cell;
(f) an embryonic stem cell;
(g) a fertilized egg cell.

(42) A process for producing an antibody composition, which comprises culturing the cell according to any one of (22) to (41) in a medium to form and accumulate an antibody composition in a culture; and recovering the antibody composition from the culture.
(43) The process according to (42), which produces an antibody composition having a higher antibody-dependent cell-mediated cytotoxic activity than an antibody composition produced by its parent cell.
(44) The process according to (43), wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region

in the antibody composition than an antibody composition produced by its parent cell.

(45) The process according to (44), wherein the ratio of a sugar chain in which fucose is not bound to *N*-acetylglucosamine in the reducing end through α-bond is 20% or more of total complex *N*-glycoside-linked sugar chains bound to the Fc region in the antibody composition.

(46) The process according to (44) or (45), wherein the sugar chain in which fucose is not bound is a sugar chain in which 1-position of the fucose is not bound to 6-position of *N*-acetylglucosamine in the reducing end through α-bond in a complex *N*-glycoside-linked sugar chain.

(47) A mouse myeloma cell resistant to a lectin which recognizes a sugar chain in which 1-position of fucose is bound to 6-position of *N*-acetylglucosamine in the reducing end through α-bond in a complex *N*-glycoside-linked sugar chain.

(48) The mouse myeloma cell according to (47), wherein the activity of a protein selected from the group consisting of the following (a), (b) and (c) is more decreased or deleted than its parent cell:

(a) an enzyme protein relating to synthesis of an intracellular sugar nucleotide, GDP-fucose;
(b) an enzyme protein relating to modification of a sugar chain in which 1-position of fucose is bound to 6-position of *N*-acetylglucosamine in the reducing end through α-bond in a complex *N*-glycoside-linked sugar chain;
(c) a protein relating to transport of an intracellular sugar nucleotide, GDP-fucose, to the Golgi body.

(49) The mouse myeloma cell according to (48), wherein the enzyme protein relating to modification of a sugar chain in which 1-position of fucose is bound to 6-position of *N*-acetylglucosamine in the reducing end through α-bond in a complex *N*-glycoside-linked sugar chain is α1,6-fucosyltransferase.

(50) The mouse myeloma cell according to (48) or (49), wherein the activity of the protein is decreased or deleted by a technique selected from the group consisting of (a) to (d):

(a) a gene disruption technique which comprises targeting a gene encoding the protein;
(b) a technique for introducing a dominant negative mutant of a gene encoding the protein;
(c) a technique for introducing mutation into the protein;
(d) a technique for suppressing transcription and/or translation of a gene encoding the protein.

(51) The mouse myeloma cell according to (50), wherein the technique for suppressing transcription and/or translation of a gene encoding the protein is an RNAi method.

(52) The mouse myeloma cell according to any one of (49) to (51), wherein the α1,6-fucosyltransferase is a protein encoding a DNA selected from the group consisting of (a) to (h) or a protein selected from the group consisting of the following (i) to (n):

(a) a DNA comprising the nucleotide sequence represented by SEQ ID NO:79;
(b) a DNA comprising the nucleotide sequence represented by SEQ ID NO:80;
(c) a DNA comprising the nucleotide sequence represented by SEQ ID NO:81;
(d) a DNA comprising the nucleotide sequence represented by SEQ ID NO:82;
(e) a DNA which hybridizes with a DNA comprising the nucleotide sequence represented by SEQ ID NO:79 under stringent conditions and encodes a protein having an α1,6-fucosyltransferase activity;
(f) a DNA which hybridizes with a DNA comprising the nucleotide sequence represented by SEQ ID NO:80 under stringent conditions and encodes a protein having an α1,6-fucosyltransferase activity;
(g) a DNA which hybridizes with a DNA comprising the nucleotide sequence represented by SEQ ID NO:81 under stringent conditions and encodes a protein having an α1,6-fucosyltransferase activity;
(h) a DNA which hybridizes with a DNA comprising the nucleotide sequence represented by SEQ ID NO:82 under stringent conditions and encodes a protein having an α1,6-fucosyltransferase activity;
(i) a protein comprising the amino acid sequence represented by SEQ ID NO:91;
(j) a protein comprising the amino acid sequence represented by SEQ ID NO:92;
(k) a protein which comprises an amino acid sequence in which at least one amino acid is deleted, substituted, inserted and/or added in the amino acid sequence represented by SEQ ID NO:91 and has an α1,6-fucosyltransferase activity;
(l) a protein which comprises an amino acid sequence in which at least one amino acid is deleted, substituted, inserted and/or added in the amino acid sequence represented by SEQ ID NO:92 and has an α1,6-fucosyltransferase activity;
(m) a protein which comprises an amino acid sequence having a homology of at least 80% with the amino acid sequence represented by SEQ ID NO:91 and has an α1,6-fucosyltransferase activity;
(n) a protein which comprises an amino acid sequence having a homology of at least 80% with the amino acid

sequence represented by SEQ ID NO:92 and has an α1,6-fucosyltransferase activity.

(53) The mouse myeloma cell according to (51) or (52), wherein the RNAi method is a method in which a double-stranded RNA comprising a RNA selected from the group consisting of (a) to (d) and its complementary RNA and being capable of decreasing the amount of mRNA of α1,6-fucosyltransferase is introduced into or expressed in the cell:

(a) an RNA corresponding to a DNA comprising a nucleotide sequence of continuous 10 to 30 nucleotides in the nucleotide sequence represented by SEQ ID NO:79;
(b) an RNA corresponding to a DNA comprising a nucleotide sequence of continuous 10 to 30 nucleotides in the nucleotide sequence represented by SEQ ID NO:80;
(c) an RNA corresponding to a DNA comprising a nucleotide sequence of continuous 10 to 30 nucleotides in the nucleotide sequence represented by SEQ ID NO:81;
(d) an RNA corresponding to a DNA comprising a nucleotide sequence of continuous 10 to 30 nucleotides in the nucleotide sequence represented by SEQ ID NO:82.

(54) The mouse myeloma cell according to (51), wherein the double-stranded RNA comprising an RNA selected from the group consisting of (a) to (d) and its complementary RNA is introduced into or expressed in the cell to thereby decrease the amount of mRNA of α1,6-fucosyltransferase and provide the cell with the resistance to a lectin which recognizes a sugar chain in which 1-position of fucose is bound to 6-position of *N*-acetylglucosamine in the reducing end through α-bond in a complex *N*-glycoside-linked sugar chain:

(a) an RNA comprising the nucleotide sequence represented by SEQ ID NO:83;
(b) an RNA comprising the nucleotide sequence represented by SEQ ID NO:84;
(c) an RNA which comprises a nucleotide sequence in which one or a few nucleotides are deleted or added in the nucleotide sequence represented by SEQ ID NO:83 and has substantially the same RNAi activity as the RNA represented by SEQ ID NO:83;
(d) an RNA which comprises a nucleotide sequence in which one or a few nucleotides are deleted or added in the nucleotide sequence represented by SEQ ID NO:84 and has substantially the same RNAi activity as the RNA represented by SEQ ID NO:84.

(55) The mouse myeloma cell according to (50) or (51), wherein the double-stranded RNA is introduced into the cell by using a vector into which a DNA corresponding to the RNA according to (53) or (54) and its complementary DNA are inserted.
(56) The mouse myeloma cell according to any one of (47) to (55), wherein the cell is resistant to at least one lectin selected from the group consisting of the following (a) to (d):

(a) a *Lens culinaris* lectin;
(b) a *Pisum sativum* lectin;
(c) a *Vicia faba* lectin;
(d) an *Aleuria aurantia* lectin.

(57) The mouse myeloma cell according to any one of (47) to (56), wherein the mouse myeloma cell is selected from the group consisting of the following (a) and (b):

(a) NSO cell;
(b) SP2/0-Ag14 cell.

(58) The cell according to any one of (47) to (57), wherein the mouse myeloma cell is a cell into which a gene encoding an antibody molecule is introduced.
(59) The cell according to (58), wherein the antibody molecule is selected from the group consisting of the following (a), (b), (c) and (d):

(a) a human antibody;
(b) a humanized antibody;
(c) an antibody fragment comprising the Fc region of (a) or (b);
(d) a fusion protein comprising the Fc region of (a) or (b).

(60) The cell according to (58) or (59), wherein the antibody molecule belongs to an IgG class.
(61) The cell according to any one of (58) to (60), wherein the antibody composition has a higher antibody-dependent cell-mediated cytotoxic activity than an antibody composition produced by its parent cell.
(62) The cell according to (61), wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by its parent cell.
(63) The cell according to (62), wherein the ratio of a sugar chain in which fucose is not bound to *N*-acetylglucosamine in the reducing end through $\alpha$-bond is 20% or more of total complex *N*-glycoside-linked sugar chains bound to the Fc region in the antibody composition.
(64) The cell according to (62), wherein the sugar chain in which fucose is not bound is a sugar chain in which 1-position of fucose is not bound to 6-position of *N*-acetylglucosamine in the reducing end in the complex *N*-glycoside-linked sugar chain through $\alpha$-bond.
(65) A process for producing an antibody composition, which comprises culturing the cell according to any one of (58) to (64) in a medium to form and accumulate an antibody composition in the culture; and recovering the antibody composition from the culture.
(66) The process according to (65), which produces an antibody composition having a higher antibody-dependent cell-mediated cytotoxic activity than an antibody composition produced by its parent cell.
(67) The process according to (66), wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by its parent cell.
(68) The process according to (67), wherein the ratio of a sugar chain in which fucose is not bound to *N*-acetylglucosamine in the reducing end through $\alpha$-bond is 20% or more of total complex *N*-glycoside-linked sugar chains bound to the Fc region in the antibody composition.
(69) The process according to (67) or (68), wherein the sugar chain in which fucose is not bound is a sugar chain in which 1-position of fucose is not bound to 6-position of *N*-acetylglucosamine in the reducing end in the complex *N*-glycoside-linked sugar chain through $\alpha$-bond.
(70) An antibody composition produced by the process according to any one of (1) to (21).
(71) An antibody composition produced by the process according to any one of (42) to (46).
(72) An antibody composition produced by the process according to any one of (65) to (69).
(73) A medicament comprising as an active ingredient the antibody composition according to any one of (70) to (72).
(74) The medicament according to (73), which is a diagnostic agent, an preventing agent or a treating agent for tumor-accompanied diseases, allergy-accompanied diseases, inflammatory-accompanied diseases, autoimmune diseases, cardiovascular diseases, viral infection-accompanied diseases or bacterial infection-accompanied diseases.
(75) A double-stranded RNA comprising an RNA selected from the group consisting of (a) to (d) and its complementary RNA:

(a) an RNA comprising the nucleotide sequence represented by SEQ ID NO:83;
(b) an RNA comprising the nucleotide sequence represented by SEQ ID NO:84;
(c) an RNA which comprises a nucleotide sequence in which one or a few nucleotides are deleted or added in the nucleotide sequence represented by SEQ ID NO:83 and has substantially the same RNAi activity as the RNA represented by SEQ ID NO:83;
(d) an RNA which comprises a nucleotide sequence in which one or a few nucleotides are deleted or added in the nucleotide sequence represented by SEQ ID NO:84 and has substantially the same RNAi activity as the RNA represented by SEQ ID NO:84.

(76) A DNA corresponding to the RNA according to (75) and its complementary DNA.
(77) The DNA according to (76), the DNA corresponding to the RNA is the nucleotide sequence represented by SEQ ID NO:63 or 64.
(78) A recombinant DNA comprising the DNA according to (76) or (77) and its complementary DNA.
(79) The recombinant DNA according to (78), which is constructed for expressing the double-stranded RNA according to (75).
(80) A transformant obtainable by introducing the recombinant DNA according to (78) or (79) into a cell.
(81) Use of the antibody composition according to any one of (70) to (72) in the manufacture of the medicament according to (73) or (74).

The process for producing an antibody composition using the cell of the present invention includes a process for producing a monoclonal antibody using a hybridoma cell, a process for producing a human antibody and a humanized antibody using a host cell into which a gene encoding an antibody is introduced, a process for producing a human antibody using a transgenic non-human animal which is developed after transplanting a non-human embryonic stem cell or fertilized egg cell into which a gene encoding an antibody is introduced into a non-human animal early stage embryo, and the like.

Accordingly, in the present invention, as the cell resistant to a lectin which recognizes a sugar chain in which 1-position of fucose is bound to 6-position of *N*-acetylglucosamine in the reducing end through α-bond in a complex *N*-glycoside-linked sugar chain, any cell can be used, so long as it is a cell such as yeast, an animal cell, an insect cell or a plant cell which can be used for producing an antibody composition and is a cell resistant to a lectin which recognizes a sugar chain in which 1-position of fucose is bound to 6-position of *N*-acetylglucosamine in the reducing end through α-bond in a complex *N*-glycoside-linked sugar chain. Examples include a hybridoma cell, a host cell for producing a human antibody and humanized antibody, an embryonic stem cell and fertilized egg cell for producing a transgenic non-human animal which produces a human antibody, a plant callus cell for producing a transgenic plant which produces a human antibody, a myeloma cell, a cell derived from a transgenic non-human animal and the like which are resistant to lectin which recognizes a sugar chain in which 1-position of fucose is bound to 6-position of *N*-acetylglucosamine in the reducing end through α-bond in a complex *N*-glycoside-linked sugar chain. The myeloma cell can be used as a fusion cell for producing a hybridoma cell. Also, a hybridoma cell can be produced by immunizing a transgenic non-human animal with an antigen and using spleen cells of the animal.

The lectin-resistant cell is a cell of which growth is not inhibited when a lectin is applied at an effective concentration.

In the present invention, the effective concentration of a lectin which does not inhibit the growth can be adjusted depending on the cell line, and is generally 10 μg/ml to 10.0 mg/ml, preferably 0.5 to 2.0 mg/ml. The effective concentration in the case where mutation is introduced into a parent cell is a concentration in which the parent cell cannot normally grow or higher than the concentration, and is a concentration which is preferably similar to, more preferably 2 to 5 times, still more preferably at least 10 times, and most preferably at least 20 times, higher than the concentration in which the parent cell cannot normally grow.

The parent cell means a cell before a certain treatment is applied, namely a cell before the step for selecting a lectin-resistant cell used in the present invention is carried out or a cell before genetic engineering techniques for decreasing or deleting the above enzyme activity are carried out.

Although the parent cell is not particularly limited, parent cells of various cell lines are exemplified below.

The parent cell of NS0 cell includes NS0 cells described in literatures such as *BIO/TECHNOLOGY*, 10, 169 (1992) and *Biotechnol. Bioeng.,* 73, 261 (2001), NS0 cell line (RCB 0213) registered at RIKEN Cell Bank, The Institute of Physical and Chemical Research, sub-cell lines obtained by acclimating these cell lines to media in which they can grow, and the like.

The parent cell of SP2/0-Ag14 cell includes SP2/0-Ag14 cells described in literatures such as *J. Immunol.*, 126, 317 (1981), *Nature*, 276, 269 (1978) and *Human Antibodies and Hybridomas*, 3, 129 (1992), SP2/0-Ag14 cell (ATCC CRL-1581) registered at ATCC, sub-cell lines obtained by acclimating these cell lines to media in which they can grow (ATCC CRL-1581.1), and the like.

The parent cell of CHO cell derived from Chinese hamster ovary tissue includes CHO cells described in literatures such as *Journal of Experimental Medicine (Jikken Igaku),* 108, 945 (1958), *Proc. Natl. Acad. Sci. USA*, 60, 1275 (1968), *Genetics*, 55, 513 (1968), *Chromosoma*, 41, 129 (1973), *Methods in Cell Science*, 18, 115 (1996), *Radiation Research,* 148, 260 (1997), *Proc. Natl. Acad Sci. USA,* 77, 4216 (1980), *Proc. Natl. Acad Sci. USA*, 60, 1275 (1968), *Cell,* 6, 121 (1975) and *Molecular Cell Genetics*, Appendix I, II (p. 883-900), cell line CHO-K1 (ATCC CCL-61), cell line DLTXB11 (ATCC CRL-9096) and cell line Pro-5 (ATCC CRL-1781) registered at ATCC, commercially available cell line CHO-S (Cat # 11619 of Life Technologies), sub-cell lines obtained by acclimating these cell lines to media in which they can grow, and the like.

The parent cell of a rat myeloma cell line YB2/3HL.P2.G11.16Ag.20 cell includes cell lines established from Y3/Ag1.2.3 cell (ATCC CRL-1631) such as YB2/3HL.P2.G11.16Ag.20 cell described in literatures such as *J. Cell. Biol.*, 93, 576 (1982) and *Methods Enzymol.*, 73B, 1 (1981), YB2/3HL.P2.G11.16Ag.20 cell (ATCC CRL-1662) registered at ATCC, sub-lines obtained by acclimating these cell lines to media in which they can grow, and the like.

As the lectin which recognizes a sugar chain structure in which 1-position of fucose is bound to 6-position of *N*-acetylglucosamine in the reducing end through α-bond in the *N*-glycoside-linked sugar chain, any lectin can be used, so long as it can recognize the sugar chain structure. Examples include a *Lens culinaris* lectin LCA (lentil agglutinin derived from *Lens culinaris*), a pea lectin PSA (pea lectin derived from *Pisum sativum*), a broad bean lectin VFA (agglutinin derived from *Vicia faba*), *an Aleuria aurantia* lectin AAL (lectin derived from *Aleuria aurantia*) and the like.

In the present invention, the lectin-resistant cell may be any cell, so long as growth of the cell is not inhibited in the presence of a lectin at a definite effective concentration. Examples include cells in which the activity of at least

one protein shown below is decreased or deleted, and the like.

(a) an enzyme protein relating to synthesis of an intracellular sugar nucleotide, GDP-fucose (hereinafter referred to "GDP-fucose synthase");
(b) an enzyme protein relating to modification of a sugar chain in which 1-position of fucose is bound to 6-position of *N*-acetylglucosamine in the reducing end through α-bond in a complex *N*-glycoside-linked sugar chain (hereinafter referred to as "α1,6-fucose modifying enzyme"); and
(c) a protein relating to transport of an intracellular sugar nucleotide, GDP-fucose, to the Golgi body (hereinafter referred to as "GDP-fucose transport protein").

The GDP-fucose synthase may be any enzyme, so long as it is an enzyme relating to the synthesis of the intracellular sugar nucleotide, GDP-fucose, as a supply source of fucose to a sugar chain, and includes an enzyme which has influence on the synthesis of the intracellular sugar nucleotide, GDP-fucose, and the like.
The intracellular sugar nucleotide, GDP-fucose, is supplied by a *de novo* synthesis pathway or a salvage synthesis pathway. Thus, all enzymes relating to the synthesis pathways are included in the GDP-fucose synthase.
The GDP-fucose synthase relating to the *de novo* synthesis pathway includes GDP-mannose 4-dehydratase (hereinafter referred to as "GMD"), GDP-keto-6-deoxymannose 3,5-epimerase, 4-reductase (hereinafter referred to as "Fx") and the like.
The GDP-fucose synthase relating to the salvage synthesis pathway includes GDP-beta-L-fucose pyrophosphorylase (hereinafter referred to as "GFPP"), fucokinase and the like.
As the enzyme which has influence on the synthesis of an intracellular sugar nucleotide, GDP-fucose, an enzyme which has influence on the activity of the enzyme relating to the synthesis of the intracellular sugar nucleotide, GDP-fucose, and an enzyme which has influence on the structure of substances as the substrate of the enzyme are also included.
In the present invention, the GMD includes:

a protein encoded by a DNA of the following (a) or (b):

(a) a DNA comprising the nucleotide sequence represented by SEQ ID NO:85;
(b) a DNA which hybridizes with the DNA comprising the nucleotide sequence represented by SEQ ID NO:85 under stringent conditions and encodes a protein having GMD activity,
or,
(c) a protein comprising the amino acid sequence represented by SEQ ID NO:86,
(d) a protein which comprises an amino acid sequence in which at least one amino acid is deleted, substituted, inserted and/or added in the amino acid sequence represented by SEQ ID NO:86 and has GMD activity,
(e) a protein which comprises an amino acid sequence having a homology of at least 80% with the amino acid sequence represented by SEQ ID NO:86 and has GMD activity, and the like.

Also, the DNA encoding the amino acid sequence of GMD includes a DNA comprising the nucleotide sequence represented by SEQ ID NO:85 and a DNA which hybridizes with the DNA comprising the nucleotide sequence represented by SEQ ID NO:85 under stringent conditions and encodes an amino acid sequence having GMD activity.
In the present invention, the Fx includes:

a protein encoded by a DNA of the following (a) or (b):

(a) a DNA comprising the nucleotide sequence represented by SEQ ID NO:87;
(b) a DNA which hybridizes with the DNA comprising the nucleotide sequence represented by SEQ ID NO:87 under stringent conditions and encodes a protein having Fx activity,
or,
(c) a protein comprising the amino acid sequence represented by SEQ ID NO:88,
(d) a protein which comprises an amino acid sequence in which at least one amino acid is deleted, substituted, inserted and/or added in the amino acid sequence represented by SEQ ID NO:88 and has Fx activity,
(e) a protein which comprises an amino acid sequence having a homology of at least 80% with the amino acid sequence represented by SEQ ID NO:88 and has Fx activity, and the like.

Also, the DNA encoding the amino acid sequence of Fx includes a DNA comprising the nucleotide sequence represented by SEQ ID NO:87 and a DNA which hybridizes with the DNA comprising the nucleotide sequence represented by SEQ ID NO:87 under stringent conditions and encodes an amino acid sequence having Fx activity.

In the present invention, the GFPP includes:

a protein encoded by a DNA of the following (a) or (b):

(a) a DNA comprising the nucleotide sequence represented by SEQ ID NO:89;
(b) a DNA which hybridizes with the DNA comprising the nucleotide sequence represented by SEQ ID NO:89 under stringent conditions and encodes a protein having GFPP activity, or,
(c) a protein comprising the amino acid sequence represented by SEQ ID NO:90,
(d) a protein which comprises an amino acid sequence in which at least one amino acid is deleted, substituted, inserted and/or added in the amino acid sequence represented by SEQ ID NO:90 and has GFPP activity,
(e) a protein which comprises an amino acid sequence having a homology of at least 80% with the amino acid sequence represented by SEQ ID NO:90 and has GFPP activity, and the like.

Also, the DNA encoding the amino acid sequence of GFPP includes a DNA comprising the nucleotide sequence represented by SEQ ID NO:89 and a DNA which hybridizes with the DNA comprising the nucleotide sequence represented by SEQ ID NO:89 under stringent conditions and encodes an amino acid sequence having GFPP activity.
The α1,6-fucose modifying enzyme includes any enzyme, so long as it is an enzyme relating to the reaction of binding of 1-position of fucose to 6-position of *N*-acetylglucosamine in the reducing end through α-bond in the complex *N*-glycoside-linked sugar chain. The enzyme relating to the reaction of binding of 1-position of fucose to 6-position of *N*-acetylglucosamine in the reducing end through α-bond in the complex *N*-glycoside-linked sugar chain includes an enzyme which has influence on the reaction of binding of 1-position of fucose to 6-position of *N*-acetylglucosamine in the reducing end through α-bond in the complex *N*-glycoside-linked sugar chain. Examples include α1,6-fucosyltransferase, α-L-fucosidase and the like.
Also, the enzyme relating to the reaction of binding of 1-position of fucose to 6-position of *N*-acetylglucosamine in the reducing end through α-bond in the complex *N*-glycoside-linked sugar chain includes an enzyme which has influence on the activity of the enzyme relating to the reaction of binding of 1-position of fucose to 6-position of *N*-acetylglucosamine in the reducing end through α-bond in the complex *N*-glycoside-linked sugar chain and an enzyme which has influence on the structure of substances as the substrate of the enzyme.
In the present invention, the α1,6-fucosyltransferase includes:

a protein encoded by a DNA of the following (a), (b), (c) or (d):

(a) a DNA comprising the nucleotide sequence represented by SEQ ID NO:79;
(b) a DNA comprising the nucleotide sequence represented by SEQ ID NO:80;
(c) a DNA comprising the nucleotide sequence represented by SEQ ID NO:81;
(d) a DNA comprising the nucleotide sequence represented by SEQ ID NO:82;
(e) a DNA which hybridizes with the DNA comprising the nucleotide sequence represented by SEQ ID NO:79 under stringent conditions and encodes a protein having α1,6-fucosyltransferase activity;
(f) a DNA which hybridizes with the DNA comprising the nucleotide sequence represented by SEQ ID NO:80 under stringent conditions and encodes a protein having α1,6-fucosyltransferase activity;
(g) a DNA which hybridizes with the DNA comprising the nucleotide sequence represented by SEQ ID NO:81 under stringent conditions and encodes a protein having α1,6-fucosyltransferase activity;
(h) a DNA which hybridizes with the DNA comprising the nucleotide sequence represented by SEQ ID NO:82 under stringent conditions and encodes a protein having α1,6-fucosyltransferase activity;
or,
(i) a protein comprising the amino acid sequence represented by SEQ ID NO:91,
(j) a protein comprising the amino acid sequence represented by SEQ ID NO:92,
(k) a protein which comprises an amino acid sequence in which at least one amino acid is deleted, substituted, inserted and/or added in the amino acid sequence represented by SEQ ID NO:91 and has α1,6-fucosyltransferase activity,
(l) a protein which comprises an amino acid sequence in which at least one amino acid is deleted, substituted, inserted and/or added in the amino acid sequence represented by SEQ ID NO:92 and has α1,6-fucosyltransferase activity,
(m) a protein which comprises an amino acid sequence having a homology of at least 80% with the amino acid sequence represented by SEQ ID NO:91 and has α1,6-fucosyltransferase activity,
(n) a protein which comprises an amino acid sequence having a homology of at least 80% with the amino acid sequence represented by SEQ ID NO:92 and has α1,6-fucosyltransferase activity, and the like.

Also, the DNA encoding the amino acid sequence of α1,6-fucosyltransferase include a DNA having the nucleotide sequence represented by SEQ ID NO:79, 80, 81 or 82 and a DNA which hybridizes with the DNA having the nucleotide sequence represented by SEQ ID NO:79, 80, 81 or 82 under stringent conditions and encodes an amino acid sequence having α1,6-fucosyltransferase activity.
The GDP-fucose transport protein may be any protein, so long as it is a protein relating to the transport of the intracellular sugar nucleotide, GDP-fucose, to the Golgi body, and includes a GDP-fucose transporter and the like.
In the present invention, the GDP-fucose transporter includes:

a protein encoded by a DNA of the following (a) to (h):

(a) a DNA comprising the nucleotide sequence represented by SEQ ID NO:93;
(b) a DNA comprising the nucleotide sequence represented by SEQ ID NO:95;
(c) a DNA comprising the nucleotide sequence represented by SEQ ID NO:97;
(d) a DNA comprising the nucleotide sequence represented by SEQ ID NO:98;
(e) a DNA which hybridizes with the DNA comprising the nucleotide sequence represented by SEQ ID NO:93 under stringent conditions and encodes a protein having GDP-transporter activity;
(f) a DNA which hybridizes with the DNA comprising the nucleotide sequence represented by SEQ ID NO:95 under stringent conditions and encodes a protein having GDP-transporter activity;
(g) a DNA which hybridizes with the DNA comprising the nucleotide sequence represented by SEQ ID NO:97 under stringent conditions and encodes a protein having GDP-transporter activity;
(h) a DNA which hybridizes with the DNA comprising the nucleotide sequence represented by SEQ ID NO:98 under stringent conditions and encodes a protein having GDP-transporter activity;

The GDP-fucose transporte of the present invention further includes the proteins selected from the following (i) to (t):

(i) a protein comprising the amino acid sequence represented by SEQ ID NO:94,
(j) a protein comprising the amino acid sequence represented by SEQ ID NO:96,
(k) a protein comprising the amino acid sequence represented by SEQ ID NO:99,
(l) a protein comprising the amino acid sequence represented by SEQ ID NO:100,
(m) a protein which comprises an amino acid sequence in which at least one amino acid is deleted, substituted, inserted and/or added in the amino acid sequence represented by SEQ ID NO:94 and has GDP-fucose transporter activity,
(n) a protein which comprises an amino acid sequence in which at least one amino acid is deleted, substituted, inserted and/or added in the amino acid sequence represented by SEQ ID NO:96 and has GDP-fucose transporter activity,
(o) a protein which comprises an amino acid sequence in which at least one amino acid is deleted, substituted, inserted and/or added in the amino acid sequence represented by SEQ ID NO:99 and has GDP-fucose transporter activity,
(p) a protein which comprises an amino acid sequence in which at least one amino acid is deleted, substituted, inserted and/or added in the amino acid sequence represented by SEQ ID NO:100 and has GDP-fucose transporter activity,
(q) a protein which comprises an amino acid sequence having a homology of at least 80% with the amino acid sequence represented by SEQ ID NO:94 and has GDP-fucose transporter activity,
(r) a protein which comprises an amino acid sequence having a homology of at least 80% with the amino acid sequence represented by SEQ ID NO:96 and has GDP-fucose transporter activity,
(s) a protein which comprises an amino acid sequence having a homology of at least 80% with the amino acid sequence represented by SEQ ID NO:99 and has GDP-fucose transporter activity,
(t) a protein which comprises an amino acid sequence having a homology of at least 80% with the amino acid sequence represented by SEQ ID NO:100 and has GDP-fucose transporter activity, and the like.

The GDP-fucose transporter protein furthermore includes a protein which has influence on the reaction to transport the intracellular sugar nucleotide, GDP-fucose, to the Golgi body, and examples thereof include a protein which has an influence on the activity of the above protein relating to the transport of the intracellular sugar nucleotide, GDP-fucose, to the Golgi body or has influence on the expression thereof
In the present invention, a DNA which is hybridizable under stringent conditions is a DNA obtained, e.g., by a method such as colony hybridization, plaque hybridization or Southern blot hybridization using a DNA such as the DNA having the nucleotide sequence represented by any one of the above SEQ ID NOs or a partial fragment thereof

as the probe, and examples thereof include a DNA which can be identified by carrying out hybridization at 65°C in the presence of 0.7 to 1.0 M sodium chloride using a filter to which colony- or plaque-derived DNA fragments are immobilized, and then washing the filter at 65°C using 0.1 to 2 × SSC solution (composition of the 1 × SSC solution comprising 150 mM sodium chloride and 15 mM sodium citrate). The hybridization can be carried out in accordance with the methods described, e.g., in *Molecular Cloning, A Laboratory Manual,* Second Edition, Cold Spring Harbor Laboratory Press (1989) (hereinafter referred to as "*Molecular Cloning,* Second Edition"), *Current Protocols in Molecular Biology,* John Wiley & Sons, 1987-1997 (hereinafter referred to as "*Current Protocols in Molecular Biology*"); *DNA Cloning I: Core Techniques, A Practical Approach,* Second Edition, Oxford University (1995); and the like. The hybridizable DNA includes a DNA having at least 60% or more, preferably 70% or more, more preferably 80% or more, still more preferably 90% or more, far more preferably 95% or more, and most preferably 98% or more, of homology with the nucleotide sequence represented by any one of the above SEQ ID NOs.

**[0043]** In the present invention, the protein which comprises an amino acid sequence in which at least one amino acid is deleted, substituted, inserted and/or added in the amino acid sequence represented by any one of the above SEQ ID NOs and has the above activity can be obtained, e.g., by introducing a site-directed mutation into a DNA encoding a protein having the amino acid sequence represented by any one of the above SEQ ID NOs using the site-directed mutagenesis described, e.g., in *Molecular Cloning,* Second Edition; *Current Protocols in Molecular Biology; Nucleic Acids Research,* 10, 6487 (1982); *Proc. Natl. Acad Sci. USA,* 79, 6409 (1982); *Gene,* 34, 315 (1985); *Nucleic Acids Research,* 13, 4431 (1985); *Proc. Natl. Acad. Sci. USA,* 82, 488 (1985); and the like. The number of amino acids to be deleted, substituted, inserted and/or added is one or more, and the number is not particularly limited, but is a number which can be deleted, substituted or added by a known technique such as the site-directed mutagenesis, e.g., it is 1 to several tens, preferably 1 to 20, more preferably 1 to 10, and most preferably 1 to 5.

Also, in order to maintain the above activity of the protein to be used in the present invention, it has at least 80% or more, preferably 85% or more, more preferably 90% or more, still more preferably 95% or more, far more preferably 97% or more, and most preferably 99% or more, of homology with the amino acid sequence represented by the above SEQ ID NOs, when calculated using an analyzing soft such as BLAST [*J. Mol. Biol*., 215, 403 (1990)] or FASTA [*Methods in Enzymology,* 183, 63 (1990)].

As a method for obtaining a cell used in the production process of the present invention, any technique can be used, so long as it is a technique which can select a cell resistant to a lectin which recognizes a sugar chain in which 1-position of fucose is bound to 6-position of *N*-acetylglucosamine in the reducing end through α-bond in a complex *N*-glycoside-linked sugar chain. Specifically, the method includes a technique for decreasing or deleting the activity of the above protein. The technique for decreasing or deleting includes:

(a) a gene disruption technique which comprises targeting a gene encoding the protein,
(b) a technique for introducing a dominant negative mutant of a gene encoding the protein,
(c) a technique for introducing mutation into the protein,
(d) a technique for suppressing transcription and/or translation of a gene encoding the protein, and the like.

In the present invention, the antibody composition is a composition which comprises an antibody molecule having a complex *N*-glycoside-linked sugar chain in the Fc region.

The antibody is a tetramer in which two molecules of each of two polypeptide chains, a heavy chain and a light chain (hereinafter referred to as "H chain" and "L chain", respectively), are respectively associated. Each of about a quarter of the *N*-terminal side of the H chain and about a half of the *N*-terminal side of the L chain (more than 100 amino acids for each) is called V region which is rich in diversity and directly relates to the binding with an antigen. The greater part of the moiety other than the V region is called a constant region (hereinafter referred to as "C region"). Based on homology with the C region, antibody molecules are classified into classes IgG, IgM, IgA, IgD and IgE.

Also, the IgG class is further classified into subclasses IgG1 to IgG4 based on homology with the C region.

The H chain is divided into four immunoglobulin domains, VH, CH1, CH2 and CH3, from its *N*-terminal side, and a highly flexible peptide region called hinge region is present between CH1 and CH2 to divide CH1 and CH2. A structural unit comprising CH2 and CH3 under the downstream of the hinge region is called Fc region to which a complex *N*-glycoside-linked sugar chain is bound. Fc region is a region to which an Fc receptor, a complement and the like are bound (*Immunology Illustrated*, the Original, 5th edition, published on February 10, 2000, by Nankodo; *Handbook of Antibody Technology (Kotai Kogaku Nyumon)*, 1st edition on January 25, 1994, by Chijin Shokan).

Sugar chains of glycoproteins such as an antibody are roughly classified into two types, namely a sugar chain which binds to asparagine (*N*-glycoside-linked sugar chain) and a sugar chain which binds to other amino acid such as serine, threonine (*O*-glycoside-linked sugar chain), based on the binding form to the protein moiety. The *N*-glycoside-linked sugar chains have a basic common core structure shown by the following structural formula (I) [*Biochemical Experimentation Method 23 - Method for Studying Glycoprotein Sugar Chain* (Gakujutsu Shuppan Center), edited by Reiko Takahashi (1989)]:

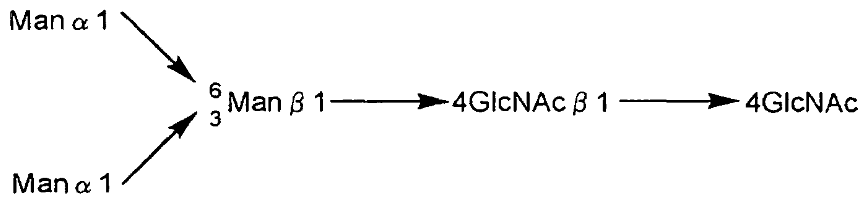

(I)

In formula (I), the sugar chain terminus which binds to asparagine is called a reducing end, and the opposite side is called a non-reducing end.

The *N*-glycoside-linked sugar chain may be any *N*-glycoside-linked sugar chain, so long as it comprises the core structure of formula (I). Examples include a high mannose type in which mannose alone binds to the non-reducing end of the core structure; a complex type in which the non-reducing end side of the core structure comprises at least one parallel branches of galactose-*N*-acetylglucosamine (hereinafter referred to as "Gal-GlcNAc") and the non-reducing end side of Gal-GlcNAc comprises a structure of sialic acid, bisecting *N*-acetylglucosamine or the like; a hybrid type in which the non-reducing end side of the core structure comprises branches of both of the high mannose type and complex type; and the like.

Since the Fc region in the antibody molecule comprises positions to which *N*-glycoside-linked sugar chains are separately bound, two sugar chains are bound per one antibody molecule. Since the *N*-glycoside-linked sugar chain which binds to an antibody molecule includes any sugar chain having the core structure represented by formula (I), there are a number of combinations of sugar chains for the two *N*-glycoside-linked sugar chains which bind to the antibody.

Accordingly, the antibody composition of the present invention which is prepared by using a lectin-resistant cell of the present invention may comprise an antibody having the same sugar chain structure or an antibody having different sugar chain structures, so long as the effect of the present invention is obtained from the composition.

The ratio of a sugar chain in which fucose is not bound to *N*-acetylglucosamine in the reducing end in the sugar chain among the total complex *N*-glycoside-linked sugar chains bound to the Fc region contained in the antibody composition is a ratio of the number of a sugar chain in which fucose is not bound to *N*-acetylglucosamine in the reducing end in the sugar chain to the total number of the complex *N*-glycoside-linked sugar chains bound to the Fc region contained in the composition.

The sugar chain in which fucose is not bound to *N*-acetylglucosamine in the reducing end in the complex *N*-glycoside-linked sugar chain is a sugar chain in which fucose is not bound to *N*-acetylglucosamine in the reducing end through α-bond in the complex *N*-glycoside-linked sugar chain. Specifically, it is a complex *N*-glycoside-linked sugar chain in which 1-position of fucose is not bound to 6-position of *N*-acetylglucosamine through α-bond.

The higher the ratio of a sugar chain in which fucose is not bound to *N*-acetylglucosamine in the reducing end in the sugar chain among the total complex *N*-glycoside-linked sugar chains bound to the Fc region contained in the antibody composition, the higher the ADCC activity of the antibody composition. The antibody composition having higher ADCC activity includes an antibody composition in which a ratio of a sugar chain in which fucose is not bound to *N*-acetylglucosamine in the reducing end in the sugar chain among the total complex *N*-glycoside-linked sugar chains bound to the Fc region contained in the antibody composition is preferably at 20% or more, more preferably 30% or more, still more preferably 40% or more, most preferably 50% or more, and far most preferably 100%.

Furthermore, the present invention relates to a process for producing an antibody composition having higher ADCC activity than an antibody composition produced by its parent cell.

When, in an antibody composition, the ratio of a sugar chain in which fucose is not bound to *N*-acetylglucosamine in the reducing end among the total complex *N*-glycoside-linked sugar chains bound to the Fc region contained in the antibody composition is higher than that of an antibody composition produced by its parent cell, the antibody composition has higher ADCC activity than the antibody composition produced by its parent cell.

The process for producing an antibody composition having higher ADCC activity than an antibody composition produced by its parent cell includes a process for producing an antibody composition using the above lectin-resistant clone.

The ADCC activity is a cytotoxic activity in which an antibody bound to a cell surface antigen on a tumor cell in the living body activate an effector cell through an Fc receptor existing on the antibody Fc region and effector cell surface and thereby obstruct the tumor cell and the like [*Monoclonal Antibodies: Principles and Applications,* Wiley-Liss, Inc., Chapter 2.1 (1995)]. The effector cell includes a killer cell, a natural killer cell, an activated macrophage and the like.

The ratio of a sugar chain in which fucose is not bound to *N*-acetylglucosamine in the reducing end in the sugar chain contained in the composition which comprises an antibody molecule having complex *N*-glycoside-linked

sugar chains in the Fc region can be determined by releasing the sugar chain from the antibody molecule, carrying out fluorescence labeling or radioisotope labeling of the released sugar chain and then separating the labeled sugar chain by chromatography using a known method such as hydrazinolysis or enzyme digestion [*Biochemical Experimentation Methods 23 - Method for Studying Glycoprotein Sugar Chain* (Japan Scientific Societies Press), edited by Reiko Takahashi (1989)]. Also, the released sugar chain can also be determined by analyzing it with the HPAED-PAD method [*J. Liq. Chromatogr.*, 6, 1577 (1983)].

The antibody molecule may be any antibody molecule, so long as it comprises the Fc region of an antibody. Examples include an antibody, an antibody fragment, a fusion protein comprising an Fc region, and the like.

Examples of the antibody include an antibody secreted by a hybridoma cell prepared from a spleen cell of an animal immunized with an antigen; an antibody prepared by a genetic recombination technique, namely an antibody obtained by introducing an antibody gene-inserted antibody expression vector into a host cell; and the like. Specific examples include an antibody produced by a hybridoma, a humanized antibody, a human antibody and the like.

A hybridoma is a cell which is obtained by cell fusion between a B cell obtained by immunizing a non-human mammal with an antigen and a myeloma cell derived from mouse or the like and which can produce a monoclonal antibody having the antigen specificity of interest.

The humanized antibody includes a human chimeric antibody, a human CDR-grafted antibody and the like.

A human chimeric antibody is an antibody which comprises H chain V region (hereinafter referred to as "HV" or "VH") and L chain V region (hereinafter referred to as "LV" or "VL"), both of a non-human animal antibody, a human antibody H chain C region (hereinafter also referred to as "CH") and a human antibody L chain C region (hereinafter also referred to as "CL"). The non-human animal may be any animal such as mouse, rat, hamster or rabbit, so long as a hybridoma can be prepared therefrom.

The human chimeric antibody can be produced by obtaining cDNAs encoding VH and VL from a monoclonal antibody-producing hybridoma, inserting them into an expression vector for host cell having genes encoding human antibody CH and human antibody CL to thereby construct a human chimeric antibody expression vector, and then introducing the vector into a host cell to express the antibody.

As the CH of human chimeric antibody, any CH can be used, so long as it belongs to human immunoglobulin (hereinafter referred to as "hIg") can be used, and those belonging to the hIgG class are preferred, and any one of the subclasses belonging to the hIgG class, such as hIgG1, hIgG2, hIgG3 and hIgG4, can be used. As the CL of human chimeric antibody, any CL can be used, so long as it belongs to the hIg class, and those belonging to the κ class or λ class can be used.

A human CDR-grafted antibody is an antibody in which amino acid sequences of CDRs of VH and VL of a non-human animal antibody are grafted into appropriate positions of VH and VL of a human antibody.

The human CDR-grafted antibody can be produced by constructing cDNAs encoding V regions in which CDRs of VH and VL of a non-human animal antibody are grafted into CDRs of VH and VL of a human antibody, inserting them into an expression vector for host cell having genes encoding human antibody CH and human antibody CL to thereby construct a human CDR-grafted antibody expression vector, and then introducing the expression vector into a host cell to express the human CDR-grafted antibody.

As the CH of human CDR-grafted antibody, any CH can be used, so long as it belongs to the hIg, and those of the hIgG class are preferred and any one of the subclasses belonging to the hIgG class, such as hIgG1, hIgG2, hIgG3 and hIgG4, can be used. As the CL of human CDR-grafted antibody, any CL can be used, so long as it belongs to the hIg class, and those belonging to the κ class or λ class can be used.

A human antibody is originally an antibody naturally existing in the human body, but it also includes antibodies obtained from a human antibody phage library, a human antibody-producing transgenic non-transgenic animal and a human antibody-producing transgenic plant, which are prepared based on the recent advance in genetic engineering, cell engineering and developmental engineering techniques.

The antibody existing in the human body can be prepared by isolating a human peripheral blood lymphocyte, immortalizing it by its infection with EB virus or the like and then cloning it to thereby obtain lymphocytes capable of producing the antibody, culturing the lymphocytes thus obtained, and collecting the antibody from the culture.

The human antibody phage library is a library in which antibody fragments such as Fab and single chain antibody are expressed on the phage surface by inserting a gene encoding an antibody prepared from a human B cell into a phage gene. A phage expressing an antibody fragment having the desired antigen binding activity can be recovered from the library, using its activity to bind to an antigen-immobilized substrate as the marker. The antibody fragment can be converted further into a human antibody molecule comprising two full H chains and two full L chains by genetic engineering techniques.

A human antibody-producing transgenic non-human animal is an animal in which a human antibody gene is introduced into cells. Specifically, a human antibody-producing transgenic non-human animal can be prepared by introducing a human antibody gene into ES cell of a mouse, transplanting the ES cell into an early stage embryo of other mouse and then developing it. By introducing a human chimeric antibody gene into a fertilized egg and developing it,

the transgenic non-human animal can be also prepared. A human antibody is prepared from the human antibody-producing transgenic non-human animal by obtaining a human antibody-producing hybridoma by a hybridoma preparation method usually carried out in non-human mammals, culturing the obtained hybridoma and accumulating the human antibody in the culture.

The transgenic non-human animal includes cattle, sheep, goat, pig, horse, mouse, rat, fowl, monkey, rabbit and the like.

In the present invention, as the antibody, preferred are an antibody which recognizes a tumor-related antigen, an antibody which recognizes an allergy- or inflammation-related antigen, an antibody which recognizes cardiovascular disease-related antigen, an antibody which recognizes an autoimmune disease-related antigen or an antibody which recognizes a viral or bacterial infection-related antigen, and a human antibody which belongs to the IgG class is preferred.

An antibody fragment is a fragment which comprises at least a part of the Fc region of an antibody. The Fc region is a region at the C-terminal of H chain of an antibody, CH2 region and CH3 region, and includes a natural type and a mutant type. "A part of the Fc region" is preferably a fragment containing CH2 region, more preferably a region containing Asp at position 1 in CH2 region. The Fc region of the IgG class is from Cys at position 226 to the C-terminal or from Pro at position 230 to the C-terminal according to the numbering of EU Index of Kabat *et al.* [*Sequences of Proteins of Immunological Interest*, $5^{th}$ Ed., Public Health Service, National Institutes of Health, Bethesda, MD. (1991)]. The antibody fragment includes an H chain monomer, an H chain dimer and the like.

A fusion protein comprising a part of an Fc region is a protein which is obtained by fusing an antibody comprising the Fc region of an antibody or the antibody fragment with a protein such as an enzyme or a cytokine (hereinafter referred to as "Fc fusion protein").

The present invention is explained below in detail.

1. Preparation of cell used for the production in the present invention

The cell used for producing the antibody composition of the present invention includes yeast, an animal cell, an insect cell, a plant cell and the like.

The cell used for producing the antibody composition of the present invention can be prepared by a technique for selecting a cell resistant to a lectin which recognizes a sugar chain in which 1-position of fucose is bound to 6-position of *N*-acetylglucosamine in the reducing end through α-bond in a complex *N*-glycoside-linked sugar chain. Furthermore, the cell of the present invention can be prepared by selecting a transformant which is resistant to a lectin which recognizes a sugar chain in which 1-position of fucose is bound to 6-position of *N*-acetylglucosamine in the reducing end through α-bond in a complex *N*-glycoside-linked sugar chain from transformants into which a gene encoding an antibody molecule is introduced in advance.

The method for selecting a clone resistant to a lectin which recognizes a sugar chain structure in which 1-position of fucose is bound to 6-position of *N*-acetylglucosamine in the reducing end through α-bond in the *N*-glycoside-linked sugar chain includes the method using lectin described in *Somatic Cell Mol. Genet.,* 12, 51 (1986) and the like.

As the lectin, any lectin can be used, so long as it recognizes a sugar chain structure in which 1-position of fucose is bound to 6-position of *N*-acetylglucosamine in the reducing end through α-bond in the *N*-glycoside-linked sugar chain. Examples include a *Lens culinaris* lectin LCA (lentil agglutinin derived from *Lens culinaris*), a pea lectin PSA (pea lectin derived from *Pisum sativum*), a broad bean lectin VFA (agglutinin derived from *Vicia faba*), an *Aleuria aurantia* lectin AAL (lectin derived from *Aleuria aurantia*) and the like.

Specifically, the cell resistant to a lectin which recognizes a sugar chain structure in which 1-position of fucose is bound to 6-position of *N*-acetylglucosamine in the reducing end through α-bond in the *N*-glycoside-linked sugar chain can be selected by culturing cells for 1 day to 2 weeks, preferably from 3 days to 1 week in a medium comprising the lectin at a concentration of 10 μg/ml to 10 mg/ml, preferably 0.5 to 2.0 μg/ml, and then by subjecting the surviving cells to subculturing or by picking up a colony and transferring it into a culture vessel, and subsequently continuing the culturing using the lectin-containing medium.

The lectin-resistant cell may be selected from a resistant clone obtained by a mutation-inducing treatment of a cell or a spontaneously generated clone.

As the mutation-inducing treatment, any treatment can be used, so long as it can induce a point mutation or a deletion or frame shift mutation in the DNA of cells of the parent cell line.

Examples of the mutation treatment include treatment with ethyl nitrosourea, nitrosoguanidine, benzopyrene or an acridine pigment and treatment with radiation. Also, various alkylating agents and carcinogens can be used as mutagens. The method for allowing a mutagen to act upon cells includes the method described in *Tissue Culture Techniques*, 3rd edition (Asakura Shoten), edited by Japanese Tissue Culture Association (1996), *Nature Genet.,* 24, 314 (2000) and the like.

The spontaneously generated mutant includes mutants which are naturally resistant to lectin, mutants which

are spontaneously formed by continuing subculture under general cell culture conditions without applying special mutation-inducing treatment, and the like.

The cell of the present invention can also be prepared by using a gene disruption technique, a technique for introducing a dominant negative mutant of the gene, or a technique for inhibiting transcription and/or translation of the gene while targeting a gene encoding GDP-fucose synthase, α1,6-fucose modifying enzyme or encoding a GDP-fucose transport protein.

The gene disruption method may be any method, so long as it can disrupt the gene of the target enzyme is included. Examples include a homologous recombination method, an RNA-DNA oligonucleotide (RDO) method, a method using retrovirus, a method using transposon, an antisense method, a ribozyme method, an RNA interference (RNAi) method and the like.

(1) Preparation of the cell of the present invention by antisense method or ribozyme method

The cell of the present invention can be prepared by the antisense method or the ribozyme method described in *Cell Technology,* 12, 239 (1993); *BIO/TECHNOLOGY,* 17, 1097 (1999); *Hum. Mol. Genet.,* 5, 1083 (1995); *Cell Technology,* 13, 255 (1994); *Proc. Natl. Acad. Sci. USA*, 96, 1886 (1999); or the like, e.g., in the following manner while targeting the above gene.

A cDNA or genomic DNA of the target gene is prepared.

The nucleotide sequence of the prepared cDNA or genomic DNA is determined.

Based on the determined DNA sequence, an antisense gene or ribozyme construct of an appropriate length comprising a part of a translation region, a part of an untranslated region or a part of an intron of the target gene is designed.

In order to express the antisense gene or ribozyme in a cell, a recombinant vector is prepared by inserting a fragment or total length of the prepared DNA into downstream of the promoter of an appropriate expression vector.

A transformant is obtained by introducing the recombinant vector into a host cell suitable for the expression vector.

The cell of the present invention can be obtained by selecting a transformant based on the activity of the protein encoded by the target gene. The cell of the present invention can also be obtained by selecting a transformant based on the sugar chain structure of a glycoprotein on the cell membrane or the sugar chain structure of the produced antibody molecule.

As the host cell used for the production of the cell of the present invention, any cell such as yeast, an animal cell, an insect cell or a plant cell can be used, so long as it has the target gene, and examples include host cells described in the following item 3.

As the expression vector, a vector which is autonomously replicable in the host cell or can be integrated into the chromosome and comprises a promoter at such a position that the designed antisense gene or ribozyme can be transferred is used. Examples include expression vectors described in the following item 3.

As the method for introducing a gene into various host cells, the methods for introducing recombinant vectors suitable for various host cells described in the following item 3 can be used.

As a method for obtaining a cDNA or genomic DNA of the target gene, the following method is exemplified.

Preparation method of cDNA:

A total RNA or mRNA is prepared from various host cells.

A cDNA library is prepared from the prepared total RNA or mRNA.

Degenerative primers are prepared based on a known amino acid sequence, such as a human amino acid sequence, of a protein encoded by the target gene, and a gene fragment of the target gene is obtained by PCR using the prepared cDNA library as the template.

A cDNA of the target gene can be obtained by screening the cDNA library using the obtained gene fragment as a probe.

The mRNA of various host cells may be a commercially available product (e.g., manufactured by Clontech) or may be prepared from various host cells as follows. The method for preparing total mRNA from various host cells include the guanidine thiocyanate-cesium trifluoroacetate method [*Methods in Enzymology*, 154, 3 (1987)], the acidic guanidine thiocyanate phenol chloroform (AGPC) method [*Analytical Biochemistry*, 162, 156 (1987); *Experimental Medicine (Jikken Igaku),* 9, 1937 (1991)] and the like.

Furthermore, a method for preparing mRNA as poly(A)$^+$ RNA from a total RNA includes the oligo(dT)-immobilized cellulose column method (*Molecular Cloning,* Second Edition)

In addition, mRNA can be prepared using a kit, such as Fast Track mRNA Isolation Kit (manufactured by Invitrogen) or Quick Prep mRNA Purification Kit (manufactured by Pharmacia).

A cDNA library is prepared from the prepared mRNA of various host cells. The method for preparing cDNA libraries includes the methods described in *Molecular Cloning,* Second Edition, *Current Protocols in Molecular Biology,* and the like, or methods using a commercially available kit, such as SuperScript Plasmid System for cDNA Synthesis and Plasmid Cloning (manufactured by Life Technologies) or ZAP-cDNA Synthesis Kit (manufactured by STRATAGENE).

As the cloning vector for the preparation of the cDNA library, any vector such as a phage vector or a plasmid vector can be used, so long as it is autonomously replicable in *Escherichia coli* K12. Examples include ZAP Express [manufactured by STRATAGENE, *Strategies,* 5, 58 (1992)], pBluescript II SK(+) [*Nucleic Acids Research,* 17, 9494 (1989)], Lambda ZAP II (manufactured by STRATAGENE), λgt10 and λgt11 [DNA Cloning, A Practical Approach, 1, 49 (1985)], λTriplEx (manufactured by Clontech), λExCell (manufactured by Pharmacia), pT7T318U (manufactured by Pharmacia), pcD2 [*Mol. Cell. Biol*., 3, 280 (1983)], pUC18 [*Gene,* 33, 103 (1985)] and the like.

Any microorganism can be used as the host microorganism, and *Escherichia coli* is preferably used. Examples include *Escherichia coli* XL1-Blue MRF' [manufactured by STRATAGENE, *Strategies,* 5, 81 (1992)], *Escherichia coli* C600 [*Genetics,* 39, 440 (1954)], *Escherichia coli* Y1088 [*Science,* 222, 778 (1983)], *Escherichia coli* Y1090 [*Science,* 222, 778 (1983)], *Escherichia coli* NM522 [*J. Mol. Biol*., 166, 1 (1983)], *Escherichia coli* K802 [*J. Mol. Biol*., 16, 118 (1966)], *Escherichia coli* JM:105 [*Gene,* 38, 275 (1985)] and the like.

The cDNA library may be used as such in the subsequent analysis, and in order to obtain a full length cDNA as efficient as possible by decreasing the ratio of an infull length cDNA, a cDNA library prepared using the oligo cap method developed by Sugano *et al.* [*Gene,* 138, 171 (1994); *Gene,* 200, 149 (1997); *Protein*, *Nucleic Acid and Protein,* 41, 603 (1996); *Experimental Medicine (Jikken Igaku)*, 11, 2491 (1993); *cDNA Cloning* (Yodo-sha) (1996); *Methods for Preparing Gene Libraries* (Yodo-sha) (1994)] may be used in the subsequent analysis.

Based on the amino acid sequence of the protein encoded by the target gene, degenerative primers specific for the 5'-terminal and 3'-terminal nucleotide sequences of a nucleotide sequence presumed to encode the amino acid sequence are prepared, and DNA is amplified by PCR [*PCR Protocols*, Academic Press (1990)] using the prepared cDNA library as the template to obtain a gene fragment of the target gene.

It can be confirmed that the obtained gene fragment is the target gene by a method generally used for analyzing a nucleotide such as the dideoxy method of Sanger *et al.* [*Proc. Natl. Acad Sci. USA*, 74, 5463 (1977)], or a nucleotide sequence analyzer such as ABIPRISM 377 DNA Sequencer (manufactured by PE Biosystems).

A cDNA of the target gene can be obtained by carrying out colony hybridization or plaque hybridization (*Molecular Cloning,* Second Edition) for the cDNA or cDNA library synthesized from the mRNA contained in various host cells, using the gene fragment as a DNA probe.

Also, a cDNA of the target gene can also be obtained by carrying out screening by PCR using the primers for obtaining a gene fragment of the target gene and using the cDNA or cDNA library synthesized from the mRNA contained in various host cells as the template.

The nucleotide sequence of the obtained DNA of the target gene is analyzed from its terminus and determined by a method generally used for analyzing a nucleotide such as the dideoxy method of Sanger *et al*. [*Proc. Natl. Acad Sci. USA,* 74, 5463 (1977)], or a nucleotide sequence analyzer such as ABIPRISM 377 DNA Sequencer (manufactured by PE Biosystems).

A gene encoding a protein having a function similar to the protein encoded by the target gene can also be determined from genes in a data base by searching a nucleotide sequence data base such as GenBank, EMBL or DDBJ using a homology retrieving program such as BLAST based on the determined cDNA nucleotide sequence.

The cDNA of the target gene can also be obtained by chemically synthesizing it with a DNA synthesizer such as DNA Synthesizer model 392 manufactured by Perkin Elmer using the phosphoamidite method, based on the determined DNA nucleotide sequence.

As an example of the method for preparing a genomic DNA of the target gene, the method described below is exemplified.

Preparation method of genomic DNA:

The method for preparing genomic DNA include known methods described in *Molecular Cloning,* Second Edition; *Current Protocols in Molecular Biology*; and the like. In addition, a genomic DNA of the target gene can also be isolated using a kit such as Genome DNA Library Screening System (manufactured by Genome Systems) or Universal GenomeWalker™ Kits (manufactured by CLONTECH).

The following method can be exemplified as the method for selecting a transformant based on the activity of a protein encoded by the target gene.

Method for selecting transformant:

The method for selecting a cell in which activity of a protein encoded by the target gene is decreased includes biochemical methods or genetic engineering techniques described in *New Biochemical Experimentation Series 3-Saccharides I*, *Glycoprotein* (Tokyo Kagaku Dojin), edited by Japanese Biochemical society (1988); *Cell Engineering, Supplement*, *Experimental Protocol Series, Glycobiology Experimental Protocol, Glycoprotein*, *Glycolipid and Proteoglycan* (Shujun-sha), edited by Naoyuki Taniguchi, Akemi Suzuki, Kiyoshi Furukawa and Kazuyuki Sugawara (1996); *Molecular Cloning,* Second Edition; *Current Protocols in Molecular Biology;* and the like. The biochemical method includes a method in which the function of the protein encoded by the target gene is evaluated. The genetic engineering technique includes the Northern analysis, RT-PCR and the like which measures the amount of mRNA of the target gene.
Furthermore, the method for selecting a cell based on morphological change caused by decrease of the activity of a protein encoded by the target gene includes a method for selecting a transformant based on the sugar structure of a produced antibody molecule as a marker, a method for selecting a transformant based on the sugar structure of a glycoprotein on a cell membrane, and the like. The method for selecting a transformant based on the sugar structure of an antibody-producing molecule includes methods described in the item 5 below. The method for selecting a transformant based on the sugar structure of a glycoprotein on a cell membrane includes the above method selecting a clone resistant to a lectin which recognizes a sugar chain structure wherein 1-position of fucose is bound to 6-position of *N*-acetylglucosamine in the reducing end through α-bond in the complex *N*-glycoside-linked sugar chain. Examples include a method using a lectin described in *Somatic Cell Mol. Genet*., 12, 51 (1986).
Furthermore, the cell of the present invention can also be obtained without using an expression vector, by directly introducing an antisense oligonucleotide or ribozyme which is designed based on the nucleotide sequence of the target gene into a host cell.
The antisense oligonucleotide or ribozyme can be prepared in the usual method or using a DNA synthesizer. Specifically, it can be prepared based on the sequence information of an oligonucleotide having a corresponding sequence of continued 5 to 150 bases, preferably 5 to 60 bases, and more preferably 10 to 40 bases, among nucleotide sequences of a cDNA and a genomic DNA of the target gene by synthesizing an oligonucleotide which corresponds to a sequence complementary to the oligonucleotide (antisense oligonucleotide) or a ribozyme comprising the oligonucleotide sequence.
The oligonucleotide includes oligo RNA and derivatives of the oligonucleotide (hereinafter referred to as "oligonucleotide derivatives").
The oligonucleotide derivatives includes oligonucleotide derivatives in which a phosphodiester bond in the oligonucleotide is converted into a phosphorothioate bond, an oligonucleotide derivative in which a phosphodiester bond in the oligonucleotide is converted into an N3'-P5' phosphoamidate bond, an oligonucleotide derivative in which ribose and a phosphodiester bond in the oligonucleotide are converted into a peptide-nucleic acid bond, an oligonucleotide derivative in which uracil in the oligonucleotide is substituted with C-5 propynyluracil, an oligonucleotide derivative in which uracil in the oligonucleotide is substituted with C-5 thiazoleuracil, an oligonucleotide derivative in which cytosine in the oligonucleotide is substituted with C-5 propynylcytosine, an oligonucleotide derivative in which cytosine in the oligonucleotide is substituted with phenoxazine-modified cytosine, an oligonucleotide derivative in which ribose in the oligonucleotide is substituted with 2'-*O*-propylribose and an oligonucleotide derivative in which ribose in the oligonucleotide is substituted with 2'-methoxyethoxyribose [*Cell Technology (Saibo Kogaku)*, 16, 1463 (1997)].

(2) Preparation of cell of the present invention by homologous recombination method

The cell of the present invention can be prepared by targeting a gene encoding the GDP-fucose synthase, the α1,6-fucose modifying enzyme or the fucose transport protein and modifying the target gene on chromosome with a homologous recombination technique.
The target gene on the chromosome can be modified by using a method described in *Manipulating the Mouse Embryo, A Laboratory Manual,* Second Edition, Cold Spring Harbor Laboratory Press (1994) (hereinafter referred to as "*Manipulating the Mouse Embryo, A Laboratory Manual*"); *Gene Targeting, A Practical Approach,* IRL Press at Oxford University Press (1993); *Biomanual Series 8, Gene Targeting, Preparation of Mutant Mice using ES Cells,* Yodosha (1995) (hereinafter referred to as "*Preparation of Mutant Mice using ES Cells*"); or the like, for example, as follows.
A genomic DNA of the target gene is prepared.
Based on the nucleotide sequence of the genomic DNA, a target vector is prepared for homologous recombination of a target gene to be modified (e.g., structural gene of the GDP-fucose synthase, the α1,6-fucose modifying enzyme or the fucose transport protein, or a promoter gene).
The cell of the present invention can be prepared by introducing the prepared target vector into a host cell and selecting a cell in which homologous recombination occurred between the target gene and target vector.
As the host cell, any cell such as yeast, an animal cell, an insect cell or a plant cell can be used, so long as

it has the target gene. Examples include the host cells described in the following item 3.
The method for preparing a genomic DNA of the target gene includes the methods described in "Preparation method of genomic DNA" in the item 1(1) and the like.
The target vector for the homologous recombination of the target gene can be prepared in accordance with a method described in *Gene Targeting, A Practical Approach,* IRL Press at Oxford University Press (1993); *Biomanual Series 8, Gene Targeting, Preparation of Mutant Mice using ES Cells,* Yodo-sha (1995) or the like. The target vector can be used as any of a replacement type, an insertion type and a gene trap type.
For introducing the target vector into various host cells, the method for introducing recombinant vectors suitable for various host cells described in the following item 3 can be used.
The method for efficiently selecting a homologous recombinant includes a method such as the positive selection, promoter selection, negative selection or polyA selection described in *Gene Targeting, A Practical Approach,* IRL Press at Oxford University Press (1993); *Biomanual Series 8, Gene Targeting, Preparation of Mutant Mice using ES Cells,* Yodo-sha (1995); or the like. The method for selecting the homologous recombinant of interest from the selected clones includes the Southern hybridization method for genomic DNA (*Molecular Cloning,* Second Edition), PCR [*PCR Protocols*, Academic Press (1990)], and the like.

(3) Preparation of cell of the present invention by RDO method

The cell of the present invention can be prepared by an RDO (RNA-DNA oligonucleotide) method by targeting a gene encoding the GDP-fucose synthase, the α1,6-fucose modifying enzyme or the fucose transport protein, for example, as follows.
A cDNA or genomic DNA of the target gene is prepared.
The nucleotide sequence of the prepared cDNA or genomic DNA is determined.
Based on the determined DNA sequence, an RDO construct of an appropriate length comprising a part of a translation region, a part of a untranslated region or a part of intron of the target gene, is designed and synthesized.
The cell of the present invention can be obtained by introducing the synthesized RDO into a host cell and then selecting a transformant in which a mutation occurred in the target protein.
As the host cell, any cell such as yeast, an animal cell, an insect cell or a plant cell can be used, so long as it has the target gene. Examples include the host cells described in the following item 3.
The method for introducing RDO into various host cells includes the method for introducing recombinant vectors suitable for various host cells, described in the following item 3.
The method for preparing the cDNA of the target gene includes the method described in "Preparation method of cDNA" in the item 1(1) and the like.
The method for preparing the genomic DNA of the target gene includes the method in "Preparation method of genomic DNA" described in the item 1(1) and the like.
The nucleotide sequence of the DNA can be determined by digesting it with appropriate restriction enzymes, cloning the fragments into a plasmid such as pBluescript SK(-) (manufactured by Stratagene), subjecting the clones to the reaction generally used as a method for analyzing a nucleotide sequence such as the dideoxy method of Sanger *et al.* [*Proc. Natl. Acad Sci. USA,* 74, 5463 (1977)] or the like, and then analyzing the clones using an automatic nucleotide sequence analyzer such as A.L.F. DNA Sequencer (manufactured by Pharmacia) or the like.
The RDO can be prepared by a usual method or using a DNA synthesizer.
The method for selecting a cell in which a mutation occurred in the gene encoding the GDP-fucose synthase, the α1,6-fucose modifying enzyme or the fucose transportation enzyme by introducing the RDO into the host cell includes the methods for directly detecting mutations in chromosomal genes described in *Molecular Cloning,* Second Edition, *Current Protocols in Molecular Biology* and the like.
Furthermore, the method described in the item 1(1) for selecting a transformant based on the activity of the protein encoded by the target gene, the method for selecting a transformant based on the sugar chain structure of a glycoprotein on the cell membrane, and the method for selecting a transformant based on the sugar structure of a produced antibody molecule described in the following item 5 can also be used.
The construct of the RDO can be designed in accordance with the methods described in *Science,* 273, 1386 (1996); *Nature Medicine,* 4, 285 (1998); *Hepatology,* 25, 1462 (1997); *Gene Therapy,* 5, 1960 (1999); *Gene Therapy,* 5, 1960 (1999); *J. Mol. Med.,* 75, 829 (1997); *Proc. Natl. Acad Sci. USA*, 96, 8774 (1999); *Proc. Natl. Acad. Sci. USA*, 96, 8768 (1999); *Nuc. Acids. Res.,* 27, 1323 (1999); *Invest. Dematol.,* 111, 1172 (1998); *Nature Biotech.,* 16, 1343 (1998); *Nature Biotech.,* 18, 43 (2000); *Nature Biotech.,* 18, 555 (2000); and the like.

(4) Preparation of cell of the present invention by RNAi method

The cell of the present invention can be prepared by an RNAi (RNA interference) method by targeting a gene

encoding the GDP-fucose synthase, the α1,6-fucose modifying enzyme or the fucose transportation protein, for example, as follows. The RNAi method means a method in which double stranded RNA is introduced into a cell and mRNA present in the cell homologous to the sequence of the RNA is decomposed and destroyed to thereby inhibit gene expression.

A cDNA of the target gene is prepared.

The nucleotide sequence of the prepared cDNA is determined.

Based on the determined DNA sequence, an RNAi gene construct of an appropriate length comprising a part of a translation region or a part of an untranslated region of the target gene, is designed.

In order to express the RNAi gene in a cell, a recombinant vector is prepared by inserting a fragment or full length of the prepared DNA into downstream of the promoter of an appropriate expression vector.

A transformant is obtained by introducing the recombinant vector into a host cell suitable for the expression vector.

The cell of the present invention can be obtained by selecting a transformant based on the activity of the protein encoded by the target gene or the sugar chain structure of the produced antibody molecule or of a glycoprotein on the cell membrane.

As the host cell, any cell such as yeast, an animal cell, an insect cell or a plant cell can be used, so long as it has the target gene. Examples include the host cells described in the following item 3.

As the expression vector, a vector which is autonomously replicable in the host cell or can be integrated into the chromosome and comprises a promoter at such a position that the designed RNAi gene can be transferred is used. Examples include expression vectors in which transcription is carried out by polymerase III and the expression vectors described in the following item 3.

As the method for introducing a gene into various host cells, the methods for introducing recombinant vectors suitable for various host cells described in the following item 3 can be used.

The method for selecting a transformant based on the activity of the protein encoded by the target gene or the method for selecting a transformant based on the sugar chain structure of a glycoprotein on the cell membrane includes the method described in the item 1(1). The method for selecting a transformant based on the sugar chain structure of a produced antibody molecule includes the method described in the following item 5.

The method for preparing cDNA of the protein encoded by the target gene includes the method described in "Preparation method of cDNA" in the item 1(1) and the like.

Furthermore, the cell of the present invention can also be obtained without using an expression vector, by directly introducing an RNAi gene designed based on the nucleotide sequence of the target gene.

The RNAi gene can be prepared in the usual method or by using a DNA synthesizer.

The RNAi gene construct can be designed in accordance with the methods described in *Nature*, 391, 806 (1998); *Proc. Natl. Acad Sci. USA,* 95, 15502 (1998); *Nature,* 395, 854 (1998); *Proc. Natl. Acad. Sci. USA*, 96, 5049 (1999); *Cell,* 95, 1017 (1998); *Proc. Natl. Acad. Sci. USA,* 96, 1451 (1999); *Proc. Natl. Acad. Sci. USA,* 95, 13959 (1998); *Nature Cell Biol.,* 2, 70 (2000); and the like.

The RNA used in the RNAi method of the present invention includes RNA corresponding to DNA encoding:

(a) GDP-fucose synthase;
(b) α1,6-fucose modifying enzyme;
(c) fucose transport protein, and the like.

The RNA is preferably RNA corresponding to DNA of α1,6-fucosyltransferase in the α1,6-fucose modifying enzyme.

The DNA encoding α1,6-fucosyltransferase includes:

(a) a DNA comprising the nucleotide sequence represented by SEQ ID NO:79;
(b) a DNA comprising the nucleotide sequence represented by SEQ ID NO:80;
(c) a DNA comprising the nucleotide sequence represented by SEQ ID NO:81;
(d) a DNA comprising the nucleotide sequence represented by SEQ ID NO:82; and the like.

The RNA used in the RNAi method of the present invention may be any double stranded RNA consisting of RNA and its complementary RNA and capable of decreasing the amount of mRNA of α1,6-fucosyltransferase. Regarding the length of the RNA, the RNA is a continuous RNA of preferably 1 to 30, more preferably 5 to 29, still more preferably 10 to 29, and most preferably 15 to 29. Examples include:

(a) an RNA corresponding to a DNA comprising the nucleotide sequence represented by 10 to 30 continuous nucleotides in the nucleotide sequence represented by SEQ ID NO:79;

(b) an RNA corresponding to a DNA comprising the nucleotide sequence represented by 10 to 30 continuous nucleotides in the nucleotide sequence represented by SEQ ID NO:80;
(c) an RNA corresponding to a DNA comprising the nucleotide sequence represented by 10 to 30 continuous nucleotides in the nucleotide sequence represented by SEQ ID NO:81; and
(d) an RNA corresponding to a DNA comprising the nucleotide sequence represented by 10 to 30 continuous nucleotides in the nucleotide sequence represented by SEQ ID NO:82. Preferable examples include:

(a) an RNA comprising the nucleotide sequence represented by SEQ ID NO:83;
(b) an RNA comprising the nucleotide sequence represented by SEQ ID NO:84;
(c) an RNA which comprises a nucleotide sequence in which one or a few nucleotides are deleted or added in the nucleotide sequence represented by SEQ ID NO:83 and has substantially the same RNAi activity as the RNA represented by SEQ ID NO:83; and
(d) an RNA which comprises a nucleotide sequence in which one or a few nucleotides are deleted or added in the nucleotide sequence represented by SEQ ID NO:84 and has substantially the same RNAi activity as the RNA represented by SEQ ID NO:84.

The above RNA having substantially the same RNAi activity as the RNA represented by SEQ ID NO:83 or 84 may be any RNA having RNAi activity to α1,6-fucosyltransferase similar to the RNA represented by SEQ ID NO: 83 or 84, and the quantitative element such as the length of the RNA may be different.
The nucleotide sequence in which one or a few nucleotides are deleted or added means a nucleotide sequence in which one or a few nucleotides are deleted and/or added at both terminals of SEQ ID NO:83 or 84. Regarding the length of the nucleotide sequence, the nucleotide sequence is a continuous RNA of preferably 1 to 30, more preferably 5 to 29, still more preferably 10 to 29, and most preferably 15 to 29.
Furthermore, a DNA corresponding to the RNA and its complementary DNA are within the scope of the present invention, and the DNA corresponding to the RNA includes a DNA comprising the nucleotide sequence represented by SEQ ID NO:63 or 64. Moreover, a recombinant DNA comprising a vector into the DNA and its complementary DNA are introduced and a transformant obtained by introducing the recombinant DNA into a cell are also within the scope of the present invention, and can be used for expressing the double stranded RNA.

(5) Preparation of cell of the present invention by method using transposon

The cell of the present invention can be prepared by selecting a mutant by using a transposon system described in *Nature Genet.,* 25, 35 (2000) or the like, based on the activity of the GDP-fucose synthase, the α1,6-fucose modifying enzyme or the fucose transport protein, or the sugar chain structure of a produced antibody molecule or of a glycoprotein on the cell membrane.
The transposon system is a system in which a mutation is induced by randomly inserting an exogenous gene into chromosome, wherein an exogenous gene interposed between transposons is generally used as a vector for inducing a mutation, and a transposase expression vector for randomly inserting the gene into chromosome is introduced into the cell at the same time.
Any transposase can be used, so long as it is suitable for the sequence of the transposon to be used.
As the exogenous gene, any gene can be used, so long as it can induce a mutation in the DNA of a host cell.
As the host cell, any cell such as yeast, an animal cell, an insect cell or a plant cell can be used, so long as it has the target gene (gene encoding the GDP-fucose synthase, gene encoding the α1,6-fucose modifying enzyme, or gene encoding the fucose transport protein). Examples include the host cells described in the following item 3. For introducing the gene into various host cells, the method for introducing recombinant vectors suitable for various host cells described in the following item 3 can be used.
The method for selecting a mutant based on the activity of the protein encoded by the target gene or the method for selecting a mutant based on the sugar chain structure of a glycoprotein on the cell membrane includes the method described in the above item 1(1). The method for selecting a mutant based on the sugar chain structure of a produced antibody molecule includes the method described in the following item 5.

(6) Preparation of cell of the present invention by method for introducing dominant negative mutant

The cell of the present invention can be prepared by targeting the GDP-fucose synthase, the α1,6-fucose modifying enzyme or the fucose transport protein using a technique for introducing a dominant negative mutant of the protein.
As the dominant negative mutant, the fucose transport protein is exemplified.
It is known that a transporter of an intracellular sugar nucleotide functions in the form of a dimer on the membrane of endoplasmic reticulum or the Golgi body [*J. Biol. Chem.*, 275, 17718 (2000)]. Also, it is reported that, when a mutant of a transporter of an intracellular sugar nucleotide is compulsorily expressed intracellularly, a heterodimer is formed with a wild type transporter, and the formed heterodimer has an activity to inhibit a wild type homodimer [*J. Biol. Chem.*, 275, 17718 (2000)]. Accordingly, a mutant of a transporter of an intracellular sugar nucleotide is prepared and introduced into a cell so that it can function as a dominant negative mutant. The mutant can be prepared using site-directed mutagenesis method described in *Molecular Cloning,* Second Edition, *Current Protocols in Molecular Biology* and the like.

The cell of the present invention can be prepared by using the prepared dominant negative mutant gene of the target enzyme in accordance with the method described in *Molecular Cloning,* Second Edition, *Current Protocols in Molecular Biology, Manipulating the Mouse Embryo* or the like, for example, as follows.

A dominant negative mutant gene of the target protein is prepared.

Based on the prepared full length DNA of the dominant negative mutant gene, a DNA fragment of an appropriate length containing a moiety encoding the protein is prepared, if necessary.

A recombinant vector is prepared by inserting the DNA fragment or full length DNA into downstream of the promoter of an appropriate expression vector.

A transformant is obtained by introducing the recombinant vector into a host cell suitable for the expression vector.

The host cell of the present invention can be prepared by selecting a transformant based on the activity of the target protein or based on the sugar chain structure of a produced antibody molecule or of a glycoprotein on the cell membrane.

As the host cell, any cell such as yeast, an animal cell, an insect cell or a plant cell can be used, so long as it has the target gene. Examples include the host cells described in the following item 3.

As the expression vector, a vector which is autonomously replicable in the host cell or can be integrated into the chromosome and comprises a promoter at a position where transcription of the DNA encoding the dominant negative mutant of interest can be effected is used. Examples include the expression vectors described in the following item 3.

For introducing the gene into various host cells, the method for introducing recombinant vectors suitable for various host cells described in the following item 3 can be used.

The method for selecting a mutant based on the activity of the target protein or the method for selecting a mutant based on the sugar chain structure of a glycoprotein on the cell membrane as a marker includes the method described in the item 1(1). The method for selecting a mutant based on the sugar chain structure of a produced antibody molecule as a marker includes the methods described in the following item 5.

2. Preparation of transgenic non-human animal or plant or the progenies thereof used in production of the present invention

The transgenic non-human animal or plant or the progenies thereof of the present invention can be prepared according to a conventional method from the embryonic stem cell, the fertilized egg cells or the plant cells of the present invention which is resistant to a lectin which recognizes a sugar chain structure wherein 1-position of fucose is bound to 6-position of *N*-acetylglucosamine in the reducing end through α-bond in the complex *N*-glycoside-linked sugar chain according to the known method, and which is prepared by the method described in the item 1.

In the case of a transgenic non-human animal, the embryonic stem cell of the present invention which is resistant to a lectin which recognizes a sugar chain structure wherein 1-position of fucose is bound to 6-position of *N*-acetylglucosamine in the reducing end through α-bond in the complex *N*-glycoside-linked sugar chain can be prepared by applying the method similar to that in the item 1 to an embryonic stem cell of a non-human animal of interest such as cattle, sheep, goat, pig, horse, mouse, rat, fowl, monkey or rabbit.

As the embryotic stem cell, mentioned is a mutant clone in which a gene encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain is inactivated or substituted with any sequence, by a known homologous recombination technique [e.g., *Nature,* 326, 6110, 295 (1987); *Cell,* 51, 3, 503 (1987); or the like]. Using the prepared embryonic stem cell (e.g., the mutant clone), a chimeric individual comprising the embryonic stem cell clone and a normal cell can be prepared by an injection chimera method into blastocyst of fertilized egg of an animal or by an aggregation chimera method. The chimeric individual is crossed with a normal individual to thereby obtain a transgenic non-human animal in which all the cells in the body ware resistant to a lectin which recognize the modification of a sugar chain wherein 1-position of fucose is bound to 6-position of *N*-acetylglucosamine in the reducing end through α-bond in the complex *N*-glycoside-linked sugar chain.

The target vector for the homologous recombination of the target gene can be prepared in accordance with

a method described in *Gene Targeting, A Practical Approach,* IRL Press at Oxford University Press (1993); *Preparation of Mutant Mice using ES Cells,* or the like. The target vector can be used as any of a replacement type, an insertion type and a gene trap type.

As the method for introducing the target vector into the embryonic stem cell, any method can be used, so long as it can introduce DNA into an animal cell. Examples include electroporation [*Cytotechnology*, 3, 133 (1990)], the calcium phosphate method (Japanese Published Unexamined Patent Application No. 227075/90), the lipofection method [*Proc. Natl. Acad Sci. USA*, 84, 7413 (1987)], the injection method (*Manipulating Mouse Embryo*, Second Edition), a method using particle gun (gene gun) (Japanese Patent No. 2606856, Japanese Patent No. 2517813), the DEAE-dextran method [*Biomanual Series 4-Gene Transfer and Expression Analysis* (Yodo-sha), edited by Takashi Yokota and Kenichi Arai (1994)], the virus vector method (*Manipulating Mouse Embryo*, Second Edition) and the like.

The method for efficiently selecting a homologous recombinant includes a method such as the positive selection, promoter selection, negative selection or polyA selection described in *Gene Targeting*, *A Practical Approach,* IRL Press at Oxford University Press (1993); or the like. Specifically, in the case of the target vector containing hprt gene, positive selection which selects the homologous recombinant of the hprt gene can be carried out by introducing the target vector into the hprt gene-defected embryonic stem cell, culturing the embryonic stem cell in a medium containing aminopterin, hypoxanthine and thymidine, and selecting an aminopterin-resistant clone. In the case of the target vector containing a neomycin-resistant gene, positive selection which selects a homologous recombinant containing neomycin-resistant gene can be carried out by culturing the vector-introduced embryonic stem cell in a medium containing G418, and selecting a G418-resistant gene. In the case of the target vector containing DT gene, negative selection which selects a DT gene-free homologous recombinant clone can be carried out by culturing the vector-introduced embryonic stem cell, and selecting the grown clone. (The recombinants in which DT gene is introduced into a chromosome at random other than the homogenous recombination cannot grow due to the toxicity of DT since the DT gene is expressed while integrated in the chromosome). The method for selecting the homogenous recombinant of interest among the selected clones include the Southern hybridization for genomic DNA (*Molecular Cloning,* Second Edition), PCR [*PCR Protocols,* Academic Press (1990)] and the like.

When the embryonic stem cell is introduced into a fertilized egg by using an aggregation chimera method, in general, a fertilized egg at the development stage before 8-cell stage is preferably used. When the embryonic stem cell is introduced into a fertilized egg by using an injection chimera method, in general, it is preferred that a fertilized egg at the development stage from 8-cell stage to batstocyst stage is preferably used.

When the fertilized egg is transplanted into a female mouse, it is preferred to artificially transplant or implant a fertilized egg obtained from a pseudopregnant female mouse in which fertility is induced by mating with a male non-human mammal which is subjected to vasoligation. Although the psuedopregnant female mouse can be obtained by natural mating, the pseudopregnant female mouse in which fertility is induced can also be obtained by mating with a male mouse after administration of a luteinizing hormone-releasing hormone (hereinafter referred to as "LHRH") or its analogue thereof. The analogue of LHRH includes [3,5-Dil-Tyr5]-LHRH, [Gln8]-LHRH, [D-Ala6]-LHRH, des-Gly10-[D-His(Bzl)6]-LHRH ethylamide and the like.

Also, a fertilized egg cell of the present invention which is resistant to a lectin which recognizes a sugar chain in which 1-position of fucose is bound to 6-position of *N*-acetylglucosamine in the reducing end through α-bond in a complex *N*-glycoside-linked sugar chain can be prepared by applying the method described in item 1 to fertilized egg of a non-human animal of interest such as cattle, sheep, goat, pig, horse, mouse, rat, fowl, monkey or rabbit.

A transgenic non-human animal in which all the cells in the body are resistant to a lectin which recognizes a sugar chain in which 1-position of fucose is bound to 6-position of *N*-acetylglucosamine in the reducing end through α-bond in a complex *N*-glycoside-linked sugar chain can be prepared by transplanting the prepared fertilized egg cell into the oviduct or uterus of a pseudopregnant female using the embryo transplantation method described in *Manipulating Mouse Embryo*, Second Edition or the like, followed by childbirth.

In the case of a transgenic plant, the callus containing plant cells which are resistant to a lectin which recognizes a sugar chain in which 1-position of fucose is bound to 6-position of *N*-acetylglucosamine in the reducing end through α-bond in a complex *N*-glycoside-linked sugar chain can be prepared by applying the method described in item 1 to a callus or cell of the plant of interest.

A transgenic plant which is resistant to a lectin which recognizes a sugar chain in which 1-position of fucose is bound to 6-position of *N*-acetylglucosamine in the reducing end through α-bond in a complex *N*-glycoside-linked sugar chain can be prepared by culturing the prepared callus using a medium comprising auxin and cytokinin to redifferentiate it in accordance with a known method [*Tissue Culture (Soshiki Baiyo),* 20 (1994); *Tissue Culture (Soshiki Baiyo),* 21 (1995); *Trends in Biotechnology*, 15, 45 (1997)].

3. Method for introducing gene encoding antibody molecule into cell

A gene encoding an antibody molecule can be introduced into a cell by the method described in *Molecular*

*Cloning*, Second Edition; *Current Protocols in Molecular Biology; Antibodies, A Laboratory Manual*, Cold Spring Harbor Laboratory, 1988 (hereinafter sometimes referred to as *"Antibodies"*); *Monoclonal Antibodies: Principles and Practice,* Third Edition, Acad. Press, 1993 (hereinafter referred to as "*Monoclonal Antibodies*"); or *Antibody Engineering, A Practical Approach,* IRL Press at Oxford University Press (hereinafter sometimes referred to as "*Antibody Engineering*"), for example, as follows.

A cDNA encoding an antibody molecule is prepared.

Based on the full length cDNA encoding the prepared antibody molecule, a DNA fragment of an appropriate length comprising a moiety encoding the protein is prepared, if necessary.

A recombinant vector is prepared by inserting the DNA fragment or the full length cDNA into downstream of the promoter of an appropriate expression vector.

A transformant of the present invention can be obtained by introducing the recombinant vector into a host cell suitable for the expression vector.

cDNA can be prepared from a tissue or cell of a human or non-human animal by using a probe primer specific to the antibody molecule of interest.

As the host cell, any of yeast, an animal cell, an insect cell, a plant cell or the like can be used, so long as it can express the gene encoding the antibody of interest.

A cell such as a yeast, an animal cell, an insect cell, a plant cell or the like into which an enzyme relating to the modification of an *N*-glycoside-linked sugar chain which binds to the Fc region of the antibody molecule is introduced by a genetic engineering technique can also be used as the host cell.

The host cell used for producing the antibody composition of the present invention includes the above-described various host cells resistant to a lectin which recognizes a sugar chain in which 1-position of fucose is bound to 6-position of *N*-acetylglucosamine in the reducing end through $\alpha$-bond in a complex *N*-glycoside-linked sugar chain.

As the expression vector, a vector which is autonomously replicable in the host cell or can be integrated into the chromosome and comprises a promoter at such a position that the DNA encoding the antibody molecule of interest can be transferred is used.

When a yeast is used as the host cell, the expression vector includes YEP13 (ATCC 37115), YEp24 (ATCC 37051), YCp50 (ATCC 37419) and the like.

Any promoter can be used, so long as it can function in yeast. Examples include a promoter of a gene of the glycolytic pathway such as a hexose kinase, PHO5 promoter, PGK promoter, GAP promoter, ADH promoter, gal 1 promoter, gal 10 promoter, heat shock protein promoter, MF $\alpha$1 promoter, CUP 1 promoter and the like.

The host cell includes yeasts belonging to the genus *Saccharomyces*, the genus *Schizosaccharomyces*, the genus *Kluyveromyces*, the genus *Trichosporon*, the genus *Schwanniomyces* and the like, such as *Saccharomyces cerevisiae*, *Schizosaccharomyces pombe*, *Kluyveromyces lactis*, *Trichosporon pullulans* and *Schwanniomyces alluvius.*

As the method for introducing the recombinant vector, any method can be used, so long as it can introduce DNA into yeast. Examples include electroporation [*Methods in Enzymology*, 194, 182 (1990)], the spheroplast method [*Proc. Natl. Acad. Sci. USA*, 84, 1929 (1978)], the lithium acetate method [*J. Bacteriol.*, 153, 163 (1983)], the method described in *Proc. Natl. Acad. Sci. USA*, 75, 1929 (1978) and the like.

When an animal cell is used as the host cell, the expression vector includes pcDNAI, pcDM8 (available from Funakoshi), pAGE107 [Japanese Published Unexamined Patent Application No. 22979/91; *Cytotechnology*, 3, 133 (1990)], pAS3-3 (Japanese Published Unexamined Patent Application No. 227075/90), pCDM8 [*Nature*, 329, 840 (1987)], pcDNAI/Amp (manufactured by Invitrogen), pREP4 (manufactured by Invitrogen), pAGE103 [*J. Biochemisny*, 101, 1307 (1987)], pAGE210 and the like.

Any promoter can be used, so long as it can function in an animal cell. Examples include a promoter of IE (immediate early) gene of cytomegalovirus (CMV), an early promoter of SV40, a promoter of retrovirus, a promoter of metallothionein, a heat shock promoter, an SR$\alpha$ promoter and the like. Also, an enhancer of the IE gene of human CMV can be used together with the promoter.

The host cell includes a human cell such as Namalwa cell, a monkey cell such as COS cell, a Chinese hamster cell such as CHO cell or HBT5637 (Japanese Published Unexamined Patent Application No. 299/88), a rat myeloma cell, a mouse myeloma cell, a cell derived from syrian hamster kidney, an embryonic stem cell, a fertilized egg cell and the like.

As the method for introducing the recombinant vector, any method can be used, so long as it can introduce DNA into an animal cell. Examples include electroporation [*Cytotechnology*, 3, 133 (1990)], the calcium phosphate method (Japanese Published Unexamined Patent Application No. 227075/90), the lipofection method [*Proc. Natl. Acad Sci. USA*, 84, 7413 (1987)], the injection method [*Manipulating the Mouse Embryo*, *A Laboratory Manual,* Second Edition, Cold Spring Harbor Laboratory Press (1994)], a method using a particle gun (gene gun) (Japanese Patent No. 2606856, Japanese Patent No. 2517813), the DEAE-dextran method [*Biomanual Series 4-Gene Transfer and Expression Analysis* (Yodo-sha), edited by Takashi Yokota and Kenichi Arai (1994)], the virus vector method [*Manipulating*

*Mouse Embryo*, Second Edition] and the like.

When an insect cell is used as the host cell, the protein can be expressed by the method described in *Current Protocols in Molecular Biology, Baculovirus Expression Vectors*, *A Laboratory Manual,* W.H. Freeman and Company, New York (1992), *Bio/Technology*, 6, 47 (1988) or the like.

That is, the protein can be expressed by co-introducing a recombinant gene-introducing vector and a baculovirus into an insect cell to obtain a recombinant virus in an insect cell culture supernatant and then infecting the insect cell with the recombinant virus.

The gene-introducing vector used in the method includes pVL1392, pVL1393, pBlueBacIII (all manufactured by Invitrogen) and the like.

The baculovirus includes *Autographa californica* nuclear polyhedrosis virus which is infected by an insect of the family *Barathra.*

The insect cell includes *Spodoptera frugiperda* oocytes Sf9 and Sf21 [*Current Protocols in Molecular Biology, Baculovirus Expression Vectors, A Laboratory Manual,* W.H. Freeman and Company, New York (1992)], a *Trichoplusia ni* oocyte High 5 (manufactured by Invitrogen) and the like.

The method for the co-introducing the recombinant gene-introducing vector and the baculovirus for preparing the recombinant virus includes the calcium phosphate method (Japanese Published Unexamined Patent Application No. 227075/90), the lipofection method [*Proc. Natl. Acad. Sci. USA*, 84, 7413 (1987)] and the like.

When a plant cell is used as the host cell, the expression vector includes Ti plasmid, tobacco mosaic virus vector and the like.

As the promoter, any promoter can be used, so long as it can function in a plant cell. Examples include cauliflower mosaic virus (CaMV) 35S promoter, rice actin 1 promoter and the like.

The host cell includes plant cells of tobacco, potato, tomato, carrot, soybean, rape, alfalfa, rice, wheat, barley, *etc*., and the like.

As the method for introducing the recombinant vector, any method can be used, so long as it can introduce DNA into a plant cell. Examples include a method using *Agrobacterium* (Japanese Published Unexamined Patent Application No. 140885/84, Japanese Published Unexamined Patent Application No. 70080/85, WO 94/00977), electroporation (Japanese Published Unexamined Patent Application No. 251887/85), a method using a particle gun (gene gun) (Japanese Patent No. 2606856, Japanese Patent No. 2517813) and the like.

As the method for expressing a gene encoding an antibody, secretion production, expression of a fusion protein and the like can be carried out in accordance with the method described in *Molecular Cloning,* Second Edition or the like, in addition to the direct expression.

When a gene relating to synthesis of a sugar chain is expressed by yeast, an animal cell, an insect cell or a plant cell into which a gene relating to the synthesis of a sugar chain is introduced, an antibody molecule to which a sugar or a sugar chain is added by the introduced gene can be obtained.

An antibody composition of interest can be obtained by culturing the obtained transformant in a medium to produce and accumulate the antibody molecule of interest in the culture and then recovering it from the resulting culture. The method for culturing the transformant using a medium can be carried out in accordance with a general method which is used for the culturing of host cells.

As the medium for culturing a transformant obtained using a eukaryote, such as yeast, as the host cell, the medium may be either a natural medium or a synthetic medium, so long as it comprises materials such as a carbon source, a nitrogen source, an inorganic salt and the like which can be assimilated by the organism and culturing of the transformant can be efficiently carried out.

As the carbon source, those which can be assimilated by the organism can be used. Examples include carbohydrates such as glucose, fructose, sucrose, molasses containing them, starch and starch hydrolysate; organic acids such as acetic acid and propionic acid; alcohols such as ethanol and propanol; and the like.

The nitrogen source includes ammonia; ammonium salts of inorganic acid or organic acid such as ammonium chloride, ammonium sulfate, ammonium acetate and ammonium phosphate; other nitrogen-containing compounds; peptone; meat extract; yeast extract; corn steep liquor; casein hydrolysate; soybean meal; soybean meal hydrolysate; various fermented cells and hydrolysates thereof; and the like.

The inorganic material includes potassium dihydrogen phosphate, dipotassium hydrogen phosphate, magnesium phosphate, magnesium sulfate, sodium chloride, ferrous sulfate, manganese sulfate, copper sulfate, calcium carbonate, and the like.

The culturing is carried out generally under aerobic conditions such as shaking culture or submerged-aeration stirring culture. The culturing temperature is preferably 15 to 40°C, and the culturing time is generally 16 hours to 7 days. During the culturing, the pH is maintained at 3.0 to 9.0. The pH is adjusted using an inorganic or organic acid, an alkali solution, urea, calcium carbonate, ammonia or the like.

If necessary, an antibiotic such as ampicillin or tetracycline can be added to the medium during the culturing.

When a microorganism transformed with a recombinant vector obtained using an inducible promoter as the

promoter is cultured, an inducer can be added to the medium, if necessary. For example, when a microorganism transformed with a recombinant vector obtained using *lac* promoter is cultured, isopropyl-β-D-thiogalactopyranoside can be added to the medium, and when a microorganism transformed with a recombinant vector obtained using *trp* promoter is cultured, indoleacrylic acid can be added to the medium.

When a transformant obtained using an animal cell as the host cell is cultured, the medium includes generally used RPMI 1640 medium [*The Journal of the American Medical Association,* 199, 519 (1967)], Eagle's MEM medium [*Science,* 122, 501 (1952)], Dulbecco's modified MEM medium [*Virology*, 8, 396 (1959)], 199 medium [*Proceeding of the Society for the Biological Medicine,* 73, 1 (1950)] and Whitten's medium [*Developmental Engineering Experimentation Manual-Preparation of Transgenic Mice* (Kodan-sha), edited by M. Katsuki (1987)], the media to which fetal calf serum, *etc.* is added, and the like.

The culturing is carried out generally at a pH of 6 to 8 and 30 to 40°C for 1 to 7 days in the presence of 5% $CO_2$.

If necessary, an antibiotic such as kanamycin or penicillin can be added to the medium during the culturing. Culturing can also be carried out by culturing a method such as fed-batch culturing or hollo-fiber culturing for several days to several months.

The medium for use in the culturing of a transformant obtained using an insect cell as the host cell includes generally used TNM-FH medium (manufactured by Pharmingen), Sf-900 II SFM medium (manufactured by Life Technologies), ExCell 400 and ExCell 405 (both manufactured by JRH Biosciences), Grace's Insect Medium [*Nature*, 195, 788 (1962)] and the like.

The culturing is carried out generally at a medium pH of 6 to 7 and 25 to 30°C for 1 to 5 days.

In addition, antibiotics such as gentamicin can be added to the medium during the culturing, if necessary.

A transformant obtained using a plant cell as the host cell can be cultured as a cell or after differentiating it into a plant cell or organ. The medium for culturing the transformant includes generally used Murashige and Skoog (MS) medium and White medium, the media to which a plant hormone such as auxin or cytokinin is added, and the like.

The culturing is carried out generally at a pH of 5 to 9 and 20 to 40°C for 3 to 60 days.

If necessary, an antibiotic such as kanamycin or hygromycin can be added to the medium during the culturing.

Thus, an antibody composition can be produced by culturing a transformant derived from yeast, an animal cell, an insect cell or a plant cell, which comprises a recombinant vector into which a DNA encoding an antibody molecule is inserted, in accordance with a general culturing method, to thereby produce and accumulate the antibody composition, and then recovering the antibody composition from the culture.

The process for producing an antibody composition includes a method of intracellular expression in a host cell, a method of extracellular secretion from a host cell, and a method of production on a host cell membrane outer envelope. The method can be selected by changing the host cell used or the structure of an antibody composition produced.

When the antibody composition of the present invention is produced in a host cell or on a host cell membrane outer envelope, it can be positively secreted extracellularly in accordance with the method of Paulson *et al.* [*J. Biol. Chem*., 264, 17619 (1989)], the method of Lowe *et al*. [*Proc. Natl. Acad Sci. USA*, 86, 8227 (1989), *Genes Develop.,* 4, 1288 (1990)], the methods described in Japanese Published Unexamined Patent Application No. 336963/93 and Japanese Published Unexamined Patent Application No. 823021/94 and the like.

That is, an antibody molecule of interest can be positively secreted extracellularly from a host cell by inserting a DNA encoding the antibody molecule and a DNA encoding a signal peptide suitable for the expression of the antibody molecule into an expression vector using a gene recombination technique, and introducing the expression vector into the host cell.

Also, the production can be increased in accordance with the method described in Japanese Published Unexamined Patent Application No. 227075/90 using a gene amplification system using a dihydrofolate reductase gene.

In addition, the antibody composition can also be produced using a gene-introduced animal individual (transgenic non-human animal) or a plant individual (transgenic plant) which is constructed by the redifferentiation of an animal or plant cell into which the gene is introduced.

When the transformant is an animal individual or a plant individual, an antibody composition can be produced in accordance with a general method by rearing or cultivating it to thereby produce and accumulate the antibody composition and then recovering the antibody composition from the animal or plant individual.

The process for producing an antibody composition using an animal individual includes a method in which the antibody composition of interest is produced in an animal constructed by introducing a gene in accordance with a known method [*American Journal of Clinical Nutrition*, 63, 639S (1996); *American Journal of Clinical Nutrition*, 63, 627S (1996); *Bio/Technology*, 9, 830 (1991)].

In the case of an animal individual, an antibody composition can be produced by rearing a transgenic non-human animal into which a DNA encoding an antibody molecule is introduced to thereby produce and accumulate the antibody composition in the animal, and then recovering the antibody composition from the animal. The place in the animal where the composition is produced and accumulated includes milk (Japanese Published Unexamined Patent

Application No. 309192/88) and eggs of the animal. As the promoter used in this case, any promoter can be used, so long as it can function in an animal. Preferred examples include mammary gland cell-specific promoters such as α casein promoter, β casein promoter, β lactoglobulin promoter, whey acidic protein promoter and the like.

The process for producing an antibody composition using a plant individual includes a method in which an antibody composition is produced by cultivating a transgenic plant into which a DNA encoding an antibody molecule is introduced by a known method [*Tissue Culture (Soshiki Baiyo),* 20 (1994); *Tissue Culture (Soshiki Baiyo),* 21 (1995); *Trends in Biotechnology*, 15, 45 (1997)] to produce and accumulate the antibody composition in the plant, and then recovering the antibody composition from the plant.

Regarding purification of an antibody composition produced by a transformant into which a gene encoding an antibody molecule is introduced, for example, when the antibody composition is intracellularly expressed in a dissolved state, the cells after culturing are recovered by centrifugation, suspended in an aqueous buffer and then disrupted using ultrasonic oscillator, French press, Manton Gaulin homogenizer, dynomill or the like to obtain a cell-free extract, which is centrifuged to obtain a supernatant, and a purified product of the antibody composition can be obtained by subjecting the supernatant to a general enzyme isolation and purification techniques such as solvent extraction; salting out and desalting with ammonium sulfate, *etc.;* precipitation with an organic solvent; anion exchange chromatography using a resin such as DIAION HPA-75 (manufactured by Mitsubishi Chemical); cation exchange chromatography using a resin such as S-Sepharose FF (manufactured by Pharmacia); hydrophobic chromatography using a resin such as butyl-Sepharose or phenyl-Sepharose; gel filtration using a molecular sieve; affinity chromatography; chromatofocusing; electrophoresis such as isoelectric focusing; and the like which may be used alone or in combination.

When the antibody composition is expressed intracellularly by forming an insoluble body, the cells are recovered, disrupted and centrifuged in the same manner, and the insoluble body of the antibody composition is recovered as a precipitation fraction. The recovered insoluble body of the antibody composition is solubilized with a protein denaturing agent. The antibody composition is made into a normal three-dimensional structure by diluting or dialyzing the solubilized solution, and then a purified product of the antibody composition is obtained by the same isolation purification method.

When the antibody composition is secreted extracellularly, the antibody composition or derivatives thereof can be recovered from the culture supernatant. That is, the culture is treated by a technique such as centrifugation to obtain a soluble fraction, and a purified preparation of the antibody composition can be obtained from the soluble fraction by the same isolation purification method.

The antibody composition thus obtained includes an antibody, the fragment of the antibody, a fusion protein comprising the Fc region of the antibody, and the like.

As examples for obtaining the antibody composition, processes for producing a humanized antibody composition and an Fc fusion protein are described below in detail, but other antibody compositions can also be obtained in a manner similar to the methods.

A. Preparation of humanized antibody composition

(1) Construction of vector for expression of humanized antibody

A vector for expression of humanized antibody is an expression vector for animal cell into which genes encoding CH and CL of a human antibody are inserted, which can be constructed by cloning each of genes encoding CH and CL of a human antibody into an expression vector for animal cell.

The C regions of a human antibody may be CH or CL of any human antibody. Examples include the C region belonging to IgG1 subclass in the H chain of a human antibody (hereinafter referred to as "hCγ1"), the C region belonging to κ class in the L chain of a human antibody (hereinafter referred to as "hCκ"), and the like.

As the genes encoding CH and CL of a human antibody, a chromosomal DNA comprising an exon and an intron can be used, and a cDNA can also be used.

As the expression vector for animal cell, any vector can be used, so long as a gene encoding the C region of a human antibody can be inserted thereinto and expressed therein. Examples include pAGE107 [*Cytotechnology*, 3, 133 (1990)], pAGE103 [*J. Biochem.,* 101, 1307 (1987)], pHSG274 [*Gene,* 27, 223 (1984)], pKCR [*Proc. Natl. Acad. Sci. USA*, 78, 1527 (1981), pSG1 β d2-4 [*Cytotechnology*, 4, 173 (1990)] and the like. The promoter and enhancer in the expression vector for animal cell include SV40 early promoter and enhancer [*J. Biochem.,* 101, 1307 (1987)], Moloney mouse leukemia virus LTR [*Biochem. Biophys. Res. Commun.*, 149, 960 (1987)], immunoglobulin H chain promoter [*Cell,* 41, 479 (1985)] and enhancer [*Cell,* 33, 717 (1983)], and the like.

The vector for expression of humanized antibody may be either of a type in which genes encoding the H chain and L chain of an antibody exist on separate vectors or of a type in which both genes exist on the same vector (tandem type). In respect of easiness of construction of a vector for expression of humanized antibody, easiness of introduction into animal cells, and balance between the expression amounts of the H and L chains of an antibody in animal cells,

a tandem type of the vector for expression of humanized antibody is more preferred [*J. Immunol. Methods,* 167, 271 (1994)].

The constructed vector for expression of humanized antibody can be used for expression of a human chimeric antibody and a human CDR-grafted antibody in animal cells.

(2) Preparation method of cDNA encoding V region of non-human animal antibody

cDNAs encoding VH and VL of a non-human animal antibody such as a mouse antibody can be obtained in the following manner.

A cDNA is synthesized from mRNA extracted from a hybridoma cell which produces the mouse antibody of interest. The synthesized cDNA is cloned into a vector such as a phage or a plasmid to obtain a cDNA library. Each of a recombinant phage or recombinant plasmid comprising a cDNA encoding VH and a recombinant phage or recombinant plasmid comprising a cDNA encoding VL is isolated from the library by using a C region part or a V region part of an existing mouse antibody as the probe. Full nucleotide sequences of VH and VL of the mouse antibody of interest on the recombinant phage or recombinant plasmid are determined, and full length amino acid sequences of VH and VL are deduced from the nucleotide sequences.

As the non-human animal, any animal such as mouse, rat, hamster, rabbit or the like can be used so long as a hybridoma cell can be produced therefrom.

The method for preparing total RNA from a hybridoma cell includes the guanidine thiocyanate-cesium trifluoroacetate method [*Methods in Enzymology*, 154, 3 (1987)] and the like, and the method for preparing mRNA from total RNA includes an oligo(dT)-immobilized cellulose column method (*Molecular Cloning,* Second Edition) and the like. In addition, a kit for preparing mRNA from a hybridoma cell includes Fast Track mRNA Isolation Kit (manufactured by Invitrogen), Quick Prep mRNA Purification Kit (manufactured by Pharmacia) and the like.

The method for synthesizing cDNA and preparing a cDNA library includes the usual methods (*Molecular Cloning,* Second Edition, *Current Protocols in Molecular Biology,* Supplement 1-34), methods using a commercially available kit such as SuperScript™, Plasmid System for cDNA Synthesis and Plasmid Cloning (manufactured by GIBCO BRL) or ZAP-cDNA Synthesis Kit (manufactured by Stratagene), and the like.

In preparing the cDNA library, the vector into which a cDNA synthesized by using mRNA extracted from a hybridoma cell as the template is inserted may be any vector so long as the cDNA can be inserted. Examples include ZAP Express [*Strategies*, 5, 58 (1992)], pBluescript II SK(+) [*Nucleic Acids Research*, 17, 9494 (1989)], λzapII (manufactured by Stratagene), λgt10 and λgt11 [*DNA Cloning, A Practical Approach,* I, 49 (1985)], Lambda BlueMid (manufactured by Clontech), λExCell, pT7T3 18U (manufactured by Pharmacia), pcD2 [*Mol. Cell. Biol.*, 3, 280 (1983)], pUC18 [*Gene,* 33, 103 (1985)] and the like.

As *Escherichia coli* into which the cDNA library constructed from a phage or plasmid vector is introduced, any *Escherichia coli* can be used, so long as the cDNA library can be introduced, expressed and maintained. Examples include XL1-Blue MRF' [*Strategies*, 5, 81 (1992)], C600 [*Genetics,* 39, 440 (1954)], Y1088 and Y1090 [*Science*, 222, 778 (1983)], NM522 [*J. Mol. Biol.*, 166, 1 (1983)], K802 [*J. Mol. Biol.*, 16, 118 (1966)], JM105 [*Gene*, 38, 275 (1985)] and the like.

As the method for selecting a cDNA clone encoding VH and VL of a non-human animal antibody from the cDNA library, a colony hybridization or a plaque hybridization using an isotope- or fluorescence-labeled probe can be used (*Molecular Cloning,* Second Edition). The cDNA encoding VH and VL can also be prepared by preparing primers and carrying out polymerase chain reaction (hereinafter referred to as "PCR"; *Molecular Cloning,* Second Edition; *Current Protocols in Molecular Biology,* Supplement 1-34) using a cDNA synthesized from mRNA or a cDNA library as the template.

The nucleotide sequences of the cDNAs can be determined by digesting the selected cDNAs with appropriate restriction enzymes, cloning the fragments into a plasmid such as pBluescript SK(-) (manufactured by Stratagene), carrying out the reaction of a generally used nucleotide sequence analyzing method such as the dideoxy method of Sanger *et al.* [*Proc. Natl. Acad Sci. USA*, 74, 5463 (1977)] or the like and then analyzing the clones using an automatic nucleotide sequence analyzer such as A.L.F. DNA Sequencer (manufactured by Pharmacia) or the like. Whether or not the obtained cDNAs encode the full length amino acid sequences of VH and VL of the antibody containing a secretory signal sequence can be confirmed by deducing the full length amino acid sequences of VH and VL from the determined nucleotide sequence and comparing them with the full length amino acid sequences of VH and VL of known antibodies [*Sequences of Proteins of Immunological Interest*, US Dep. Health and Human Services (1991) (hereinafter referred to as "*Sequences of Proteins of Immunological Interest*"].

(3) Analysis of amino acid sequence of V region of non-human animal antibody

Regarding the full length amino acid sequences of VH and VL of the antibody comprising a secretory signal

sequence, the length of the secretory signal sequence and the *N*-terminal amino acid sequences can be deduced and subgroups to which they belong can also be found, by comparing them with the full length amino acid sequences of VH and VL of known antibodies (*Sequences of Proteins of Immunological Interest*). In addition, the amino acid sequences of each CDR pf VH and VL can also be found by comparing them with the amino acid sequences of VH and VL of known antibodies (*Sequences of Proteins of Immunological Interest*).

(4) Construction of human chimeric antibody expression vector

A human chimeric antibody expression vector can be constructed by cloning cDNAs encoding VH and VL of a non-human animal antibody into upstream of genes encoding CH and CL of a human antibody in the vector for expression of humanized antibody described in the item 3(1). For example, a human chimeric antibody expression vector can be constructed by linking each of cDNAs encoding VH and VL of a non-human animal antibody to a synthetic DNA comprising nucleotide sequences at the 3'-terminals of VH and VL of a non-human animal antibody and nucleotide sequences at the 5'-terminals of CH and CL of a human antibody and also having a recognition sequence of an appropriate restriction enzyme at both terminals, and by cloning them into upstream of genes encoding CH and CL of a human antibody contained in the vector for expression of humanized antibody described in the item 3(1) in such a manner that they can be expressed in a suitable form.

(5) Construction of cDNA encoding V region of human CDR-grafted antibody

cDNAs encoding VH and VL of a human CDR-grafted antibody can be obtained as follows. First, amino acid sequences of the frameworks (hereinafter referred to as "FR") of VH and VL of a human antibody for grafting CDR of VH and VL of a non-human animal antibody is selected. As the amino acid sequences of FRs of VH and VL of a human antibody, any amino acid sequences can be used so long as they are derived from a human antibody. Examples include amino acid sequences of FRs of VH and VL of human antibodies registered at databases such as Protein Data Bank, amino acid sequences common in each subgroup of FRs of VH and VL of human antibodies (*Sequences of Proteins of Immunological Interest*) and the like. In order to produce a human CDR-grafted antibody having enough activities, it is preferred to select an amino acid sequence having a homology as high as possible (at least 60% or more) with amino acid sequences of VH and VL of a non-human animal antibody of interest.

Next, the amino acid sequences of CDRs of VH and VL of the non-human animal antibody of interest are grafted to the selected amino acid sequences of FRs of VH and VL of a human antibody to design amino acid sequences of VH and VL of the human CDR-grafted antibody. The designed amino acid sequences are converted into DNA sequences by considering the frequency of codon usage found in nucleotide sequences of antibody genes (*Sequences of Proteins of Immunological Interest*), and the DNA sequences encoding the amino acid sequences of VH and VL of the human CDR-grafted antibody are designed. Based on the designed DNA sequences, several synthetic DNAs having a length of about 100 bases are synthesized, and PCR is carried out by using them. In this case, it is preferred in each of the H chain and the L chain that 6 synthetic DNAs are designed in view of the reaction efficiency of PCR and the lengths of DNAs which can be synthesized.

Also, they can be easily cloned into the vector for expression of humanized antibody described in the item 3 (1) by introducing recognition sequences of an appropriate restriction enzyme into the 5'-terminals of the synthetic DNA present on both terminals. After the PCR, the amplified product is cloned into a plasmid such as pBluescript SK (-) (manufactured by Stratagene) and the nucleotide sequences are determined by the method in the item 3(2) to thereby obtain a plasmid having DNA sequences encoding the amino acid sequences of VH and VL of the desired human CDR-grafted antibody.

(6) Modification of amino acid sequence of V region of human CDR-grafted antibody

It is known that when a human CDR-grafted antibody is prepared by simply grafting only CDRs in VH and VL of a non-human animal antibody into FRs in VH and VL of a human antibody, its antigen-binding activity is lower than that of the original non-human animal antibody [*BIO*/*TECHNOLOGY*, 9, 266 (1991)]. As the reason, it is considered that several amino acid residues of FRs other than CDRs directly or indirectly relate to antigen-binding activity in VH and VL of the original non-human animal antibody, and that they are changed to different amino acid residues of FRs in VH and VL of a human antibody. In order to solve the problem, in human CDR-grafted antibodies, among the amino acid sequences of FRs in VH and VL of a human antibody, an amino acid residue which directly relates to binding to an antigen, or an amino acid residue which indirectly relates to binding to an antigen by interacting with an amino acid residue in CDR or by maintaining the three-dimensional structure of an antibody is identified and modified to an amino acid residue which is found in the original non-human animal antibody to thereby increase the antigen binding activity which has been decreased [*BIO*/*TECHNOLOGY*, 9, 266 (1991)].

In the preparation of a human CDR-grafted antibody, it is the most important to efficiently identify the amino acid residues relating to the antigen binding activity in FR. For identifying the amino acid residues of FR relating to the antibody-antigen binding activity, the three-dimensional structure of an antibody is constructed, and analyzed by X-ray crystallography [*J. Mol. Biol.*, 112, 535 (1977)], computer-modeling [*Protein Engineering,* 7, 1501 (1994)] or the like. Although the information of the three-dimensional structure of antibodies has been useful in the production of a human CDR-grafted antibody, method for producing a human CDR-grafted antibody which can be applied to all antibodies has not been established yet. Therefore, various attempts must be currently be necessary, for example, several modified antibodies of each antibody are produced and the relationship between each of the modified antibodies and its antibody binding activity is examined.
The amino acid sequence of FRs in VH and VL of a human antibody can be modified using various synthetic DNA for modification according to PCR as described in the item 3(5). With regard to the amplified product obtained by the PCR, the nucleotide sequence is determined according to the method as described in the item 3(2) to thereby confirm whether the objective modification has been carried out.

(7) Construction of human CDR-grafted antibody expression vector

A human CDR-grafted antibody expression vector can be constructed by cloning the cDNAs encoding VH and VL of the human CDR-grafted antibody constructed in the items 3(5) and (6) into upstream of the gene encoding CH and CL of a human antibody in the vector for expression of humanized antibody described in the item 3(1). For example, recognizing sequences of an appropriate restriction enzyme are introduced into the 5'-terminals of both terminals of a synthetic DNA fragment, among the synthetic DNA fragments which are used in the items 3(5) and (6) for constructing the VH and VL of the human CDR-grafted antibody, so that they are cloned into upstream of the genes encoding CH and CL of a human antibody in the vector for expression of humanized antibody described in the item 3 (1) in such a manner that they can be expressed in a suitable form, to thereby construct the human CDR-grafted antibody expression vector.

(8) Stable production of humanized antibody

A transformant capable of stably producing a human chimeric antibody and a human CDR-grafted antibody (both hereinafter referred to as "humanized antibody") can be obtained by introducing the vectors for humanized antibody expression described in the items 3(4) and (7) into an appropriate animal cell.
The method for introducing a humanized antibody expression vector into an animal cell includes electroporation [Japanese Published Unexamined Patent Application No. 257891/90, *Cytotechnology*, 3, 133 (1990)] and the like.
As the animal cell into which a humanized antibody expression vector is introduced, any cell can be used so long as it is an animal cell which can produce the humanized antibody.
Examples include mouse myeloma cells such as NS0 cell and SP2/0 cell, Chinese hamster ovary cells such as CHO/dhfr$^-$ cell and CHO/DG44 cell, rat myeloma such as YB2/0 cell and IR983F cell, BHK cell derived from a syrian hamster kidney, a human myeloma cell such as Namalwa cell, and the like, and the host cells of the present invention described in the item 1 are preferred.
After introduction of the humanized antibody expression vector, a transformant capable of stably producing the humanized antibody can be selected using a medium for animal cell culture comprising an agent such as G418 sulfate (hereinafter referred to as "G418"; manufactured by SIGMA) and the like in accordance with the method disclosed in Japanese Published Unexamined Patent Application No. 257891/90. The medium to culture animal cells includes RPMI 1640 medium (manufactured by Nissui Pharmaceutical), GIT medium (manufactured by Nihon Pharmaceutical), EX-CELL 302 medium (manufactured by JRH), IMDM medium (manufactured by GIBCO BRL), Hybridoma-SFM medium (manufactured by GIBCO BRL), media obtained by adding various additives such as fetal bovine serum (hereinafter referred to as "FBS") to these media, and the like. The humanized antibody can be produced and accumulated in the culture supernatant by culturing the obtained transformant in a medium. The amount of production and antigen binding activity of the humanized antibody in the culture supernatant can be measured by a method such as enzyme-linked immunosorbent assay (hereinafter referred to as "ELISA"; *Antibodies,* Chapter 14; *Monoclonal Antibodies*) or the like. Also, the amount of the humanized antibody produced by the transformant can be increased by using a DHFR gene amplification system in accordance with the method disclosed in Japanese Published Unexamined Patent Application No. 257891/90.
The humanized antibody can be purified from a culture supernatant of the transformant using a protein A column (*Antibodies,* Chapter 8; *Monoclonal Antibodies*). In addition, purification methods generally used for the purification of proteins can also be used. For example, the purification can be carried out through the combination of a gel filtration, an ion exchange chromatography and an ultrafiltration. The molecular weight of the H chain, L chain or antibody molecule as a whole of the purified humanized antibody can be measured, e.g., by polyacrylamide gel electrophoresis [hereinafter referred to as "SDS-PAGE"; *Nature,* 227, 680 (1970)], Western blotting (*Antibodies,* Chapter 12; *Monoclonal Antibodies*) or the like.

B. Preparation of Fc fusion protein

(1) Construction of Fc fusion protein expression vector

An Fc fusion protein expression vector is an expression vector for animal cell into which genes encoding the Fc region of a human antibody and a protein to be fused are inserted, which can be constructed by cloning each of genes encoding the Fc region of a human antibody and the protein to be fused into an expression vector for animal cell.

The Fc region of a human antibody includes those containing a part of a hinge region and/or CH1 in addition to regions containing CH2 and CH3 regions. Also, it can be any Fc region so long as at least one amino acid of CH2 or CH3 may be deleted, substituted, added or inserted, and substantially has the binding activity to the Fcγ receptor.

As the genes encoding the Fc region of a human antibody and the protein to be fused, a chromosomal DNA comprising an exon and an intron can be used, and a cDNA can also be used. The method for linking the genes and the Fc region includes PCR using each of the gene sequences as the template (*Molecular Cloning,* Second Edition; *Current Protocols in Molecular Biology,* Supplement 1-34).

As the expression vector for animal cell, any vector can be used, so long as a gene encoding the C region of a human antibody can be inserted thereinto and expressed therein. Examples include pAGE107 [*Cytotechnology,* 3, 133 (1990)], pAGE103 [*J. Biochem.,* 101, 1307 (1987)), pHSG274 [*Gene*, 27, 223 (1984)], pKCR [*Proc. Natl. Acad. Sci. USA*, 78, 1527 (1981), pSG1 β d2-4 [*Cytotechnology*, 4, 173 (1990)] and the like. The promoter and enhancer in the expression vector for animal cell include SV40 early promoter and enhancer [*J. Biochem*., 101, 1307 (1987)], Moloney mouse leukemia virus LTR promoter [*Biochem. Biophys. Res. Commun*., 149, 960 (1987)], immunoglobulin H chain promoter [*Cell*, 41, 479 (1985)] and enhancer [*Cell,* 33, 717 (1983)], and the like.

(2) Preparation of DNA encoding Fc region of human antibody and protein to be fused

A DNA encoding the Fc region of a human antibody and the protein to be fused can be obtained in the following manner.

A cDNA is synthesized from mRNA extracted from a cell or tissue which expresses the protein of interest to be fused with Fc. The synthesized cDNA is cloned into a vector such as a phage or a plasmid to obtain a cDNA library. A recombinant phage or recombinant plasmid comprising cDNA encoding the protein of interest is isolated from the library by using the gene sequence part of the protein of interest as the probe. A full nucleotide sequence of the antibody of interest on the recombinant phage or recombinant plasmid is determined, and a full length amino acid sequence is deduced from the nucleotide sequence.

As the non-human animal, any animal such as mouse, rat, hamster or rabbit can be used so long as a cell or tissue can be removed therefrom.

The method for preparing a total RNA from a cell or tissue includes the guanidine thiocyanate-cesium trifluoroacetate method [*Methods in Enzymology,* 154, 3 (1987)] and the like, and the method for preparing mRNA from total RNA includes an oligo (dT)-immobilized cellulose column method (*Molecular Cloning,* Second Edition) and the like. In addition, a kit for preparing mRNA from a cell or tissue includes Fast Track mRNA Isolation Kit (manufactured by Invitrogen), Quick Prep mRNA Purification Kit (manufactured by Pharmacia) and the like.

The method for synthesizing a cDNA and preparing a cDNA library includes the usual methods (*Molecular Cloning,* Second Edition; *Current Protocols in Molecular Biology,* Supplement 1-34); methods using a commercially available kit such as SuperScript™, Plasmid System for cDNA Synthesis and Plasmid Cloning (manufactured by GIBCO BRL) or ZAP-cDNA Synthesis Kit (manufactured by Stratagene); and the like.

In preparing the cDNA library, the vector into which a cDNA synthesized by using mRNA extracted from a cell or tissue as the template is inserted may be any vector so long as the cDNA can be inserted. Examples include ZAP Express [*Strategies,* 5, 58 (1992)], pBluescript II SK(+) [*Nucleic Acids Research*, 17, 9494 (1989)], λzapII (manufactured by Stratagene), λgt10 and λgt11 [*DNA Cloning, A Practical Approach*, I, 49 (1985)], Lambda BlueMid (manufactured by Clontech), λExCell, pT7T3 18U (manufactured by Pharmacia), pcD2 [*Mol. Cell. Biol*., 3, 280 (1983)], pUC18 [*Gene,* 33, 103 (1985)] and the like.

As *Escherichia coli* into which the cDNA library constructed from a phage or plasmid vector is introduced, any *Escherichia coli* can be used, so long as the cDNA library can be introduced, expressed and maintained. Examples include XL1-Blue MRF' [*Strategies,* 5, 81 (1992)], C600 [*Genetics,* 39, 440 (1954)], Y1088 and Y1090 [*Science,* 222, 778 (1983)], NM522 [*J. Mol. Biol*., 166, 1 (1983)], K802 [*J. Mol. Biol.,* 16, 118 (1966)], JM105 [*Gene,* 38, 275 (1985)] and the like.

As the method for selecting a cDNA clone encoding the protein of interest from the cDNA library, a colony hybridization or a plaque hybridization using an isotope- or fluorescence-labeled probe can be used (*Molecular Cloning,* Second Edition). The cDNA encoding the protein of interest can also be prepared by preparing primers and using a cDNA synthesized from mRNA or a cDNA library as the template according to PCR.
The method for fusing the protein of interest with the Fc region of a human antibody includes PCR. For example, synthesized oligo DNAs (primers) are designed at the 5'-terminal and 3'-terminal of the gene sequence encoding the protein of interest, and PCR is carried out to prepare a PCR product. In the same manner, primers are designed for the gene sequence encoding the Fc region of a human antibody to be fused to prepare a PCR product. At this time, the primers are designed in such a manner that the same restriction enzyme site or the same gene sequence is present between the 3'-terminal of the PCR product of the protein to be fused and the 5'-terminal of the PCR product of the Fc region. When it is necessary to modify the amino acids around the linked site, mutation is introduced by using the primer into which the mutation is introduced. PCR is further carried out by using the two kinds of the obtained PCR fragments to link the genes. Also, they can be linked by carrying out ligation after treatment with the same restriction enzyme.
The nucleotide sequence of the DNA can be determined by digesting the gene sequence linked by the above method with appropriate restriction enzymes, cloning the fragments into a plasmid such as pBluescript SK(-) (manufactured by Stratagene), carrying out analysis by using a generally used nucleotide sequence analyzing method such as the dideoxy method of Sanger *et al*. [*Proc. Natl. Acad Sci. USA*, 74, 5463 (1977)] or an automatic nucleotide sequence analyzer such as A.L.F. DNA Sequencer (manufactured by Pharmacia).
Whether or not the obtained cDNA encodes the full length amino acid sequences of the Fc fusion protein containing a secretory signal sequence can be confirmed by deducing the full length amino acid sequence of the Fc fusion protein from the determined nucleotide sequence and comparing it with the amino acid sequence of interest.

(3) Stable production of Fc fusion protein

A transformant capable of stably producing an Fc fusion protein can be obtained by introducing the Fc fusion protein expression vector described in the item (1) into an appropriate animal cell.
The method for introducing the Fc fusion protein expression vector into an animal cell include electroporation [Japanese Published Unexamined Patent Application No. 257891/90, *Cylotechnology*, 3, 133 (1990)] and the like.
As the animal cell into which the Fc fusion protein expression vector is introduced, any cell can be used, so long as it is an animal cell which can produce the Fc fusion protein.
Examples include mouse myeloma cells such as NS0 cell and SP2/0 cell, Chinese hamster ovary cells such as CHO/dhfr⁻ cell and CHO/DG44 cell, rat myeloma such as YB2/0 cell and IR983F cell, BHK cell derived from a syrian hamster kidney, a human myeloma cell such as Namalwa cell, and the like, and a Chinese hamster ovary cell CHO/DG44 cell, a rat myeloma YB2/0 cell and the host cells used in the method of the present invention described in the item 1 are preferred.
After introduction of the Fc fusion protein expression vector, a transformant capable of stably producing the Fc fusion protein expression vector can be selected using a medium for animal cell culture comprising an agent such as G418 and the like in accordance with the method disclosed in Japanese Published Unexamined Patent Application No. 257891/90. The medium to culture animal cells includes RPMI 1640 medium (manufactured by Nissui Pharmaceutical), GIT medium (manufactured by Nihon Pharmaceutical), EX-CELL 302 medium (manufactured by JRH), IMDM medium (manufactured by GIBCO BRL), Hybridoma-SFM medium (manufactured by GIBCO BRL), media obtained by adding various additives such as fetal bovine serum to these media, and the like. The Fc fusion protein can be produced and accumulated in the culture supernatant by culturing the obtained transformant in a medium. The amount of production and antigen binding activity of the Fc fusion protein in the culture supernatant can be measured by a method such as ELISA. Also, the amount of the Fc fusion protein produced by the transformant can be increased by using a *dhfr* gene amplification system in accordance with the method disclosed in Japanese Published Unexamined Patent Application No. 257891/90.
The Fc fusion protein can be purified from a culture supernatant culturing the transformant by using a protein A column or a protein G column (*Antibodies,* Chapter 8; *Monoclonal Antibodies*). In addition, purification methods generally used for the purification of proteins can also be used. For example, the purification can be carried out through the combination of a gel filtration, an ion exchange chromatography and an ultrafiltration. The molecular weight as a whole of the purified Fc fusion protein molecule can be measured by SDS-PAGE [*Nature*, 227, 680 (1970)], Western blotting (*Antibodies*, Chapter 12, *Monoclonal Antibodies*) or the like.
Thus, methods for producing an antibody composition using an animal cell as the host cell have been described, but, as described above, the antibody composition can also be produced by bacteria, yeast, an insect cell, a plant cell, an animal individual or a plant individual as described above.
When a host cell has a gene capable of expressing an antibody molecule in the host cell, the host cell is

converted to a cell which is resistant to a lectin which recognizes a sugar chain in which 1-position of fucose is bound to 6-position of *N*-acetylglucosamine in the reducing end through α-bond in a complex *N*-glycoside-linked sugar chain according to the method described in the item 1, the cell is cultured, and the antibody composition of interest is purified from the culture to obtain the antibody composition of the present invention..

4. Activity evaluation of antibody composition

As the method for measuring the amount of the purified antibody composition, the activity to bind to an antibody and the effector function of the purified antibody composition, the known method described in *Monoclonal Antibodies, Antibody Engineering* and the like can be used.

As the examples, when the antibody composition is a humanized antibody, the binding activity with an antigen and the binding activity with an antigen-positive cultured clone can be measured by ELISA, the immunofluorescent method [*Cancer Immunol. Immunother.,* 36, 373 (1993)] or the like. The cytotoxic activity against an antigen-positive cultured clone can be evaluated by measuring CDC activity, ADCC activity [*Cancer Immunol. Immunother.,* 36, 373 (1993)] and the like.

Also, safety and therapeutic effect of the antibody composition in human can be evaluated using an appropriate model of animal species relatively close to human, such as *Macaca fascicularis* or the like.

5. Analysis of sugar chains in antibody composition

The sugar chain structure binding to an antibody molecule expressed in a host cell can be analyzed in accordance with the general analysis of the sugar chain structure of a glycoprotein. For example, the sugar chain which is bound to IgG molecule comprises a neutral sugar such as galactose, mannose or fucose, an amino sugar such as *N*-acetylglucosamine and an acidic sugar such as sialic acid, and can be analyzed according to a method such as a sugar chain structure analysis by using sugar composition analysis, two dimensional sugar chain mapping or the like.

(1) Analysis of neutral sugar and amino sugar composition

The sugar chain composition binding to an antibody molecule can be analyzed by carrying out acid hydrolysis of sugar chains with an acid such as trifluoroacetic acid or the like to release a neutral sugar or an amino sugar and measuring the composition ratio.

Examples include a method using a sugar composition analyzer (BioLC) manufactured by Dionex. The BioLC is an apparatus which analyzes a sugar composition by HPAEC-PAD (high performance anion-exchange chromatography-pulsed amperometric detection) [*J. Liq. Chromatogr.,* 6, 1577 (1983)].

The composition ratio can also be analyzed by a fluorescence labeling method using 2-aminopyridine. Specifically, the composition ratio can be calculated in accordance with a known method [*Agric. Biol. Chem.*, 55(1), 283-284 (1991)], by labeling an acid-hydrolyzed sample with a fluorescence with 2-aminopyridylation and then analyzing the composition by HPLC.

(2) Analysis of sugar chain structure

The sugar chain structure binding to an antibody molecule can be analyzed by the two dimensional sugar chain mapping method [*Anal. Biochem.,* 171, 73 (1988), *Biochemical Experimentation Methods 23 - Methods for Studying Glycoprotein Sugar Chains* (Japan Scientific Societies Press) edited by Reiko Takahashi (1989)]. The two dimensional sugar chain mapping method is a method for deducing a sugar chain structure by, e.g., plotting the retention time or elution position of a sugar chain by reverse phase chromatography as the X axis and the retention time or elution position of the sugar chain by normal phase chromatography as the Y axis, respectively, and comparing them with those of known sugar chains.

Specifically, sugar chains are released from an antibody by subjecting the antibody to hydrazinolysis, and the released sugar chain is subjected to fluorescence labeling with 2-aminopyridine (hereinafter referred to as "PA") [*J. Biochem.*, 95, 197 (1984)], and then the sugar chains are separated from an excess PA-treating reagent by gel filtration, and subjected to reverse phase chromatography. Thereafter, each peak of the separated sugar chains are subjected to normal phase chromatography. The sugar chain structure can be deduced by plotting the results on a two dimensional sugar chain map and comparing them with the spots of a sugar chain standard (manufactured by Takara Shuzo) or a literature [*Anal. Biochem.*, 171, 73 (1988)].

The structure deduced by the two dimensional sugar chain mapping method can be confirmed by further carrying out mass spectrometry such as MALDI-TOF-MS of each sugar chain.

6. Immunological determination method for discriminating sugar chain structure of antibody molecule

An antibody composition comprises an antibody molecule in which sugar chains binding to the Fc region of the antibody are different in structure. The antibody composition of the present invention has a higher ratio of a sugar chain in which fucose is not bound to *N*-acetylglucosamine in the reducing end in the sugar chain among the total complex *N*-glycoside-linked sugar chains binding to the Fc region than that of the antibody composition produced by a parent cell line NS0 cell, and has high ADCC activity. The antibody composition can be identified by using the method for analyzing the sugar chain structure binding to an antibody molecule described in the item 4. Also, it can also be identified by an immunological determination method using a lectin.
The sugar chain structure binding to an antibody molecule can be identified by the immunological determination method using a lectin in accordance with the known immunological determination method such as Western staining, IRA (radioimmunoassay), VIA (viroimmunoassay), EIA (enzymoimmunoassay), FIA (fluoroimmunoassay) or MIA (metalloimmunoassay) described in *Monoclonal Antibodies: Principles and Applications,* Wiley-Liss, Inc. (1995); Immunoassay, 3rd Ed., Igakushoin (1987); *Enzyme Antibody Method,* Revised Edition, Gakusai Kikaku (1985); and the like.
A lectin which recognizes the sugar chain structure binding to an antibody molecule comprised in an antibody composition is labeled, and the labeled lectin is allowed to react with a sample antibody composition. Then, the amount of the complex of the labeled lectin with the antibody molecule is measured.
The lectin used for identifying the sugar chain structure binding to an antibody molecule includes WGA (wheat-germ agglutinin derived from *T. vulgaris*), ConA (cocanavalin A derived from *C. ensiformis*), RIC (a toxin derived from *R. communis*), L-PHA (leucoagglutinin derived from *P. vulgaris*), ACL (*Amaranthus caudatus* lectin), BPL (*Bauhinia purpurea* lectin), DSL (*Datura stramonium* lectin), DBA (*Dolichos biflorus* agglutinin), EBL (elderberry balk lectin), ECL (*Erythrina cristagalli* lectin), EEL (*Euonymus eoropaeus* lectin), GNL (*Galanthus nivalis* lectin), GSL (*Griffonia simplicifolia* lectin), HPA (*Helix pomatia* agglutinin), HHL (*Hippeastrum* hybrid lectin), Jacalin, LTL (*Lotus tetragonolobus* lectin), LEL (*Lycopersicon esculentum* lectin), MAL (*Maackia amurensis* lectin), MPL (*Maclura pomifera* lectin), NPL (*Narcissus pseudonarcissus* lectin), PNA (peanut agglutinin), E-PHA (*Phaseolus vulgaris* erythroagglutinin), PTL (*Psophocarpus tetragonolobus* lectin), RCA (*Ricinus communis* agglutinin), STL (*Solanum tuberosum* lectin), SJA (*Sophora japonica* agglutinin), SBA (soybean agglutinin), UEA (*Ulex europaeus* agglutinin), VVL (*Vicia villosa* lectin) and WFA (*Wisteria floribunda* agglutinin).
Whether or not the type of *N*-glycoside-linked sugar chain bound in the Fc region of the antibody is a complex type can be judged by using these lectins.
Furthermore, the sugar chain structure can be analyzed in detail by using a lectin which specifically recognizes a sugar chain structure wherein fucose binds to the *N*-acetylglucosamine in the reducing end in the complex *N*-glycoside-linked sugar chain. Examples include *Lens culinaris* lectin LCA (lentil agglutinin derived from *Lens culinaris*), pea lectin PSA (pea lectin derived from *Pisum sativum*), broad bean lectin VFA (agglutinin derived from *Vicia faba*) and *Aleuria aurantia* lectin AAL (lectin derived from *Aleuria aurantia*).

7. Application of antibody molecule of the present invention

The antibody composition obtained by the present invention has high ADCC activity. An antibody having high ADCC activity is useful for preventing and treating various diseases including cancers, inflammatory diseases, immune diseases such as autoimmune diseases and allergies, cardiovascular diseases and viral or bacterial infections.
In the case of cancers, namely malignant tumors, cancer cells grow. General anti-tumor agents inhibit the growth of cancer cells. In contrast, an antibody having high ADCC activity can treat cancers by injuring cancer cells through its cell killing effect, and therefore, it is more effective as a therapeutic agent than the general anti-tumor agents. At present, in the therapeutic agent for cancers, an anti-tumor effect of an antibody medicament alone is insufficient, so that combination therapy with chemotherapy has been carried out [*Science,* 280, 1197 (1998)]. If higher anti-tumor effect is found by the antibody composition of the present invention alone, the dependency on chemotherapy will be decreased and side effects will be reduced.
In immune diseases such as inflammatory diseases, autoimmune diseases and allergies, *in vivo* reactions of the diseases are induced by the release of a mediator molecule by immunocytes, so that the allergy reaction can be inhibited by eliminating immunocytes using an antibody having high ADCC activity.
The cardiovascular diseases include arteriosclerosis and the like. The arteriosclerosis is treated using balloon catheter at present, but cardiovascular diseases can be prevented and treated by inhibiting growth of arterial cells in restricture after treatment using an antibody having high ADCC activity.
Various diseases including viral and bacterial infections can be prevented and treated by inhibiting proliferation of cells infected with a virus or bacterium using an antibody having high ADCC activity.
An antibody which recognizes a tumor-related antigen, an antibody which recognizes an allergy- or inflammation-related antigen, an antibody which recognizes cardiovascular disease-related antigen and an antibody which recognizes a viral or bacterial infection-related antigen are exemplified below.

The antibody which recognizes a tumor-related antigen includes anti-GD2 antibody [*Anticancer Res.,* 13, 331-336 (1993)], anti-GD3 antibody [*Cancer Immunol. Immunother.,* 36, 260-266 (1993)], anti-GM2 antibody [*Cancer Res.,* 54, 1511-1516 (1994)], anti-HER2 antibody [*Proc. Natl. Acad. Sci. USA*, 89, 4285-4289 (1992)], anti-CD52 antibody [*Nature,* 332, 323-327 (1992)], anti-MAGE antibody [*British J. Cancer,* 83, 493-497 (2000)], anti-HM1.24 antibody [*Molecular Immunol.,* 36, 387-395 (1999)], anti-parathyroid hormone-related protein (PTHrP) antibody [*Cancer*, 88, 2909-2911 (2000)], anti-FGF8 antibody [*Proc. Natl. Acad. Sci. USA,* 86, 9911-9915 (1989)], anti-basic fibroblast growth factor antibody and anti-FGF8 receptor antibody [*J. Biol. Chem.*, 265, 16455-16463 (1990)], anti-insulin-like growth factor antibody [*J. Neurosci. Res.,* 40, 647-659 (1995)], anti-insulin-like growth factor receptor antibody [*J. Neurosci. Res.,* 40, 647-659 (1995)], anti-PMSA antibody [*J. Urology,* 160, 2396-2401 (1998)], anti-vascular endothelial cell growth factor antibody [*Cancer Res.,* 57, 4593-4599 (1997)], anti-vascular endothelial cell growth factor receptor antibody [*Oncogene*, 19, 2138-2146 (2000)] and the like.

The antibody which recognizes an allergy- or inflammation-related antigen includes anti-interleukin 6 antibody [*Immunol. Rev.,* 127, 5-24 (1992)], anti-interleukin 6 receptor antibody [*Molecular Immunol.*, 31, 371-381 (1994)], anti-interleukin 5 antibody [*Immunol. Rev.,* 127, 5-24 (1992)], anti-interleukin 5 receptor antibody and anti-interleukin 4 antibody [*Cytokine*, 3, 562-567 (1991)], anti-interleukin 4 antibody [*J. Immunol. Meth.*, 217, 41-50 (1991)], anti-tumor necrosis factor antibody [*Hybridoma*, 13, 183-190 (1994)], anti-tumor necrosis factor receptor antibody [*Molecular Pharmacol.*, 58, 237-245 (2000)], anti-CCR4 antibody [*Nature*, 400, 776-780 (1999)], anti-chemokine antibody [*J. Immuno. Meth.*, 174, 249-257 (1994)], anti-chemokine receptor antibody [*J. Exp. Med.*, 186, 1373-1381 (1997)] and the like. The antibody which recognizes a cardiovascular disease-related antigen includes anti-GpIIb/IIIa antibody [*J. Immunol.*, 152, 2968-2976 (1994)], anti-platelet-derived growth factor antibody [*Science,* 253, 1129-1132 (1991)], anti-platelet-derived growth factor receptor antibody [*J. Biol. Chem.*, 272, 17400-17404 (1997)] and anti-blood coagulation factor antibody [*Circulation*, 101, 1158-1164 (2000)] and the like.

The antibody which recognizes an antigen relating to autoimmune diseases includes an anti-auto-DNA antibody [*Immunol. Letters,* 72, 61-68 (2000)] and the like.

The antibody which recognizes a viral or bacterial infection-related antigen includes anti-gp120 antibody [*Structure*, 8, 385-395 (2000)], anti-CD4 antibody [*J. Rheumatology,* 25, 2065-2076 (1998)], anti-CCR4 antibody and anti-Vero toxin antibody [*J. Clin. Microbiol.*, 37, 396-399 (1999)] and the like.

These antibodies can be obtained from public organizations such as ATCC (The American Type Culture Collection), RIKEN Gene Bank at The Institute of Physical and Chemical Research and National Institute of Bioscience and Human Technology, Agency of Industrial Science and Technology, or private reagent sales companies such as Dainippon Pharmaceutical, R & D SYSTEMS, PharMingen, Cosmo Bio and Funakoshi.

The medicament comprising the antibody composition obtained in the present invention can be administered as a therapeutic agent alone, but generally, it is preferred to provide it as a pharmaceutical formulation produced by an appropriate method well known in the technical field of manufacturing pharmacy, by mixing it with at least one pharmaceutically acceptable carrier.

It is preferred to select a route of administration which is most effective in treatment. Examples include oral administration and parenteral administration, such as buccal, tracheal, rectal, subcutaneous, intramuscular or intravenous. In the case of an antibody preparation, intravenous administration is preferred.

The dosage form includes sprays, capsules, tablets, granules, syrups, emulsions, suppositories, injections, ointments, tapes and the like.

The pharmaceutical preparation suitable for oral administration includes emulsions, syrups, capsules, tablets, powders, granules and the like.

Liquid preparations, such as emulsions and syrups, can be produced using, as additives, water; sugars such as sucrose, sorbitol and fructose; glycols, such as polyethylene glycol and propylene glycol; oils, such as sesame oil, olive oil and soybean oil; antiseptics, such as p-hydroxybenzoic acid esters; flavors, such as strawberry flavor and peppermint; and the like.

Capsules, tablets, powders, granules and the like can be produced using, as additive, fillers, such as lactose, glucose, sucrose and mannitol; disintegrating agents, such as starch and sodium alginate; lubricants, such as magnesium stearate and talc; binders, such as polyvinyl alcohol, hydroxypropylcellulose and gelatin; surfactants, such as fatty acid ester; plasticizers, such as glycerine; and the like.

The pharmaceutical preparation suitable for parenteral administration includes injections, suppositories, sprays and the like.

Injections can be prepared using a carrier, such as a salt solution, a glucose solution, a mixture of both thereof or the like. Also, powdered injections can be prepared by freeze-drying the antibody composition in the usual way and adding sodium chloride thereto.

Suppositories can be prepared using a carrier such as cacao butter, hydrogenated fat, carboxylic acid or the

like.

Sprays can be prepared using the antibody composition as such or using the antibody composition together with a carrier which does not stimulate the buccal or airway mucous membrane of the patient and can facilitate absorption of the antibody composition by dispersing it as fine particles.

The carrier includes lactose, glycerol and the like. Depending on the properties of the antibody composition and the carrier, it is possible to produce pharmaceutical preparations such as aerosols, dry powders and the like. In addition, the components exemplified as additives for oral preparations can also be added to the parenteral preparations.

Although the clinical dose or the frequency of administration varies depending on the objective therapeutic effect, administration method, treating period, age, body weight and the like, it is usually 10 μg/kg to 20 mg/kg per day and per adult.

Also, as the method for examining antitumor effect of the antibody composition against various tumor cells, *in vitro* tests include CDC activity measuring method, ADCC activity measuring method and the like, and *in vivo* tests include antitumor experiments using a tumor system in an experimental animal such as a mouse, and the like.

CDC activity and ADCC activity measurements and antitumor experiments can be carried out in accordance with the methods described in *Cancer Immunology Immunotherapy*, 36, 373 (1993); *Cancer Research,* 54, 1511 (1994) and the like.

The present invention will be described below in detail based on Examples; however, Examples are only simple illustrations, and the scope of the present invention is not limited thereto.

BRIEF DESCRIPTION OF THE DRAWINGS

Fig. 1 shows results of antigen-biding activities of anti-CCR4 chimeric antibodies produced by NS0 cell line which is a parent cell line and lectin-resistant NS0 clone. The ordinate and the abscissa show the absorbance at a wavelength of 490 nm and the concentration of the anti-CCR4 chimeric antibody in the reaction solution, respectively. "Δ" and "●" show antigen binding activities of the NS0/CCR4 antibody and the NS0/LCA-CCR4 antibody, respectively.

Fig. 2 shows ADCC activities of anti-CCR4 chimeric antibodies produced by NS0 cell line which is a parent cell line and lectin-resistant NS0 clone. The ordinate and the abscissa show the cytotoxic activity and the antibody concentration, respectively. "Δ" and "●" show antigen binding activities of the NS0/CCR4 antibody and the NS0/LCA-CCR4 antibody, respectively.

Fig. 3 shows an elution pattern obtained by analyzing PA-treated sugar chains from each of the purified anti-CCR4 chimeric antibodies by reverse phase HPLC. The ordinate and the abscissa show the relative fluorescence intensity and the elution time, respectively.

Fig. 4 shows ADCC activities of anti-CCR4 human chimeric antibodies produced by lectin-resistant clones. The ordinate and the abscissa show the cytotoxic activity and the antibody concentration, respectively. "□", "■", "♦" and "▲" show the activities of antibodies produced by the clones 5-03, CHO/CCR4-LCA, CHO/CCR4-AAL and CHO/CCR4-PHA, respectively.

Fig. 5 shows ADCC activities of anti-CCR4 human chimeric antibodies produced by lectin-resistant clones. The ordinate and the abscissa show the cytotoxic activity and the antibody concentration, respectively. "□", "Δ" and "●" show activities of antibodies produced by the clones YB2/0 (KM2760#58-35-16), 5-03 and CHO/CCR4-LCA, respectively.

Fig. 6 shows elution patterns of PA-treated sugar chains prepared from purified anti-CCR4 human chimeric antibodies, obtained by analyzing them by reverse phase HPLC. The ordinate and the abscissa show the relative fluorescence intensity and the elution time, respectively. Fig. 6A, Fig. 6B, Fig. 6C and Fig. 6D show results of analyses of antibodies produced by the clones 5-03, CHO/CCR4-LCA, CHO/CCR4-AAL and CHO/CCR4-PHA, respectively.

Fig. 7 shows the 1st step of construction of an expression vector of CHO cell-derived GMD (6 steps in total).

Fig. 8 shows the 2nd step of construction of the expression vector of CHO cell-derived GMD (6 steps in total).

Fig. 9 shows the 3rd step of construction of the expression vector of CHO cell-derived GMD (6 steps in total).

Fig. 10 shows the 4th step of construction of the expression vector of CHO cell-derived GMD (6 steps in total).

Fig. 11 shows the 5th step of construction of the expression vector of CHO cell-derived GMD (6 steps in total).

Fig. 12 shows the 6th step of construction of the expression vector of CHO cell-derived GMD (6 steps in total).

Fig. 13 shows resistance of the GMD-expressed clone CHO/CCR4-LCA for LCA lectin. The measurement was carried out twice by defining the survival ratio of a group of cells cultured without adding LCA lectin as 100%. In the drawing, "249" shows the survival ratio of the clone CHO/CCR4-LCA introduced with an expression vector

pAGE249 for LCA lectin. GMD shows resistance of the clone CHO/CCR4-LCA introduced with a GMD expression vector pAGE249GMD for LCA lectin.

Fig. 14 shows ADCC activities of an anti-CCR4 chimeric antibody produced by cells of the GMD-expressed clone CHO/CCR4-LCA. The ordinate and the abscissa show the cytotoxic activity and the antibody concentration, respectively.

Fig. 15 shows an elution pattern of PA-treated sugar chains prepared from an anti-CCR4 human chimeric antibody purified from the GMD gene-expressed clone CHO/CCR4-LCA, obtained by analyzing them by reverse phase HPLC. The ordinate and the abscissa show the relative fluorescence intensity and the elution time, respectively.

Fig. 16 shows an elution pattern obtained by analyzing PA-treated sugar chains from each of the purified anti-GD3 chimeric antibodies by reverse phase HPLC. The ordinate and the abscissa show the relative fluorescence intensity and the elution time, respectively.

Fig. 17 shows antigen-binding activities of anti-GD3 chimeric antibodies. The ordinate and the abscissa show the absorbance at a wavelength of 490 nm and the concentration of the anti-CCR4 chimeric antibody in the reaction solution, respectively.

Fig. 18 shows ADCC activities of anti-GD3 chimeric antibodies. The ordinate and the abscissa show the degree of cell injury of target cells calculated by the equation shown in the item 2 of Example 2 and the concentration of anti-GD3 chimeric antibodies in the reaction solution, respectively.

Fig. 19 shows a construction step of plasmid pBS-2B8L.

Fig. 20 shows a construction step of plasmid pBS-2B8Hm.

Fig. 21 shows a construction step of plasmid pKANTEX2B8P.

Fig. 22 shows activities of purified anti-CD20 chimeric antibody R92-3-1 and Rituxan™ to bind to a human CD20 expressing cell Raji cell using the immunofluorescence technique by changing the concentration of the antibodies. The ordinate and the abscissa show the relative fluorescence intensity at each concentration and the antibody concentration, respectively. "■" and "○" show the activities of Rituxan™ and the R92-3-1 antibody, respectively.

Fig. 23 shows activities of purified anti-CD20 chimeric antibody R92-3-1 and Rituxan™ to bind to a human CD20-negative cell CCRF-CEM cell by using the immunofluorescence technique.

Fig. 24 shows ADCC activities of a purified anti-CD20 chimeric antibody R92-3-1 and Rituxan™ to human CD20-expressing cells. Fig. 24A, Fig. 24B and Fig. 24C show the results using Raji cell, Ramos cell and WIL2-S cell, respectively. as target cells. The ordinate and the abscissa show the cytotoxic activity and the antibody concentration, respectively. "■" and "○" show the activities of Rituxan™ and the R92-3-1 antibody, respectively.

Fig. 25 shows an elution pattern of PA-treated sugar chains prepared from a purified anti-CD20 chimeric antibody R92-3-1 and Rituxan™, obtained by analyzing them by reverse phase HPLC. The ordinate and the abscissa show the relative fluorescence intensity and the elution time, respectively.

Fig. 26 shows a construction step of plasmid U6_pre_sense.

Fig. 27 shows a construction step of plasmid pBS_BglII.

Fig. 28 shows a construction step of plasmid U6_pre_antisense.

Fig. 29 shows a construction step of plasmid U6_sense_B.

Fig. 30 shows a construction step of plasmid U6_sense_R.

Fig. 31 shows a construction step of plasmid U6_antisense_B.

Fig. 32 shows a construction step of plasmid U6_antisense_R.

Fig. 33 shows a construction step of plasmid U6_FUT8_B.

Fig. 34 shows a construction step of plasmid U6_FUT8_R.

Fig. 35 shows a construction step of plasmid U6_FUT8_B_puro.

Fig. 36 shows a construction step of plasmid U6_FUT8_R_puro.

Fig. 37 shows decrease of the amount of PUT mRNA in the FUT8 siRNA-introduced clone. The amount of FUT mRNA expressed in each clone is shown as a ratio per the amount of β-actin mRNA.

Fig. 38 shows elution patterns of PA-treated sugar chains prepared from six kinds of anti-CCR4 chimeric antibodies, obtained by analyzing them by reverse phase HPLC. The ordinates and the abscissas show the relative fluorescence intensity and the elution time, respectively.

Fig. 39 shows CCR4-binding activities of six kinds of anti-CCR4 chimeric antibodies having a different ratio of α1,6-fucose-free sugar chains measured by changing the antibody concentration. The ordinate and the abscissa show the binding activity with CCR4 and the antibody concentration, respectively. "■", "□", "▲", "△", "●" and "o" show the activities of anti-CCR4 chimeric antibody (46%), anti-CCR4 chimeric antibody (39%), anti-CCR4 chimeric antibody (27%), anti-CCR4 chimeric antibody (18%), anti-CCR4 chimeric antibody (9%) and anti-CCR4 chimeric antibody (8%), respectively.

Fig. 40 shows ADCC activities of anti-CCR4 chimeric antibodies having a different ratio of α1,6-fucose-free sugar chains against CCR4/EL-4 cell, using an effector cell of the donor A. The ordinate and the abscissa show the cytotoxic activity and the antibody concentration, respectively. "■", "□", "▲", "△", "●" and "○" show the activities of

anti-CCR4 chimeric antibody (46%), anti-CCR4 chimeric antibody (39%), anti-CCR4 chimeric antibody (27%), anti-CCR4 chimeric antibody (18%), anti-CCR4 chimeric antibody (9%) and anti-CCR4 chimeric antibody (8%), respectively.

Fig. 41 shows ADCC activities of anti-CCR4 chimeric antibodies having a different ratio of $\alpha$1,6-fucose-free sugar chains against CCR4/EL-4 cell, using an effector cell of the donor B. The ordinate and the abscissa show the cytotoxic activity and the antibody concentration, respectively. "■", "□", "▲", "△", "●" and "○" show the activities of anti-CCR4 chimeric antibody (46%), anti-CCR4 chimeric antibody (39%), anti-CCR4 chimeric antibody (27%), anti-CCR4 chimeric antibody (18%), anti-CCR4 chimeric antibody (9%) and anti-CCR4 chimeric antibody (8%), respectively.

Fig. 42 show a construction step of a plasmid CHO-GMD in which the 5'-terminal of clone 34-2 is introduced into the 5'-terminal of a CHO cell-derived GMD cDNA clone 22-8.

## BEST MODE FOR CARRYING OUT THE INVENTION

### Example 1

Preparation of lectin-resistant NS0 cell and production of antibody composition using the cell:

(1) Preparation of lectin-resistant clone NS0

A mouse myeloma NS0 cell (RCB 0213) was cultured in a suspension culture flask 75 $cm^2$ (manufactured by Iwaki Glass) using RPMI 1640 medium (manufactured by Invitrogen) to which fetal bovine serum (manufactured by Invitrogen) (hereinafter referred to as "RPMI-FBS(10) medium") had been added at 10% volume ratio, and allowed to proliferate until just before confluent. The cells in this culture were suspended in RPMI-FBS(10) medium to give a density of $1\times10^5$ cell/ml, and then 0.1 µg/ml of *N*-methyl-N'-nitro-*N*-nitrosoguanidin which was an alkylating agent (hereinafter referred to as "MNNG", manufactured by Sigma) was added or not added. After allowing to stand at 37°C for 3 days in a 5% $CO_2$ incubator (manufactured by TABAI), the cells were inoculated into a suspension culture 96 well plate (manufactured by Iwaki Glass) at a density of 1,000 cells/well. A *Lens culinaris* agglutinin (hereinafter referred to as "LCA", manufactured by Vector) was added to each well at a final concentration in medium of 1 mg/ml. After culturing at 37°C for 2 weeks in a 5% $CO_2$ incubator, the growing lectin-resistant colonies were obtained as lectin-resistant NS0 clones. Hereinafter, the clone was named clone NS0-LCA.

(2) Preparation of anti-CCR4 chimeric antibody expression cell

A cell which stably produces an anti-CCR4 chimeric antibody was prepared using the anti-CCR4 chimeric antibody tandem expression vector pKANTEX2160 described in WO 01/64754 as follows.

Into $4\times10^6$ cells of the parent clone NS0 or the clone NSO-LCA, 10 µg of the anti-CCR4 chimeric antibody expression vector pKANTEX2160 was introduced by electroporation [*Cytotechnology*, 3, 133 (1990)], and the cells were suspended in 10 ml of the RPMI-FBS(10) medium and dispensed at 200 µl/well into a 96 well culture plate (manufactured by Sumitomo Bakelite). After culturing at 37°C for 24 hours in a 5% $CO_2$ incubator, G418 was added thereto to give a concentration of 0.5 mg/ml, followed by culturing for 1 to 2 weeks. Culture supernatants were recovered from wells where colonies of transformants showing G418 resistance were formed and their growth was confirmed, and an accumulated amount of the anti-CCR4 chimeric antibody in the culture supernatant was measured by the ELISA described in the item (3) of Example 1.

Transformants in wells where production of an anti-CCR4 chimeric antibody was detected in the culture supernatant the cells were suspended to give a density of 1 to $2\times10^5$ cells/ml in the RPMI-FBS(10) medium containing 0.5 mg/ml G418 and 50 nM MTX in order to increase the antibody production using the DHFR gene amplification system, and the suspension was dispensed at 2 ml into a 24 well plate (manufactured by Greiner). After culturing at 37°C for 1 to 2 weeks in a 5% $CO_2$ incubator, transformants showing 50 nM MTX resistance were induced. Expressed level of the anti-CCR4 chimeric antibody in the culture supernatants of wells where growth of transformants were detected was measured by the ELISA described in the item (3) of Example 1. Regarding transformants in wells where production of the anti-CCR4 chimeric antibody was found in the culture supernatant, the MTX concentration was increased to 200 nM and then to 500 nM by a method similar to the above to finally obtain transformants which can grow in the RPMI-FBS(10) medium containing 0.5 mg/ml G418 and 500 nM MTX and also can highly produce the anti-CCR4 chimeric antibody. One clone of transformants was selected from each of the clone NS0 and clone NS0-LCA. Regarding the obtained transformants, the clone NS0-derived transformant was named clone NS0/CCR4, and the NS0-LCA-derived transformant was named clone NS0/LCA-CCR4. Also, the clone NS0/LCA-CCR4 has been deposited on March 14, 2002, as FERM BP-7964 in International Patent Organism Depositary, National Institute of Advanced Industrial

Science and Technology (Tsukuba Central 6, 1, Higashi 1-Chome Tsukuba-shi, Ibaraki-ken 305-8566 Japan).

(3) Determination of antibody in culture supernatant by IgG-ELISA

The concentration of the anti-CCR4 chimeric antibody in the culture supernatant was measured as follows.

In 1,200 ml of PBS, 1 mg of anti-human immunoglobulin G (hereinafter referred to as "IgG") antibody (manufactured by American Qualex) was dissolved. Into each well of a 96 well plate for ELISA (manufactured by Greiner), 50 μl of the solution was dispensed and allowed to stand at 4°C overnight, and the solution in each well was discarded, and a 1% bovine serum albumin (hereinafter referred to as "BSA"; manufactured by SIGMA)-containing PBS (hereinafter referred to as "1% BSA-PBS") was added thereto at 100 μl/well and allowed to react at room temperature for 1 hour to block the remaining active residues. After 1% BSA-PBS was discarded, culture supernatant of a transformant or variously diluted solutions of a purified human chimeric antibody were added thereto at 50 μl/well and allowed to react at room temperature for 1 hour. After the reaction, each well was washed with 0.05% Tween 20-containing PBS (hereinafter referred to as "Tween-PBS"), and then, as a secondary antibody solution, a peroxidase-labeled goat anti-human IgG (H & L) antibody solution (manufactured by American Qualex) diluted 3,000-folds with 1% BSA-PBS was added thereto at 50 μl/well and allowed to react at room temperature for 1 hour. After the reaction and subsequent washing with Tween-PBS, an ABTS substrate solution [a solution prepared by dissolving 0.55 g of 2,2'-azino-bis (3-ethylbenzothiazoline-6-sulfonic acid)ammonium in 1 liter of 0.1 M citrate buffer (pH 4.2), and adding 1 μl/ml hydrogen peroxide just before use] was added at 50 μl/well for coloration and the absorbance at 415 nm (hereinafter referred to as "OD415") was measured using Microplate Reader (manufactured by BIO-RAD).

Example 2

Purification and activity evaluation of anti-CCR4 chimeric antibody:

(1) Culturing of antibody-expressing clone and purification of antibody

The transformant cell expressing an anti-CCR4 chimeric antibody obtained in the item (2) of Example 1 was suspended in Hybridoma-SFM (manufactured by Invitrogen) medium comprising MTX and bovine serum albumin at final concentrations 500 nM and 0.2%, respectively, to give a density of $2\times10^5$ cells/ml and inoculated into a flask for suspension culture (manufactured by Iwaki Glass). After culturing at 37°C for 7 days in a 5% $CO_2$ incubator, the anti-CCR4 chimeric antibody was purified from the culture supernatant recovered using Prosep-A (manufactured by Millipore) column and gel filtration. Regarding the obtained antibody, the antibodies produced by the clone NS0/LCA-CCR4 and the clone NS0/CCR4 was named NS0/LCA-CCR4 antibody and NS0/CCR4 antibody.

(2) Evaluation of activity of anti-CCR4 chimeric antibody

(2-1) Measurement of binding activity to CCR4 partial peptide (ELISA)

(2-1-1) Preparation of an antigen peptide

Compound 1 (SEQ ID NO:1) was selected as a human CCR4 (hCCR4) extracellular domain peptide which reacts with an anti-CCR4 chimeric antibody and synthesized as follows.

<u>Abbreviations</u>

Abbreviations of the amino acids and their protecting groups used in the present invention were used according to the recommendation by IUPAC-IUB Joint Commission on Biochemical Nomenclature [*European Journal of Biochemistry*, <u>138</u>, 9 (1984)].

Unless otherwise indicated, the following abbreviations represent the following amino acids.

Ala: L-Alanine
Asn: L-Asparagine
Asp: L-Aspartic acid
Asx: L-Aspartic acid or L-asparagine
Cys: L-Cysteine
Gln: L-Glutamine
Glu: L-Glutamic acid

| | |
|---|---|
| Glx: | L-Glutamic acid or L-glutamine |
| Gly: | Glycine |
| Ile: | L-Isoleucine |
| Leu: | L-Leucine |
| Lys: | L-Lysine |
| Met: | L-Methionine |
| Phe: | L-Phenylalanine |
| Pro: | L-Proline |
| Ser: | L-Serine |
| Thr: | L-Threonine |
| Tyr: | L-Tyrosine |
| Val: | L-Valine |

The following abbreviations represent protecting groups of corresponding amino acids and side chain-protecting amino acids.

| | |
|---|---|
| Fmoc: | 9-Fluorenylmethyloxycarbonyl |
| tBu: | t-Butyl |
| Trt: | Trityl |
| Boc: | t-Butyloxycarbonyl |
| Fmoc-Thr(tBu)-OH: | Nα-9-Fluorenylmethyloxycarbonyl-O-t-butyl-L-threonine |
| Fmoc-Ser(tBu)-OH: | Nα-9-Fluorenylmethyloxycarbonyl-O-t-butyl-L-serine |
| Fmoc-Tyr(tBu)-OH: | Nα-9-Fluorenylmethyloxycarbonyl-O-t-butyl-L-tyrosine |
| Fmoc-Lys(Boc)-OH: | Nα-9-Fluorenylmethyloxycarbonyl-Nε-t-butyloxycarbonyl-L-lysine |
| Fmoc-Asn(Trt)-OH: | Nα-9-Fluorenylmethyloxycarbonyl-Nγ-trityl-L-asparagine |
| Fmoc-Gln(Trt)-OH: | Nα-9-Fluorenylmethyloxycarbonyl-Nδ-trityl-L-glutamine |
| Fmoc-Asp(OtBu)-OH: | Nα-9-Fluorenylmethyloxycarbonyl-L-aspartic acid β-t-butyl ester |
| Fmoc-Glu(OtBu)-OH: | Nα-9-Fluorenylmethyloxycarbonyl-L-glutamic acid γ-t-butyl ester |
| Fmoc-Cys(Trt)-OH: | Nα-9-Fluorenylmethyloxycarbonyl-S-trityl-L-cysteine |

The following abbreviations represent corresponding reaction solvents and reaction reagents.

| | |
|---|---|
| PyBOP: | Benzotriazol-1-yloxytripyrrolidinophosphonium hexafluorophosphate |
| HOBt: | *N*-Hydroxybenzotriazole |
| DMF: | *N*,*N*-Dimethylformamide |
| DCM: | Dichloromethane |
| TFA: | Trifluoroacetic acid |
| NMM: | *N*-Methylmorpholine |
| DTT: | Dithiothreitol |

(i) Synthesis of Compound 1 (SEQ ID NO:1) (H-Asp-Glu-Ser-Ile-Tyr-Ser-Asn-Tyr-Tyr-Leu-Tyr-Glu-Ser-Ile-Pro-Lys-Pro-Cys-OH)

Into a reaction vessel of an automatic synthesizer (manufactured by Shimadzu), 30 mg of a carrier resin (chlorotrityl resin, manufactured by AnaSpec) to which 16.8 μmol of H-Cys(Trt) had been bound was placed, 1 ml of DCM/DMF (1:1) was added thereto, followed by stirring for 10 minutes, the solution was drained away, 1 ml of DMF was further added thereto, followed by stirring for 1 minute, the solution was drained away, and then the following procedure was carried out in accordance with the synthesis program provided by Shimadzu.

(a) Fmoc-Pro-OH (168 μmol), PyBOP (168 μmol), HoBt·1$H_2O$ (168 μmol) and NMM (252 μmol) were stirred in DMF (588.2 μl) for 5 minutes, the resulting solution was added to the resin, followed by stirring for 60 minutes, and then the solution was drained away.
(b) The carrier resin was washed for 1 minute with 707 μl of DMF, and this step was repeated 5 times. In this way, Fmoc-Pro-Cys(Trt) was synthesized on the carrier.
Next, the following Fmoc group-deprotection steps were carried out.
(c) 707 μl of 30% piperidine-DMF solution was added, followed by stirring for 4 minutes, and then the solution was drained away, and this procedure was repeated again.
(d) The carrier resin was washed for 1 minute with 707 μl of DMF and then the solution was drained away, and

this step was repeated 5 times.

In this way, the carrier resin to which the Fmoc group-eliminated H-Pro-Cys(Trt) had been bound was obtained.

Next, a condensation reaction was carried out in the step (a) using Fmoc-Lys(Boc)-OH, and then H-Lys(Boc) -Pro-Cys(Trt) was synthesized on the carrier via the washing step of (b) and deprotection steps of (c) and (d). Next, the steps (a) to (d) were repeated by using Fmoc-Pro-OH, Fmoc-Ile-OH, Fmoc-Ser(tBu)-OH, Fmoc-Glu(OtBu)-OH, Fmoc-Tyr(tBu)-OH, Fmoc-Leu-OH, Fmoc-Tyr(tBu)-OH and Fmoc-Tyr(tBu)-OH in this order in the step (a). Next, the condensation reaction of step (a) was carried out by using Fmoc-Asn(Trt)-OH, the washing step of (b) was carried out, the condensation reaction of step (a) using Fmoc-Asn(Trt)-OH and the washing step of (b) were repeated and then the deprotection steps of (c) and (d) were carried out. Subsequently, the steps (a) to (d) were repeated by using Fmoc-Ser(tBu)-OH, Fmoc-Tyr(tBu)-OH, Fmoc-Ile-OH, Fmoc-Ser(tBu)-OH, Fmoc-Glu(OtBu)-OH and Fmoc-Asp(OtBu)-OH in this order, which was washed with methanol and then with butylether, followed by drying under reduced pressure for 12 hours, and a carrier resin to which a side-chain protecting group was bound wad obtained. A mixed solution (1 ml) of TFA (90%), thioanisole (5%) and 1,2-ethanedithiol (5%) was added thereto, and allowed to stand at room temperature for 2 hours to thereby remove the side chain protecting groups and simultaneously cutting out the peptide from the resin. After removing the resin by filtration, about 10 ml of ether was added to the resulting solution, and the formed precipitate was recovered by centrifugation and decantation and dried under a reduced pressure to obtain 63.7 mg of crude peptide.

The crude product was dissolved in 2 ml of DMF in the presence of 60 mg of DTT and then purified by HPLC using a reverse phase column (CAPCELL PAK C18, 30 mm I.D. × 25 mm, manufactured by Shiseido). Elution was carried out according to a linear density gradient method in which 90% acetonitrile aqueous solution containing 0.1% TFA was added to 0.1% TFA aqueous solution and detecting at 220 nm was carried out to obtain a fraction containing Compound 1. After freeze-drying of this fraction, 2.5 mg of Compound 1 was obtained.
Mass spectrometry (FAB MS): m/z = 3227.5 ($M+H^+$)
Amino acid analysis: Asx 1.2(1), Glx 2.7(2), Ser 3.1(3), Pro 2.2(2), Tyr 3.8(4), Leu 1.2(1), Lys 1.2 (1), Ile 2.0(2), Cys 1.2(1)

(2-1-2) Measurement of binding activities of antibodies to CCR4 partial peptide (ELISA)

Compound 1 (SEQ ID NO:1) was selected as a human CCR4 extracellular region peptide capable of reacting with the anti-CCR4 chimeric antibody. In order to use it in the activity measurement by ELISA, a conjugate with BSA (bovine serum albumin) (manufactured by Nacalai Tesque) was prepared by the following method and used as the antigen. That is, 100 ml of a DMSO solution comprising 25 mg/ml SMCC [4-(*N*-maleimidomethyl)-cyclohexane-1-carboxylic acid *N*-hydroxysuccinimide ester] (manufactured by Sigma)-DMSO solution was added dropwise to 900 ml of a 10 mg BSA-containing PBS solution under stirring using a vortex, followed by gently stirring for 30 minutes. To a gel filtration column such as NAP-10 column or the like equilibrated with 25 ml of PBS, 1 ml of the reaction solution was applied, and then eluted with 1.5 ml of PBS and the resulting eluate was used as a BSA-SMCC solution (BSA concentration was calculated based on $A_{280}$ measurement). Next, 250 ml of PBS was added to 0.5 mg of Compound 1 and then completely dissolved by adding 250 ml of DMF, and the BSA-SMCC solution was added thereto under vortex, followed by gently stirring for 3 hours.

The reaction solution was dialyzed against PBS at 4°C overnight, sodium azide was added thereto to give a final concentration of 0.05%, and the mixture was filtered through a 0.22 mm filter to be used as a BSA-compound 1 solution.

The prepared conjugate was dispensed at 0.05 μg/ml and 50 μl/well into a 96 well EIA plate (manufactured by Greiner) and incubated for immobilization at 4°C overnight. After washing each well with PBS, 1% BSA-PBS was added thereto in 100 μl/well and allowed to react at room temperature for one hour to block the remaining active residues. After washing each well with PBS containing 0.05% Tween 20 (hereinafter referred to as "Tween-PBS"), a culture supernatant of a transformant was added at 50 μl/well and allowed to react at room temperature for 1 hour. After the reaction, each well was washed with Tween-PBS, and then a peroxidase-labeled goat anti-human IgG(γ) antibody solution (manufactured by American Qualex) diluted 6000-fold with 1% BSA-PBS as the secondary antibody was added at 50 μl/well and allowed to react at room temperature for 1 hour. After the reaction and subsequent washing with Tween-PBS, the ABTS substrate solution was added at 50 μl/well for color development, and 20 minutes thereafter, the reaction was stopped by adding a 5% SDS solution at 50 μl/well. Thereafter, the absorbance at OD415 was measured.

Binding activities of the two purified anti-CCR4 chimeric antibodies obtained in the item (1) of Example 2 were measured by the above ELISA. Fig. 1 shows results of binding activities by changing concentrations of the added anti-CCR4 chimeric antibodies. As shown in Fig. 1, the two anti-CCR4 chimeric antibodies show similar binding activity to the CCR4 peptide.

(2-2) Measurement of *in vitro* antibody-dependent cell-mediated cytotoxic activity (ADCC activity) of anti-CCR4 chimeric antibody

The ADCC activity of the anti-CCR4 chimeric antibody was measured by using a highly human CCR4 expressing clone CCR4/EL-4 (WO 01/64754) as a target cell as follows.

(2-2-1) Preparation of target cell solution

CCR4-EL4 clone was cultured in the RPMI1640-FBS(10) medium containing 500 μg/ml G418 sulfate (manufactured by Nacalai Tesque) to prepare $1\times10^6$ cells, and the cells were radioisotope-labeled by reacting them with 3.7 MBq equivalents of a radioactive substance $Na_2{}^{51}CrO_4$ at 37°C for 90 minutes. After the reaction, the cells were washed three times through their suspension in the RPMI1640-FBS(10) medium and centrifugation, re-suspended in the medium and then incubated at 4°C for 30 minutes on ice for spontaneous dissolution of the radioactive substance. After centrifugation, the precipitate was adjusted to $2\times10^5$ cells/ml by adding 5 ml of the RPMI1640-FBS(10) medium to obtain the target cell solution.

(2-2-2) Preparation of human effector cell solution

From a healthy donor, 50 ml of venous blood was collected, and gently mixed with 0.5 ml of heparin sodium (manufactured by Takeda Pharmaceutical). The mixture was centrifuged to isolate a mononuclear cell layer using Lymphoprep (manufactured by Nycomed Pharma AS) in accordance with the manufacture's instructions. After washing with the RPMI1640-FBS(10) medium by centrifugation three times, the resulting precipitate was re-suspended in a medium at a density of $2\times10^6$ cells/ml to obtain the effector cell solution.

(2-2-3) Measurement of ADCC activity

Into each well of a 96 well U-shaped bottom plate (manufactured by Falcon), 50 μl of the target cell solution prepared in the item (2-2-1) ($1\times10^4$ cells/well) was dispensed. Next, 100 μl of the effector cell solution prepared in the item (2-2-2) was added thereto ($2\times10^5$ cells/well, the ratio of effector cells to target cells becomes 25:1). Subsequently, each of the anti-CCR4 chimeric antibodies obtained in the item (1) of Example 2 was added thereto to give a final concentration 0.025 to 2.5 μg/ml, followed by reaction at 37°C for 4 hours. After the reaction, the plate was centrifuged, and the amount of $^{51}Cr$ in the supernatant was measured using a γ-counter. The amount of spontaneously released $^{51}Cr$ was calculated by the same operation except that only the medium was used instead of the effector cell solution and the antibody solution, and measuring the amount of $^{51}Cr$ in the supernatant. The amount of total released $^{51}Cr$ was calculated by the same operation except that only the medium was used instead of the antibody solution and 1 N hydrochloric acid was added instead of the effector cell solution, and measuring the amount of $^{51}Cr$ in the supernatant. The ADCC activity was calculated from the following equation (1):

$$\text{ADCC activity (\%)} = \frac{{}^{51}\text{Cr in sample supernatant - spontaneously released } {}^{51}\text{Cr}}{\text{total released } {}^{51}\text{Cr- spontaneously released } {}^{51}\text{Cr}} \times 100 \qquad (1)$$

The results are shown in Fig. 2. It was confirmed that the activity of the NS0/LCA-CCR4 antibody was significantly improved in comparison with the NS0/CCR4 antibody.

Example 3

Analysis of sugar chain structure of anti-CCR4 chimeric antibody:

Sugar chains of the anti-CCR4 chimeric antibodies purified in the item (1) of Example 2 were analyzed. The solution of the purified antibody was exchanged to 10 mM $KH_2PO_4$ using Ultra Free 0.5-10K (manufactured by Millipore). The exchange was carried out in such a manner that the exchanging ratio became 80-fold or more. The concentration of the antibodies after the solution exchange was measured using UV-1600 (manufactured by Shimadzu). The molar absorption coefficient was calculated from the amino acid sequence of each antibody based on the following equation (2) [*Advances in Protein Chemistry*, 12, 303 (1962)], and the concentration was determined by defining the absorbance at 280 nm as 1.38 mg/ml.

$$E_{1mol/l} = A \times n1 + B \times n2 + C \times n3 \quad (2)$$

$$E_{1mol/ml} = E_{1mol/l}/MW$$

$E_{1mol/l}$: absorption coefficient at 280 nm ($mg^{-1}$ ml $cm^{-1}$)
$E_{1mol/ml}$: molar absorption coefficient at 280 nm ($M^{-1}$ $cm^{-1}$)
A: molar absorption coefficient of tryptophan at 280 nm = 5550 ($M^{-1}$ $cm^{-1}$)
B: molar absorption coefficient oftyrosine at 280 nm = 1340 ($M^{-1}$ $cm^{-1}$)
C: molar absorption coefficient of cystine at 280 nm = 200 ($M^{-1}$ $cm^{-1}$)
n1: the number of tryptophan per 1 antibody molecule
n2: the number of tyrosine per 1 antibody molecule
n3: the number of cystine per 1 antibody molecule
MW: molecular weight of antibody (g/mol)

Into Hydraclub S-204 test tube, 100 µg of each antibody was put and dried by using a centrifugal evaporator. The dried sample in the test tube was subjected to hydrazinolysis by using Hydraclub manufactured by Hohnen. The sample was allowed to react with hydrazine at 110°C for 1 hour by using a hydrazinolysis reagent manufactured by Hohnen hydrazinolysis [*Method of Enzymology*, 83, 263 (1982)]. After the reaction, hydrazine was evaporated under a reduced pressure, and the reaction tube was returned to room temperature by allowing it to stand for 30 minutes. Next, 250 µl of an acetylation reagent manufactured by Hohnen and 25 µl of acetic anhydride were added thereto, followed by thoroughly stirred for reaction at room temperature for 30 minutes. Then, 250 µl of the acetylation reagent and 25 µl of acetic anhydride were further added thereto, followed by thoroughly stirring for reaction at room temperature for 1 hour. The sample was frozen at -80°C in a freezer and freeze-dried for about 17 hours. Sugar chains were recovered from the freeze-dried sample by using Cellulose Cartridge Glycan Preparation Kit manufactured by Takara Shuzo. The sample sugar chain solution was dried by using a centrifugal evaporator and then subjected to fluorescence labeling with 2-aminopyridine [*J. Biochem*., 95, 197 (1984)]. The 2-aminopyridine solution was prepared by adding 760 µl of HCl per g of 2-aminopyridine (1 × PA solution) and diluting the solution 10-fold with reverse osmosis purified water (10-folds diluted PA solution). The sodium cyanoborohydride solution was prepared by adding 20 µl of 1 × PA solution and 430 µl of reverse osmosis purified water per 10 mg of sodium cyanoborohydride. To the sample, 67 µl of a 10 fold-diluted PA solution was added, followed by reaction at 100°C for 15 minutes and spontaneously cooled, and 2 µl of sodium cyanoborohydride was further added thereto, followed by reaction at 90°C for 12 hours for fluorescence labeling of the sample sugar chains. The fluorescence-labeled sugar chain group (PA-treated sugar chain group) was separated from excess reagent by using Superdex Peptide HR 10/30 column (manufactured by Pharmacia). This step was carried out by using 10 mM ammonium bicarbonate as the eluent at a flow rate of 0.5 ml/min and at a column temperature of room temperature, and using a fluorescence detector of 320 nm excitation wavelength and 400 nm fluorescence wavelength. The eluate was recovered 20 to 30 minutes after addition of the sample and dried by using a centrifugal evaporator obtain purified PA-treated sugar chains. Next, reverse phase HPLC analysis of the purified PA-treated sugar chains was carried out by using CLC-ODS column (manufactured by Shimadzu, ϕ 6.0 nm × 159 nm) at a column temperature of 55°C and at a flow rate of 1 ml/min by using a fluorescence detector of 320 nm excitation wavelength and 400 nm fluorescence wavelength. The column was equilibrated with a 10 mM sodium phosphate buffer (pH 3.8), and elution was carried out for 80 minutes by a 0.5% 1-butanol linear density gradient. Each of the PA-treated sugar chain was identified by post source decay analysis of each peak of the separated PA-treated sugar chains by using matrix-assisted laser ionization time of flight mass spectrometry (MALDI-TOF-MS analysis), comparison of elution positions with standards of PA-treated sugar chain manufactured by Takara Shuzo, and reverse phase HPLC analysis after digestion of each PA-treated sugar chain by using various enzymes. The analysis chart by HPLC is shown in Fig. 3. Using a 10 mM sodium phosphate buffer (pH 3.8) as buffer A and a 10 mM sodium phosphate buffer (pH 3.8) + 0.5% 1-butanol as buffer B, the analysis was carried out by the following gradient.

| Time (minute) | 0 | 80 | 90 | 90.1 | 120 |
|---|---|---|---|---|---|
| Buffer B (%) | 0 | 60 | 60 | 0 | 0 |

In the figure, peaks ① to ⑧ show the following structures (1) to (8).

(1)

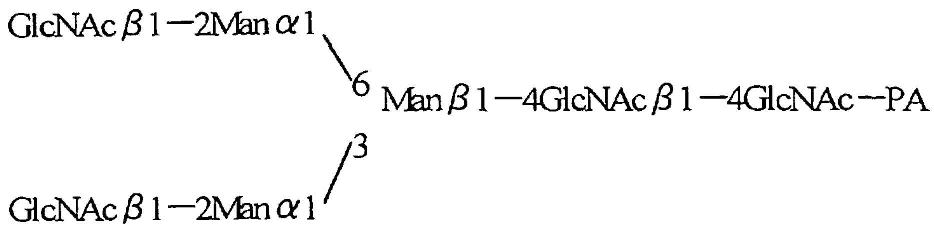

(2)

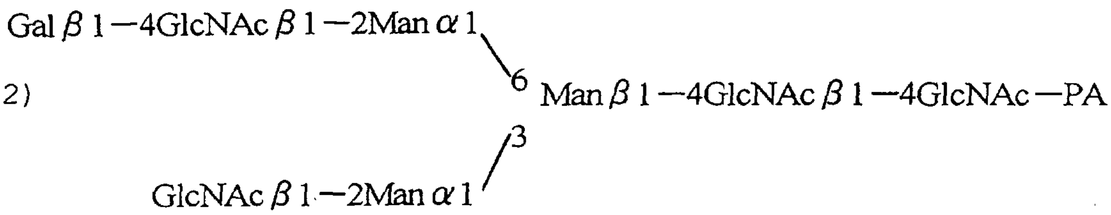

(3)

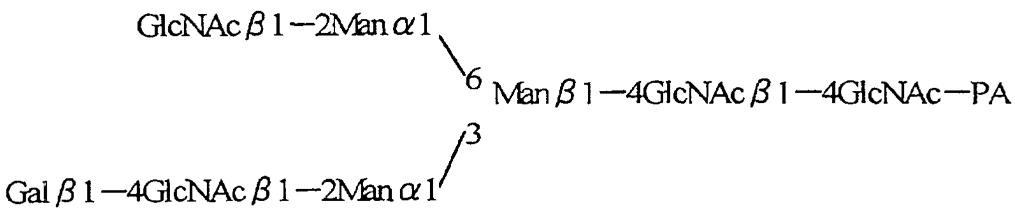

(4)

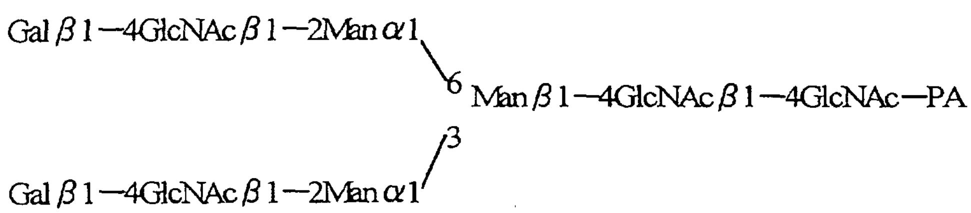

(5)

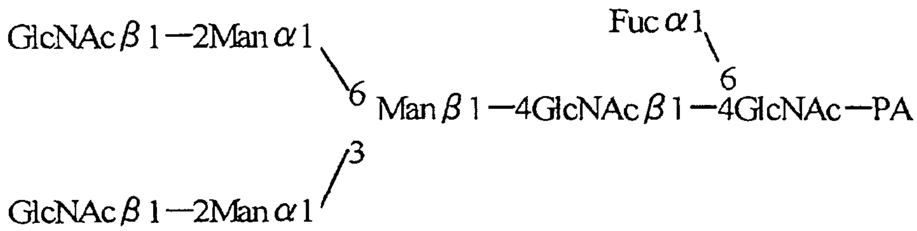

(6)

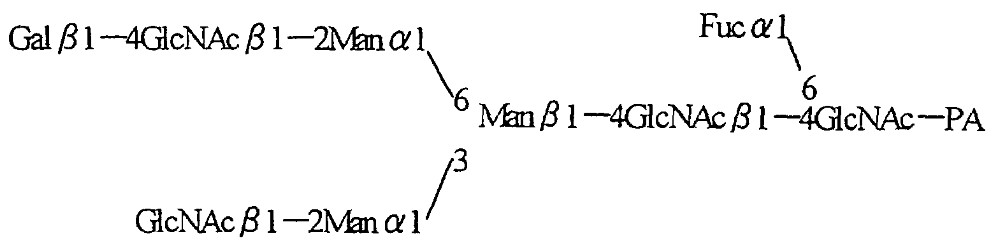

(7)

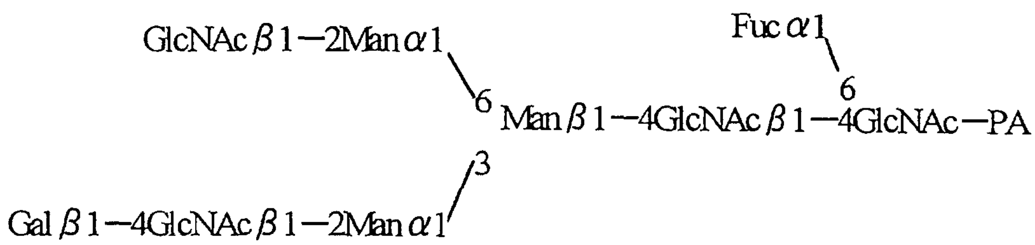

(8)

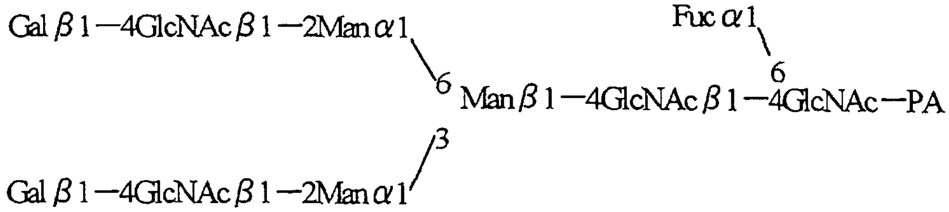

GlcNAc, Gal, Man, Fuc and PA indicate *N*-acetylglucosamine, galactose, mannose, fucose and a pyridylamino group, respectively. A sugar content is calculated based on the peak area of each PA-treated sugar chain in the reverse phase HPLC analysis. A PA-treated sugar chain whose reducing end is not *N*-acetylglucosamine was excluded from the peak area calculation, because it is an impurity or a by-product during preparation of PA-treated sugar chain.

In Fig. 3, the ratio of a sugar chain group in which fucose is not bound to 6-position of *N*-acetylglucosamine in the reducing end in the complex *N*-glycoside-linked sugar chain (hereinafter referred to as "α1,6-fucose-free sugar chain group") was calculated from the area occupied by the peaks ① to ④ among ① to ⑧ , and the ratio of a sugar chain group in which fucose is bound to 6-position of *N*-acetylglucosamine in the reducing end in the complex *N*-glycoside-linked sugar chain (hereinafter referred to as "α1,6-fucose-bound sugar chain group") from the area occupied by the peaks ⑤ to ⑧ among ① to ⑧ . The results are shown in Table 1.

Table 1

| Antibody | Ratio of α1,6-fucose-free complex biantennary sugar chain |
|---|---|
| NS0/CCR4 | 25% |
| NS/CCR4-LCA | 48% |

The ratio of the α1,6-fucose-free sugar chain of the antibody produced by the clone NS0/CCR4 was 25%, whereas the ratio of the α1,6-fucose-free sugar chain of the antibody produced by the clone NS0/CCR4-LCA was significantly increased to 48%.

Example 4

Preparation of lectin-resistant SP2/0 cell and production of antibody using the cell

1. Preparation of lectin-resistant clone SP2/0

A anti-GD3 chimeric antibody-producing transformed cell clone KM-872 (PERM BP-3512) of mouse myeloma SP2/0 cell (ATCC CRL-1581) described in Japanese Published Unexamined Patent Application No. 304989/93 was cultured in a suspension culture flask 75 $cm^2$ (manufactured by Iwaki Glass) using RPMI 1640 medium (manufactured by Invitrogen) to which fetal bovine serum (manufactured by PAA Laboratories) (hereinafter referred to as "RPMI-FBS (10) medium") had been added at 10% volume ratio and allowed to proliferate until just before confluent. The cells in this culture medium were suspended to give a density of $1 \times 10^5$ cells/ml by adding the RPMI-FBS(10) medium, and then 0.1 μg/ml of *N*-methyl-*N*'-nitro-*N* nitrosoguanidin which was an alkylating agent (MNNG, manufactured by Sigma) was added. After allowing to stand at 37°C for 3 days in a 5% $CO_2$ incubator (manufactured by NATIONAL APPLIANCE COMPANY), the cells were inoculated into a suspension culture 96 well plate (manufactured by Iwaki Glass) at a density of 1,000 cells/well. A *Lens culinaris* agglutinin (hereinafter referred to as "LCA", manufactured by Vector) was added to each well at a final concentration in the medium of 2 mg/ml. After culturing at 37°C for 2 weeks in a 5% $CO_2$ incubator, the formed lectin-resistant colony was obtained as lectin-resistant clone SP2/0. This clone was named clone SP2/0/GD3-LCA. The clone SP2/0/GD3-LCA, as a cell name of SP2/0/GD3-LCA, has been deposited on March 26, 2002, as FERM BP-7975 in International Patent Organism Depositary, National Institute of Advanced Industrial Science and Technology (Tsukuba Central 6, 1, Higashi 1-Chome Tsukuba-shi, Ibaraki-ken 305-8566 Japan).

2. Culturing of lectin-resistant clone SP2/0 and purification of antibody

Each of the anti-GD3 chimeric antibody-producing transformant clone SP2/0/GD3-LCA obtained in the item 1 of Example 4 and the clone KM-871 (PERM BP-3512) as its parent clone was suspended in Hybridoma-SFM medium (manufactured by Invitrogen) containing fetal bovine serum (manufactured by Invitrogen) at a final concentration of 0.2% to give a density of $3\times10^5$ cells/ml, and dispensed at 200 ml into 175 $cm^2$ flasks (manufactured by Greiner). After culturing at 37°C for 7 days in a 5% $CO_2$ incubator, culture supernatants were recovered. Each of the anti-GD3 chimeric antibodies was purified from the culture supernatants using Prosep-A (manufactured by Bioprocessing) column according to the manufacture's instructions. Regarding the purified anti-GD3 chimeric antibodies, the antibody produced by the clone SP2/0/GD3-LCA was named SP2/0/GD3-LCA antibody and the antibody produced by the clone KM-871 was named SP2/0/GD3 antibody.

3. Evaluation of activity of purified anti-GD3 antibody

(1) Binding activity of purified anti-ganglioside GD3 antibody against GD3

Binding activities of the purified anti-ganglioside GD3 antibodies obtained in the item 2 of Example 4 were measured according to the method described below.

In 2 ml of an ethanol solution containing 10 μg of dipalmitoylphosphatidylcholine (manufactured by SIGMA) and 5 μg of cholesterol (manufactured by SIGMA), 4 nmol ganglioside GD3 (manufactured by Snow Brand Milk Products) was dissolved. Into each well of a 96 well plate for ELISA (manufactured by Greiner), 20 μl of the solution was dispensed (40 pmol/well) and air-dried, and then 1% bovine serum albumin (hereinafter referred to as "BSA"; manufactured by SIGMA)-containing PBS (hereinafter referred to as "1% BSA-PBS") was added thereto at 100 μl/well and

allowed to react at room temperature for 1 hour to block the remaining active groups. After discarding 1% BSA-PBS, variously diluted solutions of culture supernatant of a transformant or a purified chimeric antibody were added thereto at 50 µl/well and allowed to react at room temperature for 1 hour. After the reaction, each well was washed with 0.05% Tween 20 (manufactured by Wako Pure Chemical Industries)-containing PBS (hereinafter referred to as "Tween-PBS"), and then, as a secondary antibody solution, a peroxidase-labeled goat anti-human IgG (H & L) antibody solution (manufactured by American Qualex) diluted 3,000-folds with 1% BSA-PBS was added thereto at 50 µl/well and allowed to react at room temperature for 1 hour. After the reaction and subsequent washing with Tween-PBS, an ABTS substrate solution [a solution prepared by dissolving 0.55 g of 2,2'-azino-bis(3-ethylbenzothiazoline-6-sulfonic acid)ammonium in 1 liter of 0.1 M citrate buffer (pH 4.2), and adding hydrogen peroxide at a final concentration of 1 µl/ml just before use] was dispensed at 50 µl/well for coloration and the absorbance at 490 nm was measured.

As a result, significant difference was not observed between the SP2/0GD3-LCA antibody and SP2/0/GD3 antibody, and both showed similar binding activity for GD3.

(2) *In vitro* cytotoxic activity of purified anti-GD3 antibody

*In vitro* cytotoxic activity of the purified anti-GD3 antibody obtained in the item 2 of Example 4 were measured according to the following method.

(2-1) Preparation of target cell suspension

A human melanoma culture cell line G-361 (ATCC CRL 1424) was cultured in RPMI1640 medium containing 10% in volume ratio of FBS (hereinafter referred to as "RPMI1640-FBS(10) medium") to prepare $1\times10^6$ cells, and 3.7 MBq equivalents of a radioactive substance $Na_2{}^{51}CrO_4$ were added thereto and the cells were isotope-labeled by carrying out the reaction at 37°C for 1 hour. After the reaction, the cells were washed three times by repeating their suspension in PRMI1640-FBS(10) medium and subsequent centrifugation, re-suspended in the medium and then allowed to stand at 4°C for 30 minutes on ice for spontaneous dissociation of the radioactive substance. After centrifugation, the cells were adjusted to $2\times10^5$ cells/ml by adding 5 ml of the PRMI1640-FBS(10) medium to obtain the target cell suspension.

(2-2) Preparation of effector cell suspension

From a healthy donor, 50 ml of peripheral blood was collected and gently mixed with 0.5 ml of heparin sodium (manufactured by Takeda Pharmaceutical). The resulting mixture was centrifuged by using Lymphoprep (manufactured by Nycomed Pharma AS) according to the manufacture's instructions, a mononuclear leukocyte layer was separated. After washing with PRMI1640-FBS(10) medium three times by centrifugation, the cells were re-suspended using the medium to give a density of $2\times10^6$ cells/ml to obtain the effector cell suspension.

(2-3) Measurement of ADCC activity

The target cell suspension prepared in the item (2-1) was dispensed at 50 µl ($1\times10^4$ cells/well) into wells of a 96 well U-bottom plate (manufactured by Falcon). Next, the effector cell suspension prepared in the item (2-2) was added thereto at 100 µl ($2\times10^5$ cells/well, the ratio of effector cells to target cells becomes 20:1). Subsequently, various anti-GD3 chimeric antibodies were added thereto to a respective final concentration of 2.5 to 2,500 ng/ml and allowed to react at 37°C for 4 hours. After the reaction, the plate was centrifuged, and the amount of $^{51}Cr$ in the supernatant was measured using a γ-counter (manufactured by Packard). The amount of the spontaneously dissociated $^{51}Cr$ was calculated by the same procedure using the medium alone instead of the effector cell suspension and antibody solution and measuring the amount of $^{51}Cr$ in the supernatant. The amount of the total dissociated $^{51}Cr$ was calculated by the same procedure using the medium alone instead of the antibody solution, and 1 N hydrochloric acid instead of the effector cell suspension, and measuring the amount of $^{51}Cr$ in the supernatant. The ADCC activity was calculated by equation (1) described above.

As a result, it was found that the SP2/0GD3-LCA antibody has higher activity than that of the SP2/0/GD3 antibody.

Furthermore, sugar chain structure of the purified anti-GD3 antibody obtained in the item 2 of Example 4 was analyzed according to the method described in Example 3. As a result, it was found that the ratio of a sugar chain in which 1-position of fucose is bound to 6-position of *N*-acetylglucosamine in the reducing end through α-bond in the complex *N*-glycoside-linked sugar chain is significantly decreased in the SP2/0GD3-LCA antibody in comparison with the SP2/0/GD3 antibody.

Example 5

Preparation of lectin-resistant CHO/DG44 cell and production of antibody using the cell:

(1) Preparation of lectin-resistant CHO/DG44

CHO/DG44 cells were grown until they reached a stage of just before confluent, by culturing in a 75 $cm^2$ flask for adhesion culture (manufactured by Greiner) using IMDM-FBS(10) medium [IMDM medium comprising 10% of fetal bovine serum (FBS) and 1 × concentration of HT supplement (manufactured by GIBCO BRL)]. After washing the cells with 5 ml of Dulbecco PBS (manufactured by Invitrogen), 1.5 ml of 0.05% trypsin (manufactured by Invitrogen) diluted with Dulbecco PBS was added thereto and incubated at 37°C for 5 minutes for peel the cells from the flask bottom. The peeled cells were recovered by a centrifugation operation generally used in cell culture, and suspended in IMDM-FBS(10) medium to give a density of $1\times10^5$ cells/ml, and then 0.1 μg/ml of an alkylating agent *N*-methyl-N'-nitro-*N*-nitrosoguanidine (hereinafter referred to as "MNNG", manufactured by Sigma) was added or not added thereto. After incubating them at 37°C for 3 days in a $CO_2$ incubator (manufactured by TABAI), the culture supernatant was discarded, and the cells were again washed, peeled and recovered by the same procedures described above, suspended in IMDM-FBS(10) medium and then inoculated into a 96 well plate for adhesion culture (manufactured by IWAKI Glass) to give a density of 1,000 cells/well. To each well, at the final concentration in medium, 1 mg/ml *Lens culinaris* agglutinin (hereinafter referred to as "LCA", manufactured by Vector), 1 mg/ml *Aleuria aurantia* agglutinin (*Aleuria anrantia* lectin; hereinafter referred to as "AAL", manufactured by Vector) or 1 mg/ml kidney bean agglutinin (*Phaseolus vulgaris* leucoagglutinin; hereinafter referred to as "L-PHA", manufactured by Vector) was added. After culturing at 37°C for 2 weeks in a $CO_2$ incubator, the growing colonies were obtained as lectin-resistant CHO/DG44. Regarding the obtained lectin-resistant CHO/DG44, an LCA-resistant clone was named CHO-LCA, an AAL-resistant clone was named CHO-AAL and an L-PHA-resistant clone was named CHO-PHA. When the resistance of these clones to various kinds of lectin was examined, it was found that the CHO-LCA was also resistant to AAL and the CHO-AAL was also resistant LCA. In addition, the CHO-LCA and CHO-AAL also showed a resistance to a lectin which recognizes a sugar chain structure identical to the sugar chain structure recognized by LCA and AAL, namely a lectin which recognizes a sugar chain structure in which 1-position of fucose is bound to 6-position of *N*-acetylglucosamine residue in the reducing end through α-bond in the *N*-glycoside-linked sugar chain. Specifically, it was found that the CHO-LCA and CHO-AAL can show resistance and survive even in a medium supplemented with 1 mg/ml at a final concentration of a pea agglutinin (*Pisum sativum* agglutinin; hereinafter referred to as "PSA", manufactured by Vector). In addition, even when the alkylating agent MNNG was not added, it was able to obtain lectin-resistant clones by increasing the number of cells to be treated.

(2) Preparation of anti-CCR4 human chimeric antibody-producing cell

An anti-CCR4 human chimeric antibody expression plasmid pKANTEX2160 was introduced into each of the three lectin-resistant clones obtained in the item (1) by the method described in Reference Example 1, and gene amplification by a drug MTX was carried out to prepare an anti-CCR4 human chimeric antibody-producing clone. By measuring an amount of antibody expression by the ELISA described in the item 2 of Reference Example 1, antibody-expressing transformants were obtained from each of the CHO-LCA, CHO-AAL and CHO-PHA. Regarding each of the obtained transformants, a transformant derived from CHO-LCA was named clone CHO/CCR4-LCA, a transformant derived from CHO-AAL was named clone CHO/CCR4-AAL and a transformant derived from CHO-PHA was named clone CHO/CCR4-PHA. Also, the clone CHO/CCR4-LCA, as a name of clone Nega-13, has been deposited on September 26, 2001, as FERM BP-7756 in International Patent Organism Depositary, National Institute of Advanced Industrial Science and Technology (Tsukuba Central 6, 1, Higashi 1-Chome Tsukuba-shi, Ibaraki-ken 305-8566 Japan).

(3) Production of high ADCC activity antibody by lectin-resistant CHO cell

Using the three transformants obtained in the item (2), purified antibodies were obtained by the method described in the item 3 of Reference Example 1. The antigen binding activity of each of the purified anti-CCR4 human chimeric antibodies was evaluated using the ELISA described in the item 2 of Reference Example 1. The antibodies produced by all transformants showed an antigen binding activity identical to that of the antibody produced by a recombinant clone (clone 5-03) prepared in Reference Example 1 using general CHO/DG44 cell as the host. Using these purified antibodies, ADCC activity of each of the purified anti-CCR4 human chimeric antibodies was evaluated in accordance with the method described in the item 7 of Reference Example 1. The results are shown in Fig. 4. In comparison with the antibody produced by the clone 5-03, about 100 fold-increased ADCC activity was observed in the antibodies produced by the clones CHO/CCR4-LCA and CHO/CCR4-AAL. On the other hand, no significant increase

in the ADCC activity was observed in the antibody produced by the clone CHO/CCR4-PHA. Also, when ADCC activities of the antibodies produced by the clone CHO/CCR4-LCA and YB2/0 were compared in accordance with the method described in the item 7 of Reference Example 1, it was found that the antibody produced by the clone CHO/CCR4-LCA shows higher ADCC activity than the antibody produced by the clone 5-03, similar to the case of the antibody KM2760-1 produced by the YB2/0 clone prepared in the item 1 of Reference Example 1 (Fig. 5).

(4) Sugar chain analysis of antibody produced by lectin-resistant CHO cell

Sugar chains of the anti-CCR4 human chimeric antibodies purified in the item (3) were analyzed in the same manner as in Example 3. The results of the reverse phase HPLC analysis are shown in Fig. 6. A sugar content is calculated based on the peak area of each PA-treated sugar chain in the reverse phase HPLC analysis. A PA-treated sugar chain whose reducing end is not *N*-acetylglucosamine was excluded from the peak area calculation, because it is an impurity or a by-product during preparation of PA-treated sugar chain.

Peaks (i) to (viii) in Fig. 6 correspond to the structures (1) to (8), respectively, shown in Example 3.

The analysis was carried out in the same manner as in Example 3. The ratio of α1,6-fucose-free sugar chains (%) calculated from the peak areas is shown in Table 2.

Table 2

| Antibody producing cells | α-1,6-Fucose-free complex biantennary sugar chain (%) |
|---|---|
| Clone 5-03 | 9 |
| Clone CHO/CCR4-LCA | 48 |
| Clone CHO/CCR4-AAL | 27 |
| Clone CHO/CCR4-PHA | 8 |

In comparison with the antibody produced by the clone 5-03, the ratio of the α1,6-fucose-free sugar chains was increased from 9% to 48% in the antibody produced by the clone CHO/CCR4-LCA when calculated from the analyzed peak area. The ratio of α1,6-fucose-free sugar chains was increased from 9% to 27% in the antibody produced by the clone CHO/CCR4-AAL. On the other hand, changes in the sugar chain pattern and ratio of the α1,6-fucose-free sugar chains were hardly found in the PHA-resistant clone when compared with the clone 5-03.

Example 6

Analysis of lectin-resistant CHO clone:

1. Analysis of amount of production of GMD enzyme in anti-CCR4 human chimeric antibody-producing clone CHO/CCR4-LCA

The amount of production of each of the genes of GMD, GFPP and FX known as fucose biosynthesis enzymes and α1,6-fucosyltransferase (hereinafter referred to as "FUT8") as a fucose transferase, in the anti-CCR4 human chimeric antibody-producing clone CHO/CCR4-LCA obtained in Example 5, was analyzed by RT-PCR.

(1) Preparation ofRNA from various clones

Each of the CHO/DG44 cell, the anti-CCR4 human chimeric antibody-producing clone 5-03 obtained in the item 1(2) of Reference Example 1 and the anti-CCR4 human chimeric antibody-producing clone CHO/CCR4-LCA obtained in the item (2) of Example 5 was subcultured at 37°C in a 5% $CO_2$ incubator, followed by culturing for 4 days. After culturing, total RNA was prepared from $1\times10^7$ cells of each clone using RNeasy Protect Mini Kit (manufactured by QIAGEN) in accordance with the manufacture's instructions. Subsequently, single-stranded cDNA was synthesized from 5 µg of each RNA in a 20 µl of a reaction solution using SUPER SCRIPT First-Strand Synthesis System for RT-PCR (manufactured by GIBCO BRL) in accordance with the manufacture's instructions.

(2) Analysis of amount of production of GMD gene using RT-PCR

In order to amplify GMD cDNA by PCR, a 24 mer synthetic DNA primer having the nucleotide sequence shown by SEQ ID NO:2 and a 26 mer synthetic DNA primer having the nucleotide sequence shown by SEQ ID NO:3 were prepared based on the CHO cell-derived GMD cDNA sequence shown in the item 1 of Reference Example 3.

Next, 20 µl of a reaction solution [1 × Ex Taq buffer (manufactured by Takara Shuzo), 0.2 mM dNTPs, 0.5 unit of Ex Taq polymerase (manufactured by Takara Shuzo) and each 0.5 µM of the synthetic DNA primers represented by SEQ ID NOs:2 and 3] containing 0.5 µl of the single-stranded cDNA prepared from each clone in the item (1) as the template was prepared, and PCR was carried out by using DNA Thermal Cycler 480 (manufactured by Perkin Elmer) by heating at 94°C for 5 minutes and subsequent 30 cycles of heating of 94°C for 1 minute and 68°C for 2 minutes as one cycle. After subjecting 10 µl of the PCR reaction solution to agarose electrophoresis, DNA fragments were stained using Cyber Green (manufactured by BMA) and then the amount of the DNA fragment of about 350 bp was measured using Fluor Imager SI (manufactured by Molecular Dynamics).

(3) Analysis of amount of production of GFPP gene using RT-PCR

In order to amplify GFPP cDNA by PCR, a 27 mer synthetic DNA primer having the nucleotide sequence shown by SEQ ID NO:4 and a 23 mer synthetic DNA primer having the nucleotide sequence shown by SEQ ID NO:5 were prepared based on the CHO cell-derived GFPP cDNA sequence obtained in the item 2 of Reference Example 2.
Next, 20 µl of a reaction solution [1 × Ex Taq buffer (manufactured by Takara Shuzo), 0.2 mM dNTPs, each 0.5 unit of Ex Taq polymerase (manufactured by Takara Shuzo) and 0.5 µM of the synthetic DNA primers represented by SEQ ID NOs:4 and 5] containing 0.5 µl of the single-stranded cDNA prepared from each clone in the item (1) as the template was prepared, and PCR was carried out by using DNA Thermal Cycler 480 (manufactured by Perkin Elmer) by heating at 94°C for 5 minutes and subsequent 24 cycles of heating at 94°C for 1 minute and 68°C for 2 minutes as one cycle. After subjecting 10 µl of the PCR reaction solution to agarose electrophoresis, DNA fragments were stained using Cyber Green (manufactured by BMA) and then the amount of the DNA fragment of about 600 bp was measured using Fluor Imager SI (manufactured by Molecular Dynamics).

(4) Analysis of amount of production of FX gene using RT-PCR

In order to amplify FX cDNA by PCR, a 28 mer synthetic DNA primer having the nucleotide sequence shown by SEQ ID NO:6 and a 28 mer synthetic DNA primer having the nucleotide sequence shown by SEQ ID NO:7 were prepared based on the CHO cell-derived FX cDNA sequence shown in the item 1 of Reference Example 2.
Next, 20 µl of a reaction solution [1 × Ex Taq buffer (manufactured by Takara Shuzo), 0.2 mM dNTPs, 0.5 unit of Ex Taq polymerase (manufactured by Takara Shuzo) and each 0.5 µM of the synthetic DNA primers represented by SEQ ID NO:6 and SEQ ID NO:7] containing 0.5 µl of the single-stranded cDNA prepared from each clone in the item (1) as the template was prepared, and PCR was carried out by using DNA Thermal Cycler 480 (manufactured by Perkin Elmer) by heating at 94°C for 5 minutes and subsequent 22 cycles of heating at 94°C for 1 minute and 68°C for 2 minutes as one cycle. After subjecting 10 µl of the PCR reaction solution to agarose electrophoresis, DNA fragments were stained using Cyber Green (manufactured by BMA) and then the amount of the DNA fragment of about 300 bp was measured using Fluor Imager SI (manufactured by Molecular Dynamics).

(5) Analysis of amount of production of PUT8 gene using RT-PCR

In order to amplify PUT8 cDNA by PCR, 20 µl of a reaction solution [1 × Ex Taq buffer (manufactured by Takara Shuzo), 0.2 mM dNTPs, 0.5 unit of Ex Taq polymerase (manufactured by Takara Shuzo) and each 0.5 µM of the synthetic DNA primers represented by SEQ ID NOs:42 and 43] containing 0.5 µl of the single-stranded cDNA prepared from each clone in the item (1) as the template was prepared, and PCR was carried out by using DNA Thermal Cycler 480 (manufactured by Perkin Elmer) by heating at 94°C for 5 minutes and subsequent 20 cycles of heating at 94°C for 1 minute and 68°C for 2 minutes as one cycle. After subjecting 10 µl of the PCR reaction solution to agarose electrophoresis, DNA fragments were stained using Cyber Green (manufactured by BMA) and then amount of the DNA fragment of about 600 bp was measured using Fluor Imager SI (manufactured by Molecular Dynamics).

(6) Analysis of amount of production of β-actin gene using RT-PCR

In order to amplify β-actin cDNA by PCR, 20 µl of a reaction solution [1 × Ex Taq buffer (manufactured by Takara Shuzo), 0.2 mM dNTPs, 0.5 unit of Ex Taq polymerase (manufactured by Takara Shuzo) and each 0.5 µM of the synthetic DNA primers represented by SEQ ID NOs:44 and 45] containing 0.5 µl of the single-stranded cDNA prepared from each clone in the item (1) as the template was prepared, and the reaction was carried out by using DNA Thermal Cycler 480 (manufactured by Perkin Elmer) by heating at 94°C for 5 minutes and subsequent 14 cycles of heating at 94°C for 1 minute and 68°C for 2 minutes as one cycle. After subjecting 10 µl of the PCR reaction solution to agarose electrophoresis, DNA fragments were stained using Cyber Green (manufactured by BMA) and then the amount of the DNA fragment of about 800 bp was measured using Fluor Imager SI (manufactured by Molecular Dynamics).

(7) Expression levels of GMD, GFPP, FX and FUT8 genes in each clone

The amount of the PCR-amplified fragment of each gene in the clone 5-03 and the clone CHO/CCR4-LCA was calculated by dividing values of the amounts of PCR-amplified fragments derived from GMD, GFPP, FX and FUT cDNA in each clone measured in the items (2) to (5) by the value of the amount of PCR-amplified fragment derived from β-actin cDNA in each clone, and the values were represented as the ratio against the amount of the PCR-amplified fragments in CHO/DG44 cell. The results are shown in Table 3.

Table 3

| | GMD | GEPP | FX | FUT8 |
|---|---|---|---|---|
| Clone CHO/DG44 | 1 | 1 | 1 | 1 |
| Clone 5-03 | 1.107 | 0.793 | 1.093 | 0.901 |
| Clone 5-03-derived LCA-resistant cell CHO/CCR4-LCA | 0.160 | 0.886 | 0.920 | 0.875 |

As shown in Table 3, the amount of production of GMD gene in the clone CHO/CCR4-LCA was decreased to about 1/10 in comparison with other clones. In this case, the test was independently carried out twice, and the average value was used.

2. Analysis of anti-CCR4 human chimeric antibody-producing CHO/CCR4-LCA in which GMD gene was forced to express

(1) Construction of CHO cell-derived GMD gene expression vector pAGE249GMD

Based on the CHO cell-derived GMD cDNA sequence obtained in the item 1 of Reference Example 3, a 28 mer primer having the nucleotide sequence shown by SEQ ID NO:8 and a 29 mer primer having the nucleotide sequence shown by SEQ ID NO:9 were prepared. Next, 20 μl of a reaction solution [1 × Ex Taq buffer (manufactured by Takara Shuzo), 0.2 mM dNTPs, 0.5 unit of Ex Taq polymerase (manufactured by Takara Shuzo) and each 0.5 μM of the synthetic DNA primers represented by SEQ ID NOs:8 and 9] containing 0.5 μl of the CHO cell-derived GMD single-stranded cDNA prepared in the item 1 of Reference Example 3 as the template was prepared, and PCR was carried out by using DNA Thermal Cycler 480 (manufactured by Perkin Elmer) by heating at 94°C for 5 minutes and subsequently 8 cycles of heating at 94°C for 1 minute, 58°C for 1 minute and 72°C for 1 minute as one cycle, and then 22 cycles of heating at 94°C for 1 minute and 68°C as one cycle. After completion of the reaction, the PCR reaction solution was subjected to agarose electrophoresis, and then a DNA fragment of about 600 bp was recovered using Gene Clean II Kit (manufactured by BIO 101) in accordance with the manufacture's instructions. The recovered DNA fragment was subjected to pT7Blue(R) vector (manufactured by Novagen) using DNA Ligation Kit (manufactured by Takara Shuzo), and *E. coli* DH5α (manufactured by Toyobo) was transformed using the obtained recombinant plasmid DNA to obtain a plasmid mt-C (cf. Fig. 7).

Next, based on the CHO cell-derived GMD cDNA sequence obtained in the item 1 of Reference Example 3, a 45 mer primer having the nucleotide sequence shown by SEQ ID NO:10 and a 31 mer primer having the nucleotide sequence shown by SEQ ID NO:11 were prepared. Next, 20 μl of a reaction solution [1 × Ex Taq buffer (manufactured by Takara Shuzo), 0.2 mM dNTPs, 0.5 unit of Ex Taq polymerase (manufactured by Takara Shuzo) and each 0.5 μM of the synthetic DNA primers represented by SEQ ID NOs:10 and 11] containing 0.5 μl of the CHO cell-derived GMD single-stranded cDNA prepared in the item 1 of Reference Example 3 as the template was prepared, and PCR was carried out by using DNA Thermal Cycler 480 (manufactured by Perkin Elmer) by heating at 94°C for 5 minutes and subsequently 8 cycles of heating at 94°C for 1 minute, 57°C for 1 minute and 72°C for 1 minute as one cycle, and then 22 cycles of heating at 94°C for 1 minute and 68°C for 2 minutes as one cycle. After completion of the reaction, the PCR reaction solution was subjected to agarose electrophoresis, and then a DNA fragment of about 150 bp was recovered using Gene Clean II Kit (manufactured by BIO 101) in accordance with the manufacture's instructions. The recovered DNA fragment was subcloned to pT7Blue(R) vector (manufactured by Novagen) using DNA Ligation Kit (manufactured by Takara Shuzo), and *E. coli* DH5α (manufactured by Toyobo) was transformed using the obtained recombinant plasmid DNA to obtain a plasmid ATG (cf. Fig. 8).

Next, 3 μg of the plasmid CHO-GMD prepared in the item 1 of Reference Example 3 was allowed to react with a restriction enzyme *Sac*I (manufactured by Takara Shuzo) at 37°C for 16 hours, a DNA was recovered by carrying out phenol/chloroform extraction and ethanol precipitation and allowed to react with a restriction enzyme *Eco*RI (manufactured by Takara Shuzo) at 37°C for 16 hours, a digest DNA was subjected to agarose electrophoresis and then a DNA fragment of about 900 bp was recovered using Gene Clean II Kit (manufactured by BIO 101) in accordance with the manufacture's instructions. The plasmid mt-C (1.4 μg) was allowed to react with a restriction enzyme *Sac*I (manufactured by Takara Shuzo) at 37°C for 16 hours, DNA was recovered by carrying out phenol/chloroform extraction and ethanol precipitation and allowed to react with a restriction enzyme *Eco*RI (manufactured by Takara Shuzo) at 37°C for 16 hours, the digest was subjected to agarose electrophoresis and then a DNA fragment of about 3.1 kbp was recovered using Gene Clean II Kit (manufactured by BIO 101) in accordance with the manufacture's instructions. The recovered DNA fragments were ligated using DNA Ligation Kit (manufactured by Takara Shuzo), and *E. coli* DH5α was transformed using the obtained recombinant plasmid DNA to obtain a plasmid WT-N(-) (cf. Fig. 9).

Next, 2 μg of the plasmid WT-N(-) was allowed to react with a restriction enzyme *Bam*HI (manufactured by Takara Shuzo) at 37°C for 16 hours, DNA was recovered by carrying out phenol/chloroform extraction and ethanol precipitation and allowed to react with a restriction enzyme *Eco*RI (manufactured by Takara Shuzo) at 37°C for 16 hours, the digest was subjected to agarose electrophoresis and then a DNA fragment of about 1 kbp was recovered using Gene Clean II Kit (manufactured by BIO 101) in accordance with the manufacture's instructions. The plasmid pBluescript SK(-) (3 μg; manufactured by Stratagene) was allowed to react with a restriction enzyme *Bam*HI (manufactured by Takara Shuzo) at 37°C for 16 hours, DNA was recovered by carrying out phenol/chloroform extraction and ethanol precipitation and allowed to react with a restriction enzyme *Eco*RI (manufactured by Takara Shuzo) at 37°C for 16 hours, the digest was subjected to agarose electrophoresis and then a DNA fragment of about 3 kbp was recovered using Gene Clean II Kit (manufactured by BIO 101) in accordance with the manufacture's instructions. The recovered respective DNA fragments were ligated using DNA Ligation Kit (manufactured by Takara Shuzo), and *E. coli* DH5α was transformed using the obtained recombinant plasmid DNA to obtain a plasmid WT-N(-) in pBS (cf. Fig. 10).

Next, 2 μg of the plasmid WT-N(-) in pBS was allowed to react with a restriction enzyme *Hin*dIII (manufactured by Takara Shuzo) at 37°C for 16 hours, DNA was recovered by carrying out phenol/chloroform extraction and ethanol precipitation and allowed to react with a restriction enzyme *Eco*RI (manufactured by Takara Shuzo) at 37°C for 16 hours, the digest was subjected to agarose electrophoresis and then a DNA fragment of about 4 kbp was recovered using Gene Clean II Kit (manufactured by BIO 101) in accordance with the manufacture's instructions. After 2 μg of the plasmid ATG was allowed to react with a restriction enzyme *Hin*dIII (manufactured by Takara Shuzo) at 37°C for 16 hours, DNA was recovered by carrying out phenol/chloroform extraction and ethanol precipitation and allowed to react with a restriction enzyme *Eco*RI (manufactured by Takara Shuzo) at 37°C for 16 hours, the digest was subjected to agarose electrophoresis and then a DNA fragment of about 150 bp was recovered using Gene Clean II Kit (manufactured by BIO 101) in accordance with the manufacture's instructions. The recovered respective DNA fragments were ligated using DNA Ligation Kit (manufactured by Takara Shuzo), and *E. coli* DH5α was transformed using the obtained recombinant plasmid DNA to obtain a plasmid WT in pBS (cf. Fig. 11).

Next, 2 μg of the plasmid pAGE249 was allowed to react with restriction enzymes *Hin*dIII and *Bam*HI (both manufactured by Takara Shuzo) at 37°C for 16 hours, the digest was subjected to agarose electrophoresis and then a DNA fragment of about 6.5 kbp was recovered using Gene Clean II Kit (manufactured by BIO 101) in accordance with the manufacture's instructions. After 2 μg of the plasmid WT in pBS was allowed to react with restriction enzymes *Hin*dIII and *Bam*HI (both manufactured by Takara Shuzo) at 37°C for 16 hours, the digest was subjected to agarose electrophoresis and then a DNA fragment of about 1.2 kbp was recovered using Gene Clean II Kit (manufactured by BIO 101) in accordance with the manufacture's instructions. The recovered DNA fragments were ligated using DNA Ligation Kit (manufactured by Takara Shuzo), and *E. coli* DH5α was transformed using the obtained recombinant plasmid DNA to obtain a plasmid pAGE249GMD (cf. Fig. 12).

(2) Stable expression of GMD gene in CHO/CCR4-LCA

The CHO cell-derived GMD gene expression vector pAGE249GMD (5 μg) made into linear form by digesting it with a restriction enzyme *Fsp*I (manufactured by NEW ENGLAND BIOLABS), which was introduced into $1.6 \times 10^6$ cells of CHO/CCR4-LCA by electroporation [*Cytotechnology*, 3, 133 (1990)]. Then, the cells were suspended in 30 ml of IMDM-dFBS(10) medium [IMDM medium (manufactured by GIBCO BRL) supplemented with 10% of dFBS] containing 200 nM MTX (manufactured by SIGMA), and cultured in a 182 $cm^2$ flask (manufactured by Greiner) at 37°C for 24 hours in a 5% $CO_2$ incubator. After culturing, the medium was changed to IMDM-dFBS(10) medium containing 0.5 mg/ml hygromycin and 200 nM MTX (manufactured by SIGMA), followed by culturing for 19 days to obtain colonies of hygromycin-resistant transformants.

In the same manner, the pAGE249 vector was introduced into the clone CHO/CCR4-LCA by the same method to obtain colonies of hygromycin-resistant transformants.

(3) Culturing of GMD gene-expressed CHO/CCR4-LCA and purification of antibody

Using IMDM-dFBS(10) medium comprising 200 nM MTX (manufactured by SIGMA) and 0.5 mg/ml hygromycin, the GMD-expressing transformant cells obtained in the item (2) were cultured in a 182 $cm^2$ flask (manufactured by Greiner) at 37°C in a 5% $CO_2$ incubator. Several days thereafter, when the cell density reached confluent, the culture supernatant was discarded, and the cells were washed with 25 ml of PBS buffer (manufactured by GIBCO BRL) and mixed with 35 ml of EXCELL301 medium (manufactured by JRH). After culturing at 37°C in a 5% $CO_2$ incubator for 7 days, the culture supernatant was recovered. An anti-CCR4 chimeric antibody was purified from the culture supernatant using Prosep-A (manufactured by Millipore) in accordance with the manufacture's instructions.

In the same manner, the pAGE249 vector-introduced transformant cells were cultured by the same method, and then anti-CCR4 chimeric antibody was recovered and purified from the culture supernatant.

(4) Measurement of lectin resistance in transformed cells

The GMD-expressing transformant cells obtained in the item (2) were suspended in IMDM-dFBS(10) medium comprising 200 nM MTX (manufactured by SIGMA) and 0.5 mg/ml hygromycin to give a density of $6\times10^4$ cells/ml, and the suspension was dispensed at 50 µl/well into a 96 well culture plate (manufactured by Iwaki Glass). Next, a medium prepared by suspending at concentrations of 0 mg/ml, 0.4 mg/ml, 1.6 mg/ml or 4 mg/ml LCA (*Lens culinaris* agglutinin: manufactured by Vector Laboratories) in IMDM-dFBS(10) medium containing 200 nM MTX (manufactured by SIGMA) and 0.5 mg/ml hygromycin was added to the plate at 50 µl/well, followed by culturing at 37°C for 96 hours in a 5% $CO_2$ incubator. After culturing, WST-I (manufactured by Boehringer) was added at 10 µl/well and allowed to stand at 37°C for 30 minutes in a 5% $CO_2$ incubator for color development, and then the absorbance at 450 nm and 595 nm (hereinafter referred to as "$OD_{450}$" and "$OD_{595}$", respectively) was measured using Microplate Reader (manufactured by BIO-RAD). In the same manner, the pAGE249 vector-introduced transformant cells were measured by the same method. The test was carried out twice independently.

Fig. 13 shows the percentage of survived cells in each well calculated by subtracting $OD_{595}$ from $OD_{450}$ measured in the above as the number of survived cells in each of the LCA-free wells is defined as 100%. As shown in Fig. 13, decrease in the LCA-resistance was observed in the GMD-expressed CHO/CCR4-LCA, and the survival ratio was about 40% in the presence of 0.2 mg/ml LCA and the survival ratio was about 20% in the presence of 0.8 mg/ml LCA. On the other hand, in the pAGE249 vector-introduced clone CHO/CCR4-LCA, the survival ratio was 100% in the presence of 0.2 mg/ml LCA and the survival ratio was about 80% even in the presence of 0.8 mg/ml LCA. Based on these results, it was suggested that amount of production of GMD gene in the clone CHO/CCR4-LCA was decreased and, as a result, the cell obtained the resistance against LCA.

(5) *In vitro* cytotoxic activity (ADCC activity) of anti-CCR4 chimeric antibody obtained from GMD-expressed clone CHO/CCR4-LCA

In order to evaluate *in vitro* cytotoxic activity of the purified anti-CCR4 chimeric antibody obtained in the item (3), the ADCC activity was measured in accordance with the following methods.

i) Preparation of target cell suspension

To $1\times10^6$ cells of the CCR4-EL4 (cf the item 7 of Reference Example 1) cultured in a medium prepared by adding 500 µg/ml G418 sulfate (manufactured by Nacalai Tesque) to the RPMI1640-FBS(10) medium, 3,7 MBq of a radioactive substance $Na_2{}^{51}CrO_4$ was added, followed by reaction at 37°C for 90 minutes to thereby label the cells with a radioisotope. After the reaction, the cells were washed three times by suspension in the RPMI1640-FBS(10) medium and subsequent centrifugation, re-suspended in the medium and then allowed to stand at 4°C for 30 minutes on ice for spontaneous dissociation of the radioactive substance. After centrifugation, the cells were adjusted to $2.5\times10^5$ cells/ml by adding 5 ml of the RPMI1640-FBS(10) medium to obtain a target cell suspension.

ii) Preparation of effector cell suspension

From a healthy donor, 50 ml of venous blood was collected and gently mixed with 0.5 ml of heparin sodium (manufactured by Takeda Pharmaceutical). Using Lymphoprep (manufactured by Nycomed Pharma AS), the mixture was centrifuged in accordance with the manufacture's instructions to separate a mononuclear cell layer. The cells were washed three times by centrifuging using the RPMI1640-FBS(10) medium and then resuspended in the medium to give a density of $2\times10^6$ cells/ml to obtain a effector cell suspension.

iii) Measurement of ADCC activity

The target cell suspension prepared in the i) was dispensed at 50 μl ($1\times10^4$ cells/well) into each well of a 96 well U-bottom plate (manufactured by Falcon). Next, 100 μl of the effector cell suspension prepared in the ii) was added thereto ($2\times10^5$ cells/well, ratio of the effector cells to the target cells was 25 : 1). Each of various anti-CCR4 chimeric antibodies (the anti-CCR4 chimeric antibody purified in the item (3), and KM2760-1 and KM3060 described in the item 3 of Reference Example 1) was further added thereto to give a final concentration of 0.0025 to 2.5 μg/ml, followed by reaction at 37°C for 4 hours. After the reaction, the plate was centrifuged and the amount of $^{51}Cr$ in the supernatant was measured using a γ-counter. The amount of the spontaneously dissociated $^{51}Cr$ was calculated by carrying out the same procedure using the medium alone instead of the effector cell suspension and antibody solution, and measuring the amount of $^{51}Cr$ in the supernatant. The amount of the total dissociated $^{51}Cr$ was calculated by carrying out the same procedure using the medium alone instead of the antibody solution and adding 1 N hydrochloric acid instead of the effector cell suspension and measuring the amount of $^{51}Cr$ in the supernatant. The ADCC activity was calculated based on the formula (I) shown above.

Results of the measurement of ADCC activity are shown in Fig. 14. As shown in Fig. 14, ADCC activity of the purified anti-CCR4 chimeric antibody obtained from the GMD-expressed CHO/CCR4-LCA was decreased to a similar degree to that of the KM3060 obtained in Reference Example 1. On the other hand, ADCC activity of the purified anti-CCR4 chimeric antibody obtained from the pAGE249 vector-introduced CHO/CCR4-LCA showed a similar degree of ADCC activity to that of the purified anti-CCR4 chimeric antibody obtained from the clone CHO/CCR4-LCA. Taken together, it was suggested that amount of production of GMD gene in the clone CHO/CCR4-LCA is decreased and an antibody having high ADCC activity can be produced

(6) Sugar chain analysis of anti-CCR4 chimeric antibody derived from GMD-expressed CHO/CCR4-LCA

Sugar chains binding to the purified anti-CCR4 chimeric antibody obtained in the item (3) were analyzed in accordance with the method shown in the item (4) of Example 5, and the results are shown in Fig. 15. In comparison with the purified anti-CCR4 chimeric antibody prepared from CHO/CCR4-LCA in Example 5, the ratio of sugar chain having no α1,6-fucose in the purified anti-CCR4 chimeric antibody derived from GMD-expressed CHO/CCR4-LCA was decreased to 9%. Thus, it was shown that the ratio of sugar chain having no α1,6-fucose when calculated from the peak area in the antibody produced by the cell expressing GMD gene in the clone CHO/CCR4-LCA is decreased to similar level of the antibody produced by the clone 5-03

Example 7

Preparation of anti-ganglioside GD3 human chimeric antibody using lectin-resistant CHO/DG44 cell:

1. Construction of tandem expression vector pChi641LHGM4 of anti-ganglioside GD3 human chimeric antibody

A plasmid pChi641LGM40 was constructed by ligating a fragment of about 4.03 kb fragment containing an L chain cDNA, obtained by digesting an L chain expression vector, pChi641LGM4 [*J. Immunol. Methods,* 167, 271 (1994)] for anti-ganglioside GD3 human chimeric antibody (hereinafter referred to as "anti-GD3 chimeric antibody") with restriction enzymes *Mlu*I (manufactured by Takara Shuzo) and *Sal*I (manufactured by Takara Shuzo) with a fragment of about 3.40 kb containing a G418-resistant gene and a splicing signal obtained by digesting an expression vector for animal cell pAGE107 [*Cytotechnology*, 3, 133 (1990)] with restriction enzymes *Mlu*I (manufactured by Takara Shuzo) and *Sal*I (manufactured by Takara Shuzo), using DNA Ligation Kit (manufactured by Takara Shuzo), and then transforming *E. coli* HB101 (*Molecular Cloning,* Second Edition) with the ligated product.

Next, a fragment of about 5.68 kb containing an L chain cDNA obtained by digesting the constructed plasmid pChi641LGM4 with a restriction enzyme *Cla*I (manufactured by Takara Shuzo), following blunt-terminating using DNA Blunting Kit (manufactured by Takara Shuzo) and further digesting with *Mlu*I (manufactured by Takara Shuzo), was ligated with a fragment of about 8.40 kb containing an H chain cDNA obtained by digesting an anti-GD3 chimeric antibody H chain expression vector pChi641HGM4 [*J. Immunol. Methods*, 167, 271 (1994)] with a restriction enzyme *Xho*I (manufactured by Takara Shuzo), following blunt-terminating using DNA Blunting Kit (manufactured by Takara Shuzo) and further digesting with *Mlu*I (manufactured by Takara Shuzo), using DNA Ligation Kit (manufactured by Takara Shuzo), and then *E. coli* HB101 strain (*Molecular Cloning,* Second Edition) was transformed with the ligated product to thereby construct a tandem expression vector pChi641LHGM4 for anti-GD3 chimeric antibody.

2. Preparation of cell stably producing anti-GD3 chimeric antibody

Into $1.6\times10^6$ cells of the clone CHO/DG44 and the clone CHO-LCA prepared in the item (1) of Example 5, the anti-GD3 chimeric antibody expression vector pChi641LHGM4 prepared in the item 1 of Example 7 was introduced by electroporation [*Cytotechnology*, 3, 133 (1990)], and the cells were suspended in 10 ml of IMDM medium (manufactured by Invitrogen, to be referred to as IMDM-dFBS(10) medium) containing dialyzed fetal bovine serum (manufactured by Invitrogen) at 10% volume ratio and dispensed at 200 µl/well into a 96 well culture plate (manufactured by Iwaki Glass). The cells were cultured for 2 weeks in a 5% $CO_2$ incubator. Culture supernatants were recovered from wells where colonies of transformants showing medium nucleic acid component-independent growth were formed and their growth was confirmed, and then, antigen-binding activity of the anti-GD3 chimeric antibody in the culture supernatant was measured by the ELISA shown in the item 3(1) of Example 4.

In order to increase antibody production using the DHFR gene amplification system, transformants in wells where production of an anti-GD3 chimeric antibody were detected in the culture supernatant were suspended to give a density of $1\times10^5$ cells/ml in the IIVVIDM-dFBS(10) medium containing 50 nM methotrexate (manufactured by Sigma, hereinafter referred to as "MTX"), and the suspension was dispensed at 0.5 ml into a 24 well plate (manufactured by Iwaki Glass). After culturing at 37°C for 2 weeks in a 5% $CO_2$ incubator, transformants showing 50 nM MTX resistance grew up. The transformants in wells where their growth was observed were cultured at 37°C for 2 weeks by increasing the MTX concentration to 200 nM by a method similar to the above to induce transformants showing 200 nM MTX resistance. The transformants in wells where their growth was observed were cultured at 37°C for 2 weeks by increasing the MTX concentration to 500 nM by a method similar to the above to induce transformants showing 500 nM MTX resistance. Finally, stable transformants which can grow in the IMDM-dFBS(10) medium containing 500 nM MTX and also can highly produce the anti-GD3 chimeric antibody were obtained. Regarding the thus obtained transformants, cloned clones were obtained by carrying out single cell isolation (cloning) by a limiting dilution method. Cloned clones obtained using the clone CHO-LCA as the host cell for gene introduction were named clone CHO/GD3-LCA-1 and clone CHO/GD3-LCA-2. A clone obtained using the clone CHO-DG44 as the host cell was named clone CHO/GD3. The clone CHO/GD3-LCA-1 has been deposited on November 11, 2002, as FERM BP-8236 in International Patent Organism Depositary, National Institute of Advanced Industrial Science and Technology (Tsukuba Central 6, 1, Higashi 1-Chome Tsukuba-shi, Ibaraki-ken, Japan).

3. Purification of anti-GD3 chimeric antibody

Each of the anti-GD3 chimeric antibody-producing transformant cell clone, the clone CHO/GD3-LCA-1 and the clone CHO/GD3-LCA-2 obtained in the item 2 of Example 7 was suspended in a commercially available serum-free medium, EX-CELL 301 medium (manufactured by JRH) to give a density of $1\times10^6$ cells/ml and dispensed at 35 ml into 175 $cm^2$ flasks (manufactured by Greiner). After culturing at 37°C for 7 days in a 5% $CO_2$ incubator, culture supernatants were recovered. Each of the anti-GD3 chimeric antibodies was purified from the culture supernatants by using Prosep-A (manufactured by Bioprocessing) column according to the manufacture's instructions. As the purified anti-GD3 chimeric antibodies, the antibody produced by the clone CHO/GD3-LCA-1 was named CHO/GD3-LCA-1 antibody and the antibody produced by the clone CHO/GD3-LCA-2 was named CHO/GD3-LCA-2 antibody. Also, the antibody used as a control the usual antibody produced by the clone CHO/DG44 was named CHO/GD3 antibody.

4. Analysis of purified anti-GD3 chimeric antibody

In accordance with a known method [*Nature*, 227, 680 (1970)], 4 µg of each of the three kinds of the anti-GD3 chimeric antibodies produced and purified in the item 3 of Example 7, was subjected to SDS-PAGE to analyze the molecular weight and purity. As a result, a single band of about 150 kilodaltons (hereinafter referred to as "Kd") in molecular weight was found under non-reducing conditions, and two bands of about 50 Kd and about 25 Kd under reducing conditions, in each of the purified anti-GD3 chimeric antibodies. The molecular weights almost coincided with the molecular weights deduced from the cDNA nucleotide sequences of H chain and L chain of the antibody (H chain: about 49 Kd, L chain: about 23 Kd, whole molecule: about 144 Kd), and also coincided with the reports stating that the IgG antibody has a molecular weight of about 150 Kd under non-reducing conditions and is degraded into H chains having a molecular weight of about 50 Kd and L chains having a molecular weight of about 25 Kd under reducing conditions due to cutting of the disulfide bond (hereinafter referred to as "S-S bond") in the molecule [*Antibodies,* Chapter 14; *Monoclonal Antibodies*], so that it was confirmed that each anti-GD3 chimeric antibody was expressed and purified as an antibody molecule having the correct structure.

5. Sugar chain analysis of anti-GD3 chimeric antibody

Sugar chains of the anti-GD3 chimeric antibodies purified in the item 3 of Example 7 were analyzed according to the method described in Example 5(4). Each of PA-treated sugar chains prepared from the anti-GD3 chimeric antibodies are analyzed by reverse phase HPLC and the resulting elution chart is shown in Fig. 16. In Fig. 16, peaks ① to ⑧ correspond to the structures (i) to (viii), respectively, shown in Example 3. The analysis was carried out in the same manner as in Example 3, and he ratio of α1,6-fucose-free sugar chains (%) calculated from the peak areas by the analysis chart is shown in Table 4.

Table 4

| Antibody producing cells | α1,6-Fucose-free sugar chain (%) |
|---|---|
| CHO/GD3 antibody | 9 |
| CHO/GD3-LCA-1 antibody | 42 |
| CHO/GD3-LCA-1 antibody | 80 |

As shown in Table 4, the ratio of α1,6-fucose-free complex biantennary sugar chain was increased from 9% to 42% in the CHO/GD3-LCA-1 antibody in comparison with that in the control CHO/GD3 antibody. Also, the ratio of complex biantennary sugar chains was increased from 9% to 80% in the CHO/GD3-LCA-2 antibody.

Example 8

Evaluation of activities of anti-GD3 chimeric antibodies:

1. Binding activities of anti-GD3 chimeric antibodies against GD3 (ELISA)

Binding activities of the three purified anti-GD3 chimeric antibodies obtained in the item 3 of Example 7 against GD3 were measured by the ELISA shown in the item 3(1) of Example 4. Fig. 17 shows results of binding activity tested by changing a concentration of each anti-GD3 chimeric antibody to be added. As shown in Fig. 17, the three anti-GD3 chimeric antibodies showed almost the same binding activity against the ganglioside GD3. This result shows that the antigen-binding activities of antibodies produced by LCA lectin-resistant CHO/DG44 cells are the same as that of the antibody produced by the control CHO/DG44 cell.

2. *In vitro* cytotoxic activity of anti-GD3 chimeric antibodies (ADCC activity)

In order to evaluate *in vitro* cytotoxic activity of the three purified anti-GD3 chimeric antibodies obtained in the item 3 of Example 7, the ADCC activities were measured according to the method shown in Example 4-3(2).

The results are shown in Fig. 18. As shown in Fig. 18, among the three anti-GD3 chimeric antibodies, the CHO/GD3-LCA-2 antibody showed the highest ADCC activity, followed by the CHO/GD3-LCA-1 antibody and the CHO/GD3 antibody. The above results shows that the ADCC activities of produced antibodies are increased in LCA lectin-resistant clone CHO/DG44.

Example 9

Production of anti-CD20 human chimeric antibody

1. Production of anti-CD20 vector for human chimeric antibody expression

(1) Construction of cDNA encoding L chain V region of anti-CD20 mouse monoclonal antibody

A cDNA (described in SEQ ID NO:46) encoding the amino acid sequence of VL of the anti-CD20 mouse monoclonal antibody 2B8 described in WO 94/11026 was constructed using PCR as follows.

First, PCR primer binding nucleotide sequences (also containing a restriction enzyme recognizing sequence for cloning into a vector for humanized antibody expression) for amplification by PCR were added to the 5'-terminal and 3'-terminal in the nucleotide sequence of the VL described in WO 94/11026. A designed nucleotide sequence was divided from the 5'-terminal side into a total of 6 nucleotide sequences each having about 100 nucleotides (wherein adjacent nucleotide sequences were designed in such a manner that their terminals have an overlapping sequence

of about 20 nucleotides), and 6 synthetic DNA fragments, actually those represented by SEQ ID NOs:48, 49, 50, 51, 52 and 53, were prepared from them in alternate order of a sense chain and an antisense chain (consigned to GENSET).

Each oligonucleotide was added to 50 µl of a reaction solution [PCR Buffer #1 attached to KOD DNA Polymerase (manufactured by TOYOBO), 0.2 mM dNTPs, 1 mM magnesium chloride, 0.5 µM M13 primer M4 (manufactured by Takara Shuzo) and 0.5 µM M13 primer RV (manufactured by Takara Shuzo)] to give a final concentration of 0.1 µM, and using a DNA thermal cycler GeneAmp PCR System 9600 (manufactured by Perkin Elmer), the reaction was carried out by heating at 94°C for 3 minutes, adding 2.5 units of KOD DNA Polymerase (manufactured by TOYOBO), and then carrying out 25 cycles of heating at 94°C for 30 seconds, 55°C for 30 seconds and 74°C for 1 minutes as one cycle, and then further heating at 72°C for 10 minutes. After 25 µl of the reaction solution was subjected to agarose gel electrophoresis, a VL PCR product of about 0.44 kb was recovered using QIAquick Gel Extraction Kit (manufactured by QIAGEN).

Next, 0.1 µg of a DNA fragment obtained by digesting a plasmid pBluescript II SK(-) (manufactured by Stratagene) with a restriction enzyme *Sma*I (manufactured by Takara Shuzo) and about 0.1 µg of the PCR product obtained in the above were added to sterile water to adjust the total volume to 7.5 µl, and then 7.5 µl of the solution I of TAKARA ligation kit ver. 2 (manufactured by Takara Shuzo) and 0.3 µl of a restriction enzyme *Sma*I (manufactured by Takara Shuzo) were added thereto to carry out the reaction at 22°C for 2 hours. Using the recombinant plasmid DNA solution obtained, *Escherichia coli* DH5α strain (manufactured by TOYOBO) was transformed. Each plasmid DNA was prepared from the transformant clones and allowed to react by using BigDye Terminator Cycle Sequencing Ready Reaction Kit v2.0 (manufactured by Applied Biosystems) according to the manufacture's instructions, and then the nucleotide sequence was analyzed by DNA sequencer ABI PRISM 377 manufactured by the same company. In this manner, the plasmid pBS-2B8L shown in Fig. 19 having the nucleotide sequence of interest was obtained.

(2) Construction of cDNA encoding H chain V region of anti-CD20 mouse monoclonal antibody

A cDNA (described in SEQ ID NO:47) encoding the amino acid sequence of VH of the anti-CD20 mouse monoclonal antibody 2B8 described in WO 94/11026 was constructed using PCR as follows.

First, PCR binding nucleotide sequences (also containing a restriction enzyme recognizing sequence for cloning into a vector for humanized antibody expression) for amplification by PCR were added to the 5'-terminal and 3'-terminal of the nucleotide sequence of the VH described in WO 94/11026. A designed nucleotide sequence was divided from the 5'-terminal side into a total of 6 nucleotide sequences each having about 100 nucleotides (adjacent nucleotide sequences are designed in such a manner that their terminals have an overlapping sequence of about 20 nucleotides), and 6 synthetic DNA fragments, actually those represented by SEQ ID NOs:54, 55, 56, 57, 58 and 59, were prepared from them in alternate order of sense chain and antisense chain (consigned to GENSET).

Each oligonucleotide was added to 50 µl of a reaction solution [PCR Buffer #1 attached to KOD DNA Polymerase (manufactured by TOYOBO), 0.2 mM dNTPs, 1 mM magnesium chloride, 0.5 µM M13 primer M4 (manufactured by Takara Shuzo) and 0.5 µM M13 primer RV (manufactured by Takara Shuzo)] to give a final concentration of 0.1 µM, and using a DNA thermal cycler GeneAmp PCR System 9600 (manufactured by Perkin Elmer), the reaction was carried out by heating at 94°C for 3 minutes, adding 2.5 units of KOD DNA Polymerase (manufactured by TOYOBO), and then carrying out 25 cycles of heating at 94°C for 30 seconds, 55°C for 30 seconds and 74°C for 1 minute as one cycle, and then further heating at 72°C for 10 minutes. After 25 µl of the reaction solution was subjected to agarose gel electrophoresis, and then a VH PCR product of about 0.49 kb was recovered using QIAquick Gel Extraction Kit (manufactured by QIAGEN).

Next, 0.1 µg of a DNA fragment obtained by digesting the plasmid pBluescript II SK(-) (manufactured by Stratagene) with a restriction enzyme *Sma*I (manufactured by Takara Shuzo) and about 0.1 µg of the PCR product obtained in the above were added to sterile water to adjust the total volume to 7.5 µl, and then 7.5 µl of the solution I of TAKARA ligation kit ver. 2 (manufactured by Takara Shuzo) and 0.3 µl of a restriction enzyme *Sma*I (manufactured by Takara Shuzo) were added thereto to carry out the reaction at 22°C overnight.

Using the recombinant plasmid DNA solution obtained in this manner, *Escherichia coli* DH5α (manufactured by TOYOBO) was transformed. Each plasmid DNA was prepared from the transformant clones and allowed to react by using BigDye Terminator Cycle Sequencing Ready Reaction Kit v2.0 (manufactured by Applied Biosystems) according to the manufacture's instructions, and then the nucleotide sequence was analyzed by the DNA sequencer ABI PRISM 377 manufactured by the same company. In this manner, the plasmid pBS-2B8H shown in Fig. 20 having the nucleotide sequence of interest was obtained.

Next, in order to substitute Ala at position 14 in the amino acid sequence with Pro, the synthetic DNA represented by SEQ ED NO:60 was designed and the nucleotide was substituted as follows according to PCR by using LA PCR *in vitro* Mutagenesis Primer Set for pBluescript II (manufactured by Takara Shuzo). After 50 µl of a reaction solution [LA PCR Buffer II (manufactured by Takara Shuzo), 2.5 units of TaKaRa LA Taq, 0.4 mM dNTPs, 2.5 mM magnesium chloride, 50 nM T3 BcaBEST Sequencing primer (manufactured by Takara Shuzo) and 50 nM of the primer for mutagenesis (SEQ ID NO:60, manufactured by GENSET)] containing 1 ng of the plasmid pBS-2B8H was prepared, and 25 cycles of heating at 94°C for 30 seconds, 55°C for 2 minutes and 72°C for 1.5 minutes as one cycle were repeated by using a DNA thermal cycler GeneAmp PCR System 9600 (manufactured by Perkin Elmer). After 30 µl of the reaction solution was subjected to agarose gel electrophoresis, a PCR product of about 0.44 kb was recovered by using QIAquick Gel Extraction Kit (manufactured by QIAGEN) and made into 30 µl of an aqueous solution. In the same manner, PCR was carried out by using 50 µl of a reaction solution [LA PCR Buffer II (manufactured by Takara Shuzo), 2.5 units of TaKaRa LA Taq, 0.4 mM dNTPs, 2.5 mM magnesium chloride, 50 nM T7 BcaBEST Sequencing primer (manufactured by Takara Shuzo) and 50 nM MUT B1 primer (manufactured by Takara Shuzo)] containing 1 ng of the plasmid pBS-2B8H. After 30 µl of the reaction solution was subjected to agarose gel electrophoresis, a PCR product of about 0.63 kb was recovered by using QIAquick Gel Extraction Kit (manufactured by QIAGEN) and made into 30 µl of an aqueous solution. Next, the obtained 0.44 kb PCR product and 0.63 kb PCR product were added at 0.5 µl to 47.5 µl of a reaction solution [LA PCR Buffer II (manufactured by Takara Shuzo), 0.4 mM dNTPs, and 2.5 mM magnesium chloride], and annealing of the DNA was carried out by heating the reaction solution at 90°C for 10 minutes, cooling it to 37°C spending 60 minutes and then keeping it at 37°C for 15 minutes by using a DNA thermal cycler GeneAmp PCR System 9600 (manufactured by Perkin Elmer). After the reaction at 72°C for 3 minutes by adding 2.5 units of TaKaRa LA Taq (manufactured by Takara Shuzo), 10 pmol each of T3 BcaBEST Sequencing primer (manufactured by Takara Shuzo) and T7 BcaBEST Sequencing primer (manufactured by Takara Shuzo) were added thereto and the reaction solution was adjusted to 50 µl to carry out 10 cycles, each cycle consisting of a reaction at 94°C for 30 seconds, at 55°C for 2 minutes and at 72°C for 1.5 minutes. After 25 µl of the reaction solution was purified by using QIA quick PCR purification kit (manufactured by QIAGEN), a half volume thereof was allowed to react at 37°C for 1 hour by using 10 units of a restriction enzyme *Kpn*I (manufactured by Takara Shuzo) and 10 units of a restriction enzyme *Sac*I (manufactured by Takara Shuzo). A *Kpn*I-*Sac*I fragment of about 0.59 kb was recovered by fractionating the reaction solution using agarose gel electrophoresis.

Next, 1 µg of pBluescript II SK(-) (manufactured by Stratagene) was allowed to react at 37°C for 1 hour using 10 units of a restriction enzyme *Kpn*I (manufactured by Takara Shuzo) and 10 units of a restriction enzyme *Sac*I (manufactured by Takara Shuzo), and then the reaction solution was subjected to agarose gel electrophoresis to recover a *Kpn*I-*Sac*I fragment of about 2.9 kb.

The obtained PCR product-derived *Kpn*I-*Sac*I fragment and plasmid pBluescript II SK(-)-derived *Kpn*I-*Sac*I fragment were ligated by using Solution I of DNA Ligation Kit Ver. 2 (manufactured by Takara Shuzo) according to the manufacture's instructions. Using the recombinant plasmid DNA solution obtained in this manner, *Escherichia coli* DH5α (manufactured by TOYOBO) was transformed, each plasmid DNA was prepared from the transformant clones and allowed to react by using BigDye Terminator Cycle Sequencing Ready Reaction Kit v2.0 (manufactured by Applied Biosystems) according to the manufacture's instructions, and then the nucleotide sequence was analyzed by the DNA sequencer ABI PRISM 377 manufactured by the same company.

In this manner, the plasmid pBS-2B8Hm shown in Fig. 20 having the nucleotide sequence of interest was obtained.

(3) Construction of anti-CD20 human chimeric antibody expression vector

Using a vector pKANTEX93 for humanized antibody expression [*Mol. Immunol*., 37, 1035 (2000)] and the plasmids pBS-2B8L and pBS-2B8Hm obtained in the item 1(1) and (2) of Example 1, an anti-CD20 human chimeric antibody (hereinafter referred to as "anti-CD20 chimeric antibody") expression vector pKANTEX2B8P was constructed as follows.

After 2 µg of the plasmid pBS-2B8L obtained in the item 1(1) of Example 9 was digested at 55°C for 1 hour by using 10 units of a restriction enzyme *Bsi*WI (manufactured by New England Biolabs) and then further allowed to react at 37°C for 1 hour by using 10 units of a restriction enzyme *Eco*RI (manufactured by Takara Shuzo). By fractionating the reaction solution by using an agarose gel electrophoresis, a *Bsi*WI-*Eco*RI fragment of about 0.41 kb was recovered.

Next, 2 µg of the vector pKANTEX93 for humanized antibody expression was digested at 55°C for 1 hour by using 10 units of a restriction enzyme *Bsi*WI (manufactured by New England Biolabs) and then further allowed to react at 37°C for 1 hour by using 10 units of a restriction enzyme *Eco*RI (manufactured by Takara Shuzo). *A Bsi*WI-*Eco*RI fragment of about 12.75 kb was recovered by fractionating the reaction solution using agarose gel electrophoresis.

Next, the obtained plasmid pBS-2B8L-derived *Bsi*WI-*Eco*RI fragment and plasmid pKANTEX93-derived *Bsi*WI-*Eco*RI fragment were ligated by using the Solution I of DNA Ligation Kit Ver. 2 (manufactured by Takara Shuzo) according to the manufacture's instructions. Using the recombinant plasmid DNA solution obtained in this manner, *Escherichia coli* DH5α (manufactured by TOYOBO) was transformed to obtain the plasmid pKANTEX2B8-L shown in Fig. 21.

Next, 2 µg of the plasmid pBS-2B8Hm obtained in Example 1-1(2) was digested at 37°C for 1 hour by using

10 units of a restriction enzyme *Apa*I (manufactured by Takara Shuzo) and then further allowed to react at 37°C for 1 hour by using 10 units of a restriction enzyme *Not*I (manufactured by Takara Shuzo). An *Apa*I-*Not*I fragment of about 0.45 kb was recovered by fractionating the reaction solution using agarose gel electrophoresis.

Next, 3 μg of the plasmid pKANTEX2B8-L was digested at 37°C for 1 hour using 10 units of a restriction enzyme *Apa*I (manufactured by Takara Shuzo) and then further allowed to react at 37°C for 1 hour using 10 units of a restriction enzyme *Not*I (manufactured by Takara Shuzo). An *Apa*I-*Not*I fragment of about 13.16 kb was recovered by fractionating the reaction solution using agarose gel electrophoresis.

Next, the obtained plasmid pBS-2B8Hm-derived *Apa*I-*Not*I fragment and plasmid pKANTEX2B8-L-derived *Apa*I-*Not*I fragment were ligated using the Solution I of DNA Ligation Kit Ver. 2 (manufactured by Takara Shuzo) according to the manufacture's instructions. Using the recombinant plasmid DNA solution obtained in this manner, *Escherichia coli* DH5α (manufactured by TOYOBO) was transformed, and each plasmid DNA was prepared from the transformant clones.

When the nucleotide sequence of the obtained plasmid was analyzed using BigDye Terminator Cycle Sequencing Ready Reaction Kit v2.0 (manufactured by Applied Biosystems) and the DNA sequencer 377 of the same company, it was confirmed that the plasmid pKANTEX2B8P shown in Fig. 21 into which the DNA of interest had been cloned was obtained.

2. Stable expression of anti-CD20 chimeric antibody using animal cells

(1) Preparation of antibody-producing cells using lectin-resistant CHO cell

As the lectin-resistant CHO cell, the clone CHO-LCA produced in the item (1) of Example 5 was used. Into $4\times10^6$ cells of the clone CHO-LCA, 4 μg of the anti-CD20 human chimeric antibody expression vector pKANTEX2B8P was introduced by electroporation [*Cytotechnology*, 3, 133 (1990)], and the cells were suspended in 10 ml of IMDM-dFBS(10)-HT(1) medium [IMDM medium (manufactured by Invitrogen) containing 10% dFBS (manufactured by Invitrogen) and HT supplement (manufactured by Invitrogen) at 1 × concentration] and dispensed at 100 μl/well into a 96 well microtiter plate (manufactured by Sumitomo Bakelite). After culturing at 37°C for 24 hours in a 5% $CO_2$ incubator, the medium was changed to IMDM-dFBS(10) medium (IMDM medium containing 10% dialized FBS), followed by culturing for 1 to 2 weeks. Since colonies of transformants showing HT-independent growth were found, regarding transformants in wells where growth was recognized, a produced amount of an antibody was increased by using a *dhfr* gene amplification system. Specifically, they were suspended to give a density of 1 to $2\times10^5$ cells/ml in IMDM-FBS (10) medium containing 50 nM MTX, and the suspension was dispensed at 1 ml/well into a 24 well plate (manufactured by Greiner). After culturing at 37°C for 1 to 2 weeks in a 5% $CO_2$ incubator, transformants showing 50 nM MTX resistance were induced. Regarding transformants in wells where growth was recognized, the MTX concentration was increased to 200 nM by a method similar to the above to finally obtain a transformant which can grow in IMDM-FBS(10) medium containing 200 nM MTX and can highly produce an anti-CD20 human chimeric antibody. Also, the production amounts of human IgG antibodies in culture supernatants were measured by the ELISA described in the item 2(2) of Example 9. The thus obtained LCA lectin-resistant VH0/DG44 transformant cell clone which produces an anti-CD20 chimeric antibody is named clone R92-3-1. The clone R92-3-1 has been deposited on March 26, 2002, as FERM BP-7976 in International Patent Organism Depositary, National Institute of Advanced Industrial Science and Technology (AIST Tsukuba Central 6, 1-1, Higashi 1-Chome Tsukuba-shi, Ibaraki-ken 305-8566 Japan).

(2) Measurement of human IgG antibody concentration in culture supernatant (ELISA)

A goat anti-human IgG (H & L) antibody (manufactured by American Qualex) was diluted with phosphate buffered saline (hereinafter referred to as "PBS") to give a concentration of 1 μg/ml, dispensed at 50 μl/well into a 96 well plate for ELISA (manufactured by Greiner) and then allowed to stand at 4°C overnight for immobilization. After washing with PBS, 1% bovine serum albumin (hereinafter referred to as "BSA"; manufactured by AMPC)-containing PBS (hereinafter referred to as "1% BSA-PBS") was added thereto at 100 μl/well and allowed to react at room temperature for 1 hour to block the remaining active residues. After discarding 1% BSA-PBS, culture supernatant of a transformant and variously diluted solutions of a purified human chimeric antibody were added thereto at 50 μl/well and allowed to react at room temperature for 2 hours. After the reaction, each well was washed with 0.05% Tween 20-containing PBS (hereinafter referred to as "Tween-PBS"), and then, as a secondary antibody solution, a peroxidase-labeled goat anti-human IgG (H & L) antibody solution (manufactured by American Qualex) diluted 3,000-folds with 1% BSA-PBS was added thereto at 50 μl/well and allowed to react at room temperature for 1 hour. After the reaction and subsequent washing with Tween-PBS, an ABTS substrate solution [a solution prepared by dissolving 0.55 g of 2,2'-azino-bis(3-ethylbenzothiazoline-6-sulfonic acid)ammonium in 1 liter of 0.1 M citrate buffer (pH 4.2), and adding 1 μl/ml hydrogen peroxide just before use] was dispensed at 50 μl/well for coloration and the absorbance at 415 nm

(hereinafter referred to as $OD_{415}$) was measured.

(3) Purification of an anti-CD20 chimeric antibody from culture supernatant

The transformant cell clone R92-3-1 which expresses the anti-CD20 chimeric antibody, obtained in the item 2(1) of Example 9, was suspended in IMDM-FBS(10) containing 200 nM MTX, and culturing was carried out until the cell density reached confluent. After washing with Dulbecco's PBS (manufactured by Invitrogen), the medium was changed to EX-CELL301 (manufactured by JRH). After culturing at 37°C for 7 days in a 5% $CO_2$ incubator, the culture supernatant was recovered. The anti-CD20 chimeric antibody was purified. The obtained antibody was named R92-3-1 antibody.

Example 10

Evaluation of activity of an anti-CD20 chimeric antibody:

1. Binding activity of anti-CD20 chimeric antibody on CD20-expressing cell (immunofluorescence technique)

Binding activity of the purified CD20 chimeric antibody obtained in the item 2(3) of Example 9 was evaluated by a immunofluorescence technique. A human lymphoma cell line Raji cell (JCRB 9012) which was a CD20-positive cell was dispensed at $2\times10^5$ cells into a 96 well U-shape plate (manufactured by Falcon). An antibody solution (a concentration of 0.039 to 40 µg/ml) prepared by diluting the anti-CD20 chimeric antibody with an FACS buffer (1% BSA-PBS, 0.02% EDTA, 0.05% $NaN_3$) was added thereto at 50 µl/well and allowed to react on ice for 30 minutes. After washing twice with the FACS buffer at 200 µl/well, a solution prepared by diluting a PE-labeled anti-human IgG antibody (manufactured by Coulter) 100-folds with FACS buffer was added thereto at 50 µl/well. After the reaction on ice under a shade for 30 minutes, the well were washed three times at 200 µl/well, the cells were finally suspended in 500 µl to measure the fluorescence intensity by a flow cytometer. The results are shown in Fig. 22. Increase in the fluorescence intensity depending on the antibody concentration was found in both the R92-3-1 antibody and Rituxan™, and it was confirmed that they show almost the same binding activity. Also, the binding activity to a CD20-negative cell, human CCRF-CEM cell (ATCC CCL 119), was tested by a method similar to the above at the antibody concentration of 40 µg/ml. The results are shown in Fig. 23. Since both of the R92-3-1 antibody and Rituxan™ did not bind thereto, it was suggested that the R92-3-1 antibody specifically binds to CD20.

2. *In vitro* cytotoxic activity (ADCC activity) of anti-CD20 chimeric antibody

In order to evaluate *in vitro* cytotoxic activity of the five kinds of the purified anti-CD20 chimeric antibodies obtained in the item 2(3) of Example 9, the ADCC activity was measured in accordance with the following method.

(1) Preparation of target cell solution

A human B lymphocyte cultured cell line WIL2-S cell (ATCC CRL8885), Ramos cell (ATCC CRL1596) or Raji cell (JCRB9012) cultured in RPMI1640-FCS(10) medium [RPMI1640 medium containing 10% FCS (manufactured by GIBCO BRL)] were washed with RPMI1640-FCS(5) medium [RPIM1640 medium containing 5% FCS (manufactured by GIBCO BRL)] by centrifugation and suspension, and prepared to give a density of $2\times10^5$ cells/ml with RPMI1640-FCS(5) medium as the target cell solution.

(2) Preparation of effector cell solution

From a healthy donor, 50 ml of venous blood was collected, and gently mixed with 0.5 ml of heparin sodium (manufactured by Shimizu Pharmaceutical). The mixture was centrifuged to isolate a mononuclear cell layer using Lymphoprep (manufactured by AXIS SHIELD) in accordance with the manufacture's instructions. After washing with the RPMI1640-FBS(10) medium by centrifugation three times, the resulting precipitate was re-suspended to give a density of $2\times10^6$ cells/ml using the medium and used as the effector cell solution.

(3) Measurement of ADCC activity

Into each well of a 96 well U-shaped bottom plate (manufactured by Falcon), 50 µl of the target cell solution prepared in the item (1) ($1 \times 10^4$ cells/well) was dispensed. Next, 50 µl of the effector cell solution prepared in the item (2) was added thereto ($2 \times 10^5$ cells/well, the ratio of effector cells to target cells becomes 20:1). Subsequently, each

of the anti-CD20 chimeric antibodies was added to give a final concentration from 0.3 to 3000 μg/ml, followed by reaction at 37°C for 4 hours. After the reaction, the plate was centrifuged, and the lactic acid dehydrogenase (LDH) activity in the supernatant was measured by obtaining absorbance data using CytoTox96 NonRadioactive Cytotoxicity Assay (manufactured by Promega) according to the manufacture's instructions. Absorbance data at spontaneously release from target cells were obtained by using the medium alone without using the effector cell solution and the antibody solution, and absorbance data at spontaneously release from effector cells were obtained by using the medium alone without using the target cell solution and the antibody solution, in the sama manner as the above. Regarding absorbance data of the total released target cells, the above procedures were carried out by using the medium alone without using the antibody solution and the effector cell solution, adding 15 μL of 9% Triton X-100 solution 45 minutes before completion of the reaction, and measuring the LDH activity of the supernatant. The ADCC activity was carried out by the following equation:

$$\text{Cytotoxic activity (\%)} = \frac{\left[\begin{array}{c}\text{Absorbance of}\\\text{the sample}\end{array}\right] - \left[\begin{array}{c}\text{Absorbance at}\\\text{spontanously}\\\text{release from}\\\text{effector cells}\end{array}\right] - \left[\begin{array}{c}\text{Absorbance at}\\\text{spontanously}\\\text{release from}\\\text{target cells}\end{array}\right]}{\left[\begin{array}{c}\text{Absorbance}\\\text{at total}\\\text{release from}\\\text{target cells}\end{array}\right] - \left[\begin{array}{c}\text{Absorbance at}\\\text{spontanously}\\\text{release from}\\\text{target cells}\end{array}\right]} \times 100$$

Fig. 24 shows results in which the three clones were used as the target. Fig. 24A, Fig. 24B and Fig. 25C show the results using Raji cell (JCRB9012), Ramos cell (ATCC CRL1596) and WIL2-S cell (ATCC CRL8885), respectively. As shown in Fig. 24, the R92-3-1 antibody has higher ADCC activity than Rituxan™ at all antibody concentrations, and has the highest maximum cytotoxic activity value.

Example 11

Sugar chain analysis of an anti-CD20 chimeric antibody

Sugar chains of the anti-CD20 antibodies purified in the item 2(3) of Example 9 were analyzed. The sugar chains were cleaved from the proteins by subjecting the R92-3-1 antibody and Rituxan™ to hydrazinolysis [*Method of Enzymology,* 83, 263 (1982)]. After removing hydrazine by evaporation under a reduced pressure, *N*-acetylation was carried out by adding an aqueous ammonium acetate solution and acetic anhydride. After freeze-drying, fluorescence labeling by 2-aminopyridine was carrying out [*Journal of Biochemistry*, 95, 197 (1984)]. A fluorescence-labeled sugar chain group (PA-treated sugar chain group) was separated from excess reagents using Superdex Peptide HR 10/30 column (manufactured by Pharmacia). The sugar chain fractions were dried using a centrifugation concentrator and used as a purified PA-treated sugar chain group. Next, the purified PA-treated sugar chain group was subjected to reverse phase HPLC analysis using a CLC-ODS column (manufactured by Shimadzu).

Fig. 25 shows elution patterns obtained by carrying out reverse phase HPLC analysis of each of PA-treated sugar chains prepared from the anti-CD20 chimeric antibodies. Fig. 25A and Fig. 25B show elution patterns of the R92-3-1 antibody and Rituxan™, respectively. The analysis was carried out by gradient similar to that in Example 3.

Peaks ① to ⑧ shown in Fig. 25 show the structures (1) to (8), respectively, shown in Example 3.

GlcNAc, Gal, Man, Fuc and PA indicate *N*-acetylglucosamine, galactose, mannose, fucose and a pyridylamino group, respectively. In Fig. 25, the ratio of the α1,6-fucose-free sugar chain group was calculated from the area occupied by the peaks ① to ④ among ① to ⑧, and the ratio of the α1,6-fucose-bound sugar chain group from the area occupied by the peaks ⑤ to ⑧ among ① to ⑧.

As a result, in Rituxan™, the sugar chain content of α1,6-fucose-free sugar chains was 6%, and the sugar chain content of α1,6-fucose-bound sugar chains was 94%. In the R92-3-1 antibody, the sugar chain content of α1,6-fucose-free sugar chains was 33%, and the sugar chain content of α1,6-fucose-bound sugar chains was 67%. Based on these results, it was found that the sugar chain content of α1,6-fucose-free sugar chains in the R92-3-1 antibody is higher than that of Rituxan™.

Example 12

Preparation of lectin-resistant CHO/DG44 cell by introduction of siRNA expression plasmid targeting FUT8 and preparation of antibody composition using the cell

1. Construction of siRNA expression plasmids U6_FUT8_B_puro and U6_FUT8_R_puro targeting FUT8

(1) Selection of nucleotide sequence of CHO-derived FUT8 gene as target ofRNAi

According to the description of WO00/61739, the nucleotide sequence of a Chinese hamster-derived FUT8 cDNA was determined from a single-strand cDNA prepared from CHO/DG44 cells by the following method. A polymerase chain reaction (PCR) was carried out by using a primer specific for the 5'-terminal of the untranslated region (SEQ ID NO:61) and another primer specific for the 3'-terminal of the untranslated region (SEQ ID NO:62) designed from the cDNA sequence of mouse FUT8 (GenBank No. AB025198) to amplify the full length Chinese hamster-derived FUT8 cDNA gene. In the PCR, 25 μL of a reaction solution containing 1 μL of the above single-strand cDNA obtained from CHO/DG44 cell [1×EX Taq Buffer (manufactured by Takara Shuzo), 0.2 mM dNTP's, 4% DMSO (manufactured by Nacalai Tesque), 0.5 unit of EX Taq polymerase (manufactured by Takara Shuzo), and 0.5 μM of each of the above primers (SEQ ID NOs:61 and 62 as mentioned above)] was prepared, followed by heating at 94°C for 5 minutes and 30 cycles of heating at 94°C for 30 seconds, 55°C for 30 seconds and 72°C for 2 minutes as one cycle by using GeneAmp PCR system 9700 (manufactured by Perkin Elmer). Furthermore, the heating was carried out at 72°C for 10 minutes.

The PCR solution was extracted with phenol/chloroform, and the PCR amplified fragment was purified by ethanol precipitation. The nucleotide sequence of the PCR amplified fragment was determined by using a DNA sequencer ABI PRISM377 (manufactured by Perkin Elmer) according to the conventional method. The nucleotide sequences of PCR amplified fragments (totally, 10 clones) were determined by carrying out the experiment independently, and the nucleotide sequence of Chinese hamster-derived PUT8 cDNA was determined after removal of the nucleotide mutation caused by PCR.

Based on the nucleotide sequence of Chinese hamster-derived FUT8 cDNA, the nucleotide sequences represented by SEQ ID NOs:63 and 64 were used as the target sequences in RNAi. Method for constructing an expression plasmid U6_FUT8_B_puro of the siRNA molecule having SEQ ID NO:63 and an expression plasmid U6_FUT8_R_puro of the siRNA molecule having SEQ ID NO:64 are described below. The basic structure of the siRNA expression plasmid was designed according to the method of Miyagishi [*Nature Biotechnology*, 20, 5 (2002)].

(2) Construction of plasmid U6_pre_sense

Plasmid U6_pre_sense was constructed according to the following method (Fig. 26). A polymerase chain reaction (PCR) was carried out by using primers (SEQ ID NOs:65 and 66) designed from the gene sequence of human U6 snRNP registered in GenBank (GenBank Nos. X07425 and M14486) to amplify the promoter region of the human U6 snRNP gene. In the PCR, 50 μL of a reaction solution containing 200 ng of Human Genomic DNA (manufactured by Clontech) [1×EX Taq Buffer (manufactured by Takara Shuzo), 0.2 mM dNTP's, 2.5 unit of EX Taq polymerase (manufactured by Takara Shuzo), and 0.5 μM each of the above primers (SEQ ID NOs:65 and 66 as described above)] was prepared, followed by heating at 94°C for 5 minutes and 30 cycles of heating at 94°C for 1 minute and 68°C for 2 minutes as one cycle by using GeneAmp PCR system 9700 (manufactured by Perkin Elmer).

The PCR solution was extracted with phenol/chloroform, and the PCR amplified fragment was recovered by ethanol precipitation. The amplified fragment was digested with *Xba*I (manufactured by Takara Shuzo), extracted with phenol/chloroform, and subjected to ethanol precipitation to recover a DNA fragment. The DNA fragment was then digested with *Bam*HI (manufactured by Takara Shuzo), and the reaction mixture was subjected to agarose gel electrophoresis. The DNA fragment of about 300 bp was purified by Gel Extraction Kit (manufactured by QIAGEN). The recovered DNA fragment was linked with pBluescript SK(-) vector (STRATAGENE) which had been digested in advance with *Xba*I (manufactured by Takara Shuzo) and *Bam*HI (manufactured by Takara Shuzo) by using DNA Ligation Kit (manufactured by Takara Shuzo). Using the resulting recombinant plasmid DNA, *Escherichia coli* DH5α strain (manufactured by Toyobo) was transformed according to the method of Cohen *et al.* [*Proc. Natl. Acad Sci. USA.,* 69, 2110 (1972)] (hereinafter, this method was used in transformation of *Escherichia coli*). A recombinant plasmid DNA was isolated from the resulting multiple ampicillin resistant colonies by using QIAprep Spin Miniprep Kit (manufactured by QIAGEN). The nucleotide sequence of U6 promoter contained in the plasmid was determined by using DNA sequencer ABI PRISM 377 (manufactured by Perkin Elmer) according to the conventional method. The plasmid in which no mutation was occurred in the nucleotides during the PCR was selected and named U6_pre_sense.

(3) Construction of plasmid pBS_BglII

Plasmid pBS_BglII was constructed according to the following method (Fig. 27). In distilled water, 10 pmol of syntheticoligo DNAs (each phosphorylated at the 5' terminals) represented by SEQ ID NOs:67 and 68 was dissolved, followed by heating at 90°C for 10 minutes, and the mixture was allowed to stand to room temperature for annealing. The annealed syntheticoligomer (0.2 pmol) isolated from the reaction solution was linked with pBluescript SK(-) vector (manufactured by STRATAGENE) which had been digested in advance with *Sac*I (manufactured by Takara Shuzo) by using DNA Ligation Kit (manufactured by Takara Shuzo). *Escherichia coli* DH5α strain (manufactured by Toyobo) was transformed with the resulting recombinant plasmid DNA. The recombinant plasmid DNA was isolated from the resulting multiple ampicillin-resistant colonies by using QIAprep Spin Miniprep Kit (manufactured by QIAGEN). The plasmid which was digested with *Bgl*II (manufactured by Takara Shuzo) was selected from the respective clones and named pBS_BglII.

(4) Construction of plasmid U6_pre_antisense

Plasmid U6_pre_antisense was constructed according to the following method (Fig. 28). A polymerase chain reaction (PCR) was carried out by using primers (SEQ ID NOs:69 and 70) designed from the gene sequence of human U6 snRNP registered in GenBank (GenBank Accession Nos. X07425 and M14486) to amplify the promoter region of the human U6 snRNP gene. In the PCR, 50 μL of a reaction solution containing 200 ng of Human Genomic DNA (manufactured by Clontech) [1×EX Taq Buffer (manufactured by Takara Shuzo), 0.2 mM dNTP's, 2.5 unit of EX Taq polymerase (manufactured by Takara Shuzo), and 0.5 μM each of the above primers (SEQ ID NOs:69 and 70 as described above)] was prepared, followed by heating at 94°C for 5 minutes and then 30 cycles of heating at 94°C for 1 minute and 68°C for 2 minutes as one cycle by using GeneAmp PCR system 9700 (manufactured by Perkin Elmer),.

The PCR solution was extracted with phenol/chloroform, and the PCR amplified fragment was recovered by ethanol precipitation. The amplified fragment was digested with *Bam*HI (manufactured by Takara Shuzo), extracted with phenol/chloroform, and subjected to ethanol precipitation to recover a DNA fragment. The DNA fragment was then digested with *Eco*RI (manufactured by Takara Shuzo), and the reaction mixture was subjected to agarose gel electrophoresis. The DNA fragment of about 300 bp was purified by using Gel Extraction Kit (manufactured by QIAGEN). The recovered DNA fragment was linked with plasmid pBS_BglII (manufactured by Takara Shuzo) which had been digested in advance with *Bam*HI (manufactured by Takara Shuzo) and *Eco*RI (manufactured by Takara Shuzo) by using DNA Ligation Kit (manufactured by Takara Shuzo). *Escherichia coli* DH5α strain (manufactured by Toyobo) was transformed with the resulting recombinant plasmid DNA. The recombinant plasmid DNA was isolated from the resulting multiple ampicillin resistant colonies by using a QIAprep Spin Miniprep Kit (manufactured by QIAGEN). The nucleotide sequence of U6 promoter contained in the plasmid was determined by using DNA sequencer ABI PRISM 377 (manufactured by Perkin Elmer) according to the conventional method. The plasmid in which no mutation was occurred in the nucleotides during the PCR was selected from the determined clones and named U6_pre_antisense.

(5) Construction of a plasmid U6_sense_B

Plasmid U6_sense_B was constructed according to the following method (Fig. 29). In distilled water, 10 pmol of each of syntheticoligo DNAs (each phosphorylated at the 5' terminal) represented by SEQ ID NOs:71 and 72 was dissolved, followed by heating at 90°C for 10 minutes, and the mixture was allowed to stand to room temperature for annealing. The annealed syntheticoligomer (0.2 pmol) isolated from the reaction solution was linked with a plasmid U6_pre_sense which had been digested in advance with *Pma*CI (manufactured by Takara Shuzo) and *Bam*HI (manufactured by Takara Shuzo) by using DNA Ligation Kit (manufactured by Takara Shuzo). *Escherichia coli* DH5α strain (manufactured by Toyobo) was transformed with the resulting recombinant plasmid DNA. A recombinant plasmid DNA was isolated from the resulting multiple ampicillin resistant colonies by using QIAprep Spin Miniprep Kit (manufactured by QIAGEN). The nucleotide sequence derived from the syntheticoligomer contained in the plasmid was determined by using DNA sequencer ABI PRISM 377 (manufactured by Perkin Elmer) according to the conventional method. The plasmid into which the nucleotide sequences represented by SEQ ID NOs: 71 and 72 were correctly introduced was selected from the determined clones and named U6_sense_B.

(6) Construction of a plasmid U6_sense_R

Plasmid U6_sense_R was constructed according to the following method (Fig. 30). In distilled water, 10 pmol of each of syntheticoligo DNAs (each phosphorylated at the 5' terminals) represented by SEQ ID NOs:73 and 74 was dissolved, followed by heating at 90°C for 10 minutes, and the mixture was allowed to stand to room temperature for annealing. The annealed syntheticoligomer (0.2 pmol) isolated from the reaction solution was linked with plasmid

U6_pre_sense which had been digested in advance with *Pma*CI (manufactured by Takara Shuzo) and *Bam*HI (manufactured by Takara Shuzo) by using DNA Ligation Kit (manufactured by Takara Shuzo). *Escherichia coli* DH5α strain (manufactured by Toyobo) was transformed with the resulting recombinant plasmid DNA. The recombinant plasmid DNA was isolated from the resulting multiple ampicillin resistant colonies by using QIAprep Spin Miniprep Kit (manufactured by QIAGEN). The nucleotide sequence derived from the syntheticoligomer contained in the plasmid was determined by using a DNA sequencer ABI PRISM 377 (manufactured by Perkin Elmer) according to the conventional method. The plasmid into which the nucleotide sequences represented by SEQ ID NOs: 73 and 74 were correctly introduced was selected from the determined clones and designated as U6_sense_R.

(7) Construction of plasmid U6_antisense_B

Plasmid U6_antisense_B was constructed according to the following method (Fig. 31). In distilled water, 10 pmol of each of syntheticoligo DNAs (each phosphorylated at the 5' terminals) represented by SEQ ID NOs:75 and 76 was dissolved, followed by heating at 90°C for 10 minutes, and the mixture was allowed to cool to room temperature for annealing. The annealed syntheticoligomer (0.2 pmol) isolated from the reaction solution was linked with plasmid U6_pre_antisense which had been digested in advance with *Pma*CI (manufactured by Takara Shuzo) and *Eco*RI (manufactured by Takara Shuzo) by using DNA Ligation Kit (manufactured by Takara Shuzo). *Escherichia coli* DH5α strain (manufactured by Toyobo) was transformed with the resulting recombinant plasmid DNA. The recombinant plasmid DNA was isolated from the resulting multiple ampicillin resistant colonies by using QIAprep Spin Miniprep Kit (manufactured by QIAGEN). The nucleotide sequence derived from the syntheticoligomer contained in the plasmid was determined by using DNA sequencer ABI PRISM 377 (manufactured by Perkin Elmer) according to the conventional method. The plasmid into which the nucleotide sequences represented by SEQ ID NOs:75 and 76 were correctly introduced was selected from the determined clones and named U6_antisense_B.

(8) Construction of plasmid U6_antisense_R

Plasmid U6_antisense_R was constructed according to the following method (Fig. 32). In distilled water, 10 pmol of each of syntheticoligo DNAs (each phosphorylated at the 5' terminal) represented by SEQ ID NOs:77 and 78 was dissolved in distilled water, followed by heating at 90°C for 10 minutes, and the mixture was allowed to stand to room temperature for annealing. The annealed syntheticoligomer (0.2 pmol) isolated from the reaction mixture was linked with plasmid U6_pre_antisense which had been digested in advance with *Pma*CI (manufactured by Takara Shuzo) and *Eco*RI (manufactured by Takara Shuzo) by using DNA Ligation Kit (manufactured by Takara Shuzo). *Escherichia coli* DH5α strain (manufactured by Toyobo) was transformed with the resulting recombinant plasmid DNA. The recombinant plasmid DNA was isolated from the resulting multiple ampicillin resistant colonies by using QIAprep Spin Miniprep Kit (manufactured by QIAGEN). The nucleotide sequence derived from the syntheticoligomer contained in the plasmid was determined by using DNA sequencer ABI PRISM 377 (manufactured by Perkin Elmer) according to the conventional method. The plasmid into which the nucleotide sequences represented by SEQ ID NOs: 77 and 78 were correctly introduced was selected from the determined clones and named U6_antisense_R.

(9) Construction of plasmid U6_FUT8_B

Plasmid U6_FUT8_B was constructed according to the following method (Fig. 33). Plasmid U6_antisense_B was digested with *Sal*I (manufactured by Takara Shuzo) and extracted with phenol/chloroform. The DNA fragment was recovered by ethanol precipitation and then digested with *Bgl*II (manufactured by Takara Shuzo). The reaction solution was subjected to agarose gel electrophoresis and the DNA fragment of about 370 bp was purified by using Gel Extraction Kit (manufactured by QIAGEN). The recovered DNA fragment was linked with plasmid U6_sense_B which had been digested in advance with *Sal*I (manufactured by Takara Shuzo) and *Bam*HI (manufactured by Takara Shuzo) by using DNA Ligation Kit (manufactured by Takara Shuzo). *Escherichia coli* DH5α strain (manufactured by Toyobo) was transformed with the resulting recombinant plasmid DNA. The recombinant plasmid DNA was isolated from the resulting multiple ampicillin resistant colonies by using QIAprep Spin Miniprep Kit (manufactured by QIAGEN). The nucleotide sequence contained in the plasmid was determined by using DNA sequencer ABI PRISM 377 (manufactured by Perkin Elmer) according to the conventional method. The plasmid having the nucleotide sequence of interest was selected from the determined clones and named U6_FUT8_B.

(10) Construction of plasmid U6_FUT8_R

Plasmid U6_FUT8_R was constructed according to the following method (Fig. 34). Plasmid U6_antisense_R was digested with *Sal*I (manufactured by Takara Shuzo) and extracted with phenol/chloroform. The DNA fragment was

recovered by ethanol precipitation and then digested with *Bgl*II (manufactured by Takara Shuzo). The reaction solution was subjected to agarose gel electrophoresis and the DNA fragment of about 370 bp was purified by using Gel Extraction Kit (manufactured by QIAGEN). The recovered DNA fragment was linked with plasmid U6_sense_R which had been digested in advance with *Sal*I (manufactured by Takara Shuzo) and *Bam*HI (manufactured by Takara Shuzo) by using DNA Ligation Kit (manufactured by Takara Shuzo). *Escherichia coli* DH5α strain (manufactured by Toyobo) was transformed with the resulting recombinant plasmid DNA. The recombinant plasmid DNA was isolated from the resulting multiple ampicillin resistant colonies by using QIAprep Spin Miniprep Kit (manufactured by QIAGEN). The nucleotide sequence contained in the plasmid was determined by using a DNA sequencer ABI PRISM 377 (manufactured by Perkin Elmer) according to the conventional method. The plasmid having the nucleotide sequence of interest was selected from the determined clones and named U6_FUT8_R.

(11) Construction of plasmid U6_FUT8_B_puro

Plasmid U6_FUT8_B_puro was constructed according to the following method (Fig. 35). Plasmid U6_FUT8_B was digested with *Pvu*II (manufactured by Takara Shuzo) and the reaction solution was subjected to agarose gel electrophoresis. The DNA fragment of about 1150 bp was purified by using Gel Extraction Kit (manufactured by QIAGEN). The recovered DNA fragment was linked plasmid pPUR (manufactured by Clontech) which had been digested in advance with *Pvu*II (manufactured by Takara Shuzo) by using DNA Ligation Kit (manufactured by Takara Shuzo). *Escherichia coli* DH5α strain (manufactured by Toyobo) was transformed with the resulting recombinant plasmid DNA. The recombinant plasmid DNA was isolated from the resulting multiple ampicillin resistant colonies by using QIAprep Spin Miniprep Kit (manufactured by QIAGEN). The nucleotide sequence contained in the plasmid was determined by using DNA sequencer ABI PRISM 377 (manufactured by Perkin Elmer) according to the conventional method. The plasmid having the nucleotide sequence of interest was selected from the determined clones and named U6_FUT8_B_puro.

(12) Construction of plasmid U6_FUT8_R_puro

Plasmid U6_FUT8_R_puro was constructed according to the following method (Fig. 36). Plasmid U6_FUT8_R was digested with *Pvu*II (manufactured by Takara Shuzo) and the reaction solution was subjected to agarose gel electrophoresis. The DNA fragment of about 1150 bp was purified by using Gel Extraction Kit (manufactured by QIAGEN). The recovered DNA fragment was linked with plasmid pPUR (manufactured by Clontech) which had been digested in advance with *Pvu*II (manufactured by Takara Shuzo) by using DNA Ligation Kit (manufactured by Takara Shuzo). *Escherichia coli* DH5α strain (manufactured by Toyobo) was transformed with the resulting recombinant plasmid DNA. The recombinant plasmid DNA was isolated from the resulting multiple ampicillin resistant colonies by using QIAprep Spin Miniprep Kit (manufactured by QIAGEN). The nucleotide sequence contained in the plasmid was determined by using DNA sequencer ABI PRISM 377 (manufactured by Perkin Elmer) according to the conventional method. The plasmid having the nucleotide sequence of interest was selected from the determined clones and named U6_FUT8_R_puro.

(13) Preparation of linearized plasmids U6_FUT8_B_puro and U6_FUT8_R_puro

Plasmids U6_FUT8_B_puro and U6_FUT8_R_puro were digested with a restriction enzyme *Fsp*I (manufactured by NEW ENGLAND BIOLABS) for linearization. After the digestion, the reaction solution was subjected to agarose gel electrophoresis to confirm that the plasmids were correctly linearized.

2. Preparation of lectin-resistant clone into which FUT8 siRNA expression plasmid has been introduced

Subsequently, the siRNA expression plasmid constructed in the item 1 was introduced into the clone 32-05-09 or the clone 32-05-12 to obtain LCA, α1,6-fucose specific lectin-resistant clones. The clone 32-05-09 and the clone 32-05-12 are clones of CHO/DG44 cell producing an anti-CCR4 humanized antibody, which has been obtained according to the method as described in the item 1(2) of Reference Example 1.

The siRNA expression plasmid into the clone 32-05-09 or the clone 32-05-12 was introduced by electroporation [*Cytotechnology*, 3, 133 (1990)] according to the following method. First, the clone 32-05-09 and the clone 32-05-12 were suspended into a K-PBS buffer solution (137 mmol/L KCl, 2.7 mmol/L NaCl, 8.1 mmol/L $Na_2HPO_4$, 1.5 mmol/L $KH_2PO_4$, 4.0 mmol/L $MgCl_2$) at $8\times10^6$ cells/ml. The cell suspension (200 μl) ($1.8\times10^6$ cells) was mixed with 10 μg of the linearized plasmid U6_FUT8_B_puro or U6_FUT8_R_puro prepared in the item 1 of Example 12. The resulting cell/DNA mixture was moved into Gene Pulser Cuvette (2 mm in distance between the electrodes) (manufactured by BIO-RAD) and subjected to gene transduction at 0.35 KV of pulse voltage and 250 μF of electric capacity

on a cell fusion apparatus, Gene Pulser (manufactured by BIO-RAD).

The cell suspension was added to 30 ml of a basic culture medium [Iscove's Modified Dulbecco's Medium (manufactured by Life Technologies) supplemented with 10% fetal bovine dialyzed serum (manufactured by Life Technologies) and 50 μg/ml gentamicin (manufactured by Nacalai Tesque)] and then inoculated at 10 ml on a 10 cm dish for cell adhesion (manufactured by Iwaki Glass) and cultured in 5% $CO_2$ at 37°C for 24 hours. After removal of the culture medium, 10 ml of a basic medium supplemented with 12 μg/ml puromycin (manufactured by SIGMA) was added thereto and further cultured for 6 days. The culture medium was removed, and then 10 ml of a basic culture medium supplemented with 0.5 mg/ml LCA (manufactured by EY Labo.) and 12 μg/ml puromycin (manufactured by SIGMA) was added thereto. Incubation was continued for additional 8 days until colonies appeared.

Eight days thereafter, the formed lectin-resistant colonies were collected according to the following method. First, the supernatant was removed from the 10 cm dish, 7 ml of a phosphate buffer saline was then added thereto, and the dish was placed under a stereoscopic microscope. Then, the colony was scratched and sucked up with Pipetteman (manufactured by GILSON) and placed in a round-bottom 96 well plate (manufactured by Falcon). After treatment with trypsin, each clone was inoculated on a flat-bottom 96 well plate for adhesion cells (manufactured by Iwaki Glass) and cultured with a basic medium supplemented with 12 mg/ml puromycin (manufactured by SIGMA) for 1 week. After completion of the incubation, each clone cultured on the above plate was further cultured on a basic medium supplemented with 12 μg/ml puromycin (manufactured by SIGMA) for scale-up culture. Thus, lectin-resistant clone 9R-3 into which U6_FUT8_R_puro was introduced in the clone 32-05-09 and lectin-resistant clone 12B-5 in which U6_FUT8_B_puro was expressed in the clone 32-05-12 were obtained. These clones were adapted to the analyses as described in the items 3 and 4 described below and frozen vials in which $1\times10^7$ cells were suspended in 1 ml of 10% DMSO (manufactured by SIGMA)/FBS (manufactured by Life Technologies) per vial were prepared. The clone 9R-3 and the clone 12B-5 have been deposited in the names of CHO/9R-3 and CHO/12B-5, respectively, on March 4, 2003, as PERM BP-8311 and FERM BP-8312, respectively, in International Patent Organism Depositary, National Institute of Advanced Industrial Science and Technology (AIST Tsukuba Central 6, 1-1, Higashi 1-Chome Tsukuba-shi, Ibaraki-ken 305-8566 Japan).

3. Determination of FUT8 mRNA in lectin-resistant clone into which FUT8-targeting siRNA expression plasmid was introduced

The lectin-resistant clone 9R-3 or 12B-5 obtained in the item 2 was suspended in a basic culture medium supplemented with 12 μg/ml puromycin at a cell density of $3\times10^5$ cells/ml, inoculated in a T25 flask for adhesion cells (manufactured by Greiner), and cultured for 3 days. Each cell suspension was recovered by trypsin treatment and centrifuged at 3000 rpm at 4°C for 5 minutes to remove the supernatant. The cells were suspended in a PBS buffer (manufactured by GIBCO), again centrifuged at 3000 rpm at 4°C twice for 5 minutes, and frozen at -80°C. The parent clones 32-05-09 and 32-05-12 were also treated in the same manner by using the basic medium.

The cells were thawed at room temperature and total RNA was extracted by using RNAeasy (manufactured by QIAGEN) according to the attached manufacture's instruction. The total RNA was dissolved in 45 μl of sterilized water, 1 μl of RQ1 RNase-Free DNase (manufactured by Promega), 5 μl of the attached 10×DNase buffer, and 0.5 μl of RNasin Ribonuclease inhibitor (manufactured by Promega) were added thereto, and the mixture was allowed to react at 37°C for 30 minutes to decompose genomic DNA contaminated in the sample. After the reaction, the total RNA was purified again using RNAeasy (manufactured by QIAGEN) and dissolved in 40 μl of sterilized water.

For 3 μg of each of the obtained total RNAs, a reverse transcription reaction was carried out by using oligo (dT) as a primer in a 20 μl system with SUPERSCRIPT™ Preamplification System for First Strand cDNA Synthesis (manufactured by Life Technologies) according to the attached manufacture's instruction to synthesize a single-strand cDNA. In determining the amount of transcription of FUT8 gene or of β-actin gene, each of the reaction solution diluted 50-folds with water was used and stocked at -80°C before use. According to the method for determining the amount of transcription by competitive PCR as described in WO00/61739, a competitive PCR was carried out by using the total cDNA of each of the cell clones to determine the amount of FUT8 mRNA and β-actin mRNA in the total RNA of each of the cell clones. Considering that the amount of transcription of β-actin is even between different cells, the relative value of the amount of FUT8 mRNA was calculated for that of β-actin mRNA. Fig. 37 shows the results of the comparison of these values. Thus, it was found that the amount of FUT8 mRNA was reduced to 20% and 30% in the clone 9R-3 and the clone 12B-5, respectively, which were prepared by introduction of the FUT8-targeting siRNA expression plasmid, in comparison with that of the respective parent clones.

4. Preparation of antibody produced by lectin-resistant clone into which FUT8-targeting siRNA expression plasmid was transformed

An anti-CCR4 humanized antibody produced by the lectin-resistant clone obtained above was prepared according to the following method. First, the lectin-resistant clone 9R-3 or 12B-5 obtained in the item 2 was suspended in a basic culture medium supplemented with 12 μg/ml puromycin at a density of $3 \times 10^5$ cells/ml, and 30 ml of the mixture was inoculated in a T182 flask for culturing adhesion cell (manufactured by Greiner) and cultured to become 100% confluent. The whole amount of the culture medium was removed in each clone, and the same amount of PBS (manufactured by GIBCO) was added and removed again to replace with 30 ml of EXCELL301 (manufactured by JRH Biosciences). After culturing for further 7 days, each of the cell suspensions was recovered. The suspension was centrifuged at 3000 rpm and at 4°C for 10 minutes to recover the supernatant, followed by filtration through PES Membrane of 0.22 mm pore size (manufactured by Asahi Technoglass).

In a column of 0.8 cm diameter, 0.5 ml of Mab Select (manufactured by Amersham Pharmacia Biotech) was packed, and 3.0 ml of purified water and 3.0 ml of 0.2 mol/L borate-0.15 mol/L NaCl buffer (pH 7.5) were successively passed in the column. The column was further washed successively with 2.0 ml of 0.1 mol/L citrate buffer (pH 3.5) and 1.5 ml of 0.2 mol/L borate-0.15 mol/L NaCl buffer (pH 7.5) to equilibrate the carrier. Then, the supernatant (30 ml) of the above cultured medium was applied to the column, and then the column was washed with 3.0 ml of 0.2 mol/L borate-0.15 mol/L NaCl buffer (pH 7.5). After washing, the antibody adsorbed on the column was eluted with 1.25 ml of 0.1 mol/L citrate buffer (pH 3.5). A fraction of 250 μl first eluted was discarded, and the second fraction (1 ml) was collected and neutralized with 200 ml of 2 mol/L Tris-HCl (pH 8.5). The recovered eluate was dialyzed in 10 mol/L citrate-0.15 mol/L NaCl buffer (pH 6.0) at 4°C overnight. After the dialysis, the antibody solution was recovered and subjected to sterile filtration by using Millex GV (manufactured by MILLIPORE).

5. Monosaccharide composition analysis in antibody composition produced by lectin-resistant clone into which FUT8-targeting siRNA expression plasmid was introduced

For the anti-CCR4 humanized antibody purified in the item 4, the monosaccharide composition was analyzed according to a known method [*Journal of Liquid Chromatography,* 6, 1577 (1983)]. Table 5 shows the ratio of fucose-free complex sugar chains in the total complex sugar chains, calculated from the monosaccharide composition ratio contained in each of the antibodies. The results show that the ratios of the fucose-free sugar chains in the antibodies produced by the parent clones 32-05-09 and 32-05-12 used in the siRNA introduction were 8% and 12%, respectively, whereas those in the siRNA-introduced lectin-resistant stains 9R-3 and 12B-5 were markedly increased up to 56% and 65%, respectively.

From the above results, it was shown that the introduction with siRNA targeting-FUT8 can control the content of α1,6-fucose in the antibody produced by the host cell.

Table 5

Ratio of fucose-free sugar chains of antibody produced by each clone

| Clone | Ratio of fucose-free sugar chain |
|---|---|
| 32-05-09 | 8% |
| 9R-3 | 56% |
| 32-05-12 | 6% |
| 12B-5 | 65% |

Example 13

Preparation of lectin-resistant SP2/0 cell and production of anti-GM2 chimeric antibody using the cell:

1. Preparation of lectin-resistant SP2/0 clone

Transformant clone (KM968) from mouse myeloma SP2/0 cell (ATCC CRL-1581) producing an anti-GM2 chimeric antibody as described in Japanese Published Unexamined Patent Application No. 205694/94 was cultured on an RPMI1640 medium (manufactured by Invitrogen) containing 10 v/v% fetal bovine serum (manufactured by JRH Biosciences), 500 μg/mL G418 and 500 nM MTX (hereinafter referred to as "RPMI-FBS(10) medium") in a 25 $cm^2$ flask for suspension culture (manufactured by Iwaki Glass), and multiplied just before confluent state. Into this culture,

RPMI-FBS(10) medium was suspended to give a cell density of 1×10[5] cells/ml, and then 0.1 μg/ml of an alkylating agent, *N*-methyl-N'-nitro-*N*-nitrosoguanidine (hereinafter referred to as "MNNG"; manufactured by Sigma) was added thereto. The mixture was allowed to stand at 37°C in a $CO_2$ incubator (manufactured by Tabai ESPEC) and then inoculated on a 96-well plate for adhesion culture (manufactured by Greiner) at a density of $1\times10^4$ cells/well. *Lens culinaris* agglutinin (hereinafter referred to as "LCA"; manufactured by Vector Corp.) was added to each well at a final concentration of 2 mg/ml in the medium. The mixture was cultured at 37°C in a $CO_2$ incubator for 2 weeks, and the formed lectin-resistant colony was recovered as lectin-resistant clone SP2/0n. The clone was hereinafter named clone SP2/0/GM2-LCA. The clone SP2/0/GM2-LCA has been deposited in the names of SP2/0/GM2-LCA on March 4, 2003, as FERM BP-8313 in International Patent Organism Depositary, National Institute of Advanced Industrial Science and Technology (AIST Tsukuba Central 6, 1-1, Higashi 1-Chome Tsukuba-shi, Ibaraki-ken 305-8566 Japan).

2. Culturing of lectin-resistant SP2/0 clone and purification of antibody

The transformant cell SP2/0/GM2-LCA clone producing an anti-GM2 chimeric antibody obtained in the item 1 of Example 13 or clone KM968 of the parent clone was suspended into Hybridoma-SFM medium containing 5% Ultra-Low IgG fetal bovine serum (manufactured by Invitrogen) as the final concentration, 500 μg/ml G418 and 500 nM MTX, to give the density of $3\times10^5$ cells/ml and was distributed into 225 $cm^2$ flasks for suspension culture (manufactured by Iwaki Glass) at 50 ml. The mixture was cultured at 37°C in a $CO_2$ incubator for 7 days, and the cultured supernatant was recovered. An anti-GM2 chimeric antibody was purified from the supernatant by using Prosep-A column (Millipore) according to the attached manufacture's instruction. The purified anti-GM2 chimeric antibody produced by the clone SP2/0/GM2-LCA was named antibody SP2/0/GM2-LCA, and that produced by clone KM968 was named as antibody SP2/0/GM2.

3. Sugar chain structure analysis of purified anti-GM2 antibody

The sugar chain structure of the purified anti-GM2 antibody obtained in the item 2 of Example 13 was analyzed in the same manner as in Example 3. As a result, it was found that the ratio of α1,6-fucose-bound sugar chain in the antibody SP2/0/GM2-LCA was significantly reduced to less than 10% in comparison with that in the antibody SP2/0/GM2.

Reference Example 1

Evaluation of activity of anti-CCR4 chimeric antibody having different ratio of α1,6-fucose-free sugar chains:

1. Production of cell stably producing anti-CCR4 chimeric antibody

Cells capable of stably producing an anti-CCR4 chimeric antibody were prepared as follows using a tandem type expression vector pKANTEX2160 for an anti-CCR4 chimeric antibody described in WO 01/64754.

(1) Preparation of antibody-producing cell using rat myeloma YB2/0 cell

After introducing 10 μg of the anti-CCR4 chimeric antibody expression vector pKANTEX2160 into $4\times10^6$ cells of rat myeloma YB2/0 cell (ATCC CRL 1662) by electroporation [*Cytotechnology*, 3, 133 (1990)], the cells were suspended in 40 ml of Hybridoma-SFM-FBS(5) [Hybridoma-SFM medium (manufactured by Invitrogen) comprising 5% FBS (manufactured by PAA Laboratories)] and dispensed in 200 μl/well into 96 well culture plates (manufactured by Sumitomo Bakelite). After culturing at 37°C for 24 hours in a 5% $CO_2$ incubator, G418 was added to give a concentration of 1 mg/ml, followed by culturing for 1 to 2 weeks. Culture supernatant was recovered from wells in which growth of transformants showing G418 resistance was observed by the formation of colonies, and antigen binding activity of the anti-CCR4 chimeric antibody in the supernatant was measured by the ELISA described in the item 2 of Reference Example 1.

Regarding the transformants in wells in which production of the anti-CCR4 chimeric antibody was observed in culture supernatants, in order to increase an amount of the antibody production using a DHFR gene amplification system, each of them was suspended in the Hybridoma-SFM-FBS(5) medium comprising 1 mg/ml G418 and 50 nM DHFR inhibitor MTX (manufactured by SIGMA) to give a density of 1 to $2\times10^5$ cells/ml, and the suspension was dispensed at 1 ml into wells of 24 well plates (manufactured by Greiner). After culturing at 37°C for 1 to 2 weeks in a 5% $CO_2$ incubator, transformants showing 50 nM MTX resistance were induced. Antigen binding activity of the anti-CCR4 chimeric antibody in culture supernatants in wells in which growth of transformants was observed was measured by the ELISA described in the item 2 of Reference Example 1.

Regarding the transformants in wells in which production of the anti-CCR4 chimeric antibody was observed in culture supernatants, the MTX concentration was increased by the same method, and a transformant capable of growing in the Hybridoma-SFM-FBS(5) medium comprising 200 nM MTX and of producing the anti-CCR4 chimeric antibody in a large amount was finally obtained. The obtained transformant was made into a single cell (cloning) by limiting dilution twice, and the obtained cloned clone was named KM2760#58-35-16. In this case, using the method for determining the transcription product of FUT8 gene shown in WO 00/61739, a clone producing a relatively small amount of the transcription product was selected and used as a suitable clone.

(2) Preparation of antibody-producing cell using CHO/DG44 cell

After introducing 4 μg of the anti-CCR4 chimeric antibody expression vector pKANTEX2160 into $1.6\times10^6$ cells of CHO/DG44 cell by electroporation [*Cytotechnology*, 3, 133 (1990)], the cells were suspended in 10 ml of IMDM-dFBS(10)-HT(1) [IMDM medium (manufactured by Invitrogen) comprising 10% dFBS (manufactured by Invitrogen) and 1 × concentration of HT supplement (manufactured by Invitrogen)] and dispensed in 100 μl/well into 96 well culture plates (manufactured by Iwaki Glass). After culturing at 37°C for 24 hours in a 5% $CO_2$ incubator, the medium was changed to IMDM-dFBS(10) (IMDM medium comprising 10% of dialyzed FBS), followed by culturing for 1 to 2 weeks. Culture supernatant was recovered from wells in which the growth was observed due to formation of a transformant showing HT-independent growth, and an amount of production of the anti-CCR4 chimeric antibody in the supernatant was measured by the ELISA described in the item 2 of Reference Example 1.

Regarding the transformants in wells in which production of the anti-CCR4 chimeric antibody was observed in culture supernatants, in order to increase an amount of the antibody production using a DHFR gene amplification system, each of them was suspended in the IMDM-dFBS(10) medium comprising 50 nM MTX to give a density of 1 to $2\times10^5$ cells/ml, and the suspension was dispensed in 0.5 ml into wells of 24 well plates (manufactured by Iwaki Glass). After culturing at 37°C for 1 to 2 weeks in a 5% $CO_2$ incubator, transformants showing 50 nM MTX resistance were induced. Regarding the transformants in wells in which the growth was observed, the MTX concentration was increased to 200 nM by the same method, and a transformant capable of growing in the IMDM-dFBS(10) medium comprising 200 nM MTX and of producing the anti-CCR4 chimeric antibody in a large amount was finally obtained. The obtained transformant was named clone 5-03.

2. Binding activity to CCR4 partial peptide (ELISA)

Compound 1 (SEQ ID NO:1) was selected as a human CCR4 extracellular region peptide capable of reacting with the anti-CCR4 chimeric antibody. In order to use it in the activity measurement by ELISA, a conjugate with BSA (bovine serum albumin) (manufactured by Nacalai Tesque) was prepared by the following method and used as the antigen. That is, 100 ml of a DMSO solution comprising 25 mg/ml SMCC [4-(*N*-maleimidomethyl)-cyclohexane-1-carboxylic acid *N*-hydroxysuccinimide ester] (manufactured by Sigma) was added dropwise to 900 ml of a 10 mg BSA-containing PBS solution under stirring using a vortex, followed by gently stirring for 30 minutes. To a gel filtration column such as NAP-10 column or the like equilibrated with 25 ml of PBS, 1 ml of the reaction solution was applied and then eluted with 1.5 ml of PBS and the resulting eluate was used as a BSA-SMCC solution (BSA concentration was calculated based on $A_{280}$ measurement). Next, 250 ml of PBS was added to 0.5 mg of Compound 1 and then completely dissolved by adding 250 ml of DMF, and the BSA-SMCC solution was added thereto under vortex, followed by gently stirring for 3 hours. The reaction solution was dialyzed against PBS at 4°C overnight, sodium azide was added thereto to give a final concentration of 0.05%, and the mixture was filtered through a 0.22 mm filter to be used as a BSA-compound 1 solution.

The prepared conjugate was dispensed at 0.05 μg/ml and 50 μl/well into 96 well EIA plates (manufactured by Greiner) and allowed to stand for adhesion at 4°C overnight. After washing each well with PBS, 1% BSA-PBS was added thereto in 100 μl/well and allowed to react at room temperature to block the remaining active groups. After washing each well with PBS containing 0.05% Tween 20 (hereinafter referred to as "Tween-PBS"), a culture supernatant of a transformant was added at 50 μl/well and allowed to react at room temperature for 1 hour. After the reaction, each well was washed with Tween-PBS, and then a peroxidase-labeled goat anti-human IgG(γ) antibody solution (manufactured by American Qualex) diluted 6000 times with 1% BSA-PBS as the secondary antibody was added at 50 μl/well and allowed to react at room temperature for 1 hour. After the reaction and subsequent washing with Tween-PBS, the ABTS substrate solution was added at 50 μl/well for color development, and 20 minutes thereafter, the reaction was stopped by adding a 5% SDS solution at 50 μl/well. Thereafter, the absorbance at $OD_{415}$ was measured. The anti-CCR4 chimeric antibody obtained in the item 1 of Reference Example 1 showed the binding activity to CCR4.

3. Purification of anti-CCR4 chimeric antibody

(1) Culturing of antibody-producing cell derived from YB2/0 cell and purification of antibody

The anti-CCR4 chimeric antibody-expressing transformant cell clone KM2760#58-35-16 obtained in the item 1(1) of Reference Example 1 was suspended in Hybridoma-SFM (manufactured by Invitrogen) medium comprising 200 nM MTX and 5% of Daigo's GF21 (manufactured by Wako Pure Chemical Industries) to give a density of $2 \times 10^5$ cells/ml and subjected to fed-batch shaking culturing using a spinner bottle (manufactured by Iwaki Glass) in a constant temperature chamber of 37°C. After culturing for 8 to 10 days, the anti-CCR4 chimeric antibody was purified from the culture supernatant recovered using Prosep-A (manufactured by Millipore) column and gel filtration. The purified anti-CCR4 chimeric antibody was named KM2760-1.

(2) Culturing of antibody-producing cell derived from CHO-DG44 cell and purification of antibody

The anti-CCR4 chimeric antibody-producing transformant clone 5-03 obtained in the item 1(2) of Reference Example 1 was cultured at 37°C in a 5% $CO_2$ incubator using IMDM-dFBS(10) medium in a 182 $cm^2$ flask (manufactured by Greiner). When the cell density reached confluent after several days, the culture supernatant was discarded, and the cells were washed with 25 ml of PBS buffer and then mixed with 35 ml of EXCELL 301 medium (manufactured by JRH). After culturing at 37°C for 7 days in a 5% $CO_2$ incubator, the culture supernatant was recovered. The anti-CCR4 chimeric antibody was purified from the culture supernatant by using Prosep-A (manufactured by Millipore) column in accordance with the manufacture's instructions. The purified anti-CCR4 chimeric antibody was named KM3060.

When the binding activity to CCR4 of KM2760-1 and KM3060 was measured by ELISA, they showed equivalent binding activity.

4. Analysis of purified anti-CCR4 chimeric antibody

Each (4 µg) of the two kinds of the anti-CCR4 chimeric antibodies produced by and purified from in different animal cells, obtained in the item 1 of Reference Example 1 was subjected to SDS-PAGE in accordance with a known method [*Nature*, 227, 680 (1970)], and the molecular weight and purity were analyzed. In each of the purified anti-CCR4 chimeric antibodies, a single band corresponding to the molecular weight of about 150 Kd was found under non-reducing conditions, and two bands of about 50 Kd and about 25 Kd were found under reducing conditions. The molecular weights almost coincided with the molecular weights deduced from the cDNA nucleotide sequences of antibody H chain and L chain (H chain: about 49 Kd, L chain: about 23 Kd, whole molecule: about 144 Kd) and coincided with reports showing that an IgG type antibody has a molecular weight of about 150 Kd under non-reducing conditions and is degraded into H chain having a molecular weight of about 50 Kd and L chain having a molecular weight of about 25 Kd under reducing conditions caused by cutting an S-S bond in the molecule (*Antibodies*, Chapter 14, *Monoclonal Antibodies: Principles and Practice*), thus confirming that the anti-CCR4 chimeric antibody was expressed and purified as an antibody molecule having a correct structure.

5. Preparation of anti-CCR4 chimeric antibody having a different ratio of α1,6-fucose-free sugar chains

Sugar chains which were bound to anti-CCR4 chimeric antibody KM2760-1 derived from YB2/0 cell prepared in the item 3(1) of Reference Example 1 and the anti-CCR4 chimeric antibody KM3060 derived from CHO/DG44 cell prepared in the item 3(2) of Reference Example 1 were analyzed in accordance with the method of Example 3. The ratio of α1,6-fucose-free sugar chains was 87% and 8% in KM2760-1 and KM3060, respectively. Herein, the samples are referred to as anti-CCR4 chimeric antibody (87%) and anti-CCR4 chimeric antibody (8%).

The anti-CCR4 chimeric antibody (87%) and anti-CCR4 chimeric antibody (8%) were mixed at a ratio of anti-CCR4 chimeric antibody (87%): anti-CCR4 chimeric antibody (8%) = 1 : 39, 16 : 67, 22 : 57, 32 : 47 or 42 : 37. Sugar chains of these samples were analyzed in accordance with the method of Example 3. The ratio of α1,6-fucose-free sugar chains was 9%, 18%, 27%, 39% and 46%, respectively. Herein, these samples are referred to as anti-CCR4 chimeric antibody (9%), anti-CCR4 chimeric antibody (18%), anti-CCR4 chimeric antibody (27%), anti-CCR4 chimeric antibody (39%) and anti-CCR4 chimeric antibody (46%).

Results of the sugar chain analysis of each of the samples are shown in Fig. 38. In Fig. 38, the peaks (i) to (viii) correspond to the structures (1) to (8), respectively, shown in Example 3. The ratio of α1,6-fucose-free sugar chains was shown as an average value of the result of two sugar chain analyses.

6. Evaluation of binding activity to a CCR4 partial peptide (ELISA)

Binding activity of the six kinds of the different anti-CCR4 chimeric antibodies having a different α1,6-fucose-free sugar chain ratio prepared in the item 5 of Reference Example 1 to a CCR4 partial peptide was measured in accordance with the method described in the item 2 of Reference Example 1.

As a result, as shown in Fig. 39, the six kinds of the anti-CCR4 chimeric antibodies showed almost the same CCR4-binding activity, it was found that the ratio of α1,6-fucose-free sugar chains does not have influence on the antigen-binding activity of the antibody.

7. Evaluation of ADCC activity on human CCR4-high expressing clone

The ADCC activity of the anti-CCR4 chimeric antibodies against a human CCR4-high expressing cell CCR4/EL-4 cell (WO 01/64754) was measured as follows.

(1) Preparation of target cell suspension

Cells ($1.5\times10^6$) of a human CCR4-expressing cell, CCR4/EL-4 cell, described in WO 01/64754 were prepared and a 5.55 MBq equivalent of a radioactive substance $Na_2{}^{51}CrO_4$ was added thereto, followed by reaction at 37°C for 1.5 hours to thereby label the cells with a radioisotope. After the reaction, the cells were washed three times by suspension in a medium and subsequent centrifugation, resuspended in the medium and then allowed to stand at 4°C for 30 minutes on ice for spontaneous dissociation of the radioactive substance. After centrifugation, the cells were adjusted to give a density of $2\times10^5$ cells/ml by adding 7.5 ml of the medium and used as a target cell suspension.

(2) Preparation of human effector cell suspension

From a healthy donor, 60 ml of peripheral blood was collected, 0.6 ml of heparin sodium (manufactured by Shimizu Pharmaceutical) was added thereto, followed by gently mixing. The mixture was centrifuged (800 g, 20 minutes) to isolate a mononuclear cell layer by using Lymphoprep (manufactured by AXIS SHIELD) in accordance with the manufacture's instructions. The cells were washed by centrifuging (1,400 rpm, 5 minutes) three times by using a medium and then re-suspended in the medium to give a density of $5\times10^6$ cells/ml and used as a human effector cell suspension.

(3) Measurement of ADCC activity

The target cell suspension prepared in the (1) was dispensed at 50 μl ($1\times10^4$ cells/well) into each well of a 96 well U-bottom plate (manufactured by Falcon). Next, 100 μl of the human effector cell suspension prepared in the (2) was added thereto ($5\times10^5$ cells/well, ratio of the human effector cells to the target cells was 50: 1). Furthermore, each of the anti-CCR4 chimeric antibodies was added thereto to give a final concentration of 0.0001 to 10 μg/ml, followed by reaction at 37°C for 4 hours. After the reaction, the plate was centrifuged and the amount of $^{51}Cr$ in the supernatant was measured by using a γ-counter. An amount of the spontaneously dissociated $^{51}Cr$ was calculated by carrying out the same procedure by using the medium alone instead of the human effector cell suspension and antibody solution, and measuring the amount of $^{51}Cr$ in the supernatant. An amount of the total dissociated $^{51}Cr$ was calculated by carrying out the same procedure by using a 1 mol/L hydrochloric acid solution instead of the antibody solution and human effector cell suspension, and measuring the amount of $^{51}Cr$ in the supernatant. The ADCC activity (%) was calculated based on equation (1).

Figs. 40 and 41 show results of the measurement of ADCC activity of the anti-CCR4 chimeric antibodies having a different ratio of α1,6-fucose-free sugar chains at various concentrations (0.001 to 10 μg/ml) by using effector cells of two healthy donors (A and B), respectively. As shown in Figs. 40 and 41, the ADCC activity of the anti-CCR4 chimeric antibodies showed a tendency to increase in proportion to the ratio of α1,6-fucose-free sugar chains at each antibody concentration. The ADCC activity decreases when the antibody concentration is low. At an antibody concentration of 0.01 μg/ml, the antibody in which the α1,6-fucose-free sugar chains is 27%, 39% or 46% showed almost the same high ADCC activity but the ADCC activity was low in the antibody in which the ratio of α1,6-fucose-free sugar chains is less than 20%. The results were the same as the case when the effector cell donor was changed.

Reference Example 2

Preparation of various genes encoding enzymes relating to the sugar chain synthesis in CHO cell:

1. Determination of CHO cell-derived FX cDNA sequence

(1) Extraction of total RNA from CHO/DG44 cell

CHO/DG44 cells were suspended in IMDM medium containing 10% fetal bovine serum (manufactured by Life Technologies) and 1 × concentration HT supplement (manufactured by Life Technologies), and 15 ml of the suspension was inoculated into a T75 flask for adhesion cell culture use (manufactured by Greiner) to give a density of $2\times10^5$ cells/ml. On the second day after culturing them at 37°C in a 5% $CO_2$ incubator, $1\times10^7$ of the cells were recovered and total RNA was extracted therefrom by using RNAeasy (manufactured by QIAGEN) in accordance with the manufacture's instructions.

(2) Preparation of a total single-stranded cDNA from CHO/DG44 cell

The total RNA prepared in the (1) was dissolved in 45 μl of sterile water, and 1 μl of RQ1 RNase-Free DNase (manufactured by Promega), 5 μl of the attached 10 × DNase buffer and 0.5 μl of RNasin Ribonuclease Inhibitor (manufactured by Promega) were added thereto, followed by reaction at 37°C for 30 minutes to degrade genomic DNA contaminated in the sample. After the reaction, the total RNA was purified again by using RNAeasy (manufactured by QIAGEN) and dissolved in 50 μl of sterile water.

In 20 μl of reaction mixture using oligo(dT) as a primer, single-stranded cDNA was synthesized from 3 μg of the obtained total RNA samples by carrying out reverse transcription reaction using SUPERSCRIPT™ Preamplification System for First Strand cDNA Synthesis (manufactured by Life Technologies) in accordance with the manufacture's instructions. A 50 fold-diluted aqueous solution of the reaction solution was used in the cloning of GFPP and FX. This was stored at -80°C until use.

(3) Preparation method of cDNA partial fragment of Chinese hamster-derived FX

FX cDNA partial fragment derived from Chinese hamster was prepared by the following procedure.

First, primers (represented by SEQ ID NOs:12 and 13) specific for common nucleotide sequences registered at a public data base, namely a human FX cDNA (Genebank Accession No. U58766) and a mouse cDNA (Genebank Accession No. M30127), were designed.

Next, 25 μl of a reaction solution [ExTaq buffer (manufactured by Takara Shuzo), 0.2 mmol/l dNTPs and 0.5 μmol/l gene-specific primers (SEQ ID NOs:12 and 13)] containing 1 μl of the CHO/DG44-derived single-stranded cDNA prepared in the item (2) was prepared, and a polymerase chain reaction (PCR) was carried out by using a DNA polymerase ExTaq (manufactured by Takara Shuzo). The PCR was carried out by heating at 94°C for 5 minutes, subsequent 30 cycles of heating at 94°C for 1 minute, 58°C for 2 minutes and 72°C for 3 minutes as one cycle, and final heating at 72°C for 10 minutes.

After the PCR, the reaction solution was subjected to 2% agarose gel electrophoresis, and a specific amplified fragment of 301 bp was purified by using QuiaexII Gel Extraction Kit (manufactured by QIAGEN) and eluted with 20 μl of sterile water (hereinafter, the method was used for the purification of DNA fragments from agarose gel). Into a plasmid pCR2.1, 4 μl of the amplified fragment was employed to insert in accordance with the instructions attached to TOPO TA Cloning Kit (manufactured by Invitrogen), and *E. coli* DH5α was transformed with the reaction solution by the method of Cohen *et al*. [*Proc. Natl. Acad. Sci. USA*, 69, 2110 (1972)] (hereinafter, the method was used for the transformation of *E. coli*). Plasmid DNA was isolated in accordance with a known method [*Nucleic Acids Research,* 7, 1513 (1979)] (hereinafter, the method was used for the isolation of plasmid) from the obtained several kanamycin-resistant colonies to obtain 2 clones into which FX cDNA partial fragments were respectively inserted. They are referred to as pCRFX clone 8 and pCRFX clone 12.

The nucleotide sequence of the cDNA inserted into each of the FX clone 8 and FX clone 12 was determined by using DNA Sequencer 377 (manufactured by Parkin Elmer) and BigDye Terminator Cycle Sequencing FS Ready Reaction kit (manufactured by Parkin Elmer) in accordance with the method of the manufacture's instructions. It was confirmed that each of the inserted cDNA whose sequence was determined encodes open reading frame (ORF) partial sequence of the Chinese hamster FX.

(4) Synthesis of a single-stranded cDNA for RACE

Single-stranded cDNA samples for 5' and 3' RACE were prepared from the CHO/DG44 total RNA extracted in the item (1) using SMART™ RACE cDNA Amplification Kit (manufactured by CLONTECH) in accordance with the manufacture's instructions. In the case, PowerScript™ Reverse Transcriptase (manufactured by CLONTECH) was used as the reverse transcriptase. Each single-stranded cDNA after the preparation was diluted 10-fold with the Tricin-EDTA buffer attached to the kit and used as the template of PCR.

(5) Determination of a Chinese hamster-derived FX full length cDNA by RACE method

Based on the FX partial sequence derived from Chinese hamster determined in the item (3), primers FXGSP1-1 (SEQ ID NO:14) and FXGSP1-2 (SEQ ID NO:15) for the Chinese hamster FX-specific 5' RACE and primers FXGSP2-1 (SEQ ID NO:16) and FXGSP2-2 (SEQ ID NO:17) for the Chinese hamster FX-specific 3' RACE were designed.

Next, polymerase chain reaction (PCR) was carried out by using Advantage2 PCR Kit (manufactured by CLONTECH), by preparing 50 µl of a reaction solution [Advantage2 PCR buffer (manufactured by CLONTECH), 0.2 mM dNTPs, 0.2 µmol/l Chinese hamster FX-specific primers for RACE and 1 × concentration of common primers (manufactured by CLONTECH)] containing 1 µl of the CHO/DG44-derived single-stranded cDNA for RACE prepared in the item (4).

The PCR was carried out by repeating 20 cycles of heating at 94°C for 5 seconds, 68°C for 10 seconds and 72°C for 2 minutes as one cycle.

After completion of the reaction, 1 µl of the reaction solution was diluted 50-folds with the Tricin-EDTA buffer, and 1 µl of the diluted solution was used as a template. The reaction solution was again prepared and the PCR was carried out under the same conditions. The templates, the combination of primers used in the first and second PCRs and the length of amplified DNA fragments by the PCRs are shown in Table 6.

Table 6

| Combination of primers used in Chinese hamster FX cDNA RACE PCR and size of PCR product | | | |
|---|---|---|---|
| 5' RACE | FX-specific primers | Common primers | Size of PCR-amplified product |
| First<br>Second | FXGSP1-1<br>FXGSP1-2 | UPM (Universal primer mix)<br>NUP (Nested Universal primer) | 300 bp |
| | | | |
| 3' RACE | FX-specific primers | Common primers | Size of PCR-amplified product |
| First<br>Second | FXGSP2-1<br>FXGSP2-2 | UPM (Universal primer mix)<br>NUP (Nested Universal primer) | 1,100 bp |

After the PCR, the reaction solution was subjected to 1% agarose gel electrophoresis, and the specific amplified fragment of interest was purified by using QiaexII Gel Extraction Kit (manufactured by QIAGEN) and eluted with 20 µl of sterile water. Into a plasmid pCR2.1, 4 µl of the amplified fragment was inserted, and *E. coli* DH5α was transformed by using the reaction solution in accordance with the instructions attached to TOPO TA Cloning Kit (manufactured by Invitrogen).

Plasmid DNAs were isolated from the appeared several kanamycin-resistant colonies to obtain 5 cDNA clones containing Chinese hamster FX 5' region. They are referred to as FX5' clone 25, FX5' clone 26, FX5' clone 27, FX5' clone 28, FX5' clone 31 and FX5' clone 32.

In the same manner, 5 cDNA clones containing Chinese hamster FX 3' region were obtained. These FX3' clones are referred to as FX3' clone 1, FX3' clone 3, FX3' clone 6, FX3' clone 8 and FX3' clone 9.

The nucleotide sequence of the cDNA moiety of each of the clones obtained by the 5' and 3' RACE was determined by using DNA Sequencer 377 (manufactured by Parkin Elmer) in accordance with the method described in the manufacture's instructions. By comparing the cDNA nucleotide sequences determined by the method, reading errors of bases due to PCR were excluded and the full length nucleotide sequence of Chinese hamster FX cDNA was determined. The determined sequence is represented by SEQ ID NO:18.

2. Determination of CHO cell-derived GFPP cDNA sequence

(1) Preparation of GFPP cDNA partial fragment derived from a Chinese hamster

A GFPP cDNA partial fragment derived from Chinese hamster was prepared by the following procedure.

First, nucleotide sequences of a human GFPP cDNA (Genebank Accession No. AF017445), mouse EST sequences having high homology with the sequence (Genebank Accession Nos. AI467195, AA422658, BE304325 and AI466474) and rat EST sequences (Genebank Accession Nos. BF546372, AI058400 and AW144783), registered at public data bases, were compared, and primers GFPP FW9 and GFPP RV9 (SEQ ID NOs:19 and 20) specific for rat GFPP were designed on a highly preserved region among these three species.

Next, by using a DNA polymerase ExTaq (manufactured by Takara Shuzo), a polymerase chain reaction (PCR) was carried out by preparing 25 μl of a reaction solution [ExTaq buffer (manufactured by Takara Shuzo), 0.2 mM dNTPs and 0.5 μmol/l GFPP-specific primers GFPP FW9 and GFPP RV9 (SEQ ID NOs:19 and 20)] containing 1 μl of the CHO/DG44-derived single-stranded cDNA prepared in the item 1(2). The PCR was carried out by heating at 94°C for 5 minutes, subsequent 30 cycles of heating at 94°C for 1 minute, 58°C for 2 minutes and 72°C for 3 minutes as one cycle, and final heating at 72°C for 10 minutes.

After the PCR, the reaction solution was subjected to 2% agarose gel electrophoresis, and a specific amplified fragment of 1.4 Kbp was purified by using QuiaexII Gel Extraction Kit (manufactured by QIAGEN) and eluted with 20 μl of sterile water. Into a plasmid pCR2.1, 4 μl of the amplified fragment was employed to insert in accordance with the instructions attached to TOPO TA Cloning Kit (manufactured by Invitrogen), and *E. coli* DH5α was transformed by using the reaction solution.

Plasmid DNAs were isolated from the appeared several kanamycin-resistant colonies to obtain 3 clones into which GFPP cDNA partial fragments were respectively integrated. They are referred to as GFPP clone 8, GFPP clone 11 and GFPP clone 12.

The nucleotide sequence of the cDNA inserted into each of the GFPP clone 8, GFPP clone 11 or GFPP clone 12 was determined by using DNA Sequencer 377 (manufactured by Parkin Elmer) and BigDye Terminator Cycle Sequencing FS Ready Reaction Kit (manufactured by Parkin Elmer) in accordance with the method described in the manufacture's instructions. It was confirmed that the inserted cDNA whose sequence was determined encodes a partial sequence of the open reading frame (ORF) of the Chinese hamster GFPP.

(2) Determination of Chinese hamster GFPP full length cDNA by RACE method

Based on the Chinese hamster FX partial sequence determined in the item 2(1), primers GFPP GSP1-1 (SEQ ID NO:22) and GFPP GSP1-2 (SEQ ID NO:23) for the Chinese hamster FX-specific 5' RACE and primers GFPP GSP2-1 (SEQ ID NO:24) and GFPP GSP2-2 (SEQ ID NO:25) for the Chinese hamster GFPP-specific 3' RACE were designed.

Next, by using Advantage2 PCR Kit (manufactured by CLONTECH), a polymerase chain reaction (PCR) was carried out by preparing 50 μl of a reaction solution [Advantage2 PCR buffer (manufactured by CLONTECH), 0.2 mM dNTPs, 0.2 μmol/l Chinese hamster GFPP-specific primers for RACE and 1 × concentration of common primers (manufactured by CLONTECH)] containing 1 μl of the CHO/DG44-derived single-stranded cDNA for RACE prepared in the item (4).

The PCR was carried out by repeating 20 cycles of heating at 94°C for 5 seconds, 68°C for 10 seconds and 72°C for 2 minutes as one cycle.

After completion of the reaction, 1 μl of the reaction solution was diluted 50-folds with the Tricin-EDTA buffer, and 1 μl of the diluted solution was used as a template. The reaction solution was again prepared and the PCR was carried out under the same conditions. The templates, the combination of primers used in the first and second PCRs and the size of amplified DNA fragments by the PCRs are shown in Table 7.

Table 7

| Combination of primers used in Chinese hamster GFPP cDNA RACE PCR and size of PCR products | | | |
|---|---|---|---|
| 5'RACE | GFPP-specific primers | Common primers | Size of PCR-amplified product |
| First<br>Second | GFPPGSP1-1<br>GFPPGSP1-2 | UPM (Universal primer mix)<br>NUP (Nested Universal primer) | <br>1,100 bp |
| | | | |

Table 7 (continued)

| Combination of primers used in Chinese hamster GFPP cDNA RACE PCR and size of PCR products | | | |
|---|---|---|---|
| 3' RACE | GFPP-specific primers | Common primers | Size of PCR-amplified product |
| First<br>Second | GFPPGSP2-1<br>GFPPGSP2-2 | UPM (Universal primer mix)<br>NUP (Nested Universal primer) | 1,400 bp |

After the PCR, the reaction solution was subjected to 1% agarose gel electrophoresis, and the specific amplified fragment of interest was purified by using QiaexII Gel Extraction Kit (manufactured by QIAGEN) and eluted with 20 μl of sterile water. Into a plasmid pCR2.1, 4 μl of the amplified fragment was inserted, and *E. coli* DH5α was transformed with the reaction solution in accordance with the instructions attached to TOPO TA Cloning Kit (manufactured by Invitrogen).

Plasmid DNAs were isolated from the obtained several kanamycin-resistant colonies to obtain 4 cDNA clones containing Chinese hamster GFPP 5' region. They are referred to as GFPP5' clone 1, GFPP5' clone 2, GFPP5' clone 3 and GFPP5' clone 4.

In the same manner, 5 cDNA clones containing Chinese hamster GFPP 3' region were obtained. They are referred to as GFPP3' clone 10, GFPP3' clone 16 and GFPP3' clone 20.

The nucleotide sequence of the cDNA of each of the clones obtained by the 5' and 3' RACE was determined by using DNA Sequencer 377 (manufactured by Parkin Elmer) in accordance with the method described in the manufacture's instructions. By comparing the cDNA nucleotide sequences after the nucleotide sequence determination, reading errors of bases due to PCR were excluded and the full length nucleotide sequence of Chinese hamster GFPP cDNA was determined. The determined sequence is represented by SEQ ID NO:21.

Reference Example 3

Preparation of CHO cell-derived GMD gene:

1. Determination of CHO cell-derived GMD cDNA sequence

(1) Preparation of CHO cell-derived GMD gene cDNA (preparation of partial cDNA excluding 5'- and 3'-terminal sequences)

A rodents-derived GMD cDNA was searched in a public data base (BLAST) using a human-derived GMD cDNA sequence (GenBank Accession No. AF042377) registered at GenBank as a query, and three kinds of mouse EST sequences were obtained (GenBank Accession Nos. BE986856, BF158988 and BE284785). By ligating these EST sequences, a deduced mouse GMD cDNA sequence was determined.

Based on the mouse-derived GMD cDNA sequence, a 28 mer primer having the sequence represented by SEQ ID NO:26, a 27 mer primer having the sequence represented by SEQ ID NO:27, a 25 mer primer having the sequence represented by SEQ ID NO:28, a 24 mer primer having the sequence represented by SEQ ID NO:29 and a 25 mer primer having the sequence represented by SEQ ID NO:30 were generated.

Next, in order to amplify the CHO cell-derived GMD cDNA, PCR was carried out by the following method.

A reaction solution (20 μl) [1 × Ex Taq buffer (manufactured by Takara Shuzo), 0.2 mM dNTPs, 0.5 unit of Ex Taq polymerase (manufactured by Takara Shuzo) and 0.5 μM of two synthetic DNA primers] containing 0.5 μl of the CHO cell-derived single-stranded cDNA prepared in the item 1(1) of Example 6 as the template was prepared. In this case, combinations represented by SEQ ID NO:26 with SEQ ID NO:27, SEQ ID NO:28 with SEQ ID NO:27, SEQ ID NO:26 with SEQ ID NO:29 and SEQ ID NO:26 with SEQ ID NO:30 were used as the synthetic DNA primers. The reaction of heating at 94°C for 5 minutes and subsequent 30 cycles of heating at 94°C for 1 minute and 68°C for 2 minutes as one cycle was carried out by DNA Thermal Cycler 480 (manufactured by Perkin Elmer).

The PCR reaction solution was subjected to agarose electrophoresis to find that a DNA fragment of about 1.2 kbp was amplified in the PCR product when synthetic DNA primers represented by SEQ ID NOs:26 and 27 were used, a fragment of about 1.1 kbp was amplified in the PCR product when synthetic DNA primers represented by SEQ ID NOs:27 and 29 were used, a fragment of about 350 bp was amplified in the PCR product when synthetic DNA primers represented by SEQ ID NOs:26 and 29 were used and a fragment of about 1 kbp was amplified in the PCR product when synthetic DNA primers represented by SEQ ID NOs:26 and 30 were used. The DNA fragments were recovered by using Gene Clean II Kit (manufactured by BIO 101) in accordance with the manufacture's instructions. The recovered DNA fragments were ligated to a pT7Blue(R) vector (manufactured by Novagen) by using DNA Ligation Kit (manufactured by Takara Shuzo), and *E. coli* DH (manufactured by Toyobo) was transformed by using the obtained

recombinant plasmid DNA samples to thereby obtain plasmids 22-8 (having a DNA fragment of about 1.2 kbp amplified from synthetic DNA primers represented by SEQ ID NO:26 and SEQ ID NO:27), 23-3 (having a DNA fragment of about 1.1 kbp amplified from synthetic DNA primers represented by SEQ ID NO:28 and SEQ ID NO:27), 31-5 (a DNA fragment of about 350 bp amplified from synthetic DNA primers represented by SEQ ID NO:26 and SEQ ID NO:29) and 34-2 (having a DNA fragment of about 1 kbp amplified from synthetic DNA primers represented by SEQ ID NO:26 and SEQ ID NO:30). The CHO cell-derived GMD cDNA sequence contained in these plasmids was determined in the usual way by using a DNA sequencer ABI PRISM 377 (manufactured by Parkin Elmer) (since a sequence of 28 bases in downstream of the initiation codon methionine in the 5'-terminal side and a sequence of 27 bases in upstream of the termination codon in the 3'-terminal side are originated from syntheticoligo DNA sequences, they are mouse GMD cDNA sequences).

In addition, the following steps were carried out in order to prepare a plasmid in which the CHO cell-derived GMD cDNA fragments contained in the plasmids 22-8 and 34-2 are combined. The plasmid 22-8 (1 μg) was allowed to react with a restriction enzyme *Eco*RI (manufactured by Takara Shuzo) at 37°C for 16 hours, the digest was subjected to agarose electrophoresis and then a DNA fragment of about 4 kbp was recovered by using Gene Clean II Kit (manufactured by BIO 101) in accordance with the manufacture's instructions. The plasmid 34-2 (2 μg) was allowed to react with a restriction enzyme *Eco*RI at 37°C for 16 hours, the digest was subjected to agarose electrophoresis and then a DNA fragment of about 150 bp was recovered by using Gene Clean II Kit (manufactured by BIO 101) in accordance with the manufacture's instructions. The recovered DNA fragments were respectively subjected to terminal dephosphorylation by using Calf Intestine Alkaline Phosphatase (manufactured by Takara Shuzo) and then ligated by using DNA Ligation Kit (manufactured by Takara Shuzo), and *E. coli* DH5α (manufactured by Toyobo) was transformed by using the obtained recombinant plasmid DNA to obtain a plasmid CHO-GMD (cf. Fig. 42).

(2) Determination of 5'-terminal sequence of CHO cell-derived GMD cDNA

A 24 mer primer having the nucleotide sequence represented by SEQ ID NO:31 was prepared from 5'-terminal side non-coding region nucleotide sequences of CHO cell-derived human and mouse GMD cDNA, and a 32 mer primer having the nucleotide sequence represented by SEQ ID NO:32 from CHO cell-derived GMD cDNA sequence were prepared, and PCR was carried out by the following method to amplify cDNA. Then, 20 μl of a reaction solution [1 × Ex Taq buffer (manufactured by Takara Shuzo), 0.2 mM dNTPs, 0.5 unit of Ex Taq polymerase (manufactured by Takara Shuzo) and 0.5 μM of the synthetic DNA primers represented by SEQ ID NO:31 and SEQ ID NO:32] containing 0.5 μl of the single-stranded cDNA prepared in the item 1(1) of Example 15 was prepared as the template, and the reaction was carried out therein by using DNA Thermal Cycler 480 (manufactured by Perkin Elmer) by heating at 94°C for 5 minutes, subsequent 20 cycles of heating at 94°C for 1 minute, 55°C for 1 minute and 72°C for 2 minutes as one cycle and further 18 cycles of heating at 94°C for 1 minute and 68°C for 2 minutes as one cycle. After fractionation of the PCR reaction solution by agarose electrophoresis, a DNA fragment of about 300 bp was recovered by using Gene Clean II Kit (manufactured by BIO 101) in accordance with the manufacture's instructions. The recovered DNA fragment was ligated to a pT7Blue(R) vector (manufactured by Novagen) by using DNA Ligation Kit (manufactured by Takara Shuzo), and *E. coli* DH5α (manufactured by Toyobo) was transformed by using the obtained recombinant plasmid DNA samples to thereby obtain a plasmid 5'GMD. Using DNA Sequencer 377 (manufactured by Parkin Elmer), the nucleotide sequence of 28 bases in downstream of the initiation methionine of CHO cell-derived GMD cDNA contained in the plasmid was determined.

(3) Determination of 3'-terminal sequence of CHO cell-derived GMD cDNA

In order to obtain 3'-terminal cDNA sequence of CHO cell-derived GMD, RACE method was carried out by the following method. A single-stranded cDNA for 3' RACE was prepared from the CHO cell-derived RNA obtained in the item 1(1) of Example 6 by using SMART™ RACE cDNA Amplification Kit (manufactured by CLONTECH) in accordance with the manufacture's instructions. In the case, PowerScript™ Reverse Transcriptase (manufactured by CLONTECH) was used as the reverse transcriptase. The single-stranded cDNA after the preparation was diluted 10-fold with the Tricin-EDTA buffer attached to the kit and used as the template of PCR.

Next, 20 μl of a reaction solution [ExTaq buffer (manufactured by Takara Shuzo), 0.2 mM dNTPs, 0.5 unit of EX Taq polymerase (manufactured by Takara Shuzo), 0.5 μM of the 25 mer synthetic DNA primer represented by SEQ ID NO:33 [generated on the base of the CHO cell-derived GMD cDNA sequence determined in the item (1)] and 1 × concentration of Universal Primer Mix (attached to SMART™ RACE cDNA Amplification Kit; manufactured by CLONTECH] containing 1 μl of the cDNA for 3' RACE as the template was prepared, and PCR was carried out by using DNA Thermal Cycler 480 (manufactured by Perkin Elmer) by heating at 94°C for 5 minutes and subsequent 30 cycles of heating at 94°C for 1 minute and 68°C for 2 minutes as one cycle.

After completion of the reaction, 1 μl of the PCR reaction solution was diluted 20-fold with Tricin-EDTA buffer

(manufactured by CLONTECH). Then, 20 µl of a reaction solution [ExTaq buffer (manufactured by Takara Shuzo), 0.2 mM dNTPs, 0.5 unit of EX Taq polymerase (manufactured by Takara Shuzo), 0.5 µM of the 25 mer synthetic DNA primer represented by SEQ ID NO:34 [generated on the base of the CHO cell-derived GMD cDNA sequence determined in the item (1)] and 0.5 µM of Nested Universal Primer (attached to SMART™ RACE cDNA Amplification Kit; manufactured by CLONTECH) containing 1 µl of the 20 fold-diluted aqueous solution as the template] was prepared, and the reaction of heating at 94°C for 5 minutes and subsequent 30 cycles at 94°C for 1 minute and 68°C for 2 minutes as one cycle was carried out by using DNA Thermal Cycler 480 (manufactured by Perkin Elmer).

After completion of the reaction, the PCR reaction solution was subjected to agarose electrophoresis and then a DNA fragment of about 700 bp was recovered by using Gene Clean II Kit (manufactured by BIO 101) in accordance with the manufacture's instructions. The recovered DNA fragment was ligated to a pT7Blue(R) vector (manufactured by Novagen) by using DNA Ligation Kit (manufactured by Takara Shuzo), and *E. coli* DH5α (manufactured by Toyobo) was transformed by using the obtained recombinant plasmid DNA to obtain a plasmid 3'GMD. Using DNA Sequencer 377 (manufactured by Parkin Elmer), the nucleotide sequence of 27 bases in upstream of the termination codon of CHO cell-derived GMD cDNA contained in the plasmid was determined.

The full length cDNA sequence of the CHO-derived GMD gene determined by the items (1), (2) and (3) and the corresponding amino acid sequence are represented by SEQ ID NOs:35 and 41, respectively.

2. Determination of genome sequence containing CHO/DG44 cell-derived GMD gene

A 25 mer primer having the nucleotide sequence represented by SEQ ID NO:36 was prepared from the mouse GMD cDNA sequence determined in the item 1 of Reference Example 3. Next, a CHO cell-derived genomic DNA was obtained by the following method. A CHO/DG44 cell-derived KC861 was suspended in IMDM-dFBS(10)-HT(1) medium [IMDM-dFBS(10) medium comprising 1 × concentration of HT supplement (manufactured by Invitrogen)] to give a density of $3\times10^5$ cells/ml, and the suspension was dispensed at 2 ml/well into a 6 well flat bottom plate for adhesion cell use (manufactured by Greiner). After culturing at 37°C in a 5% $CO_2$ incubator until the cells became confluent on the plate, genomic DNA was prepared from the cells on the plate by a known method [*Nucleic Acids Research*, 3, 2303 (1976)] and dissolved overnight in 150 µl of TE-RNase buffer (pH 8.0) (10 mmol/l Tris-HCl, 1 mmol/l EDTA, 200 µg/ml RNase A).

A reaction solution (20 µl) [1 × Ex Taq buffer (manufactured by Takara Shuzo), 0.2 mM dNTPs, 0.5 unit of EX Taq polymerase (manufactured by Takara Shuzo) and 0.5 µM of synthetic DNA primers represented by SEQ ID NO:29 and SEQ ID NO:36] containing 100 ng of the obtained CHO/DG44 cell-derived genomic DNA was prepared, and the reaction of heating at 94°C for 5 minutes and subsequent 30 cycles of heating at 94°C for 1 minute and 68°C for 2 minutes as one cycle was carried out by using DNA Thermal Cycler 480 (manufactured by Perkin Elmer). After completion of the reaction, the PCR reaction solution was subjected to agarose electrophoresis and then a DNA fragment of about 100 bp was recovered by using Gene Clean II Kit (manufactured by BIO 101) in accordance with the manufacture's instructions. The recovered DNA fragment was ligated to a pT7Blue(R) vector (manufactured by Novagen) by using DNA Ligation Kit (manufactured by Takara Shuzo), and *E. coli* DH5α (manufactured by Toyobo) was transformed by using the obtained recombinant plasmid DNA to obtain a plasmid ex3. Using DNA Sequencer 377 (manufactured by Parkin Elmer), the nucleotide sequence of CHO cell-derived genomic DNA contained in the plasmid was determined. The result is represented by SEQ ID NO:37.

Next, a 25 mer primer having the nucleotide sequence represented by SEQ ID NO:38 and a 25 mer primer having the nucleotide sequence represented by SEQ ID NO:39 were generated on the base of the CHO cell-derived GMD cDNA sequence determined in the item 1 of Reference Example 3. Next, 20 µl of a reaction solution [1 × Ex Taq buffer (manufactured by Takara Shuzo), 0.2 mM dNTPs, 0.5 unit of EX Taq polymerase (manufactured by Takara Shuzo) and 0.5 µM of the synthetic DNA primers represented by SEQ ID NO:38 and SEQ ID NO:39] containing 100 ng of the CHO/DG44-derived genomic DNA was prepared, and PCR was carried out by using DNA Thermal Cycler 480 (manufactured by Perkin Elmer) by heating at 94°C for 5 minutes and subsequent 30 cycles of heating at 94°C for 1 minute and 68°C for 2 minutes as one cycle.

After completion of the reaction, the PCR reaction solution was subjected to agarose electrophoresis and then a DNA fragment of about 200 bp was recovered by using Gene Clean II Kit (manufactured by BIO 101) in accordance with the manufacture's instructions. The recovered DNA fragment was ligated to a pT7Blue(R) vector (manufactured by Novagen) by using DNA Ligation Kit (manufactured by Takara Shuzo), and *E. coli* DH5α (manufactured by Toyobo) was transformed by using the obtained recombinant plasmid DNA to obtain a plasmid ex4. Using DNA Sequencer 377 (manufactured by Parkin Elmer), the nucleotide sequence of CHO cell-derived genomic DNA contained in the plasmid was determined, and represented by SEQ ID NO:40.

INDUSTRIAL APPLICABILITY

The present invention can provide a process for producing an antibody composition using a cell, which comprises using a cell resistant to a lectin which recognizes a sugar chain in which 1-position of fucose is bound to 6-position of *N*-acetylglucosamine in the reducing end through α-bond in a complex *N*-glycoside-linked sugar chain.

Free Text in Sequence Listing

SEQ ID NO:2 - Explanation of synthetic sequence: synthetic DNA
SEQ ID NO:3 - Explanation of synthetic sequence: synthetic DNA
SEQ ID NO:4 - Explanation of synthetic sequence: synthetic DNA
SEQ ID NO:5 - Explanation of synthetic sequence: synthetic DNA
SEQ ID NO:6 - Explanation of synthetic sequence: synthetic DNA
SEQ ID NO:7 - Explanation of synthetic sequence: synthetic DNA
SEQ ID NO:8 - Explanation of synthetic sequence: synthetic DNA
SEQ ID NO:9 - Explanation of synthetic sequence: synthetic DNA
SEQ ID NO:10 - Explanation of synthetic sequence: synthetic DNA
SEQ ID NO:11 - Explanation of synthetic sequence: synthetic DNA
SEQ ID NO:12 - Explanation of synthetic sequence: synthetic DNA
SEQ ID NO:13 - Explanation of synthetic sequence: synthetic DNA
SEQ ID NO:14 - Explanation of synthetic sequence: synthetic DNA
SEQ ID NO:15 - Explanation of synthetic sequence: synthetic DNA
SEQ ID NO:16 - Explanation of synthetic sequence: synthetic DNA
SEQ ID NO:17 - Explanation of synthetic sequence: synthetic DNA
SEQ ID NO:19 - Explanation of synthetic sequence: synthetic DNA
SEQ ID NO:22 - Explanation of synthetic sequence: synthetic DNA
SEQ ID NO:23 - Explanation of synthetic sequence: synthetic DNA
SEQ ID NO:24 - Explanation of synthetic sequence: synthetic DNA
SEQ ID NO:25 - Explanation of synthetic sequence: synthetic DNA
SEQ ID NO:26 - Explanation of synthetic sequence: synthetic DNA
SEQ ID NO:27 - Explanation of synthetic sequence: synthetic DNA
SEQ ID NO:28 - Explanation of synthetic sequence: synthetic DNA
SEQ ID NO:29 - Explanation of synthetic sequence: synthetic DNA
SEQ ID NO:30 - Explanation of synthetic sequence: synthetic DNA
SEQ ID NO:31 - Explanation of synthetic sequence: synthetic DNA
SEQ ID NO:32 - Explanation of synthetic sequence: synthetic DNA
SEQ ID NO:33 - Explanation of synthetic sequence: synthetic DNA
SEQ ID NO:34 - Explanation of synthetic sequence: synthetic DNA
SEQ ID NO:36 - Explanation of synthetic sequence: synthetic DNA
SEQ ID NO:38 - Explanation of synthetic sequence: synthetic DNA
SEQ ID NO:39 - Explanation of synthetic sequence: synthetic DNA
SEQ ID NO:42 - Explanation of synthetic sequence: synthetic DNA
SEQ ID NO:43 - Explanation of synthetic sequence: synthetic DNA
SEQ ID NO:44 - Explanation of synthetic sequence: synthetic DNA
SEQ ID NO:45 - Explanation of synthetic sequence: synthetic DNA
SEQ ID NO:48 - Explanation of synthetic sequence: synthetic DNA
SEQ ID NO:49 - Explanation of synthetic sequence: synthetic DNA
SEQ ID NO:50 - Explanation of synthetic sequence: synthetic DNA
SEQ ID NO:51 - Explanation of synthetic sequence: synthetic DNA
SEQ ID NO:52 - Explanation of synthetic sequence: synthetic DNA
SEQ ID NO:53 - Explanation of synthetic sequence: synthetic DNA
SEQ ID NO:54 - Explanation of synthetic sequence: synthetic DNA
SEQ ID NO:55 - Explanation of synthetic sequence: synthetic DNA
SEQ ID NO:56 - Explanation of synthetic sequence: synthetic DNA
SEQ ID NO:57 - Explanation of synthetic sequence: synthetic DNA
SEQ ID NO:58 - Explanation of synthetic sequence: synthetic DNA
SEQ ID NO:59 - Explanation of synthetic sequence: synthetic DNA
SEQ ID NO:60 - Explanation of synthetic sequence: synthetic DNA
SEQ ID NO:61 - Explanation of synthetic sequence: synthetic DNA

SEQ ID NO:62 - Explanation of synthetic sequence: synthetic DNA
SEQ ID NO:65 - Explanation of synthetic sequence: synthetic DNA
SEQ ID NO:66 - Explanation of synthetic sequence: synthetic DNA
SEQ ID NO:67 - Explanation of synthetic sequence: synthetic DNA
SEQ ID NO:68 - Explanation of synthetic sequence: synthetic DNA
SEQ ED NO:69 - Explanation of synthetic sequence: synthetic DNA
SEQ ID NO:70 - Explanation of synthetic sequence: synthetic DNA
SEQ ID NO:71 - Explanation of synthetic sequence: synthetic DNA
SEQ IID NO:72 - Explanation of synthetic sequence: synthetic DNA
SEQ II7 NO:73 - Explanation of synthetic sequence: synthetic DNA
SEQ ID NO:74 - Explanation of synthetic sequence: synthetic DNA
SEQ ID NO:75 - Explanation of synthetic sequence: synthetic DNA
SEQ ID NO:76 - Explanation of synthetic sequence: synthetic DNA
SEQ ID NO:77 - Explanation of synthetic sequence: synthetic DNA
SEQ ID NO:78 - Explanation of synthetic sequence: synthetic DNA
SEQ ID NO:83 - Explanation of synthetic sequence: synthetic RNA
SEQ ID NO:84 - Explanation of synthetic sequence: synthetic RNA

## Claims

**1.** A process for producing an antibody composition using a cell, which comprises using a cell resistant to a lectin which recognizes a sugar chain in which 1-position of fucose is bound to 6-position of *N*-acetylglucosamine in the reducing end through α-bond in a complex *N*-glycoside-linked sugar chain.

**2.** The process according to claim 1, wherein the cell is a cell in which the activity of a protein selected from the group consisting of the following (a), (b) and (c) is decreased or deleted:

(a) an enzyme protein relating to synthesis of an intracellular sugar nucleotide, GDP-fucose;
(b) an enzyme protein relating to modification of a sugar chain in which 1-position of fucose is bound to 6-position of *N*-acetylglucosamine in the reducing end through α-bond in a complex *N*-glycoside-linked sugar chain;
(c) a protein relating to transport of an intracellular sugar nucleotide, GDP-fucose, to the Golgi body.

**3.** The process according to claim 2, wherein the enzyme protein relating to modification of a sugar chain in which 1-position of fucose is bound to 6-position of *N*-acetylglucosamine in the reducing end through α-bond in a complex *N*-glycoside-linked sugar chain is α1,6-fucosyltransferase.

**4.** The process according to claim 2 or 3, wherein the activity of the protein is decreased or deleted by a technique selected from the group consisting of (a) to (d):

(a) a gene disruption technique which comprises targeting a gene encoding the protein;
(b) a technique for introducing a dominant negative mutant of a gene encoding the protein;
(c) a technique for introducing mutation into the protein;
(d) a technique for suppressing transcription and/or translation of a gene encoding the protein.

**5.** The process according to claim 4), wherein the technique for suppressing transcription and/or translation of a gene encoding the protein is an RNAi method.

**6.** The process according to any one of claims 3 to 5, wherein the α1,6-fucosyltransferase is a protein encoded by a DNA selected from the group consisting of (a) to (h) or a protein selected from the group consisting of the following (i) to (n):

(a) a DNA comprising the nucleotide sequence represented by SEQ ID NO:79;
(b) a DNA comprising the nucleotide sequence represented by SEQ ID NO:80;
(c) a DNA comprising the nucleotide sequence represented by SEQ ID NO:81;
(d) a DNA comprising the nucleotide sequence represented by SEQ ID NO:82;
(e) a DNA which hybridizes with a DNA comprising the nucleotide sequence represented by SEQ ID NO:79 under stringent conditions and encodes a protein having an α1,6-fucosyltransferase activity;
(f) a DNA which hybridizes with a DNA comprising the nucleotide sequence represented by SEQ ID NO:80 under stringent conditions and encodes a protein having an α1,6-fucosyltransferase activity;

(g) a DNA which hybridizes with a DNA comprising the nucleotide sequence represented by SEQ ID NO:81 under stringent conditions and encodes a protein having an α1,6-fucosyltransferase activity;
(h) a DNA which hybridizes with a DNA comprising the nucleotide sequence represented by SEQ ID NO:82 under stringent conditions and encodes a protein having an α1,6-fucosyltransferase activity;
(i) a protein comprising the amino acid sequence represented by SEQ ID NO:91;
(j) a protein comprising the amino acid sequence represented by SEQ ID NO:92;
(k) a protein which comprises an amino acid sequence in which at least one amino acid is deleted, substituted, inserted and/or added in the amino acid sequence represented by SEQ ID NO:91 and has an α1,6-fucosyltransferase activity;
(l) a protein which comprises an amino acid sequence in which at least one amino acid is deleted, substituted, inserted and/or added in the amino acid sequence represented by SEQ ID NO:92 and has an α1,6-fucosyltransferase activity;
(m) a protein which comprises an amino acid sequence having a homology of at least 80% with the amino acid sequence represented by SEQ ID NO:91 and has an α1,6-fucosyltransferase activity;
(n) a protein which comprises an amino acid sequence having a homology of at least 80% with the amino acid sequence represented by SEQ ID NO:92 and has an α1,6-fucosyltransferase activity.

**7.** The process according to claim 5 or 6, wherein the RNAi method is a method in which a double-stranded RNA comprising an RNA selected from the group consisting of (a) to (d) and its complementary RNA and being capable of decreasing the amount of mRNA of α1,6-fucosyltransferase is introduced into or expressed in the cell:

(a) an RNA corresponding to a DNA comprising a nucleotide sequence of continuous 10 to 30 nucleotides in the nucleotide sequence represented by SEQ ID NO:79;
(b) an RNA corresponding to a DNA comprising a nucleotide sequence of continuous 10 to 30 nucleotides in the nucleotide sequence represented by SEQ ID NO:80;
(c) an RNA corresponding to a DNA comprising a nucleotide sequence of continuous 10 to 30 nucleotides in the nucleotide sequence represented by SEQ ID NO:81;
(d) an RNA corresponding to a DNA comprising a nucleotide sequence of continuous 10 to 30 nucleotides in the nucleotide sequence represented by SEQ ID NO:82.

**8.** The process according to claim 7, wherein the double-stranded RNA comprising an RNA selected from the group consisting of (a) to (d) and its complementary RNA is introduced into or expressed in the cell to thereby decrease the amount of mRNA of α1,6-fucosyltransferase and provide the cell with the resistance to a lectin which recognizes a sugar chain in which 1-position of fucose is bound to 6-position of *N*-acetylglucosamine in the reducing end through α-bond in a complex *N*-glycoside-linked sugar chain:

(a) an RNA comprising the nucleotide sequence represented by SEQ ID NO:83;
(b) an RNA comprising the nucleotide sequence represented by SEQ ID NO:84;
(c) an RNA which comprises a nucleotide sequence in which one or a few nucleotides are deleted or added in the nucleotide sequence represented by SEQ ID NO:83 and has substantially the same RNAi activity as the RNA represented by SEQ ID NO:83;
(d) an RNA which comprises a nucleotide sequence in which one or a few nucleotides are deleted or added in the nucleotide sequence represented by SEQ ID NO:84 and has substantially the same RNAi activity as the RNA represented by SEQ ID NO:94.

**9.** The process according to claim 7 or 8, wherein the double-stranded RNA is introduced into the cell by using a vector into which a DNA corresponding to the RNA according to claim 7 or 8 and its complementary DNA are inserted.

**10.** The process according to any one of claims 1 to 9, wherein the cell is resistant to at least one lectin selected from the group consisting of the following (a) to (d):

(a) a *Lens culinaris* lectin;
(b) a *Pisum sativum* lectin;
(c) a *Vicia faba* lectin;
(d) an *Aleuria aurantia* lectin.

**11.** The process according to any one of claims 1 to 10, wherein the cell is a cell into which a gene encoding an

antibody molecule is introduced.

**12.** The process according to claim 11, wherein the antibody molecule is selected from the group consisting of the following (a) to (d):

(a) a human antibody;
(b) a humanized antibody;
(c) an antibody fragment comprising the Fc region of (a) or (b);
(d) a fusion protein comprising the Fc region of (a) or (b).

**13.** The process according to claim 11 or 12, wherein the antibody molecule belongs to an IgG class.

**14.** The process according to any one of claims 1 to 13, wherein the antibody composition has a higher antibody-dependent cell-mediated cytotoxic activity than an antibody composition produced by its parent cell.

**15.** The process according to claim 14, wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by its parent cell.

**16.** The process according to claim 15, wherein the ratio of a sugar chain in which fucose is not bound to *N*-acetylglucosamine in the reducing end through $\alpha$-bond is 20% or more of total complex *N*-glycoside-linked sugar chains bound to the Fc region in the antibody composition.

**17.** The process according to claim 15 or 16, wherein the sugar chain in which fucose is not bound is a sugar chain in which 1-position of fucose is not bound to 6-position of *N*-acetylglucosamine in the reducing end in the complex *N*-glycoside-linked sugar chain through $\alpha$-bond.

**18.** The process according to any one of claims 1 to 17, wherein the cell is selected from the group consisting of a yeast, an animal cell, an insect cell and a plant cell.

**19.** The process according to any one of claims 1 to 18, wherein the cell is a mouse myeloma cell.

**20.** The process according to claim 19, wherein the mouse myeloma cell is NS0 cell or SP2/0-Ag14 cell.

**21.** The process according to any one of claims 1 to 18, wherein the cell is selected from the group consisting of the following (a) to (g):

(a) a CHO cell derived from a Chinese hamster ovary tissue;
(b) a rat myeloma cell line YB2/3HL.P2.G11.16Ag.20 cell;
(c) a BHK cell derived from a Syrian hamster kidney tissue;
(d) a hybridoma cell which produces an antibody;
(e) a human leukemic cell line Namalwa cell;
(f) an embryonic stem cell;
(g) a fertilized egg cell.

**22.** A cell into which a gene encoding an antibody composition is introduced, which is resistant to a lectin which recognizes a sugar chain in which 1-position of fucose is bound to 6-position of *N*-acetylglucosamine in the reducing end through $\alpha$-bond in a complex *N*-glycoside-linked sugar chain.

**23.** The cell according to claim 22, wherein the activity of a protein selected from the group consisting of the following (a), (b) and (c) is more decreased or deleted than its parent cell:

(a) an enzyme protein relating to synthesis of an intracellular sugar nucleotide, GDP-fucose;
(b) an enzyme protein relating to modification of a sugar chain in which 1-position of fucose is bound to 6-position of *N*-acetylglucosamine in the reducing end through $\alpha$-bond in a complex *N*-glycoside-linked sugar chain;
(c) a protein relating to transport of an intracellular sugar nucleotide, GDP-fucose, to the Golgi body.

**24.** The cell according to claim 23, wherein the enzyme protein relating to modification of a sugar chain in which 1-position of fucose is bound to 6-position of *N*-acetylglucosamine in the reducing end through α-bond in a complex *N*-glycoside-linked sugar chain is α1,6-fucosyltransferase.

**25.** The cell according to claim 23 or 24, wherein the activity of the protein is decreased or deleted by a technique selected from the group consisting of (a) to (d):

(a) a gene disruption technique which comprises targeting a gene encoding the protein;
(b) a technique for introducing a dominant negative mutant of a gene encoding the protein;
(c) a technique for introducing mutation into the protein;
(d) a technique for suppressing transcription and/or translation of a gene encoding the protein.

**26.** The cell according to claim 25, wherein the technique for suppressing transcription and/or translation of a gene encoding the protein is an RNAi method.

**27.** The cell according to any one of claims 24 to 26, wherein the α1,6-fucosyltransferase is a protein encoded by a DNA selected from the group consisting of (a) to (h) or a protein selected from the group consisting of the following (i) to (n):

(a) a DNA comprising the nucleotide sequence represented by SEQ ID NO:79;
(b) a DNA comprising the nucleotide sequence represented by SEQ ID NO:80;
(c) a DNA comprising the nucleotide sequence represented by SEQ ID NO:81;
(d) a DNA comprising the nucleotide sequence represented by SEQ ID NO:82;
(e) a DNA which hybridizes with a DNA comprising the nucleotide sequence represented by SEQ ID NO:79 under stringent conditions and encodes a protein having an α1,6-fucosyltransferase activity;
(f) a DNA which hybridizes with a DNA comprising the nucleotide sequence represented by SEQ ID NO:80 under stringent conditions and encodes a protein having an α1,6-fucosyltransferase activity;
(g) a DNA which hybridizes with a DNA comprising the nucleotide sequence represented by SEQ ID NO:81 under stringent conditions and encodes a protein having an α1,6-fucosyltransferase activity;
(h) a DNA which hybridizes with a DNA comprising the nucleotide sequence represented by SEQ ID NO:82 under stringent conditions and encodes a protein having an α1,6-fucosyltransferase activity;
(i) a protein comprising the amino acid sequence represented by SEQ ID NO:91;
(j) a protein comprising the amino acid sequence represented by SEQ ID NO:92;
(k) a protein which comprises an amino acid sequence in which at least one amino acid is deleted, substituted, inserted and/or added in the amino acid sequence represented by SEQ ID NO:91 and has an α1,6-fucosyltransferase activity;
(l) a protein which comprises an amino acid sequence in which at least one amino acid is deleted, substituted, inserted and/or added in the amino acid sequence represented by SEQ ID NO:92 and has an α1,6-fucosyltransferase activity;
(m) a protein which comprises an amino acid sequence having a homology of at least 80% with the amino acid sequence represented by SEQ ID NO:91 and has an α1,6-fucosyltransferase activity;
(n) a protein which comprises an amino acid sequence having a homology of at least 80% with the amino acid sequence represented by SEQ ID NO:92 and has an α1,6-fucosyltransferase activity.

**28.** The cell according to claim 26 or 27, wherein the RNAi method is a method in which a double-stranded RNA comprising an RNA selected from the group consisting of (a) to (d) and its complementary RNA and being capable of decreasing the amount of mRNA of α1,6-fucosyltransferase is introduced into or expressed in the cell:

(a) an RNA corresponding to a DNA comprising a nucleotide sequence of continuous 10 to 30 nucleotides in the nucleotide sequence represented by SEQ ID NO:79;
(b) an RNA corresponding to a DNA comprising a nucleotide sequence of continuous 10 to 30 nucleotides in the nucleotide sequence represented by SEQ ID NO:80;
(c) an RNA corresponding to a DNA comprising a nucleotide sequence of continuous 10 to 30 nucleotides in the nucleotide sequence represented by SEQ ID NO:81;
(d) an RNA corresponding to a DNA comprising a nucleotide sequence of continuous 10 to 30 nucleotides in the nucleotide sequence represented by SEQ ID NO:82.

**29.** The cell according to claim 28, wherein the double-stranded RNA comprising an RNA selected from the group

consisting of (a) to (d) and its complementary RNA is introduced into or expressed in the cell to thereby decrease the amount of mRNA of α1,6-fucosyltransferase and provide the cell with the resistance to a lectin which recognizes a sugar chain in which 1-position of fucose is bound to 6-position of *N*-acetylglucosamine in the reducing end through α-bond in a complex *N*-glycoside-linked sugar chain:

(a) an RNA comprising the nucleotide sequence represented by SEQ ID NO:83;
(b) an RNA comprising the nucleotide sequence represented by SEQ ID NO:84;
(c) an RNA which comprises a nucleotide sequence in which one or a few nucleotides are deleted or added in the nucleotide sequence represented by SEQ ID NO:83 and has substantially the same RNAi activity as the RNA represented by SEQ ID NO:83;
(d) an RNA which comprises a nucleotide sequence in which one or a few nucleotides are deleted or added in the nucleotide sequence represented by SEQ ID NO:84 and has substantially the same RNAi activity as the RNA represented by SEQ ID NO:84.

**30.** The process according to claim 28 or 29, wherein the double-stranded RNA is introduced into the cell by using a vector into which a DNA corresponding to the RNA according to claim 28 or 29 and its complementary DNA are inserted.

**31.** The cell according to any one of claims 22 to 30, wherein the cell is resistant to at least one lectin selected from the group consisting of the following (a) to (d):

(a) a *Lens culinaris* lectin;
(b) a *Pisum sativum* lectin;
(c) a *Vicia faba* lectin;
(d) an *Aleuria auranlia* lectin.

**32.** The cell according to any one of claims 22 to 31, wherein the antibody molecule is selected from the group consisting of the following (a), (b), (c) and (d):

(a) a human antibody;
(b) a humanized antibody;
(c) an antibody fragment comprising the Fc region of (a) or (b);
(d) a fusion protein comprising the Fc region of (a) or (b).

**33.** The cell according to any one of claims 22 to 32, wherein the antibody molecule belongs to an IgG class.

**34.** The cell according to any one of claims 22 to 33, wherein the antibody composition has a higher antibody-dependent cell-mediated cytotoxic activity than an antibody composition produced by its parent cell.

**35.** The cell according to claim 34, wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by its parent cell.

**36.** The cell according to claim 35, wherein the ratio of a sugar chain in which fucose is not bound to *N*-acetylglucosamine in the reducing end through α-bond is 20% or more of total complex *N*-glycoside-linked sugar chains bound to the Fc region in the antibody composition.

**37.** The cell according to claim 35, wherein the sugar chain in which fucose is not bound is a sugar chain in which 1-position of fucose is not bound to 6-position of *N*-acetylglucosamine in the reducing end in the complex *N*-glycoside-linked sugar chain through α-bond.

**38.** The cell according to any one of claims 22 to 37, wherein the cell is selected from the group consisting of a yeast, an animal cell, an insect cell and a plant cell.

**39.** The cell according to any one of claims 22 to 37, wherein the cell is a mouse myeloma cell.

**40.** The cell according to claim 39, wherein the mouse myeloma cell is NS0 cell or SP2/0-Ag14 cell.

**41.** The cell according to any one of claims 22 to 38, wherein the cell is selected from the group consisting of the following (a) to (g):

(a) a CHO cell derived from a Chinese hamster ovary tissue;
(b) a rat myeloma cell line YB2/3HL.P2.G11.16Ag.20 cell;
(c) a BHK cell derived from a Syrian hamster kidney tissue;
(d) a hybridoma cell which produces an antibody;
(e) a human leukemic cell line Namalwa cell;
(f) an embryonic stem cell;
(g) a fertilized egg cell.

**42.** A process for producing an antibody composition, which comprises culturing the cell according to any one of claims 22 to 41 in a medium to form and accumulate an antibody composition in a culture; and recovering the antibody composition from the culture.

**43.** The process according to claim 42, which produces an antibody composition having a higher antibody-dependent cell-mediated cytotoxic activity than an antibody composition produced by its parent cell.

**44.** The process according to claim 43, wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by its parent cell.

**45.** The process according to claim 44, wherein the ratio of a sugar chain in which fucose is not bound to *N*-acetylglucosamine in the reducing end through α-bond is 20% or more of total complex *N*-glycoside-linked sugar chains bound to the Fc region in the antibody composition.

**46.** The process according to claim 44 or 45, wherein the sugar chain in which fucose is not bound is a sugar chain in which 1-position of the fucose is not bound to 6-position of *N*-acetylglucosamine in the reducing end through α-bond in a complex *N*-glycoside-linked sugar chain.

**47.** A mouse myeloma cell resistant to a lectin which recognizes a sugar chain in which 1-position of fucose is bound to 6-position of *N*-acetylglucosamine in the reducing end through α-bond in a complex *N*-glycoside-linked sugar chain.

**48.** The mouse myeloma cell according to claim 47, wherein the activity of a protein selected from the group consisting of the following (a), (b) and (c) is more decreased or deleted than its parent cell:

(a) an enzyme protein relating to synthesis of an intracellular sugar nucleotide, GDP-fucose;
(b) an enzyme protein relating to modification of a sugar chain in which 1-position of fucose is bound to 6-position of *N*-acetylglucosamine in the reducing end through α-bond in a complex *N*-glycoside-linked sugar chain;
(c) a protein relating to transport of an intracellular sugar nucleotide, GDP-fucose, to the Golgi body.

**49.** The mouse myeloma cell according to claim 48, wherein the enzyme protein relating to modification of a sugar chain in which 1-position of fucose is bound to 6-position of *N*-acetylglucosamine in the reducing end through α-bond in a complex *N*-glycoside-linked sugar chain is α1,6-fucosyltransferase.

**50.** The mouse myeloma cell according to claim 48 or 49, wherein the activity of the protein is decreased or deleted by a technique selected from the group consisting of (a) to (d):

(a) a gene disruption technique which comprises targeting a gene encoding the protein;
(b) a technique for introducing a dominant negative mutant of a gene encoding the protein;
(c) a technique for introducing mutation into the protein;
(d) a technique for suppressing transcription and/or translation of a gene encoding the protein.

**51.** The mouse myeloma cell according to claim 50, wherein the technique for suppressing transcription and/or translation of a gene encoding the protein is an RNAi method.

**52.** The mouse myeloma cell according to any one of claims 49 to 51, wherein the α1,6-fucosyltransferase is a protein encoding a DNA selected from the group consisting of (a) to (h) or a protein selected from the group consisting of the following (i) to (n):

(a) a DNA comprising the nucleotide sequence represented by SEQ ID NO:79;
(b) a DNA comprising the nucleotide sequence represented by SEQ ID NO:80;
(c) a DNA comprising the nucleotide sequence represented by SEQ ID NO:81;
(d) a DNA comprising the nucleotide sequence represented by SEQ ID NO:82;
(e) a DNA which hybridizes with a DNA comprising the nucleotide sequence represented by SEQ ID NO:79 under stringent conditions and encodes a protein having an α1,6-fucosyltransferase activity;
(f) a DNA which hybridizes with a DNA comprising the nucleotide sequence represented by SEQ ID NO:80 under stringent conditions and encodes a protein having an α1,6-fucosyltransferase activity;
(g) a DNA which hybridizes with a DNA comprising the nucleotide sequence represented by SEQ ID NO:81 under stringent conditions and encodes a protein having an α1,6-fucosyltransferase activity;
(h) a DNA which hybridizes with a DNA comprising the nucleotide sequence represented by SEQ ID NO:82 under stringent conditions and encodes a protein having an α1,6-fucosyltransferase activity;
(i) a protein comprising the amino acid sequence represented by SEQ ID NO:91;
(j) a protein comprising the amino acid sequence represented by SEQ ID NO:92;
(k) a protein which comprises an amino acid sequence in which at least one amino acid is deleted, substituted, inserted and/or added in the amino acid sequence represented by SEQ ID NO:91 and has an α1,6-fucosyltransferase activity;
(l) a protein which comprises an amino acid sequence in which at least one amino acid is deleted, substituted, inserted and/or added in the amino acid sequence represented by SEQ ID NO:92 and has an α1,6-fucosyltransferase activity;
(m) a protein which comprises an amino acid sequence having a homology of at least 80% with the amino acid sequence represented by SEQ ID NO:91 and has an α1,6-fucosyltransferase activity;
(n) a protein which comprises an amino acid sequence having a homology of at least 80% with the amino acid sequence represented by SEQ ID NO:92 and has an α1,6-fucosyltransferase activity.

**53.** The mouse myeloma cell according to claim 51 or 52, wherein the RNAi method is a method in which a double-stranded RNA comprising a RNA selected from the group consisting of (a) to (d) and its complementary RNA and being capable of decreasing the amount of mRNA of α1,6-fucosyltransferase is introduced into or expressed in the cell:

(a) an RNA corresponding to a DNA comprising a nucleotide sequence of continuous 10 to 30 nucleotides in the nucleotide sequence represented by SEQ ID NO:79;
(b) an RNA corresponding to a DNA comprising a nucleotide sequence of continuous 10 to 30 nucleotides in the nucleotide sequence represented by SEQ ID NO:80;
(c) an RNA corresponding to a DNA comprising a nucleotide sequence of continuous 10 to 30 nucleotides in the nucleotide sequence represented by SEQ ID NO:81;
(d) an RNA corresponding to a DNA comprising a nucleotide sequence of continuous 10 to 30 nucleotides in the nucleotide sequence represented by SEQ ID NO:82.

**54.** The mouse myeloma cell according to claim 51, wherein the double-stranded RNA comprising an RNA selected from the group consisting of (a) to (d) and its complementary RNA is introduced into or expressed in the cell to thereby decrease the amount of mRNA of α1,6-fucosyltransferase and provide the cell with the resistance to a lectin which recognizes a sugar chain in which 1-position of fucose is bound to 6-position of *N*-acetylglucosamine in the reducing end through α-bond in a complex *N*-glycoside-linked sugar chain:

(a) an RNA comprising the nucleotide sequence represented by SEQ ID NO:83;
(b) an RNA comprising the nucleotide sequence represented by SEQ ID NO:84;
(c) an RNA which comprises a nucleotide sequence in which one or a few nucleotides are deleted or added in the nucleotide sequence represented by SEQ ID NO:83 and has substantially the same RNAi activity as the RNA represented by SEQ ID NO:83;
(d) an RNA which comprises a nucleotide sequence in which one or a few nucleotides are deleted or added in the nucleotide sequence represented by SEQ ID NO:84 and has substantially the same RNAi activity as the RNA represented by SEQ ID NO:84.

**55.** The mouse myeloma cell according to claim 50 or 51, wherein the double-stranded RNA is introduced into the cell by using a vector into which a DNA corresponding to the RNA according to claim 53 or 54 and its complementary DNA are inserted.

**56.** The mouse myeloma cell according to any one of claims 47 to 55, wherein the cell is resistant to at least one lectin selected from the group consisting of the following (a) to (d):

(a) a *Lens culinaris* lectin;
(b) a *Pisum sativum* lectin;
(c) a *Vicia faba* lectin;
(d) an *Aleuria aurantia* lectin.

**57.** The mouse myeloma cell according to any one of claims 47 to 56, wherein the mouse myeloma cell is selected from the group consisting of the following (a) and (b):

(a) NS0 cell;
(b) SP2/0-Ag14 cell.

**58.** The cell according to any one of claims 47 to 57, wherein the mouse myeloma cell is a cell into which a gene encoding an antibody molecule is introduced.

**59.** The cell according to claim 58, wherein the antibody molecule is selected from the group consisting of the following (a), (b), (c) and (d):

(a) a human antibody;
(b) a humanized antibody;
(c) an antibody fragment comprising the Fc region of (a) or (b);
(d) a fusion protein comprising the Fc region of (a) or (b).

**60.** The cell according to claim 58 or 59, wherein the antibody molecule belongs to an IgG class.

**61.** The cell according to any one of claims 58 to 60, wherein the antibody composition has a higher antibody-dependent cell-mediated cytotoxic activity than an antibody composition produced by its parent cell.

**62.** The cell according to claim 61, wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by its parent cell.

**63.** The cell according to claim 62, wherein the ratio of a sugar chain in which fucose is not bound to *N*-acetylglucosamine in the reducing end through α-bond is 20% or more of total complex *N*-glycoside-linked sugar chains bound to the Fc region in the antibody composition.

**64.** The cell according to claim 62, wherein the sugar chain in which fucose is not bound is a sugar chain in which 1-position of fucose is not bound to 6-position of *N*-acetylglucosamine in the reducing end in the complex *N*-glycoside-linked sugar chain through α-bond.

**65.** A process for producing an antibody composition, which comprises culturing the cell according to any one of claims 58 to 64 in a medium to form and accumulate an antibody composition in the culture; and recovering the antibody composition from the culture.

**66.** The process according to claim 65, which produces an antibody composition having a higher antibody-dependent cell-mediated cytotoxic activity than an antibody composition produced by its parent cell.

**67.** The process according to claim 66, wherein the antibody composition having a higher antibody-dependent cell-mediated cytotoxic activity has a higher ratio of a sugar chain in which fucose is not bound to *N*-acetylglucosamine in the reducing end in the sugar chain among total complex *N*-glycoside-linked sugar chains bound to the Fc region in the antibody composition than an antibody composition produced by its parent cell.

**68.** The process according to claim 67, wherein the ratio of a sugar chain in which fucose is not bound to *N*-acetylglucosamine in the reducing end through $\alpha$-bond is 20% or more of total complex *N*-glycoside-linked sugar chains bound to the Fc region in the antibody composition.

**69.** The process according to claim 67 or 68, wherein the sugar chain in which fucose is not bound is a sugar chain in which 1-position of fucose is not bound to 6-position of *N*-acetylglucosamine in the reducing end in the complex *N*-glycoside-linked sugar chain through $\alpha$-bond.

**70.** An antibody composition produced by the process according to any one of claims 1 to 21.

**71.** An antibody composition produced by the process according to any one of claims 42 to 46.

**72.** An antibody composition produced by the process according to any one of claims 65 to 69.

**73.** A medicament comprising as an active ingredient the antibody composition according to any one of claims 70 to 72.

**74.** The medicament according to claim 73, which is a diagnostic agent, an preventing agent or a treating agent for tumor-accompanied diseases, allergy-accompanied diseases, inflammatory-accompanied diseases, autoimmune diseases, cardiovascular diseases, viral infection-accompanied diseases or bacterial infection-accompanied diseases.

**75.** A double-stranded RNA comprising an RNA selected from the group consisting of (a) to (d) and its complementary RNA:

(a) an RNA comprising the nucleotide sequence represented by SEQ ID NO:83;
(b) an RNA comprising the nucleotide sequence represented by SEQ ID NO:84;
(c) an RNA which comprises a nucleotide sequence in which one or a few nucleotides are deleted or added in the nucleotide sequence represented by SEQ ID NO:83 and has substantially the same RNAi activity as the RNA represented by SEQ ID NO:83;
(d) an RNA which comprises a nucleotide sequence in which one or a few nucleotides are deleted or added in the nucleotide sequence represented by SEQ ID NO:84 and has substantially the same RNAi activity as the RNA represented by SEQ ID NO:84.

**76.** A DNA corresponding to the RNA according to claim 75 and its complementary DNA.

**77.** The DNA according to claim 76, the DNA corresponding to the RNA is the nucleotide sequence represented by SEQ ID NO:63 or 64.

**78.** A recombinant DNA comprising the DNA according to claim 76 or 77 and its complementary DNA.

**79.** The recombinant DNA according to claim 78, which is constructed for expressing the double-stranded RNA according to claim 75.

**80.** A transformant obtainable by introducing the recombinant DNA according to claim 78 or 79 into a cell.

**81.** Use of the antibody composition according to any one of claims 70 to 72 in the manufacture of the medicament according to claim 73 or 74.

FIG. 1

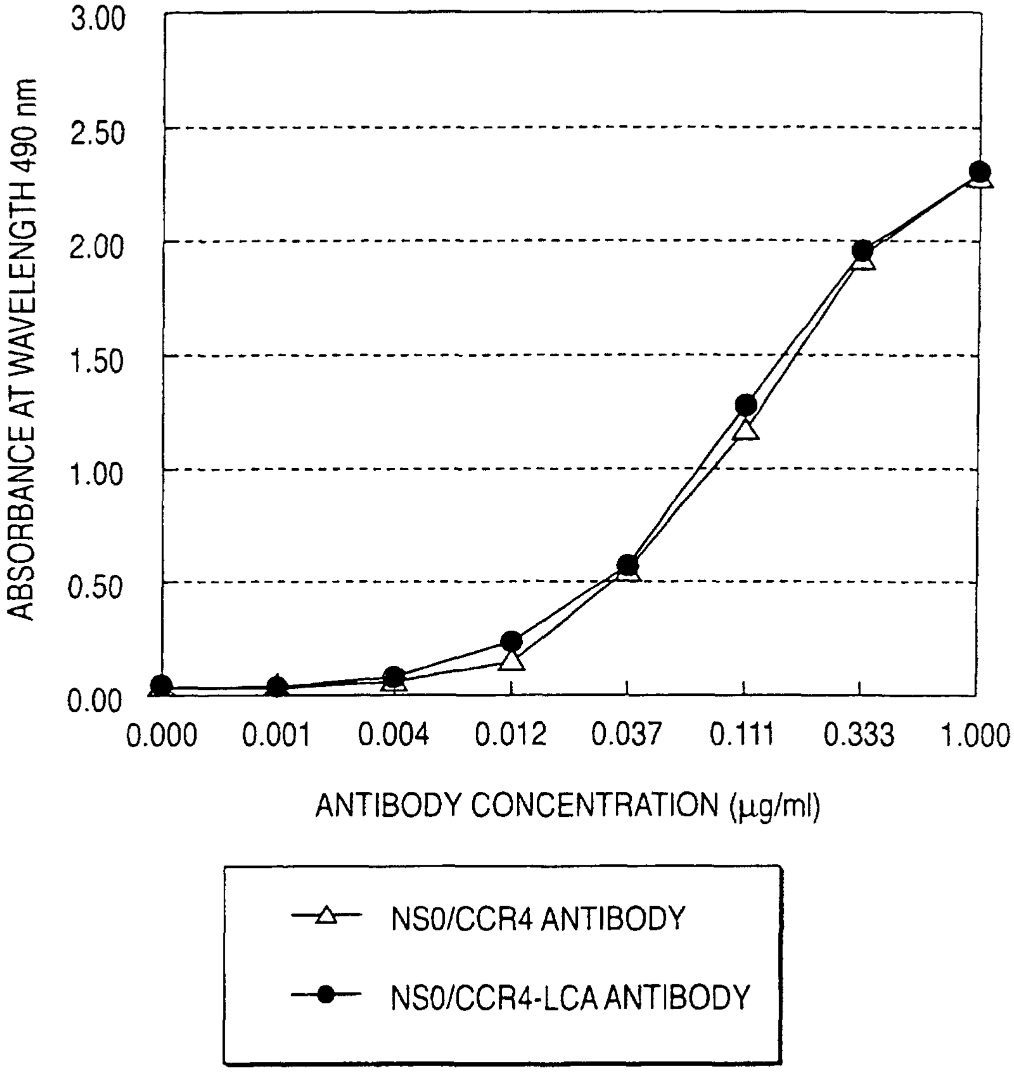
3.00
2.50
2.00
1.50
1.00
0.50
0.00
ABSORBANCE AT WAVELENGTH 490 nm
0.000
0.001
0.004
0.012
0.037
0.111
0.333
1.000
ANTIBODY CONCENTRATION (μg/ml)
NS0/CCR4 ANTIBODY
NS0/CCR4-LCA ANTIBODY

FIG. 2

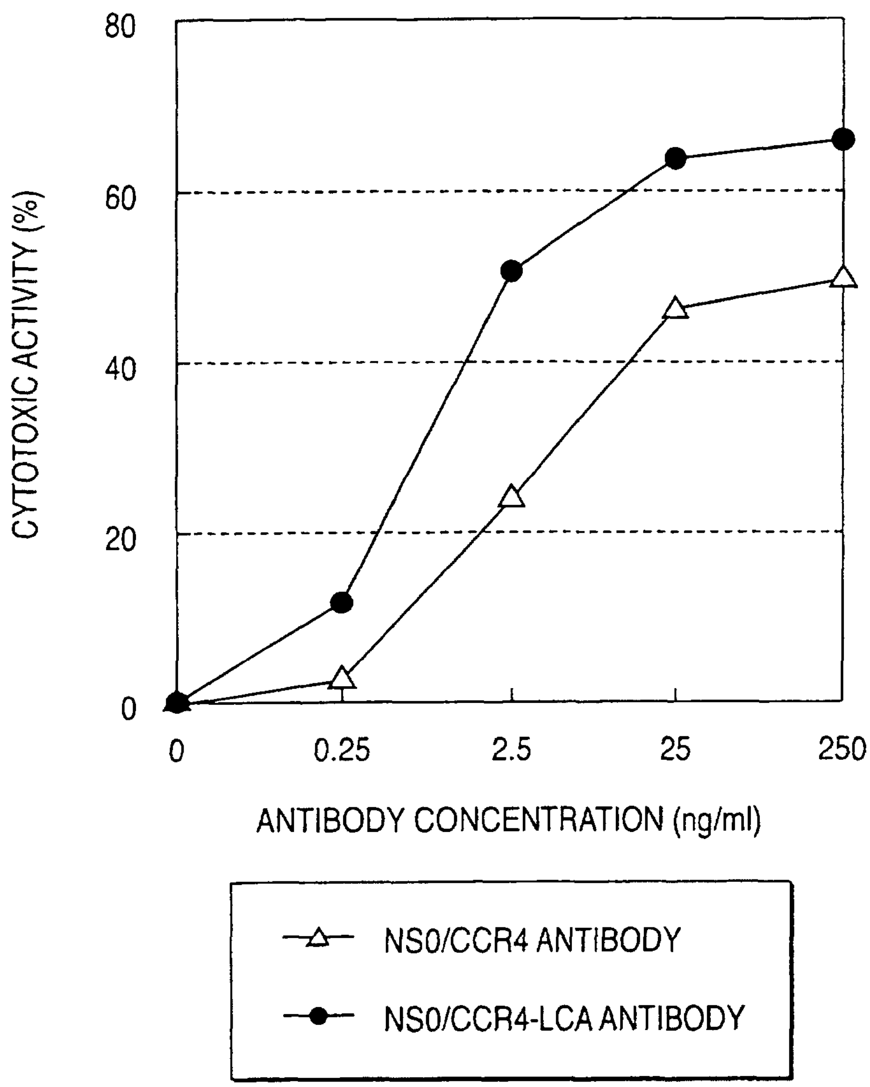
80
60
40
20
0
CYTOTOXIC ACTIVITY (%)
0
0.25
2.5
25
250
ANTIBODY CONCENTRATION (ng/ml)
NS0/CCR4 ANTIBODY
NS0/CCR4-LCA ANTIBODY

FIG. 3

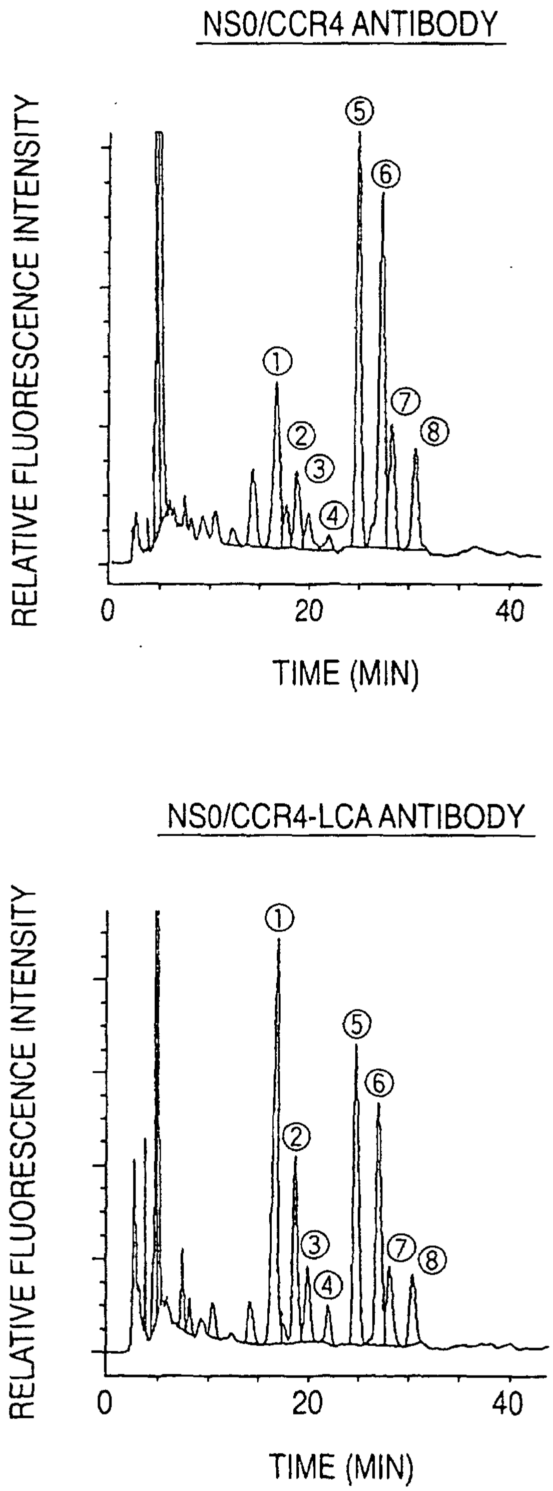
NS0/CCR4 ANTIBODY
RELATIVE FLUORESCENCE INTENSITY
①
②
③
④
⑤
⑥
⑦
⑧
0
20
40
TIME (MIN)
NS0/CCR4-LCA ANTIBODY
RELATIVE FLUORESCENCE INTENSITY
①
②
③
④
⑤
⑥
⑦
⑧
0
20
40
TIME (MIN)

FIG. 4

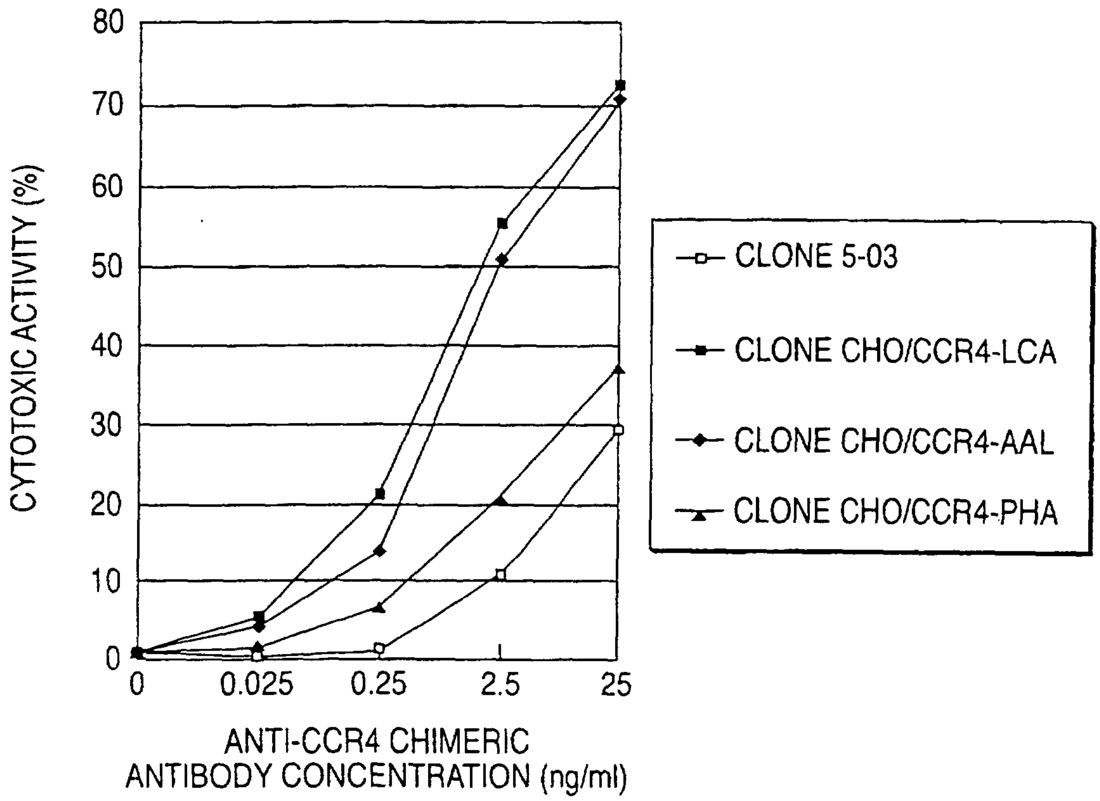
80
70
60
50
40
30
20
10
0
CYTOTOXIC ACTIVITY (%)
0
0.025
0.25
2.5
25
ANTI-CCR4 CHIMERIC
ANTIBODY CONCENTRATION (ng/ml)
CLONE 5-03
CLONE CHO/CCR4-LCA
CLONE CHO/CCR4-AAL
CLONE CHO/CCR4-PHA

FIG. 5

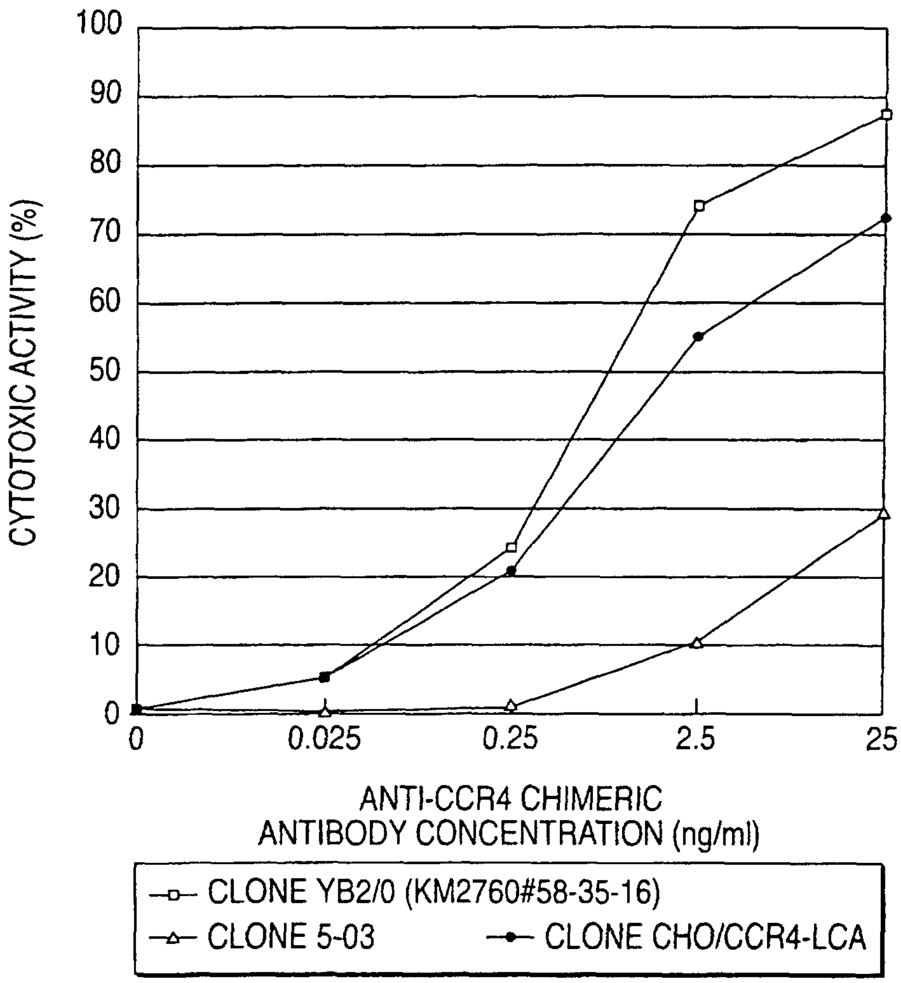
100
90
80
70
60
50
40
30
20
10
0
CYTOTOXIC ACTIVITY (%)
0
0.025
0.25
2.5
25
ANTI-CCR4 CHIMERIC
ANTIBODY CONCENTRATION (ng/ml)
CLONE YB2/0 (KM2760#58-35-16)
CLONE 5-03
CLONE CHO/CCR4-LCA

FIG. 6

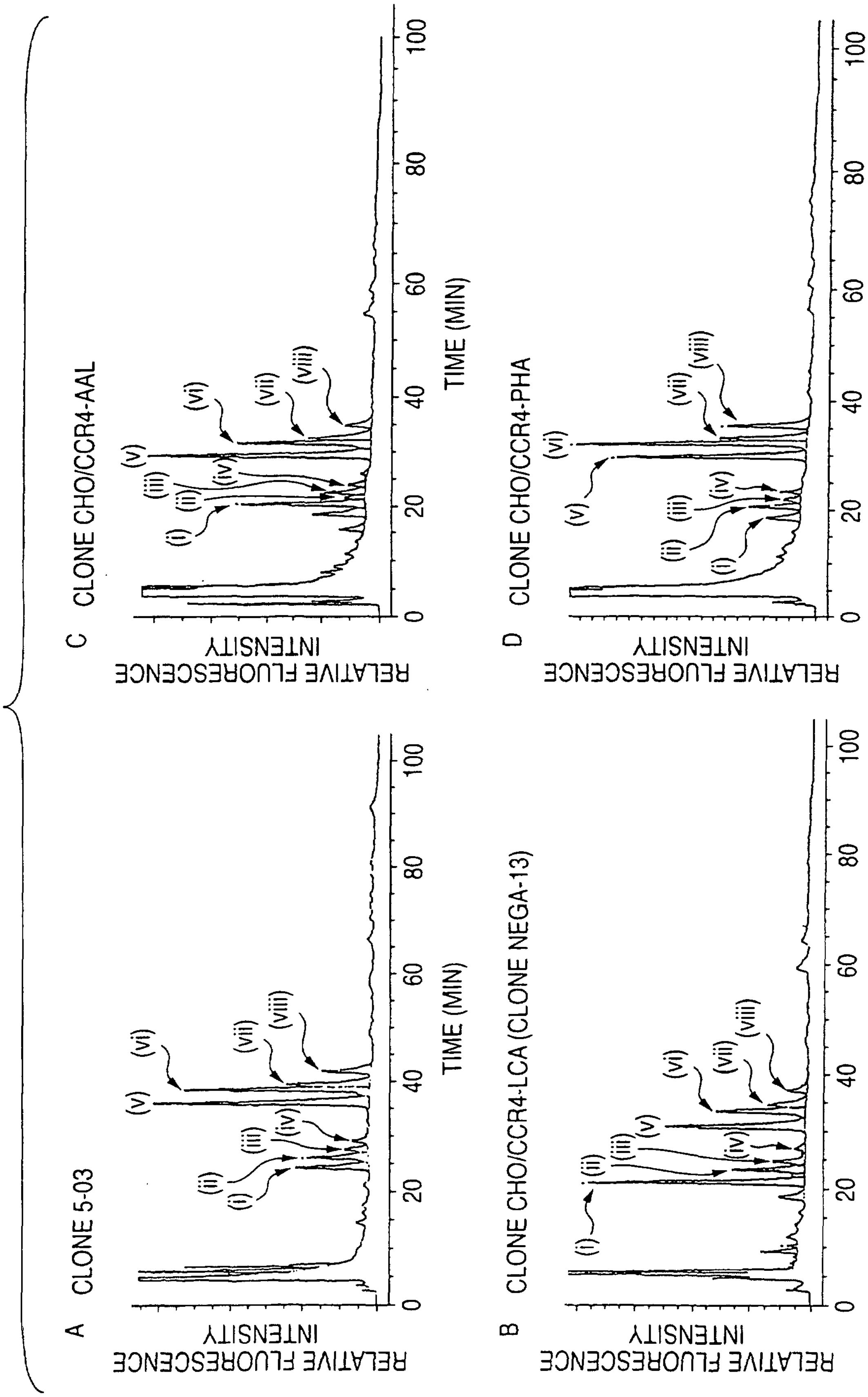
A
CLONE 5-03
B
CLONE CHO/CCR4-LCA (CLONE NEGA-13)
C
CLONE CHO/CCR4-AAL
D
CLONE CHO/CCR4-PHA
RELATIVE FLUORESCENCE INTENSITY
TIME (MIN)
0
20
40
60
80
100
(i)
(ii)
(iii)
(iv)
(v)
(vi)
(vii)
(viii)

FIG. 7

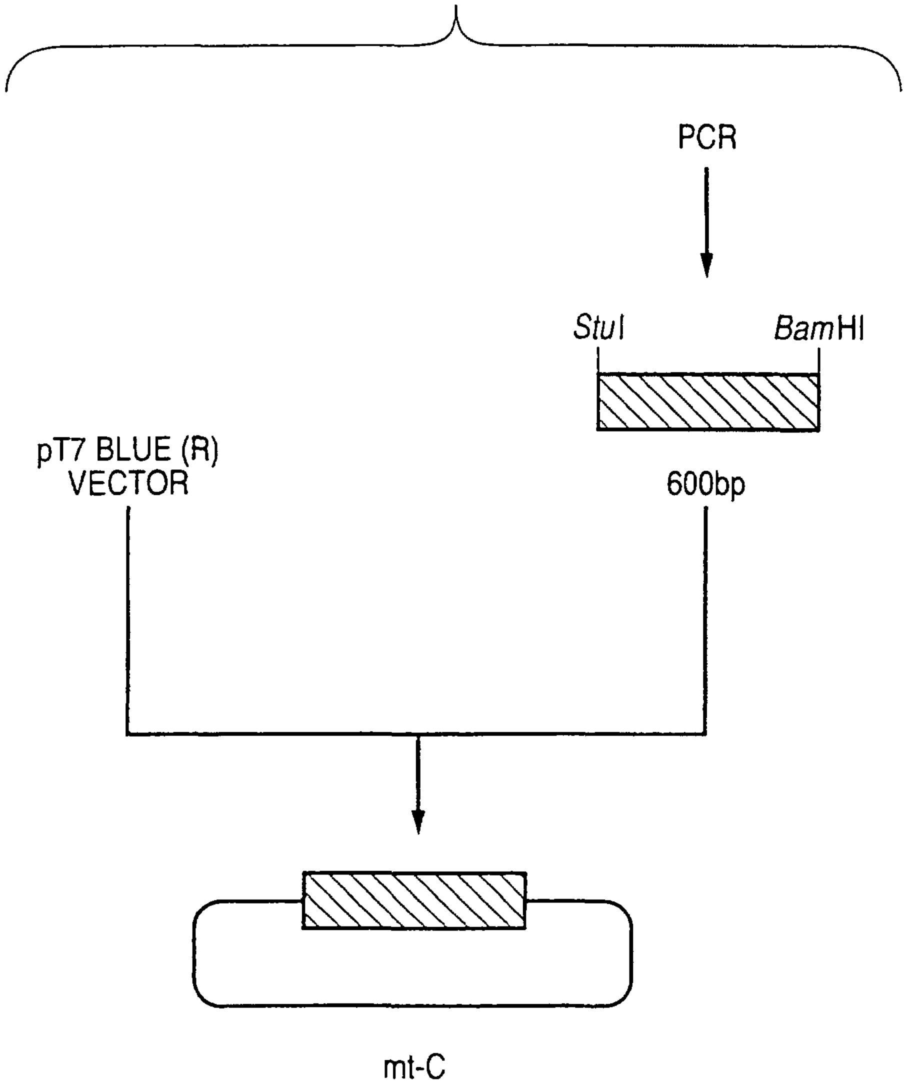

FIG. 8

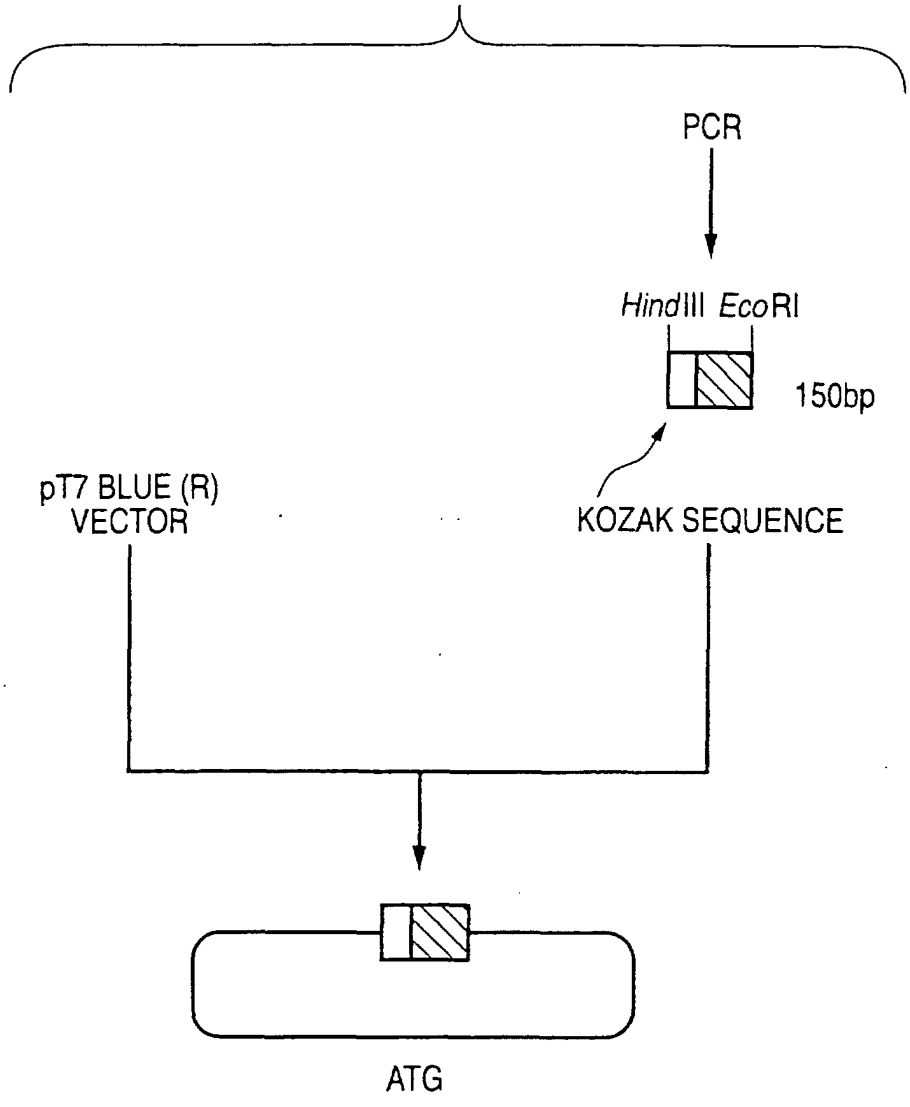
PCR
*Hind*III *Eco*RI
150bp
pT7 BLUE (R)
VECTOR
KOZAK SEQUENCE
ATG

FIG. 9

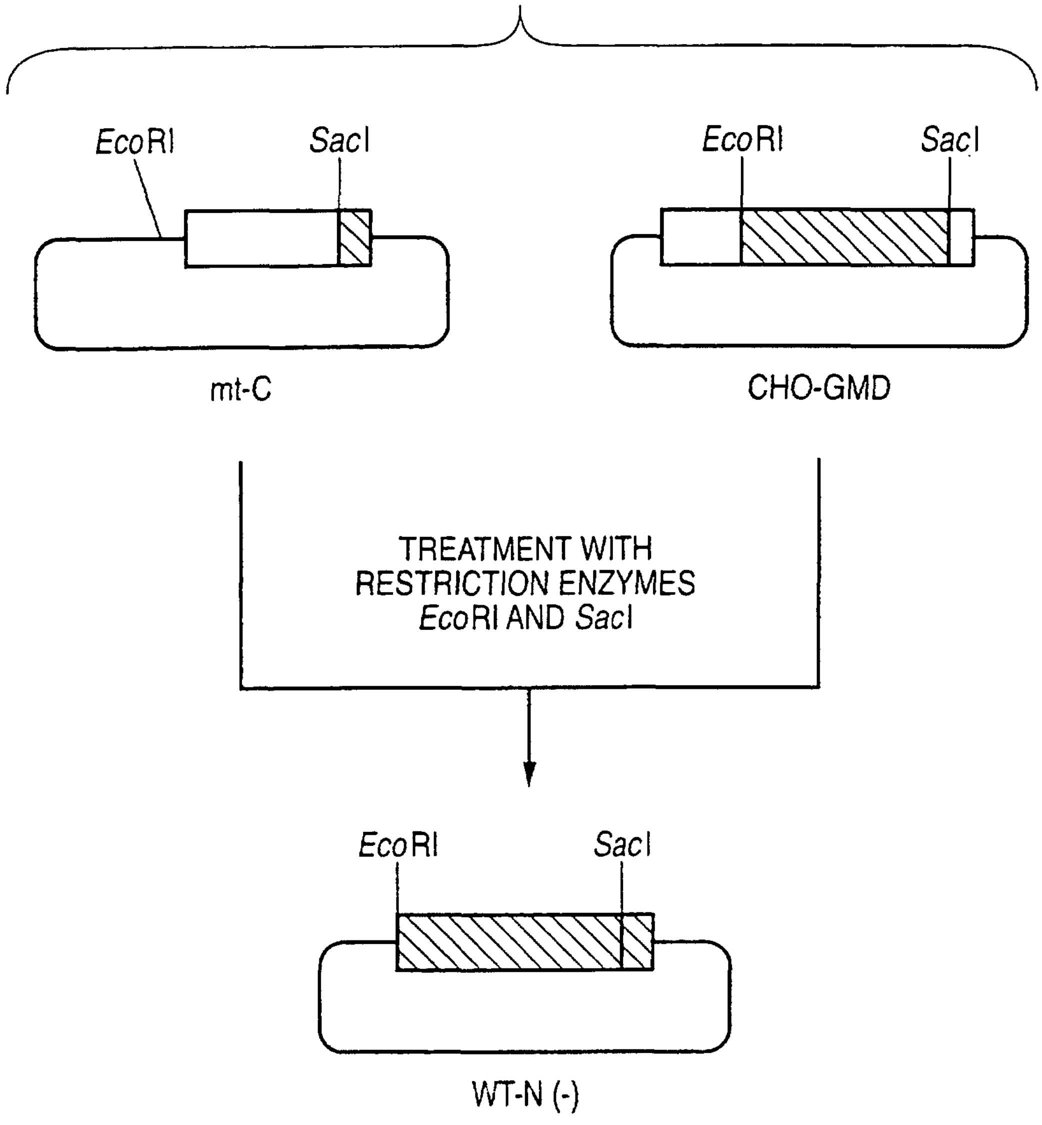
EcoRI
SacI
mt-C
EcoRI
SacI
CHO-GMD
TREATMENT WITH
RESTRICTION ENZYMES
EcoRI AND SacI
EcoRI
SacI
WT-N (-)

FIG. 10

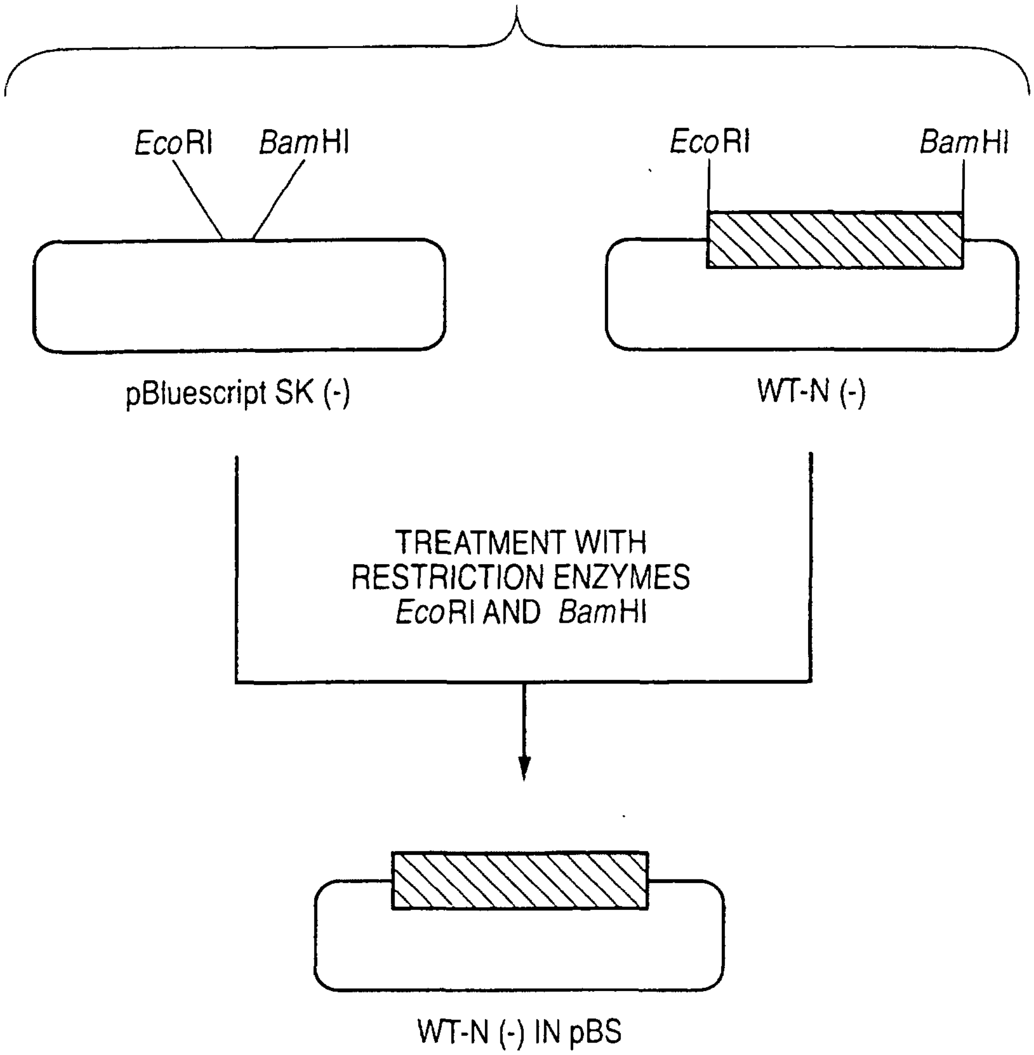
*Eco*RI
*Bam*HI
*Eco*RI
*Bam*HI
pBluescript SK (-)
WT-N (-)
TREATMENT WITH
RESTRICTION ENZYMES
*Eco*RI AND *Bam*HI
WT-N (-) IN pBS

FIG. 11

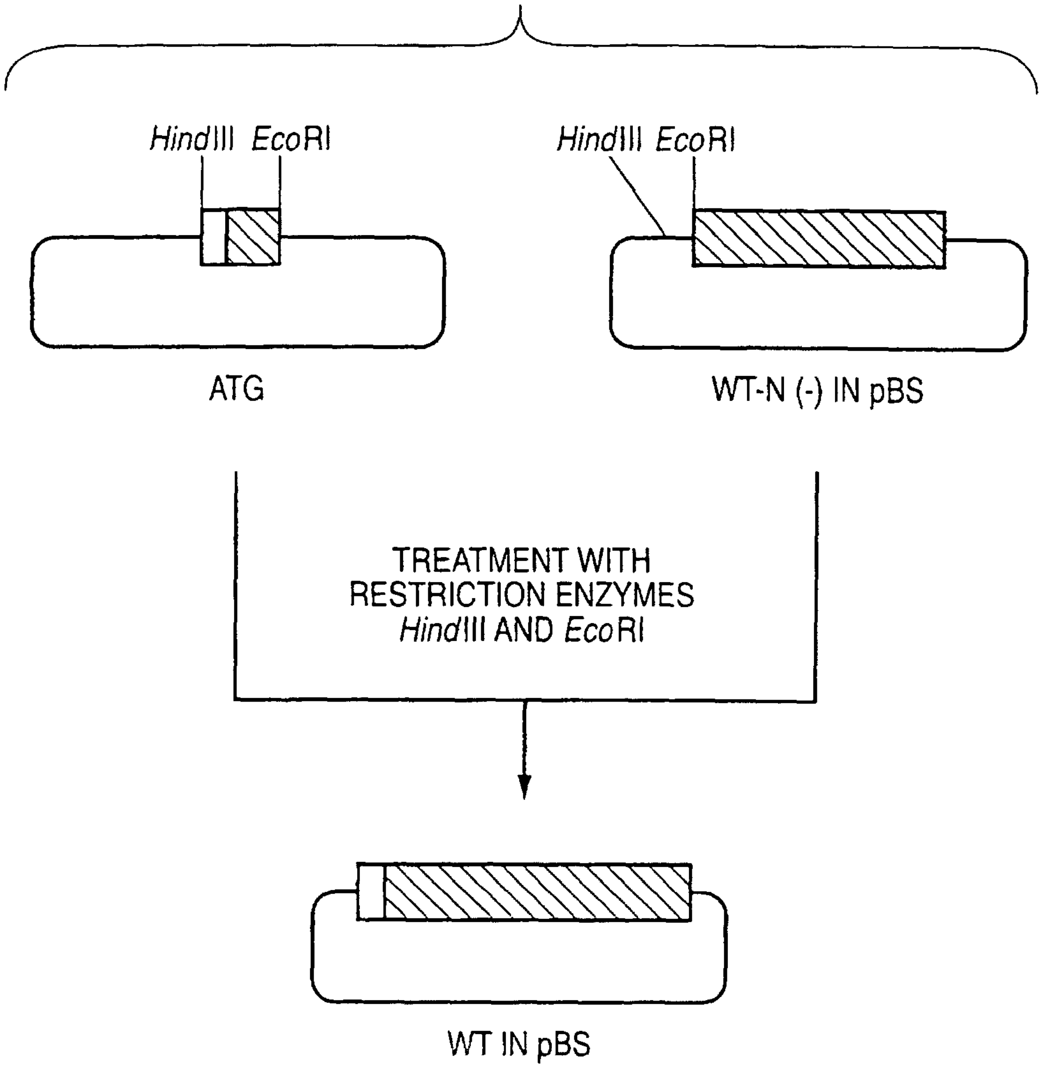
*Hind*III *Eco*RI
*Hind*III *Eco*RI
ATG
WT-N (-) IN pBS
TREATMENT WITH
RESTRICTION ENZYMES
*Hind*III AND *Eco*RI
WT IN pBS

FIG.12

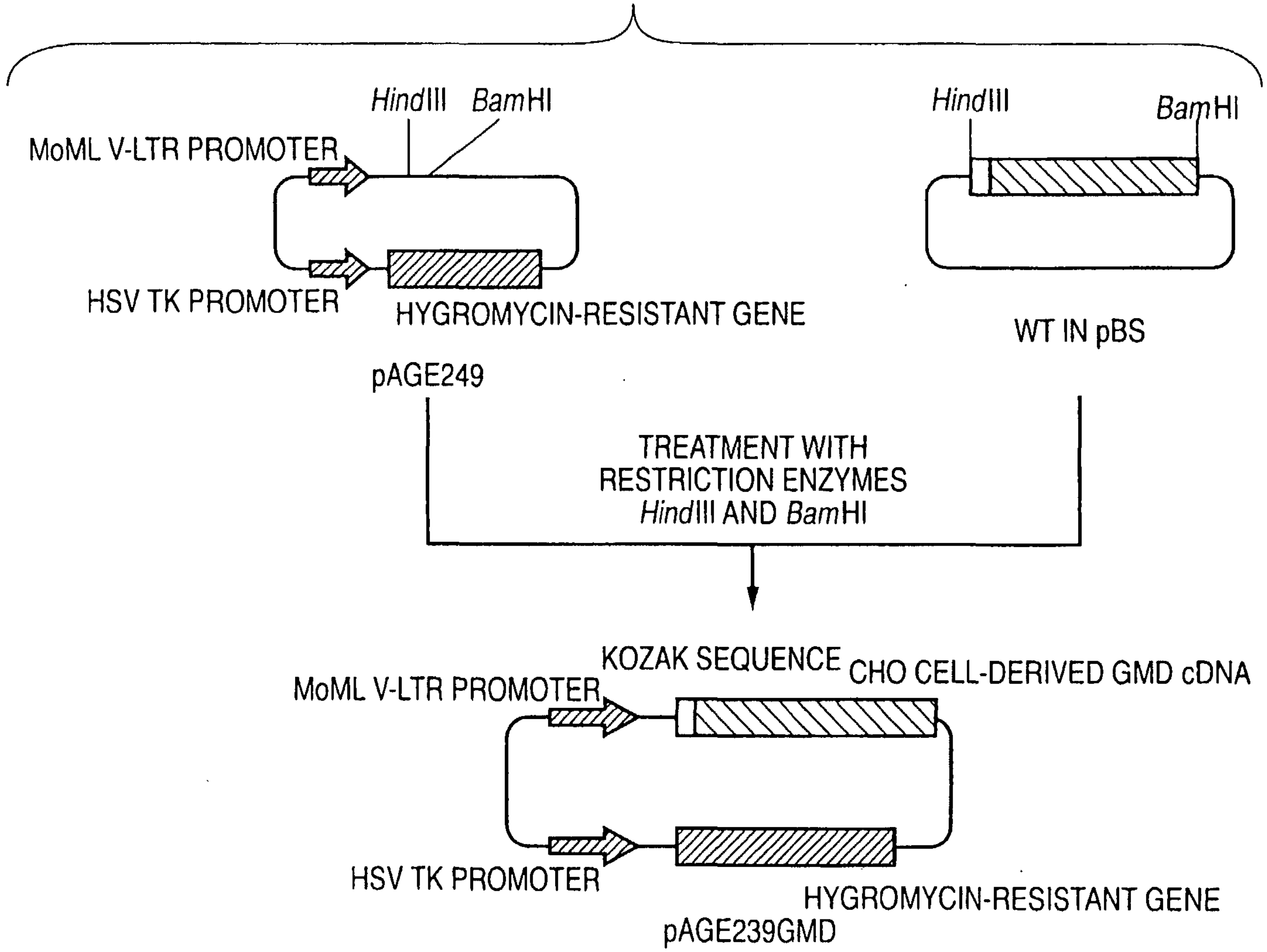
HindIII
BamHI
MoML V-LTR PROMOTER
HSV TK PROMOTER
HYGROMYCIN-RESISTANT GENE
pAGE249
HindIII
BamHI
WT IN pBS
TREATMENT WITH
RESTRICTION ENZYMES
HindIII AND BamHI
KOZAK SEQUENCE
CHO CELL-DERIVED GMD cDNA
MoML V-LTR PROMOTER
HSV TK PROMOTER
HYGROMYCIN-RESISTANT GENE
pAGE239GMD

FIG. 13

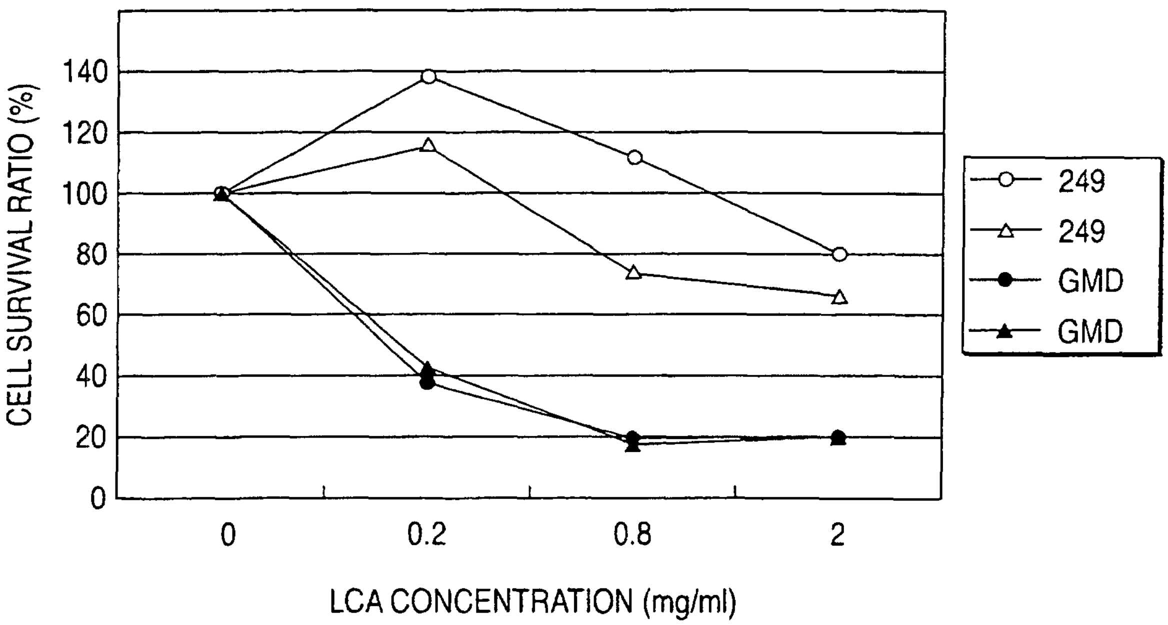
CELL SURVIVAL RATIO (%)
140
120
100
80
60
40
20
0
0
0.2
0.8
2
LCA CONCENTRATION (mg/ml)
249
249
GMD
GMD

FIG. 14

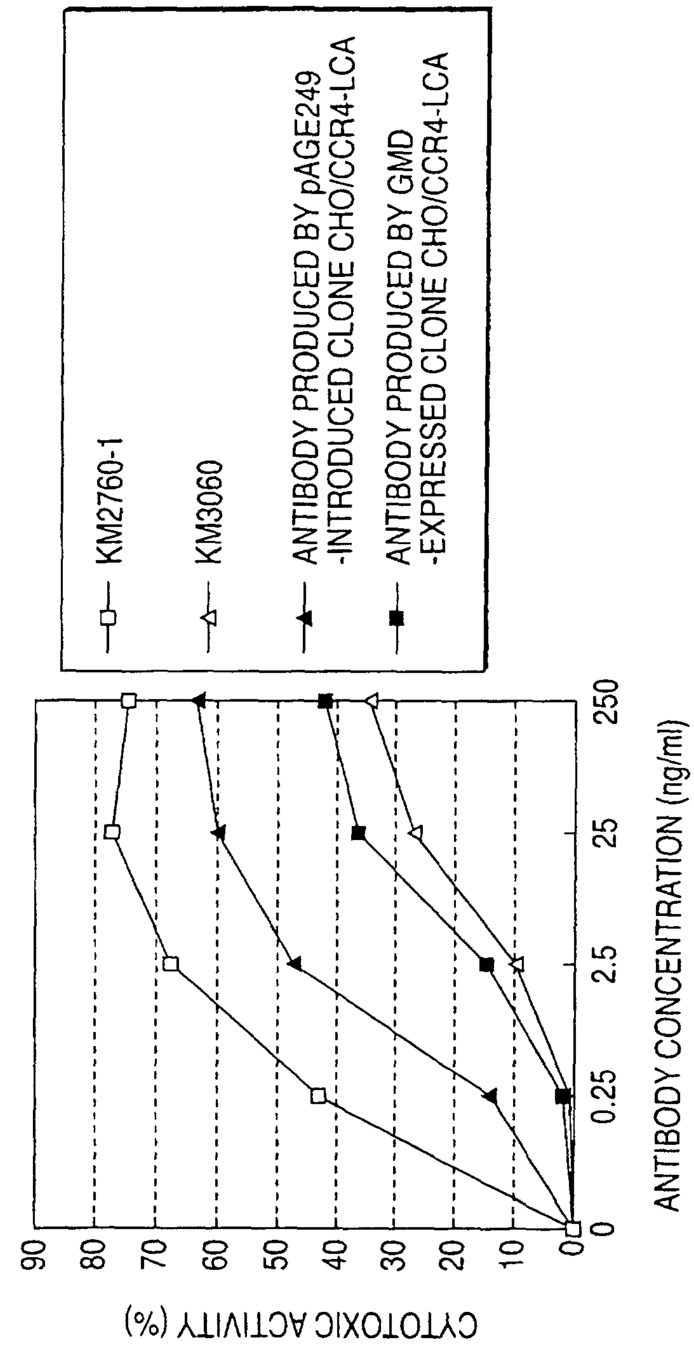
CYTOTOXIC ACTIVITY (%)
90
80
70
60
50
40
30
20
10
0
0
0.25
2.5
25
250
ANTIBODY CONCENTRATION (ng/ml)
KM2760-1
KM3060
ANTIBODY PRODUCED BY pAGE249 -INTRODUCED CLONE CHO/CCR4-LCA
ANTIBODY PRODUCED BY GMD -EXPRESSED CLONE CHO/CCR4-LCA

FIG. 15

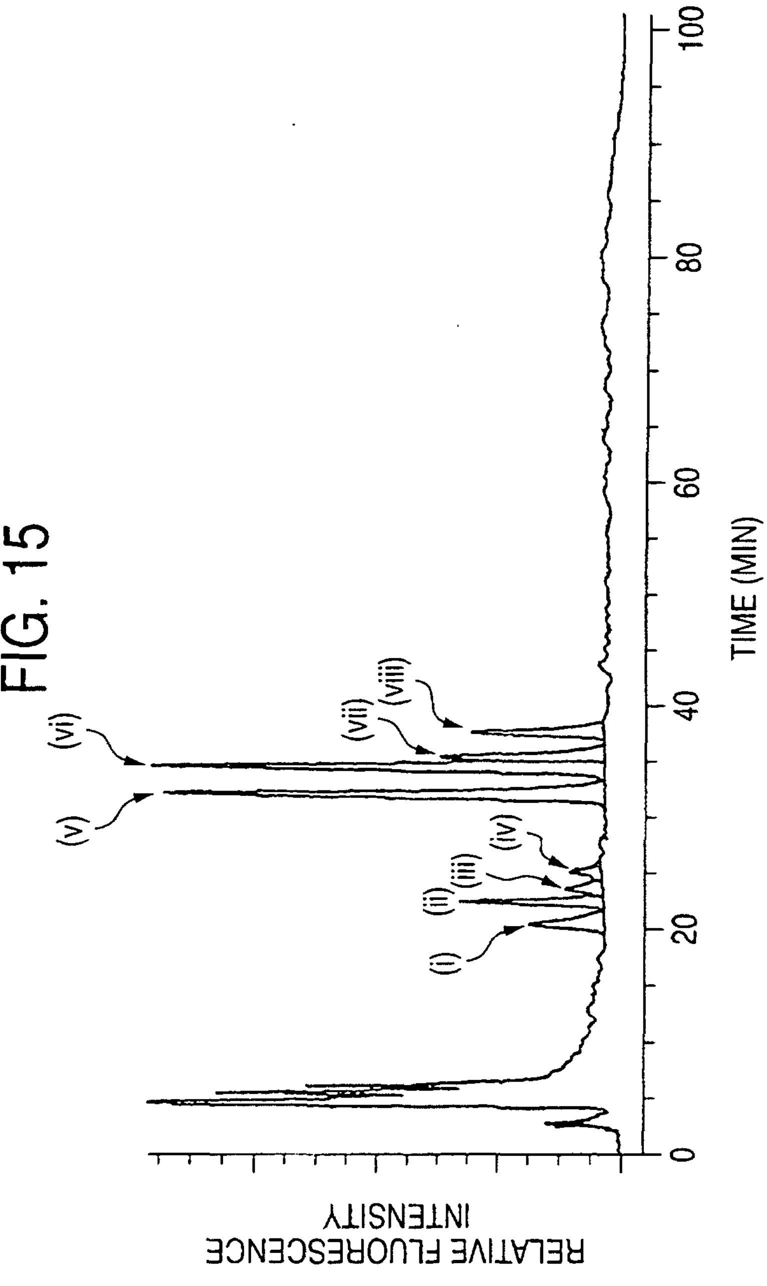
(i)
(ii)
(iii)
(iv)
(v)
(vi)
(vii)
(viii)
0
20
40
60
80
100
TIME (MIN)
RELATIVE FLUORESCENCE INTENSITY

FIG. 16

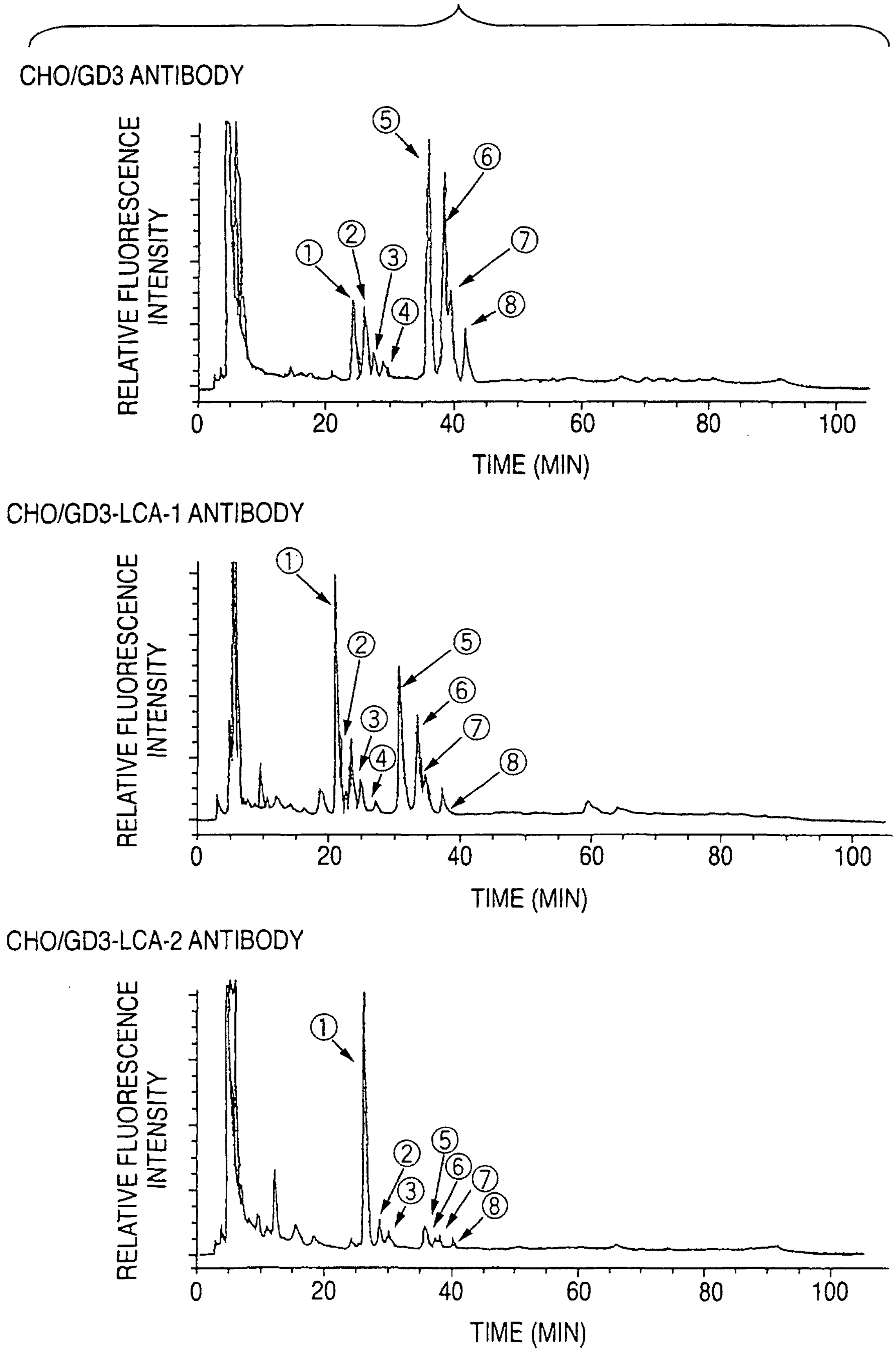
CHO/GD3 ANTIBODY
RELATIVE FLUORESCENCE INTENSITY
TIME (MIN)
CHO/GD3-LCA-1 ANTIBODY
RELATIVE FLUORESCENCE INTENSITY
TIME (MIN)
CHO/GD3-LCA-2 ANTIBODY
RELATIVE FLUORESCENCE INTENSITY
TIME (MIN)

FIG. 17

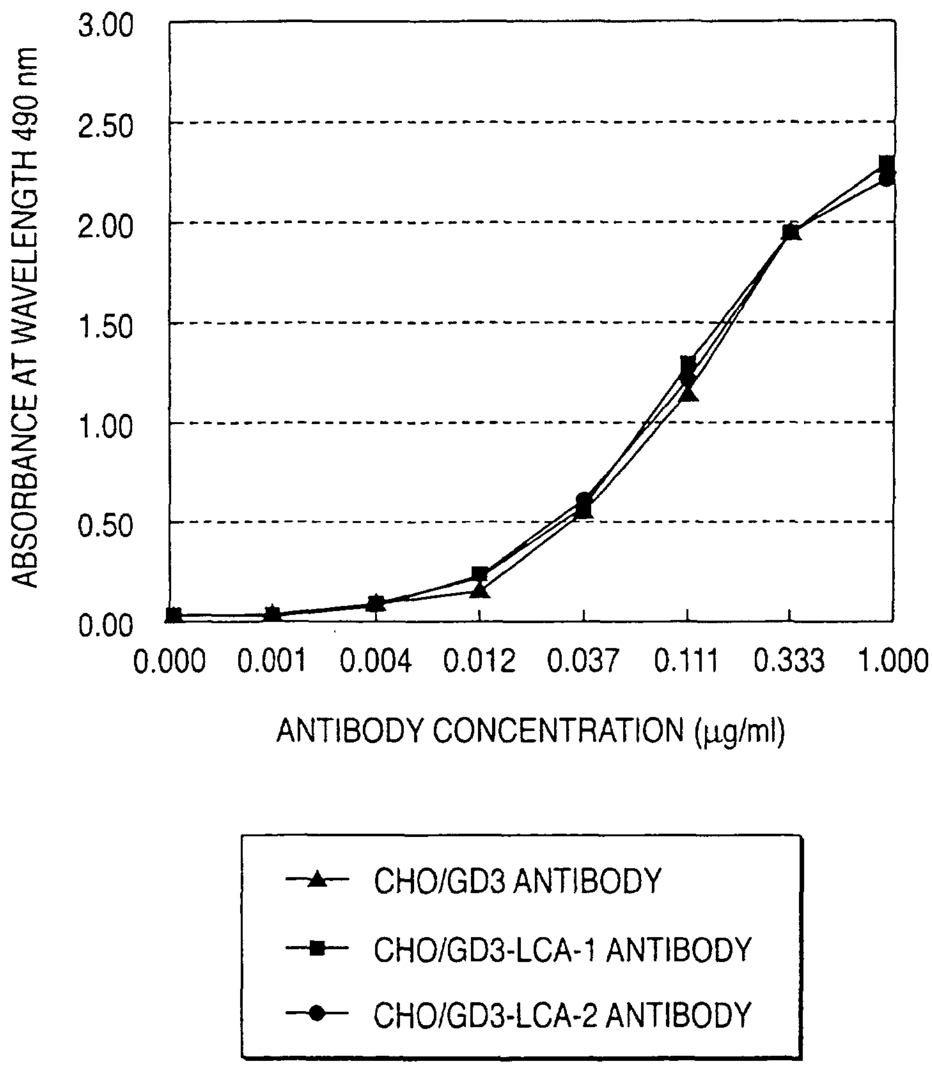
3.00
2.50
2.00
1.50
1.00
0.50
0.00
ABSORBANCE AT WAVELENGTH 490 nm
0.000
0.001
0.004
0.012
0.037
0.111
0.333
1.000
ANTIBODY CONCENTRATION (μg/ml)
CHO/GD3 ANTIBODY
CHO/GD3-LCA-1 ANTIBODY
CHO/GD3-LCA-2 ANTIBODY

FIG. 18

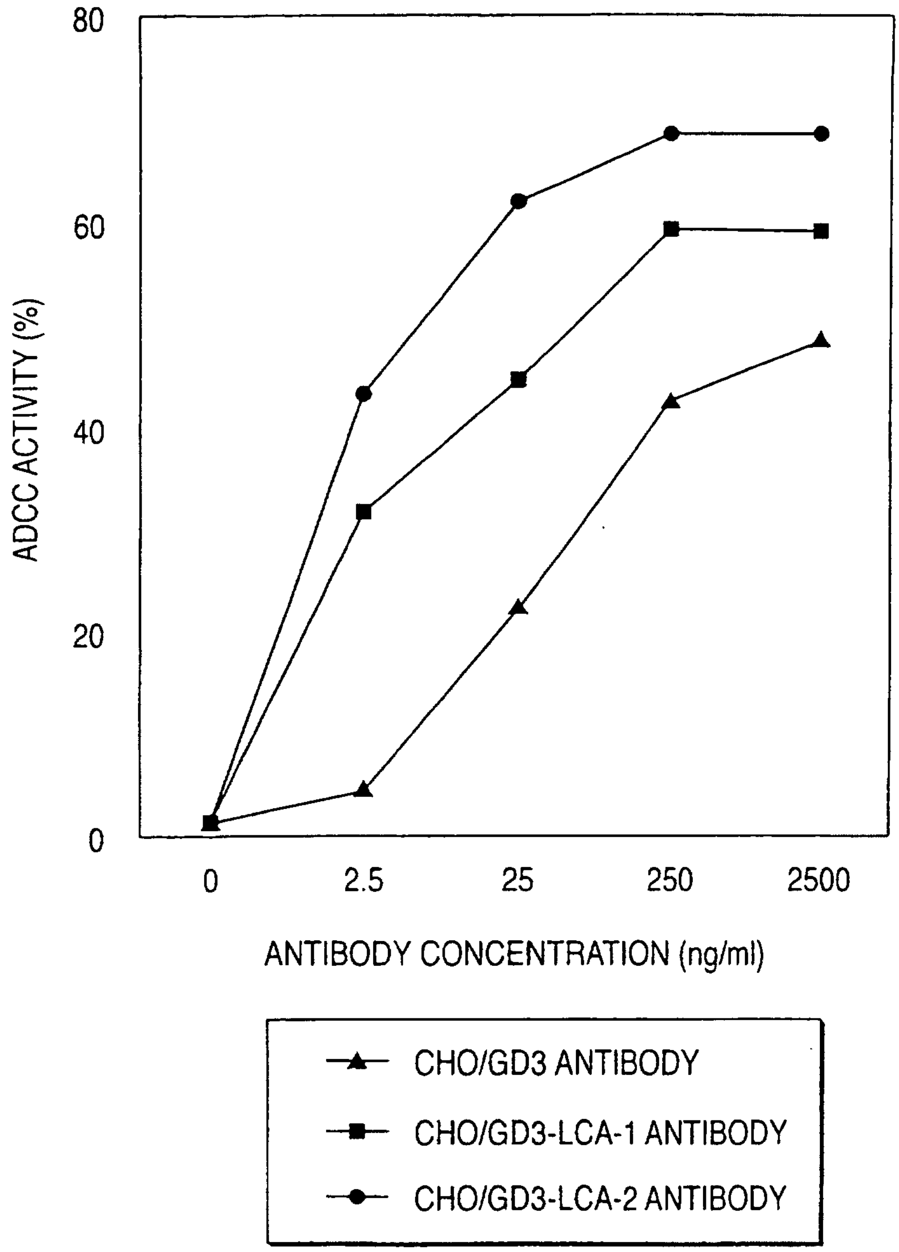
80
60
40
20
0
ADCC ACTIVITY (%)
0
2.5
25
250
2500
ANTIBODY CONCENTRATION (ng/ml)
CHO/GD3 ANTIBODY
CHO/GD3-LCA-1 ANTIBODY
CHO/GD3-LCA-2 ANTIBODY

FIG. 19

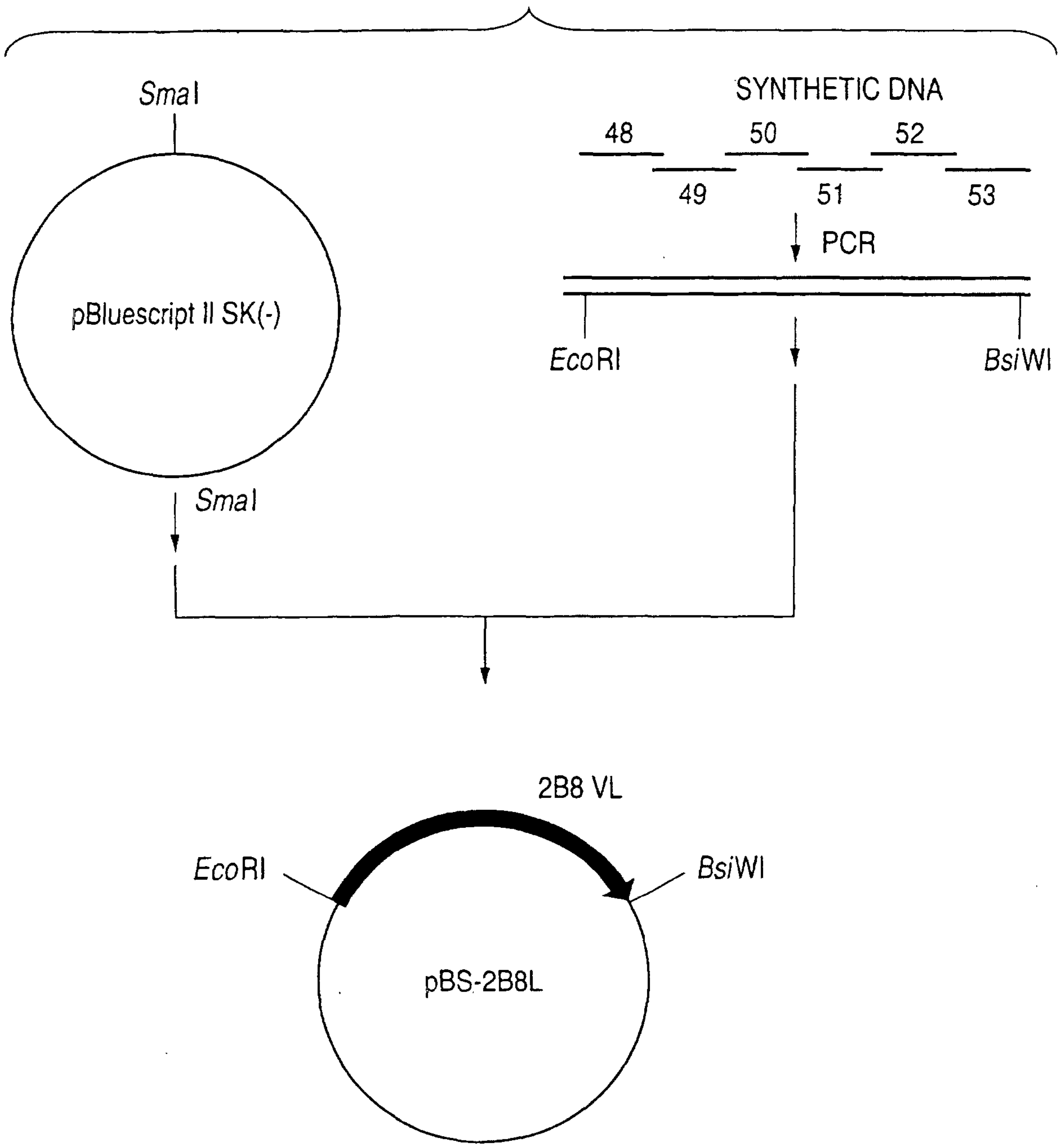
SmaI
pBluescript II SK(-)
SmaI
SYNTHETIC DNA
48
50
52
49
51
53
PCR
EcoRI
BsiWI
2B8 VL
EcoRI
BsiWI
pBS-2B8L

FIG. 20

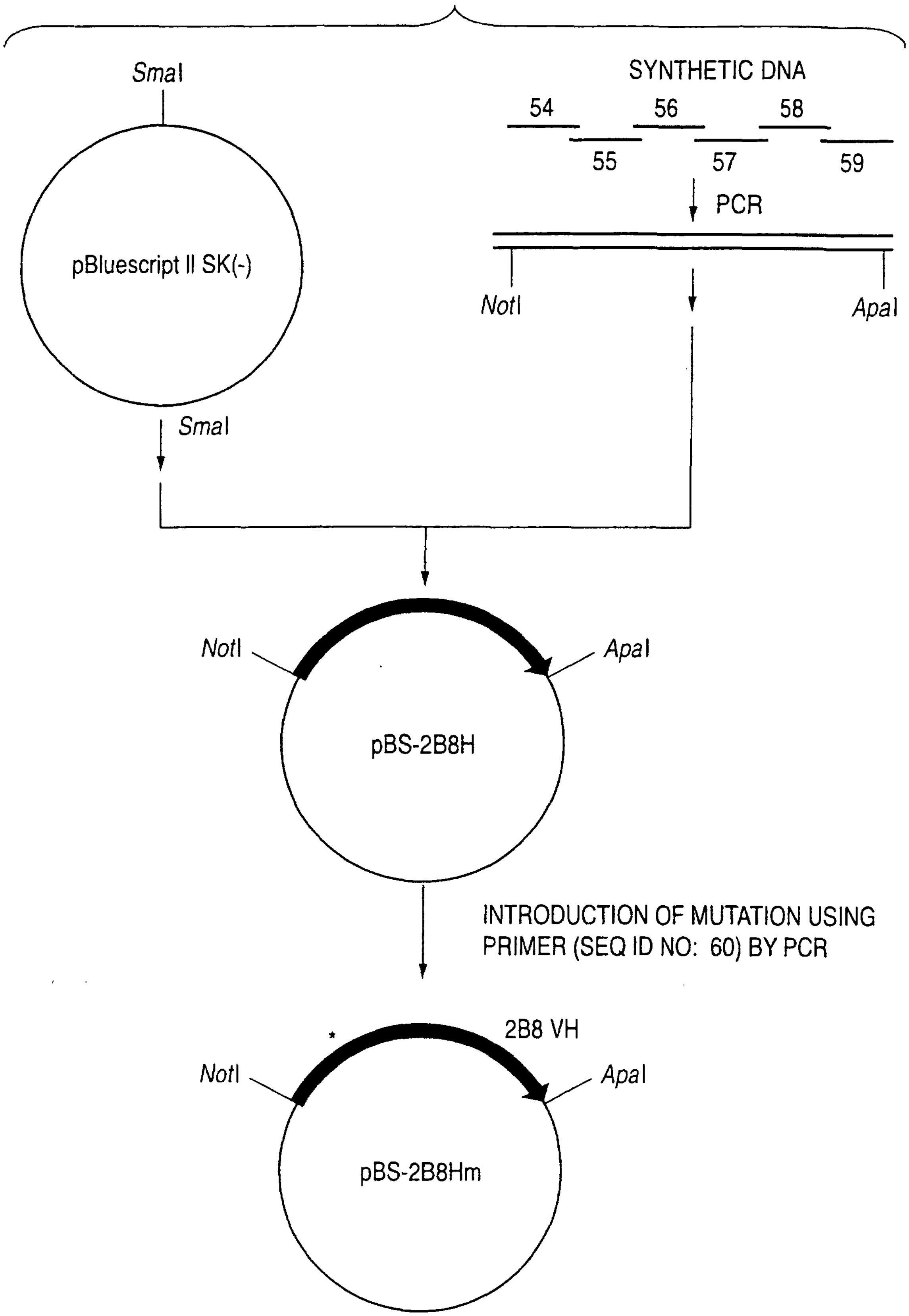
SmaI
SYNTHETIC DNA
54
56
58
55
57
59
PCR
pBluescript II SK(-)
NotI
ApaI
SmaI
NotI
ApaI
pBS-2B8H
INTRODUCTION OF MUTATION USING PRIMER (SEQ ID NO: 60) BY PCR
2B8 VH
NotI
ApaI
pBS-2B8Hm

FIG. 21

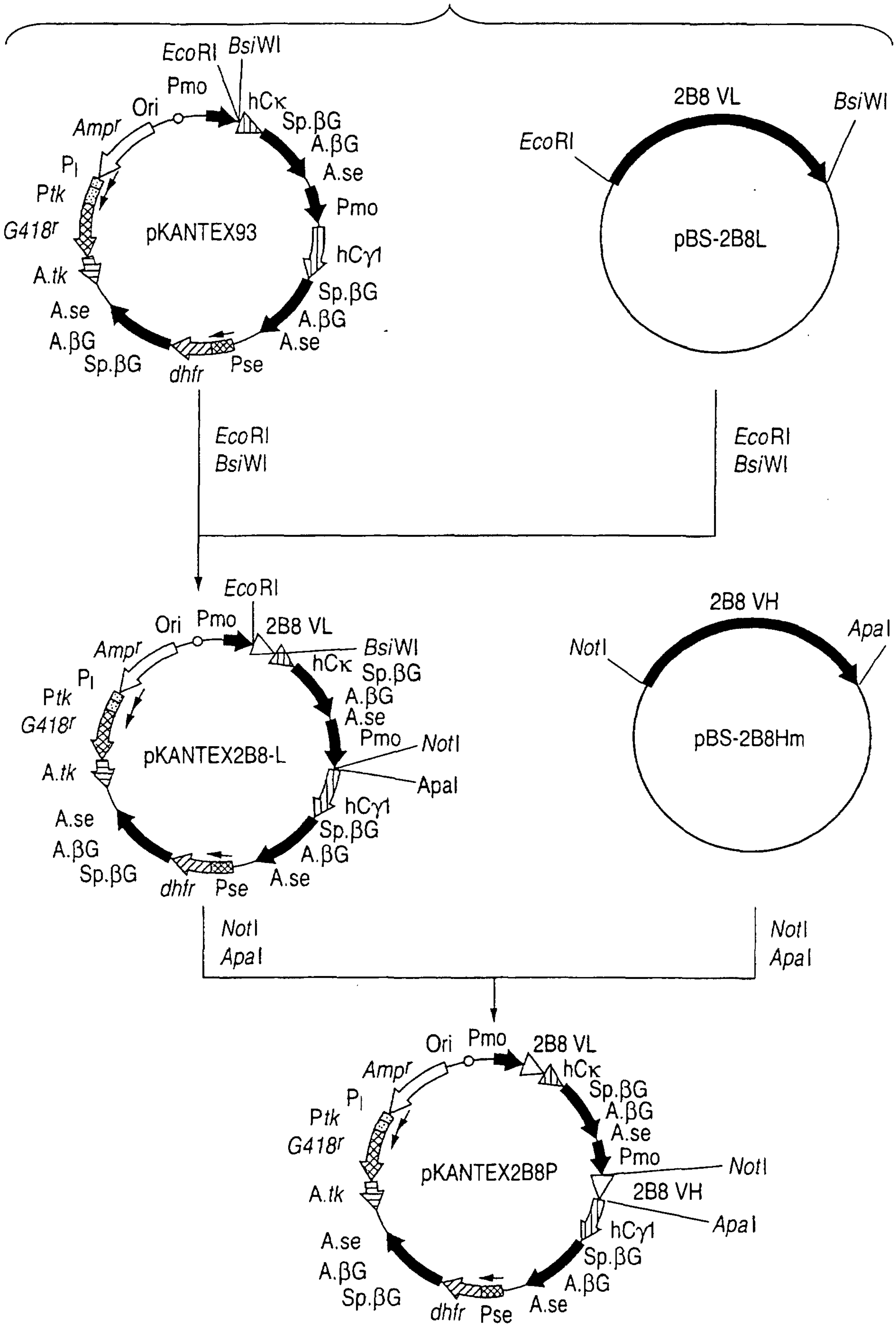

EcoRI
BsiWI
Pmo
Ori
Ampr
hCκ
Sp.βG
A.βG
A.se
Pl
Ptk
G418r
pKANTEX93
Pmo
hCγ1
A.tk
Sp.βG
A.βG
A.se
A.se
A.βG
Sp.βG
dhfr
Pse
2B8 VL
BsiWI
EcoRI
pBS-2B8L
EcoRI
BsiWI
EcoRI
BsiWI
EcoRI
Ori
Pmo
2B8 VL
Ampr
BsiWI
hCκ
Sp.βG
Pl
Ptk
G418r
A.βG
A.se
Pmo
NotI
pKANTEX2B8-L
A.tk
ApaI
hCγ1
A.se
Sp.βG
A.βG
A.βG
Sp.βG
dhfr
Pse
A.se
2B8 VH
NotI
ApaI
pBS-2B8Hm
NotI
ApaI
NotI
ApaI
Ori
Pmo
2B8 VL
Ampr
hCκ
Pl
Sp.βG
A.βG
Ptk
G418r
A.se
Pmo
NotI
pKANTEX2B8P
A.tk
2B8 VH
ApaI
hCγ1
A.se
Sp.βG
A.βG
A.βG
Sp.βG
dhfr
Pse
A.se

FIG. 22

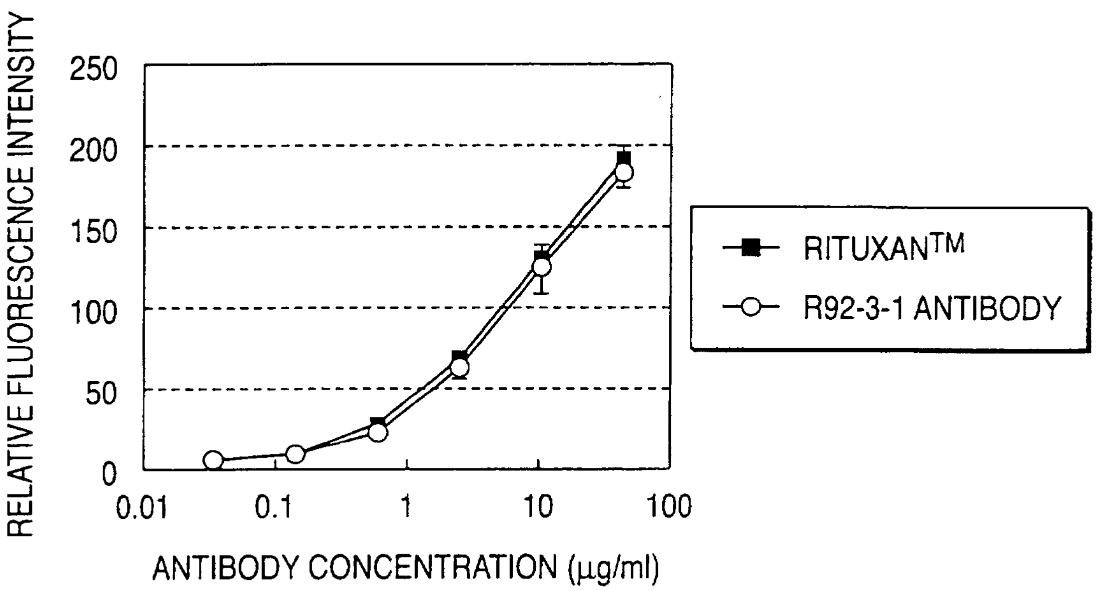
RELATIVE FLUORESCENCE INTENSITY
250
200
150
100
50
0
0.01
0.1
1
10
100
ANTIBODY CONCENTRATION (μg/ml)
RITUXANTM
R92-3-1 ANTIBODY

FIG. 23

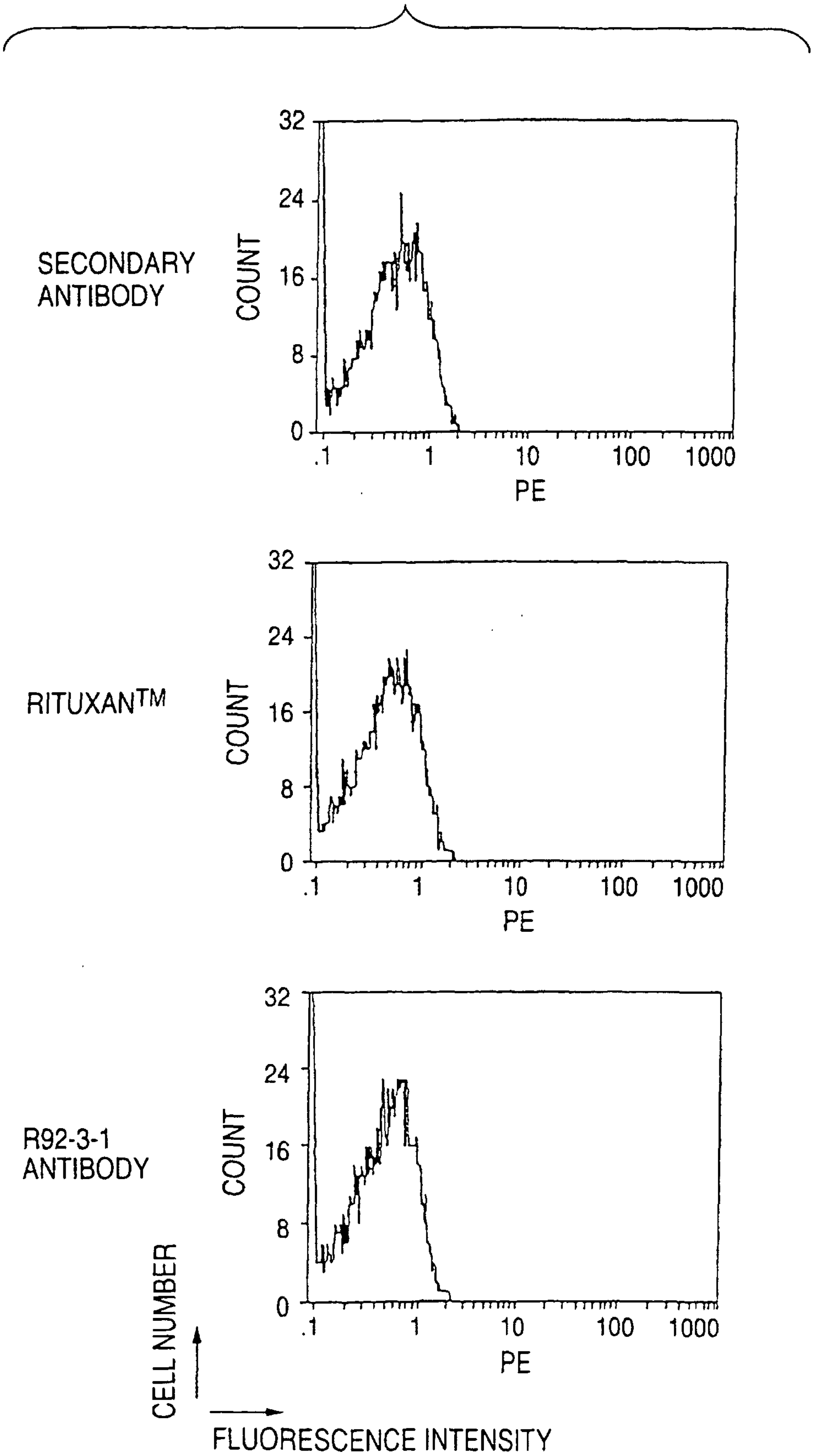
SECONDARY
ANTIBODY
COUNT
32
24
16
8
0
.1
1
10
100
1000
PE
RITUXAN™
COUNT
32
24
16
8
0
.1
1
10
100
1000
PE
R92-3-1
ANTIBODY
COUNT
32
24
16
8
0
.1
1
10
100
1000
PE
CELL NUMBER
FLUORESCENCE INTENSITY

FIG. 24A

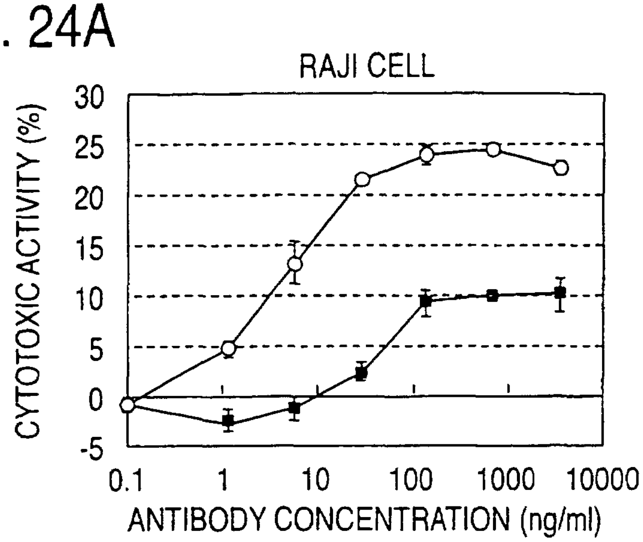


FIG. 24B

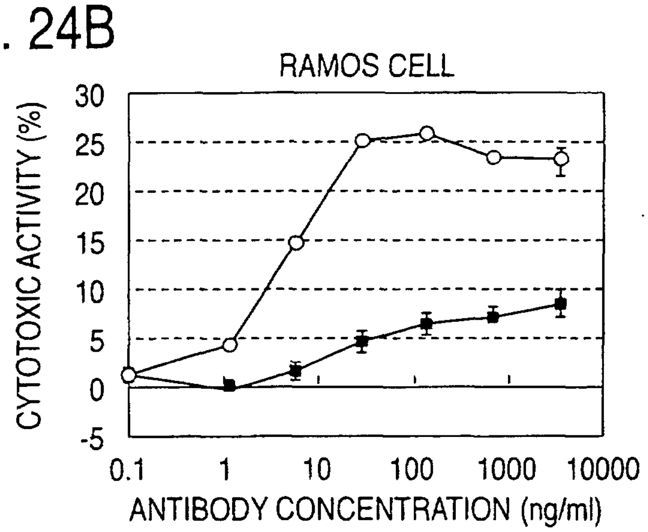


FIG. 24C

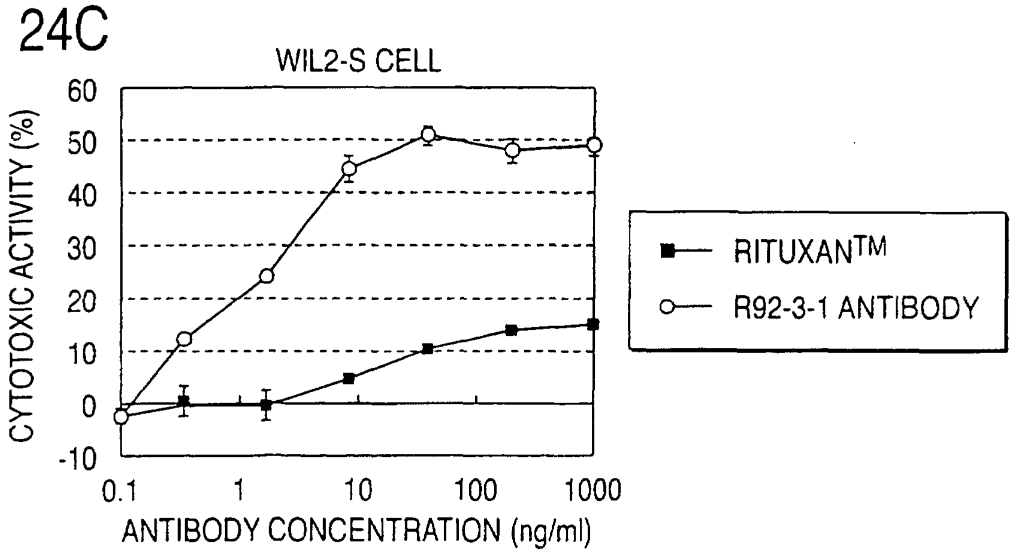

FIG. 25A

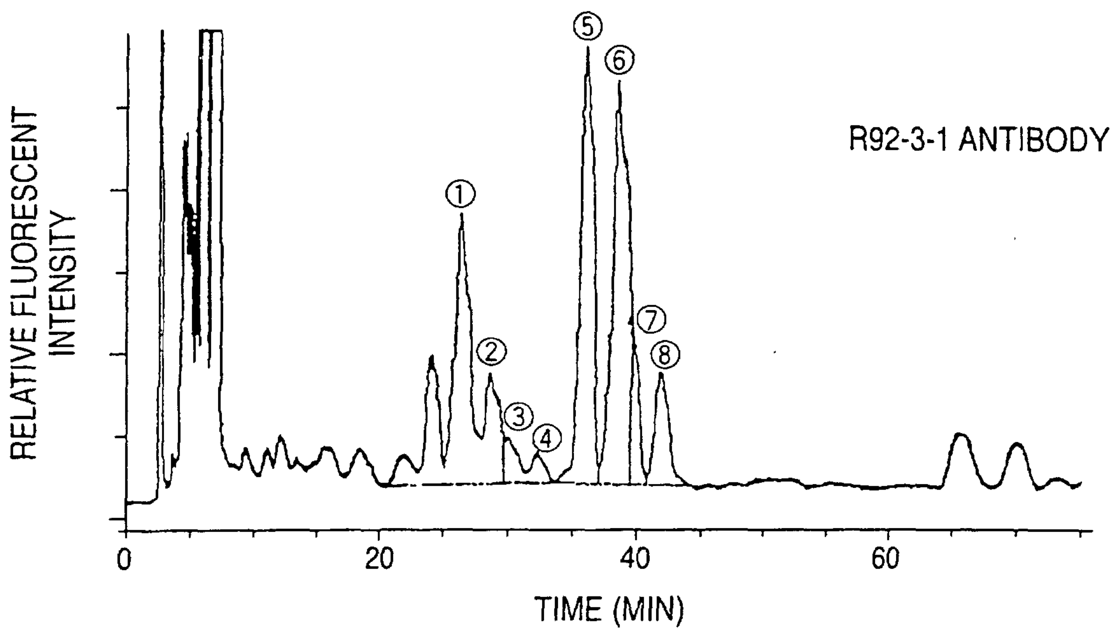


FIG. 25B

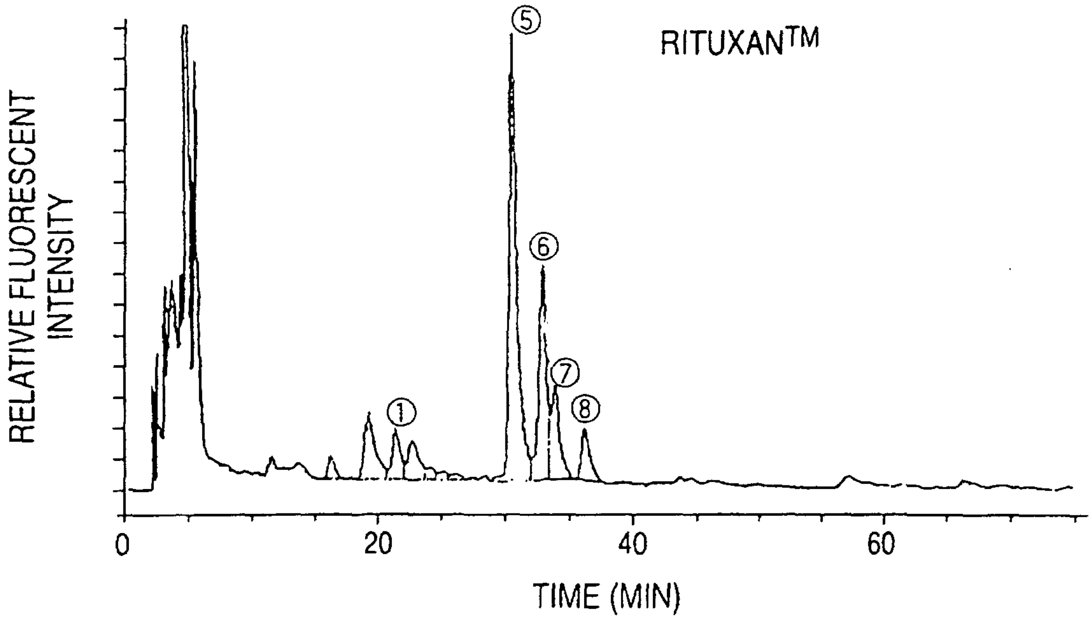

FIG. 26

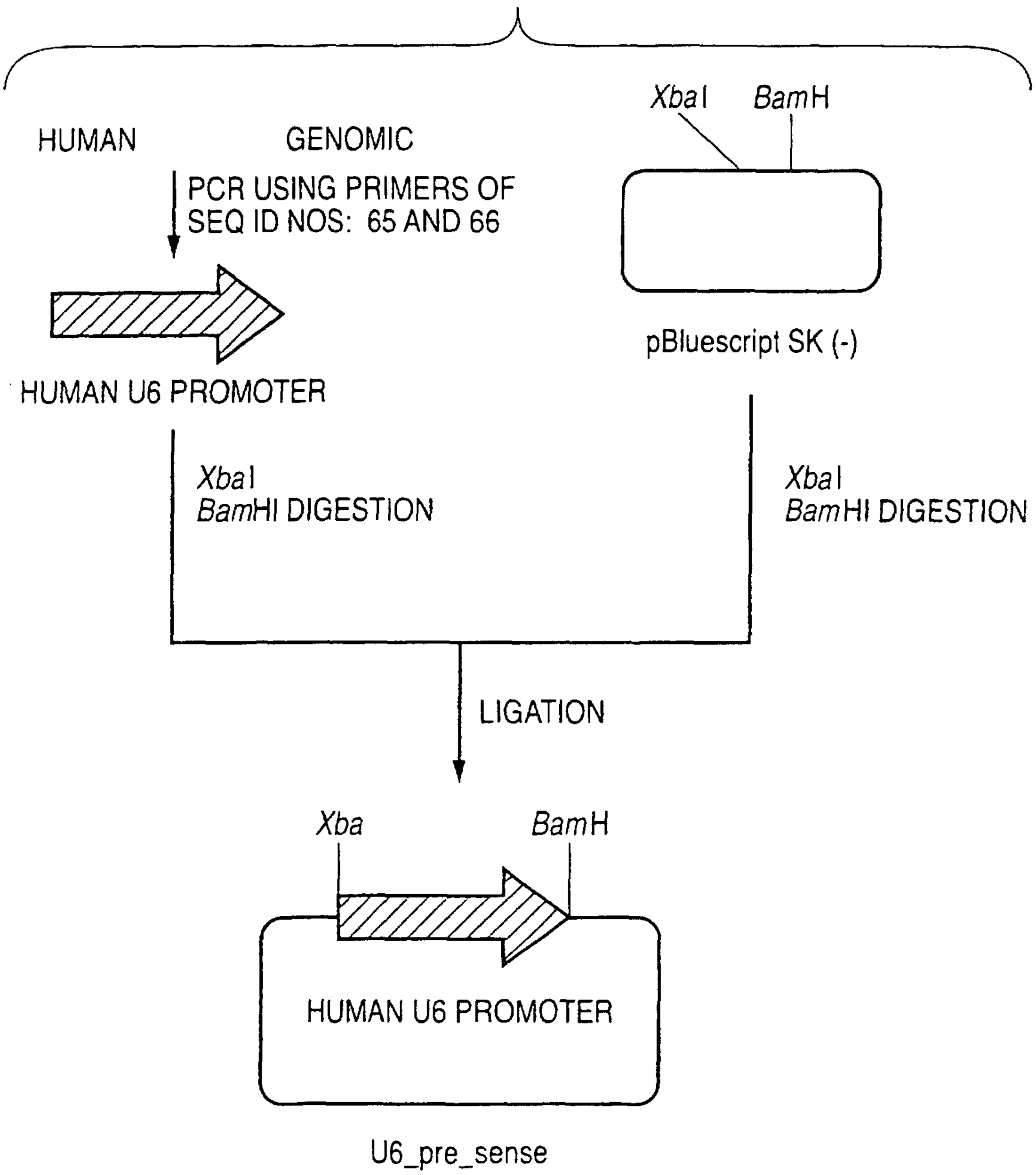
HUMAN GENOMIC
PCR USING PRIMERS OF SEQ ID NOS: 65 AND 66
HUMAN U6 PROMOTER
XbaI BamH
pBluescript SK (-)
XbaI
BamHI DIGESTION
XbaI
BamHI DIGESTION
LIGATION
Xba BamH
HUMAN U6 PROMOTER
U6_pre_sense

FIG. 27

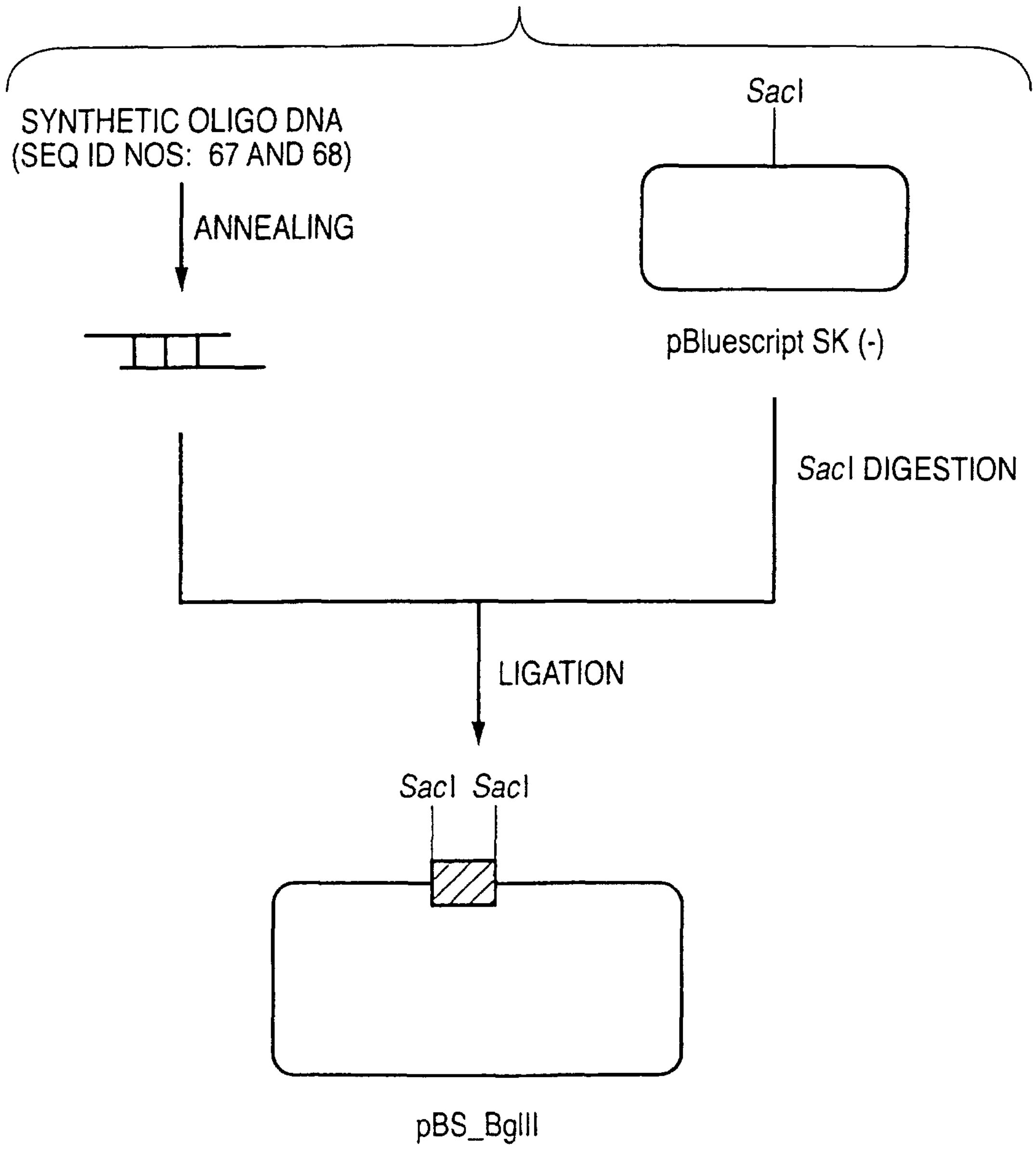
SYNTHETIC OLIGO DNA
(SEQ ID NOS: 67 AND 68)
ANNEALING
SacI
pBluescript SK (-)
SacI DIGESTION
LIGATION
SacI SacI
pBS_BglII

FIG. 28

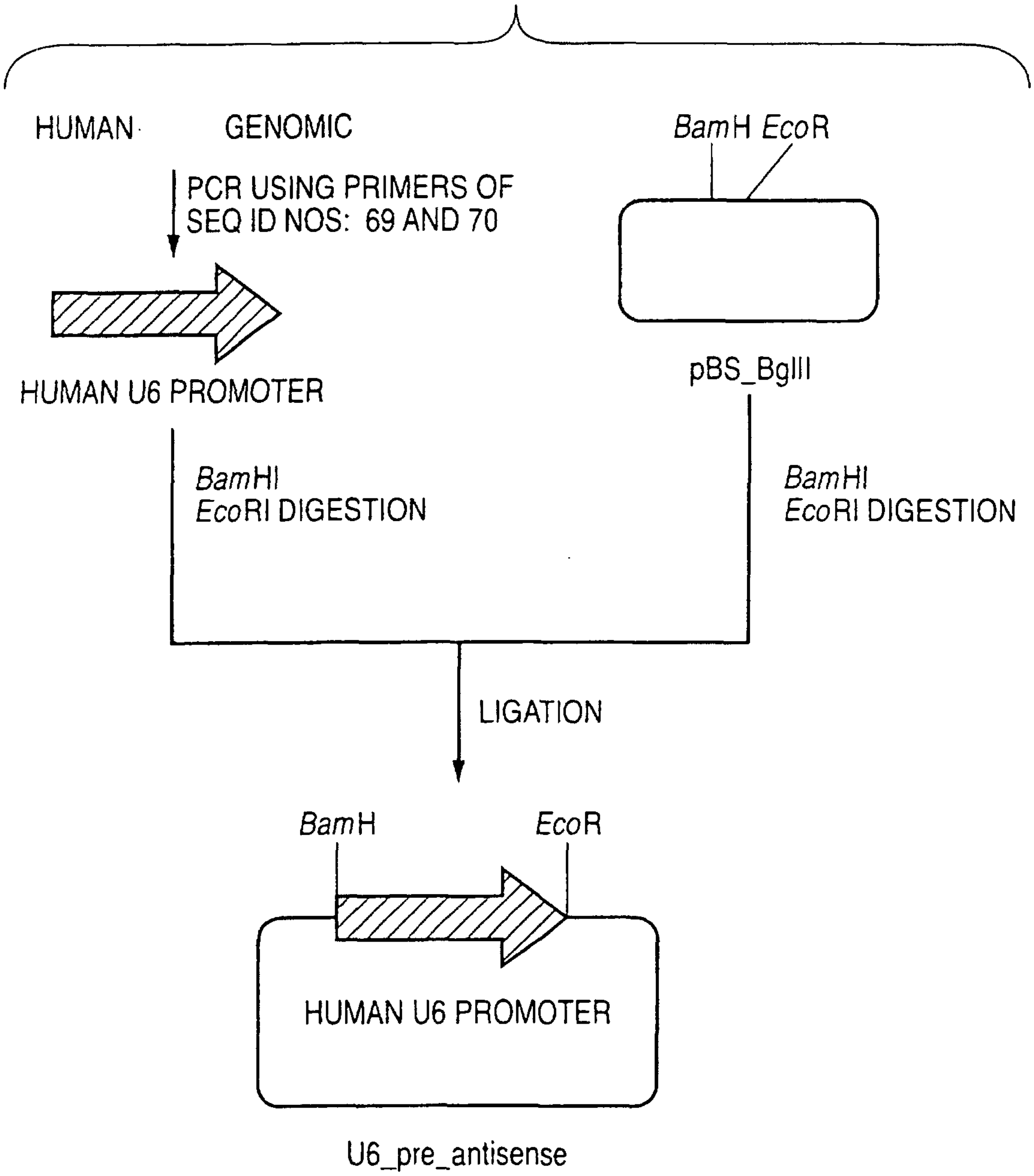
HUMAN GENOMIC
PCR USING PRIMERS OF SEQ ID NOS: 69 AND 70
HUMAN U6 PROMOTER
BamH EcoR
pBS_BglII
BamHI EcoRI DIGESTION
BamHI EcoRI DIGESTION
LIGATION
BamH
EcoR
HUMAN U6 PROMOTER
U6_pre_antisense

FIG. 29

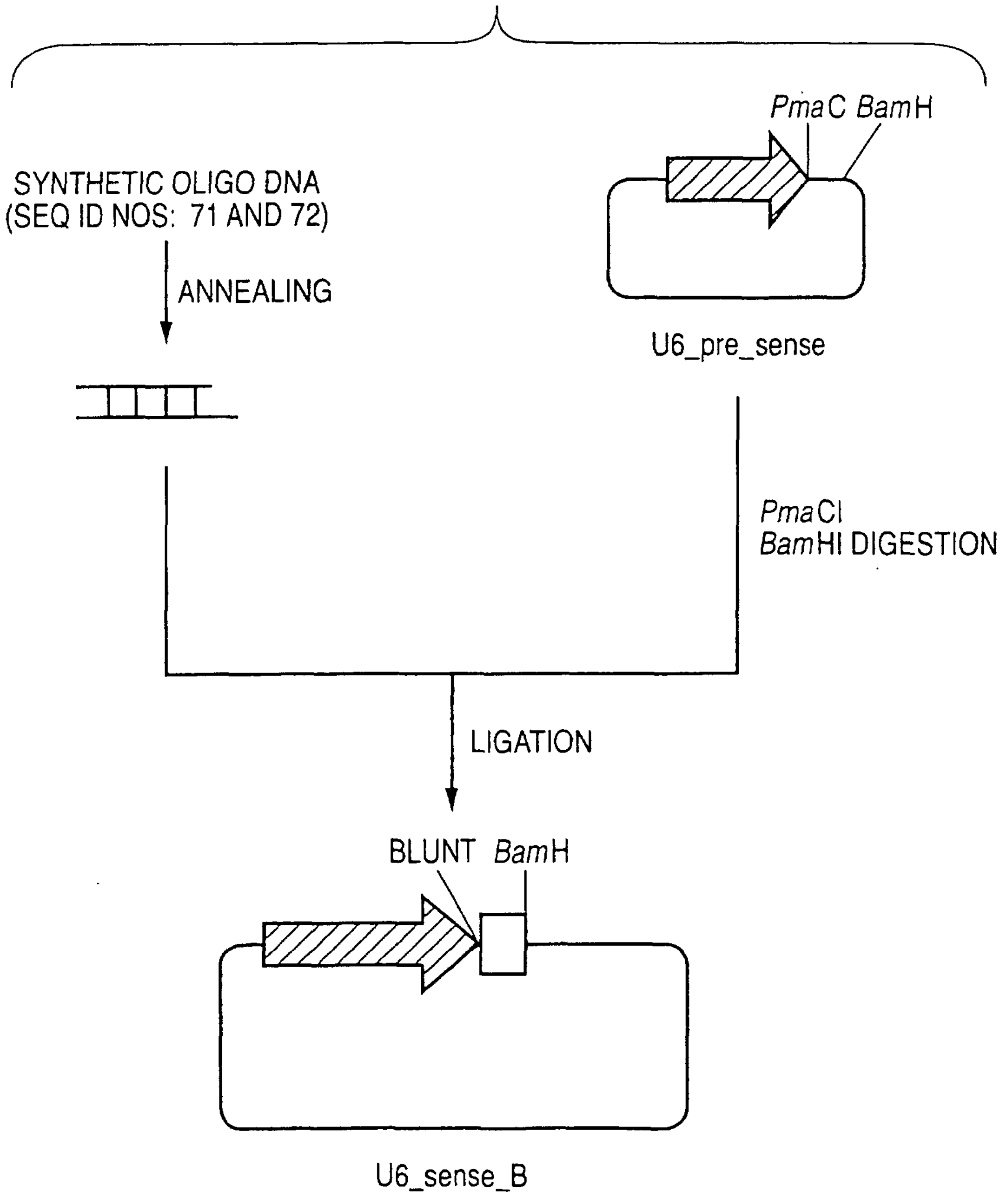
SYNTHETIC OLIGO DNA
(SEQ ID NOS: 71 AND 72)
ANNEALING
PmaC BamH
U6_pre_sense
PmaCI
BamHI DIGESTION
LIGATION
BLUNT BamH
U6_sense_B

FIG. 30

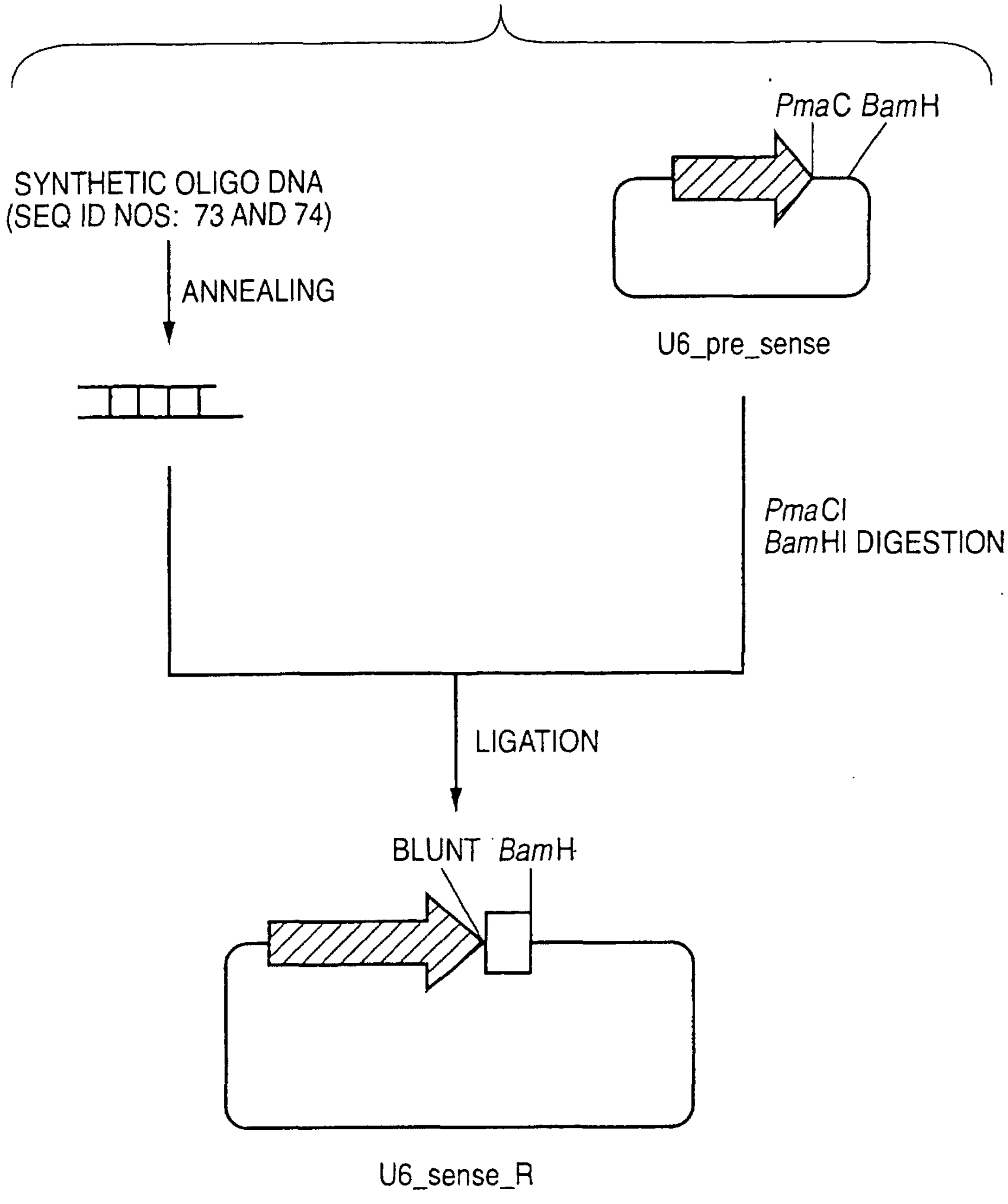
SYNTHETIC OLIGO DNA
(SEQ ID NOS: 73 AND 74)
ANNEALING
PmaC BamH
U6_pre_sense
PmaCI
BamHI DIGESTION
LIGATION
BLUNT BamH
U6_sense_R

FIG. 31

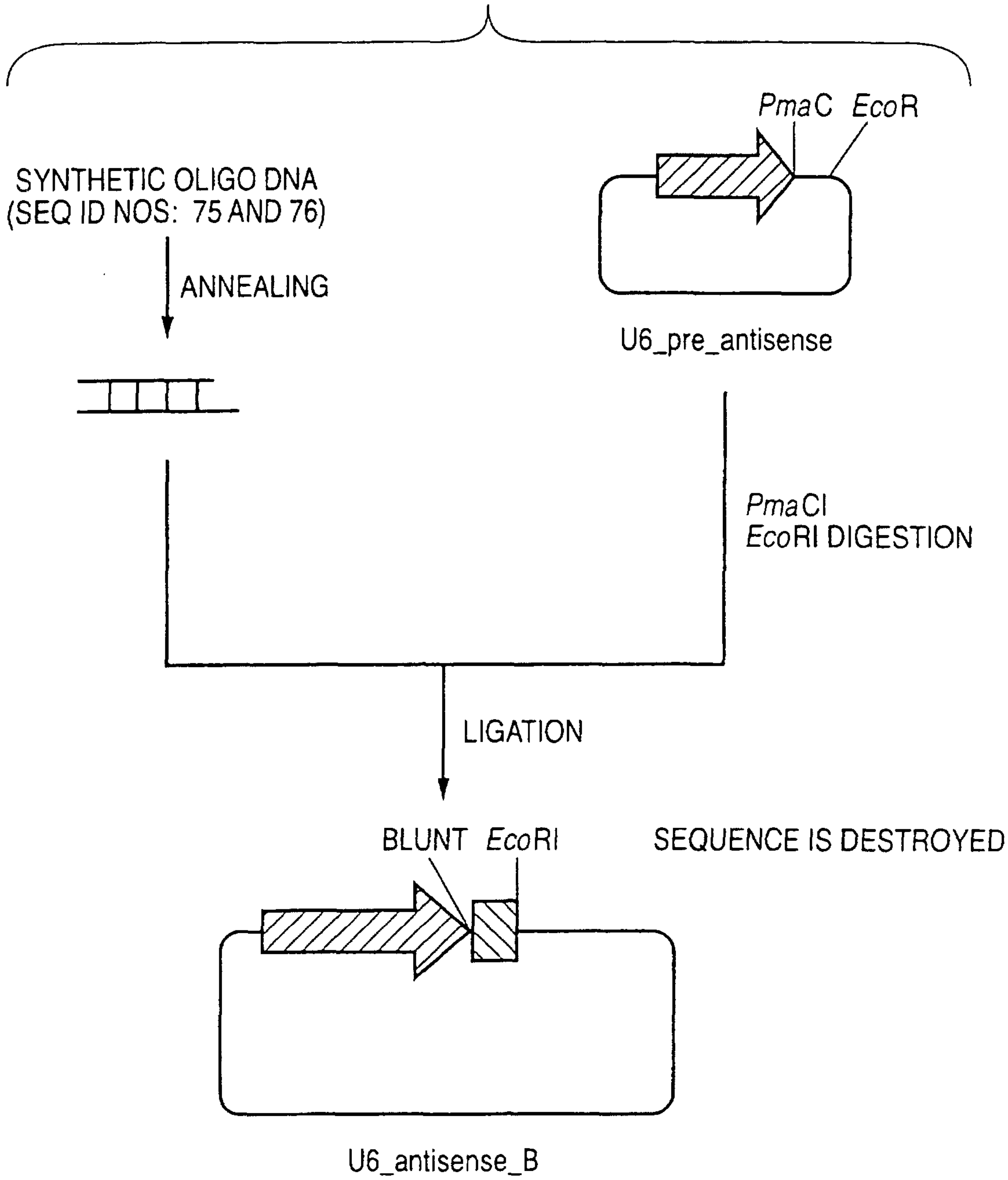
SYNTHETIC OLIGO DNA
(SEQ ID NOS: 75 AND 76)
ANNEALING
PmaC EcoR
U6_pre_antisense
PmaCI
EcoRI DIGESTION
LIGATION
BLUNT EcoRI
SEQUENCE IS DESTROYED
U6_antisense_B

FIG. 32

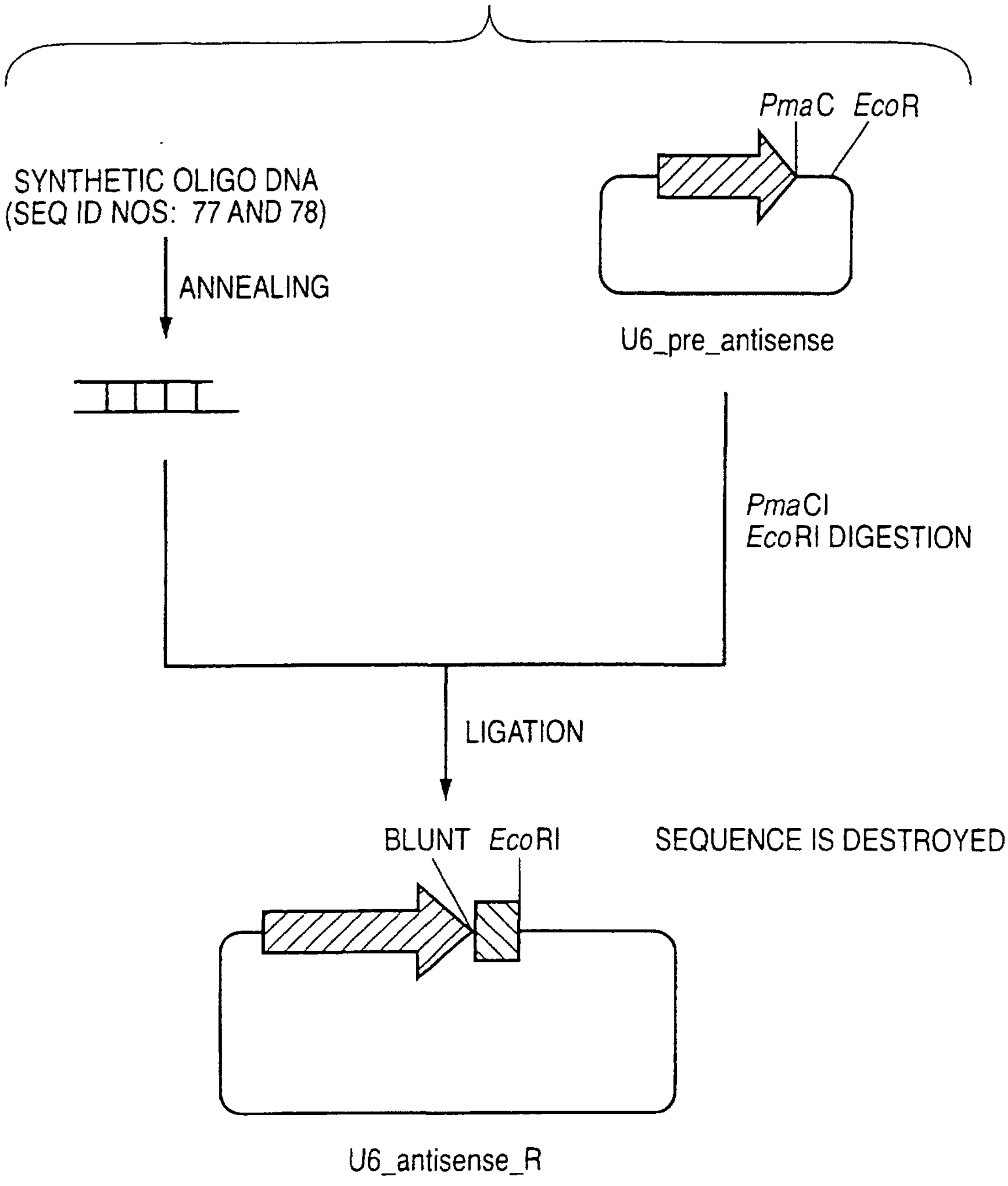
SYNTHETIC OLIGO DNA
(SEQ ID NOS: 77 AND 78)
ANNEALING
PmaC EcoR
U6_pre_antisense
PmaCI
EcoRI DIGESTION
LIGATION
BLUNT EcoRI
SEQUENCE IS DESTROYED
U6_antisense_R

FIG. 33

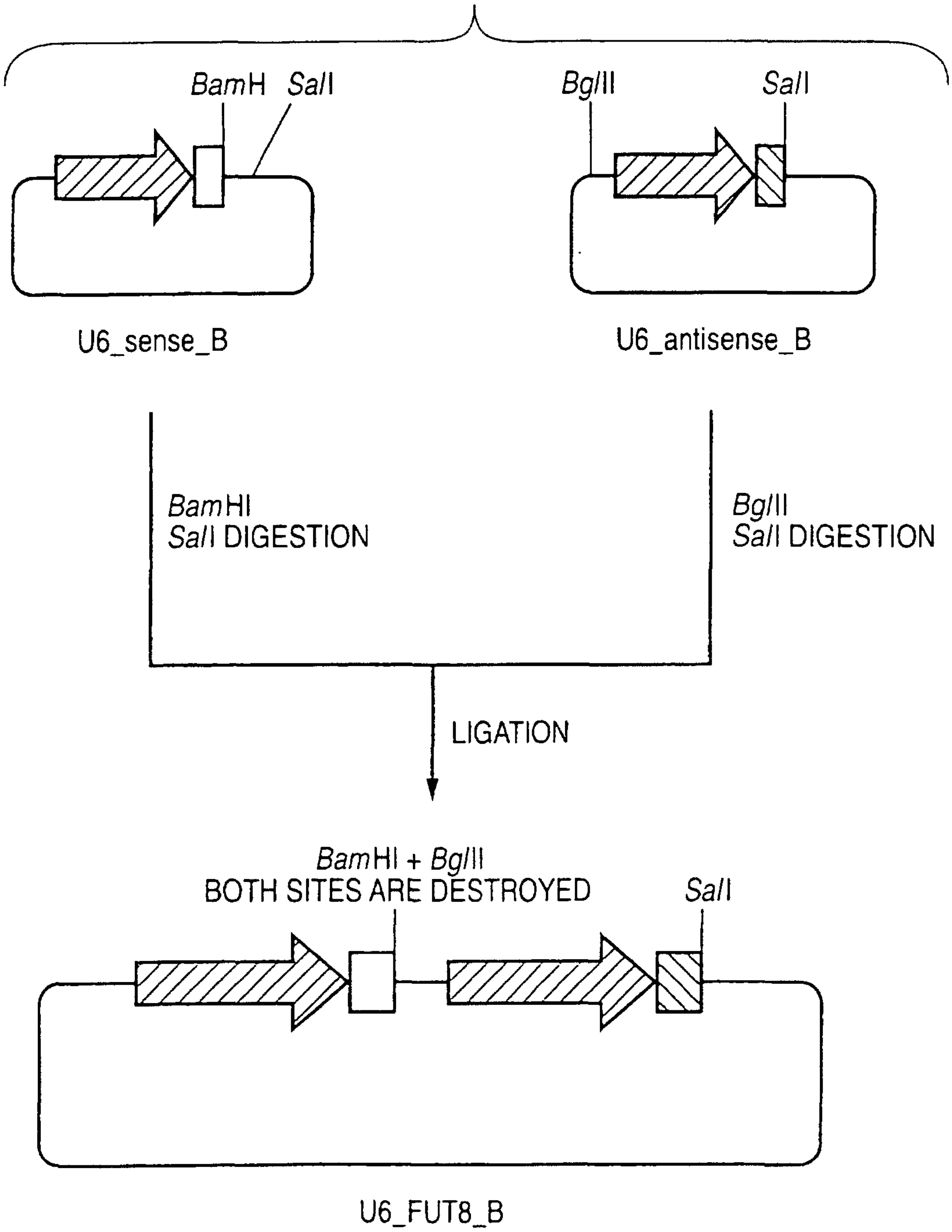
*Bam*H *Sal*I
*Bgl*II *Sal*I
U6_sense_B
U6_antisense_B
*Bam*HI
*Sal*I DIGESTION
*Bgl*II
*Sal*I DIGESTION
LIGATION
*Bam*HI + *Bgl*II
BOTH SITES ARE DESTROYED
*Sal*I
U6_FUT8_B

FIG. 34

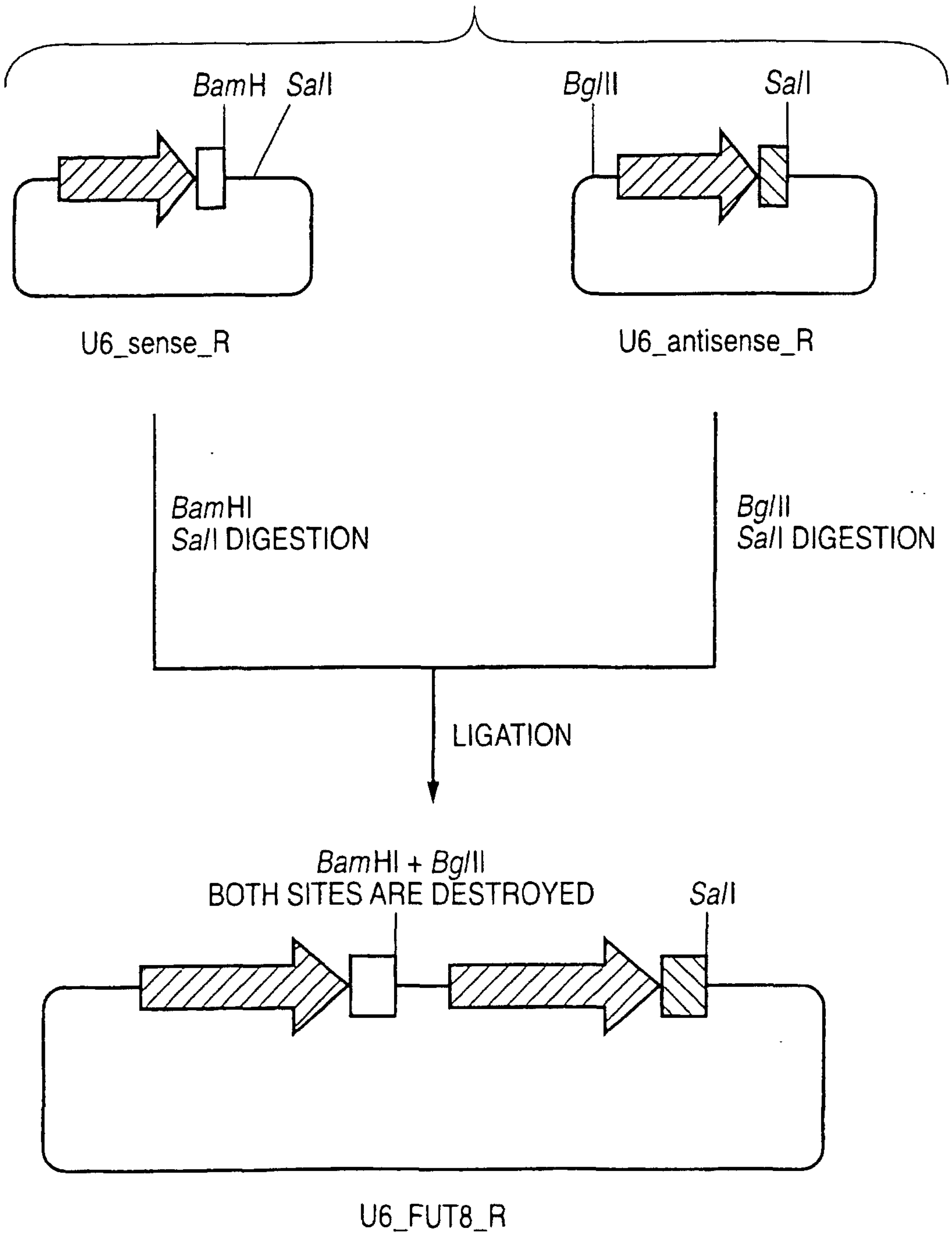
BamH Sall
BglII Sall
U6_sense_R
U6_antisense_R
BamHI
SalI DIGESTION
BglII
SalI DIGESTION
LIGATION
BamHI + BglII
BOTH SITES ARE DESTROYED
SalI
U6_FUT8_R

FIG. 35

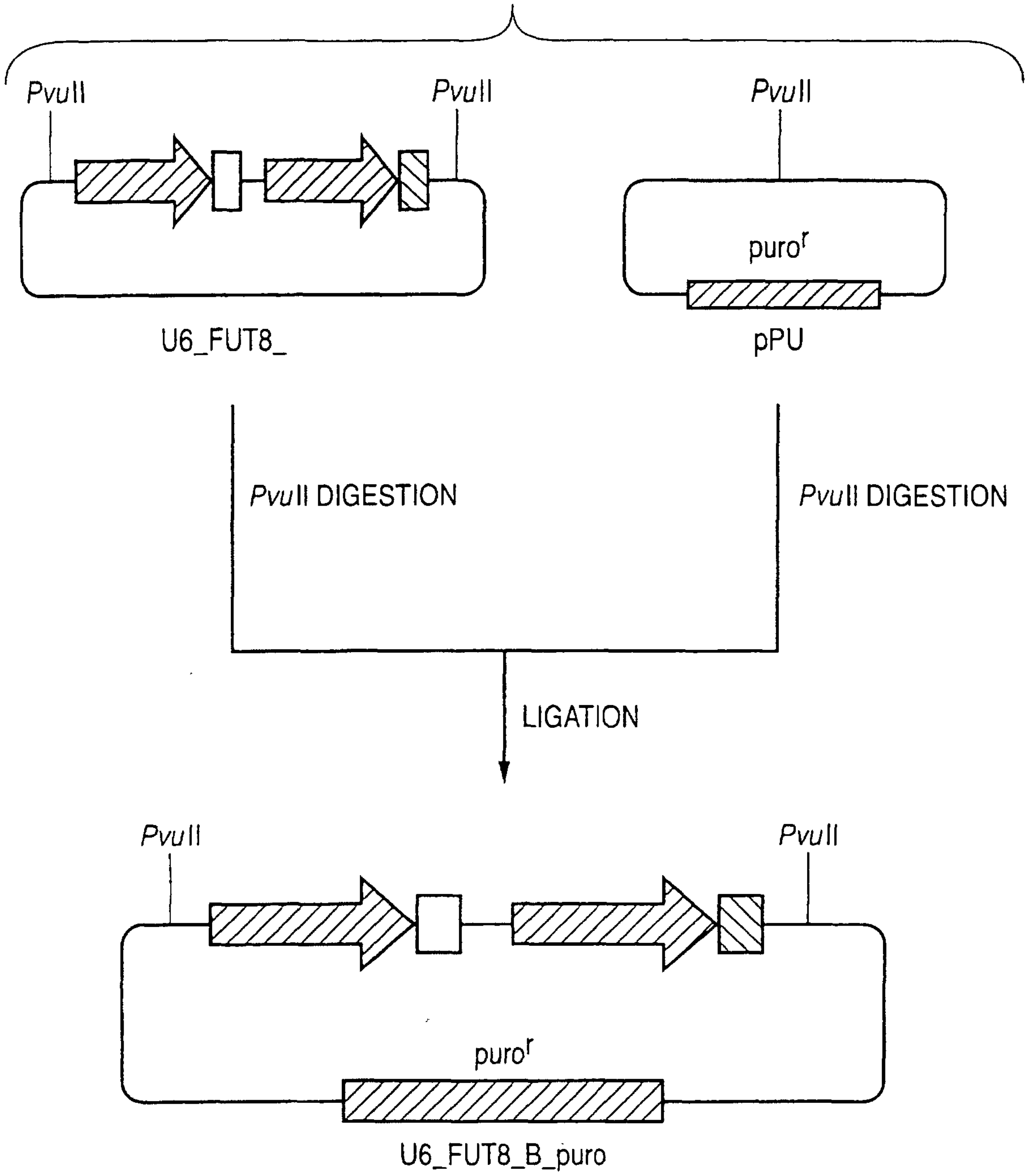
PvuII
PvuII
PvuII
puro^r
U6_FUT8_
pPU
PvuII DIGESTION
PvuII DIGESTION
LIGATION
PvuII
PvuII
puro^r
U6_FUT8_B_puro

FIG. 36

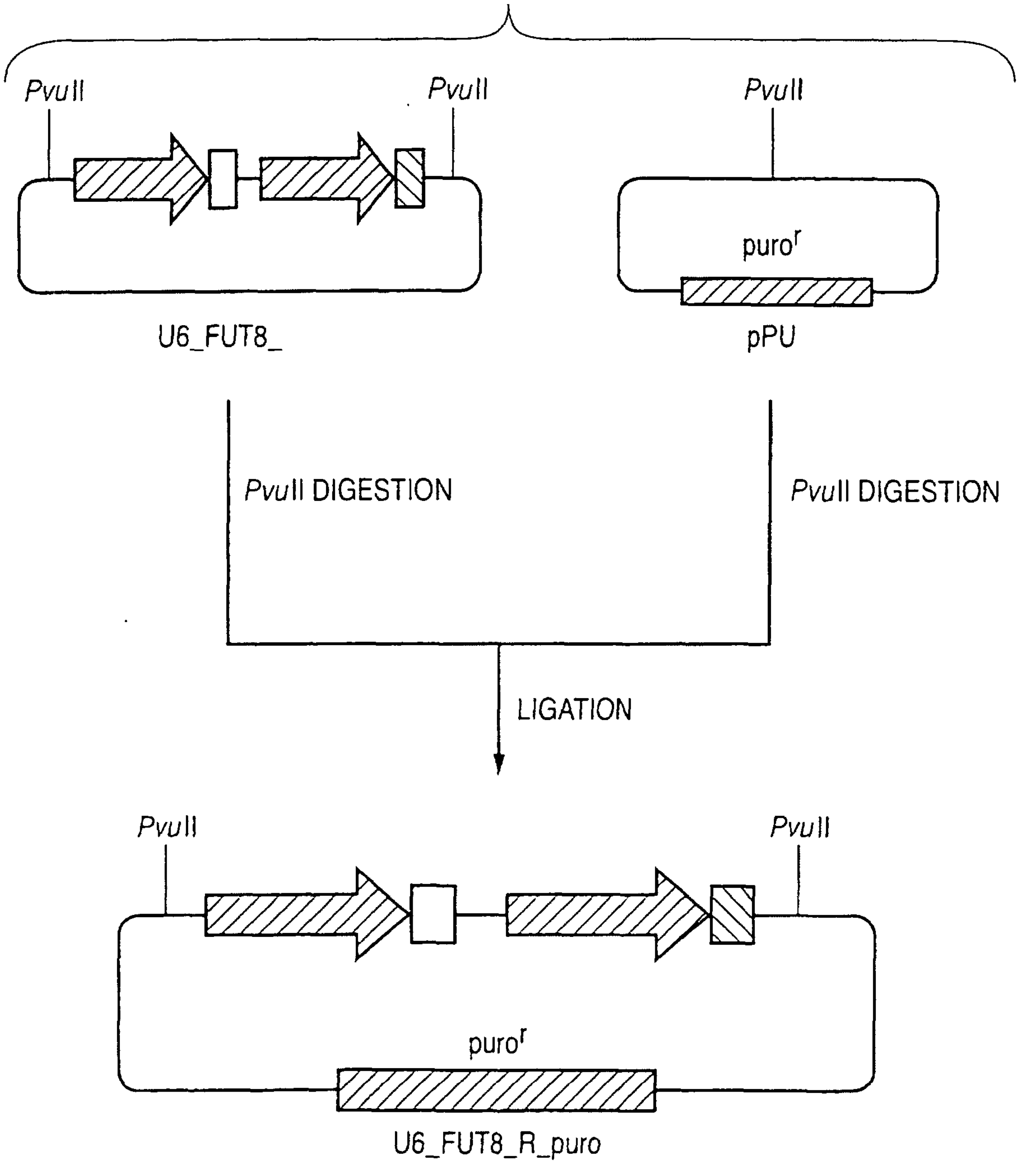

PvuII
PvuII
PvuII
puro^r
U6_FUT8_
pPU
PvuII DIGESTION
PvuII DIGESTION
LIGATION
PvuII
PvuII
puro^r
U6_FUT8_R_puro

FIG. 37

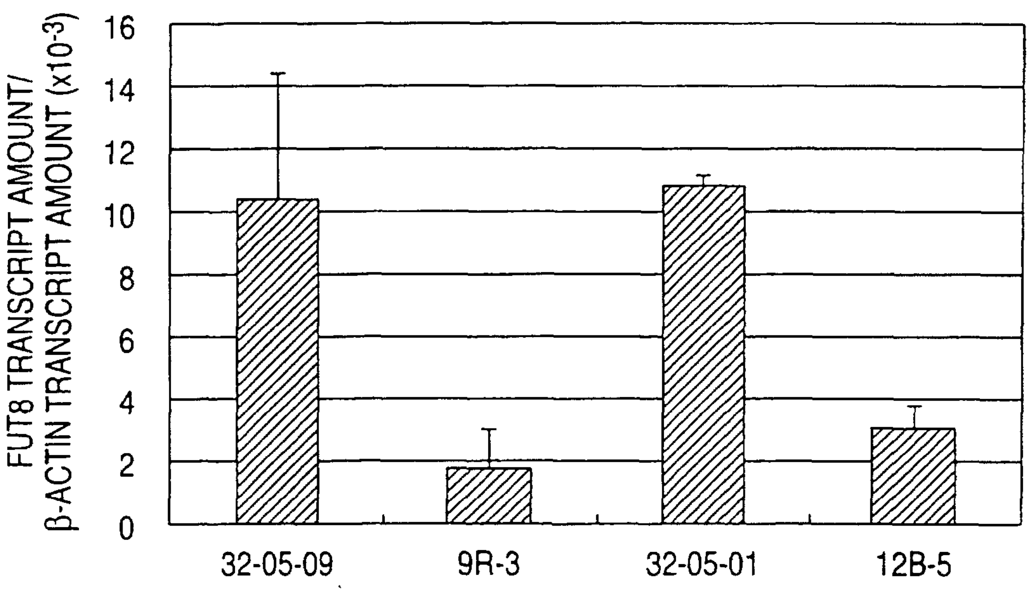
FUT8 TRANSCRIPT AMOUNT/
β-ACTIN TRANSCRIPT AMOUNT (x10-3)
16
14
12
10
8
6
4
2
0
32-05-09
9R-3
32-05-01
12B-5

FIG. 38A

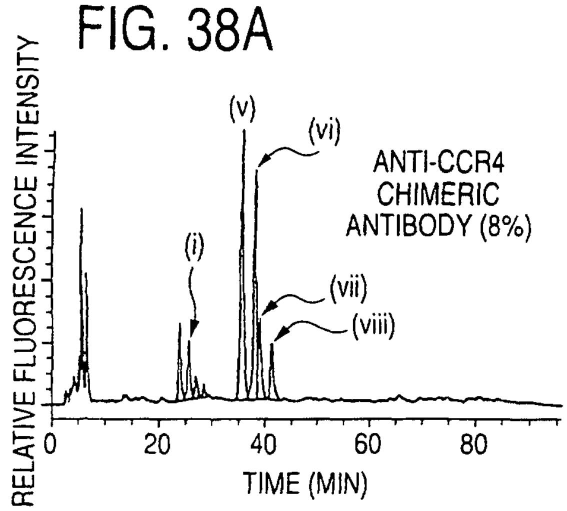


FIG. 38B

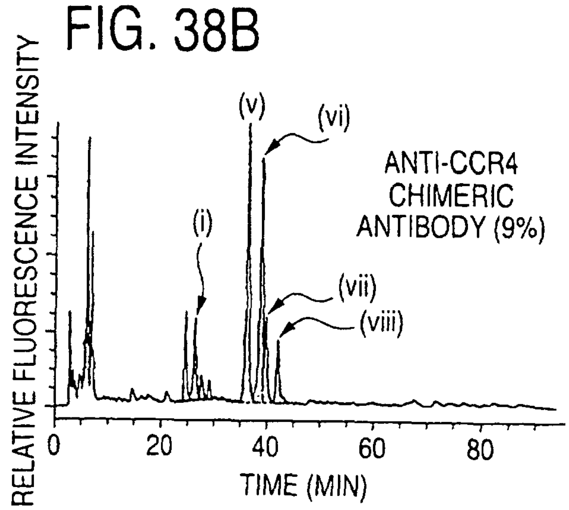


FIG. 38C

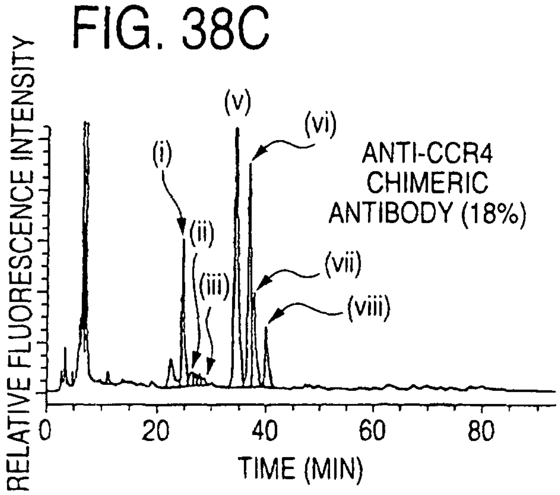


FIG. 38D

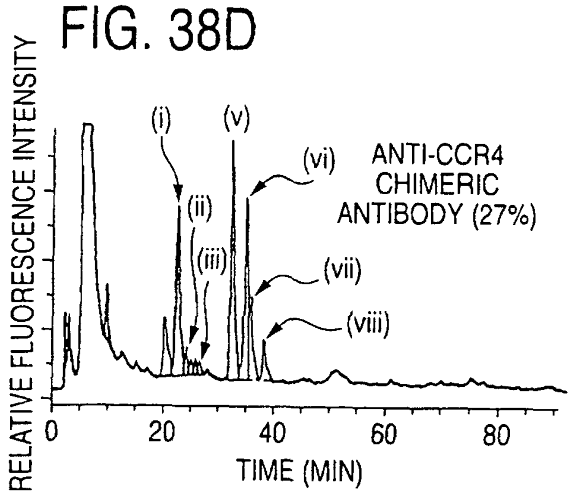


FIG. 38E

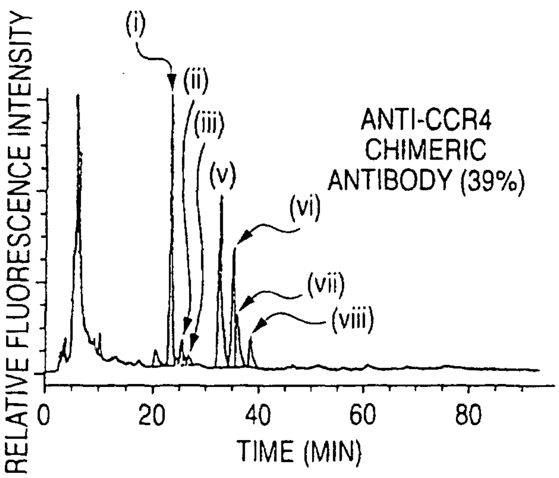


FIG. 38F

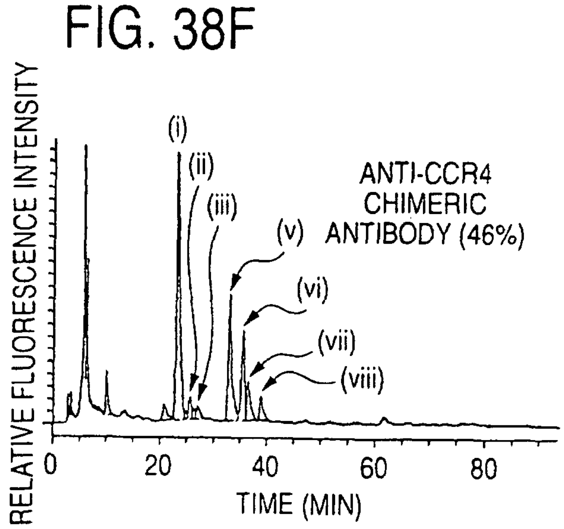

FIG. 39

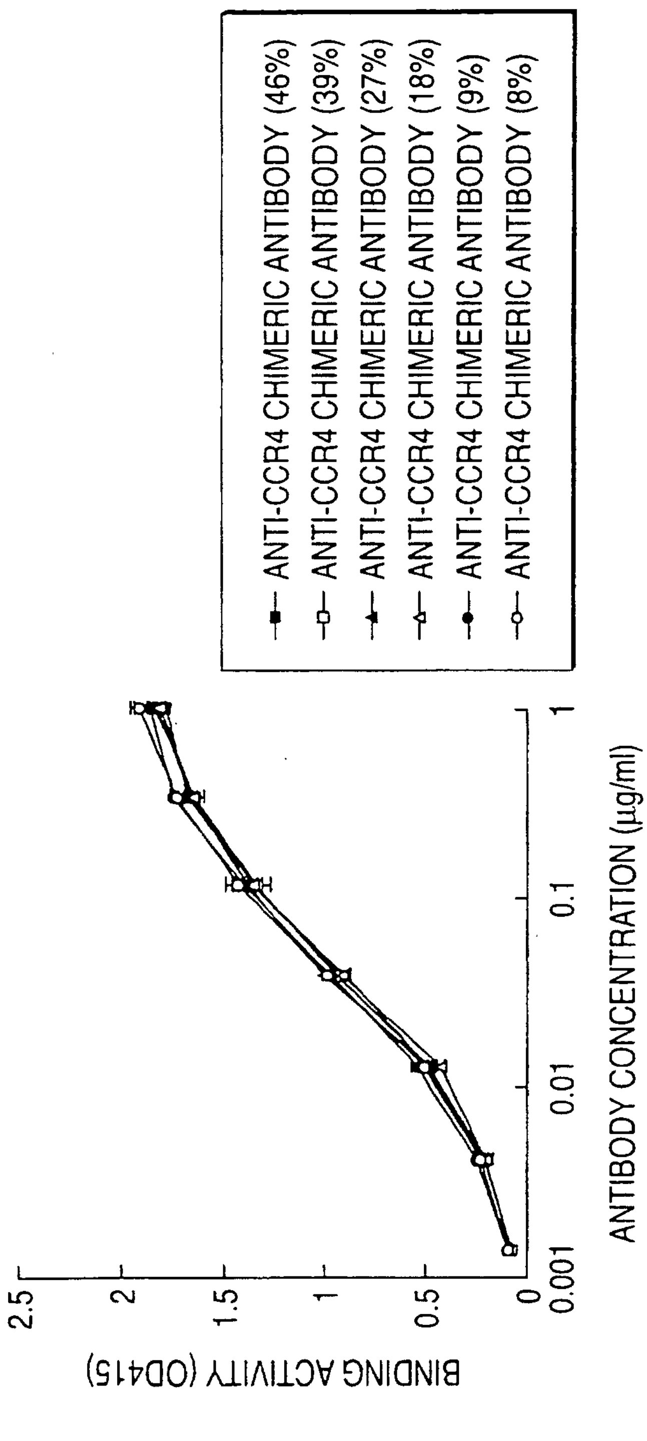
BINDING ACTIVITY (OD415)
2.5
2
1.5
1
0.5
0
0.001
0.01
0.1
1
ANTIBODY CONCENTRATION (μg/ml)
ANTI-CCR4 CHIMERIC ANTIBODY (46%)
ANTI-CCR4 CHIMERIC ANTIBODY (39%)
ANTI-CCR4 CHIMERIC ANTIBODY (27%)
ANTI-CCR4 CHIMERIC ANTIBODY (18%)
ANTI-CCR4 CHIMERIC ANTIBODY (9%)
ANTI-CCR4 CHIMERIC ANTIBODY (8%)

FIG. 40

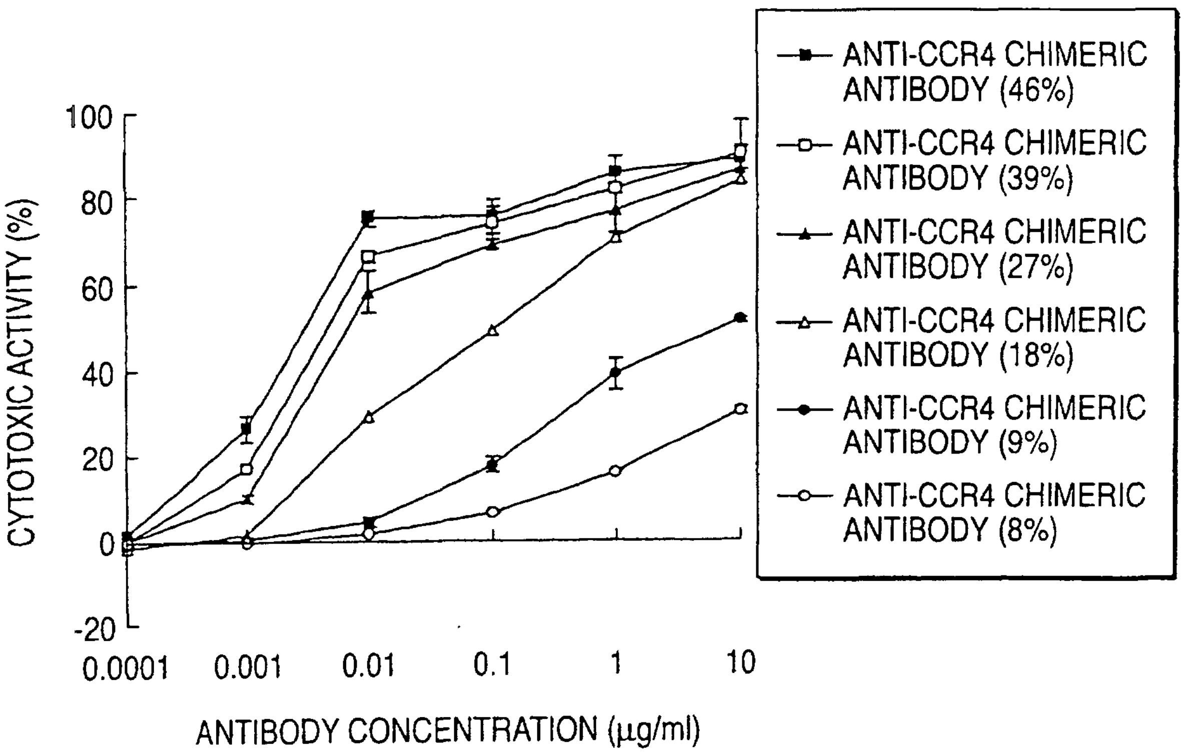

CYTOTOXIC ACTIVITY (%)
100
80
60
40
20
0
-20
0.0001
0.001
0.01
0.1
1
10
ANTIBODY CONCENTRATION (μg/ml)
ANTI-CCR4 CHIMERIC ANTIBODY (46%)
ANTI-CCR4 CHIMERIC ANTIBODY (39%)
ANTI-CCR4 CHIMERIC ANTIBODY (27%)
ANTI-CCR4 CHIMERIC ANTIBODY (18%)
ANTI-CCR4 CHIMERIC ANTIBODY (9%)
ANTI-CCR4 CHIMERIC ANTIBODY (8%)

FIG. 41

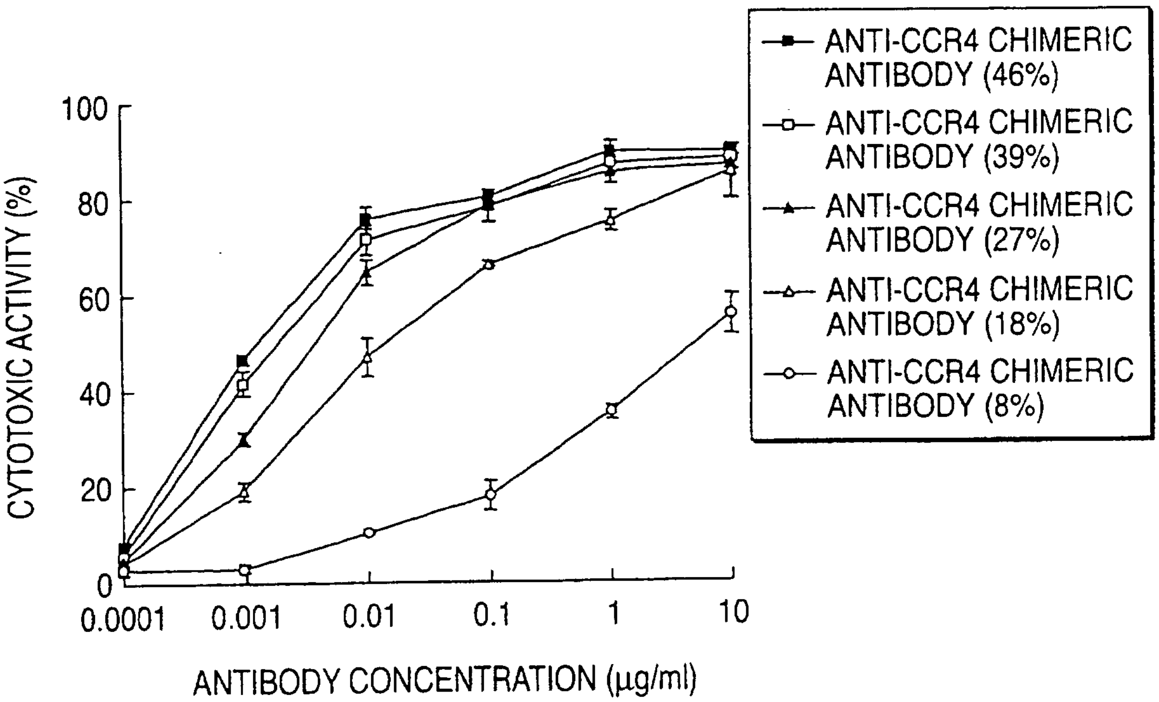
CYTOTOXIC ACTIVITY (%)
100
80
60
40
20
0
0.0001
0.001
0.01
0.1
1
10
ANTIBODY CONCENTRATION (μg/ml)
ANTI-CCR4 CHIMERIC ANTIBODY (46%)
ANTI-CCR4 CHIMERIC ANTIBODY (39%)
ANTI-CCR4 CHIMERIC ANTIBODY (27%)
ANTI-CCR4 CHIMERIC ANTIBODY (18%)
ANTI-CCR4 CHIMERIC ANTIBODY (8%)

FIG. 42

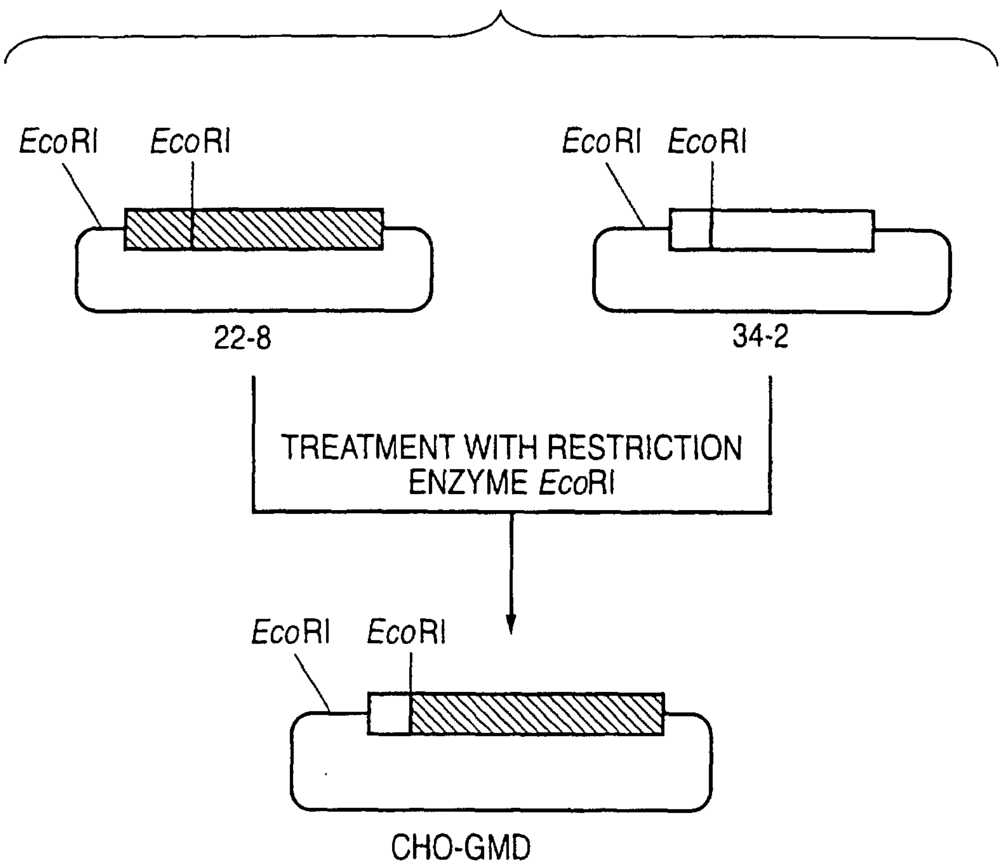
EcoRI
EcoRI
22-8
EcoRI
EcoRI
34-2
TREATMENT WITH RESTRICTION ENZYME EcoRI
EcoRI
EcoRI
CHO-GMD

**INTERNATIONAL SEARCH REPORT**

International application No.
PCT/JP03/04502

A. CLASSIFICATION OF SUBJECT MATTER
Int.Cl[7] C12P21/08, C12N15/09, C12N5/10, C07K16/18//A61K39/395, A61P9/00, A61P29/00, A61P31/04, A61P31/12, A61P31/14, A61P35/00, A61P37/02, A61P37/04, A61P37/08, A61P43/00,

According to International Patent Classification (IPC) or to both national classification and IPC

B. FIELDS SEARCHED

Minimum documentation searched (classification system followed by classification symbols)
Int.Cl[7] C12P21/08, C12N15/09, C12N5/10, C07K16/18

Documentation searched other than minimum documentation to the extent that such documents are included in the fields searched

Electronic data base consulted during the international search (name of data base and, where practicable, search terms used)
BIOSIS/MEDLINE/WPIDS(STN), CA(STN), Genbank/EMBL/DDBJ/GeneSeq

C. DOCUMENTS CONSIDERED TO BE RELEVANT

| Category* | Citation of document, with indication, where appropriate, of the relevant passages | Relevant to claim No. |
|---|---|---|
| X/Y | WO 00/61739 A1 (Kyowa Hakko Kogyo Co., Ltd.), 19 October, 2000 (19.10.00), & EP 117619 A1 & AU 3672800 A | 1-3,11-19, 21-24,32-39, 41-49,58-74, 81/4-10,20, 25-31,40, 50-57,75-80 |
| Y | Ripka J. et al., Two Chinese hamster ovary glycosylation mutants affected in the conversion of GDP-mannose to GDP-fucose, Arch.Biochem. Biophys., 1986, 249(2), pages 533 to 545 | 4-10,20, 25-31,40, 50-57,75-80 |
| X | Shitara K. et al., A new vector for the high level expression of chimeric antibodies in myeloma cells, J.Immunol.Methods, 1994, 167(1-2), pages 271 to 278 | 1-3,10-19, 21-24,31-39, 41-49,56-72 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 May, 2003 (23.05.03) | 22 July, 2003 (22.07.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

**INTERNATIONAL SEARCH REPORT**

International application No.
PCT/JP03/04502

C (Continuation). DOCUMENTS CONSIDERED TO BE RELEVANT

| Category* | Citation of document, with indication, where appropriate, of the relevant passages | Relevant to claim No. |
|---|---|---|
| A | Davies J. et al., Expression of GnTIII in a recombinant anti-CD20 CHO production cell line: Expression of antibodies with altered glycoforms leads to an increase in ADCC through higher affinity for FC gamma RIII, Biotechnol.Bioeng., 2001, 74(4), pages 288 to 294 | 1-81 |
| Y | Hackett J. Jr. et al., Recombinant mouse-human chimeric antibodies as calibrators in immunoassays that measure antibodies to Toxoplasma gondii, J. Clin.Microbiol., 1998, 36(5), pages 1277 to 1284 | 20,40,57 |
| Y | Elbashir SM. et al., Duplexes of 21-nucleotide RNAs mediate RNA interference in cultured mammalian cells, Nature, 2001, 411(6836), pages 494 to 498 | 4-10,20, 25-31,40, 50-57,75-80 |
| A | WO 97/27303 A (Toyobo Co., Ltd.), 31 July, 1997 (31.07.97), & US 6054304 A & EP 816503 A1 & JP 9-201191 A | 1-81 |
| A | Shields RL. et al., High resolution mapping of the binding site on human IgG1 for Fc gamma RI, Fc gamma RII, Fc gamma RIII, and FcRn and design of IgG1 variants with improved binding to the Fc gamma R, J.Biol.Chem., 2001, 276(9), pages 6591 to 6604 | 1-81 |
| P,X | Shields RL. et al., Lack of fucose on human IgG1 N-linked oligosaccharide improves binding to human Fc gamma RIII and antibody-dependent cellular toxicity, J.Biol.Chem., 2002 Jul., 277(30), pages 26733 to 26740 | 1-81 |
| P,X | SHINKAWA, T. et al., The absence of fucose but not the presence of galactose or bisecting N-acetylglucosamine of human IgG1 complex-type oligosaccharides shows the critical role of enhancing antibody-dependent cellular cytotoxicity, J.Biol.Chem., 2003, 278(5), pages 3466 to 3473 | 1-81 |
| P,X | WO 02/31140 A1 (Kyowa Hakko Kogyo Co., Ltd.), 18 April, 2002 (18.04.02), & AU 9419801 A | 1-81 |

Form PCT/ISA/210 (continuation of second sheet) (July 1998)

**INTERNATIONAL SEARCH REPORT**

International application No.
PCT/JP03/04502

Continuation of A. CLASSIFICATION OF SUBJECT MATTER
(International Patent Classification (IPC))

Int.Cl$^7$ G01N33/15, G01N33/50(C12P21/08, C12R1:91),
(C12N5/10, C12R1:91)

(According to International Patent Classification (IPC) or to both national classification and IPC)

Continuation of B. FIELDS SEARCHED
Minimum Documentation Searched(International Patent Classification (IPC))

Int.Cl$^7$

Minimum documentation searched (classification system followed by classification symbols)

Form PCT/ISA/210 (extra sheet) (July 1998)

SEQUENCE LISTING

<110> KYOWA HAKKO KOGYO CO., LTD.

<120> Production process for antibody composition

<130> P44075

<150> JP 2002-106820
<151> 2002-04-09

<150> JP 2003-024685
<151> 2003-01-31

<160> 100

<170> Patenting Veer. 2.1

<210> 1
<211> 18
<212> PRT
<213> Homo sapiens

<400> 1

```
Asp Glu Ser Ile Tyr Ser Asn Tyr Tyr Leu Tyr Glu Ser Ile Pro Lys
 1               5                  10                  15
Pro Cys
```

<210> 2
<211> 24
<212> DNA
<213> Artificial Sequence

<220>
<223> Description of Artificial Sequence: Synthetic DNA

<400> 2
aggaaggtgg cgctcatcac gggc 24

<210> 3
<211> 26
<212> DNA
<213> Artificial Sequence

<220>
<223> Description of Artificial Sequence: Synthetic DNA

<400> 3
taaggccaca agtcttaatt gcatcc 26

<210> 4
<211> 27
<212> DNA
<213> Artificial Sequence

<220>
<223> Description of Artificial Sequence: Synthetic DNA

<400> 4
caggggtgtt cccttgagga ggtggaa 27

<210> 5
<211> 23
<212> DNA
<213> Artificial Sequence

<220>
<223> Description of Artificial Sequence: Synthetic DNA

<400> 5

cccctcacgc atgaagcctg gag 23

<210> 6
<211> 28
<212> DNA
<213> Artificial Sequence

<220>
<223> Description of Artificial Sequence: Synthetic DNA

<400> 6
ggcaggagac caccttgcga gtgcccac 28

<210> 7
<211> 28
<212> DNA
<213> Artificial Sequence

<220>
<223> Description of Artificial Sequence: Synthetic DNA

<400> 7
ggcgctggct tacccggaga ggaatggg 28

<210> 8
<211> 28
<212> DNA
<213> Artificial Sequence

<220>
<223> Description of Artificial Sequence: Synthetic DNA

<400> 8
aaaaggcctc agttagtgaa ctgtatgg 28

<210> 9
<211> 29
<212> DNA
<213> Artificial Sequence

<220>
<223> Description of Artificial Sequence: Synthetic DNA

<400> 9
cgcggatcct caagcgttgg ggttggtcc 29

<210> 10
<211> 45
<212> DNA
<213> Artificial Sequence

<220>
<223> Description of Artificial Sequence: Synthetic DNA

<400> 10
cccaagcttg ccaccatggc tcacgctccc gctagctgcc cgagc 45

<210> 11
<211> 31
<212> DNA
<213> Artificial Sequence

<220>
<223> Description of Artificial Sequence: Synthetic DNA

<400> 11
ccggaattct gccaagtatg agccatcctg g 31

<210> 12
<211> 17
<212> DNA
<213> Artificial Sequence

<220>
<223> Description of Artificial Sequence: Synthetic DNA

<400> 12
gccatccaga aggtggt 17

<210> 13
<211> 17
<212> DNA
<213> Artificial Sequence

<220>
<223> Description of Artificial Sequence: Synthetic DNA

<400> 13
gtcttgtcag ggaagat 17

<210> 14
<211> 28
<212> DNA
<213> Artificial Sequence

<220>
<223> Description of Artificial Sequence: Synthetic DNA

<400> 14
ggcaggagac caccttgcga gtgcccac 28

<210> 15
<211> 28
<212> DNA
<213> Artificial Sequence

<220>
<223> Description of Artificial Sequence: Synthetic DNA

<400> 15
gggtgggctg taccttctgg aacagggc 28

<210> 16
<211> 28
<212> DNA
<213> Artificial Sequence

<220>
<223> Description of Artificial Sequence: Synthetic DNA

<400> 16
ggcgctggct tacccggaga ggaatggg 28

<210> 17
<211> 28
<212> DNA
<213> Artificial Sequence

<220>
<223> Description of Artificial Sequence: Synthetic DNA

<400> 17
ggaatgggtg tttgtctcctc caaagatgc 28

<210> 18

<211> 1316
<212> DNA
<213> Cricetulus griseus

<400> 18
gccccgcccc ctccacctgg accgagagta gctggagaat tgtgcaccgg aagtagctct 60
tggactggtg gaaccctgcg caggtgcagc aacaatgggt gagccccagg gatccaggag 120
gatcctagtg acagggggct ctggactggt gggcagagct atccagaagg tggtcgcaga 180
tggcgctggc ttacccggag aggaatgggt gtttgtctcc tccaaagatg cagatctgac 240
ggatgcagca caaacccaag ccctgttcca gaaggtacag cccacccatg tcatccatct 300
tgctgcaatg gtaggaggcc ttttccggaa tatcaaatac aacttggatt tctggaggaa 360
gaatgtgcac atcaatgaca acgtcctgca ctcagctttc gaggtgggca ctcgcaaggt 420
ggtctcctgc ctgtccacct gtatcttccc tgacaagacc acctatccta ttgatgaaac 480
aatgatccac aatggtccac cccacagcag caattttggg tactcgtatg ccaagaggat 540
gattgacgtg cagaacaggg cctacttcca gcagcatggc tgcaccttca ctgctgtcat 600
ccctaccaat gtctttggac ctcatgacaa cttcaacatt gaagatggcc atgtgctgcc 660
tggcctcatc cataaggtgc atctggccaa gagtaatggt tcagccttga ctgtttgggg 720
tacagggaaa ccacggaggc agttcatcta ctcactggac ctagcccggc tcttcatctg 780
ggtcctgcgg gagtacaatg aagttgagcc catcatcctc tcagtgggcg aggaagatga 840
agtctccatt aaggaggcag ctgaggctgt agtggaggcc atggacttct gtggggaagt 900
cacttttgat tcaacaaagt cagatgggca gtataagaag acagccagca atggcaagct 960
tcgggcctac ttgcctgatt tccgtttcac acccttcaag caggctgtga aggagacctg 1020
tgcctggttc accgacaact atgagcaggc ccggaagtga agcatgggac aagcgggtgc 1080
tcagctggca atgcccagtc agtaggctgc agtctcatca tttgcttgtc aagaactgag 1140
gacagtatcc agcaacctga gccacatgct ggtctctctg ccagggggct tcatgcagcc 1200
atccagtagg gcccatgttt gtccatcctc gggggaaggc cagaccaaca ccttgtttgt 1260
ctgcttctgc cccaacctca gtgcatccat gctggtcctg ctgtcccttg tctaga 1316

<210> 19
<211> 23
<212> DNA
<213> Artificial Sequence

<220>
<223> Description of Artificial Sequence: Synthetic DNA

<400> 19
gatcctgctg ggaccaaaat tgg 23

<210> 20
<211> 22
<212> DNA
<213> Artificial Sequence

<220>
<223> Description of Artificial Sequence: Synthetic DNA

<400> 20
cttaacatcc caagggatgc tg 22

<210> 21
<211> 1965
<212> DNA
<213> Cricetulus griseus

<400> 21
acggggggct cccggaagcg gggaccatgg cgtctctgcg cgaagcgagc ctgcggaagc 60
tgcggcgctt ttccgagatg agaggcaaac ctgtggcaac tgggaaattc tgggatgtag 120
ttgtaataac agcagctgac gaaaagcagg agcttgctta caagcaacag ttgtcggaga 180
agctgaagag aaaggaattg ccccttggag ttaactacca tgttttcact gatcctcctg 240
gaaccaaaat tggaaatgga ggatcaacac tttgttctct tcagtgcctg gaaagcctct 300
atggagacaa gtggaattcc ttcacagtcc tgttaattca ctctggtggc tacagtcaac 360
gacttcccaa tgcaagcgct ttaggaaaaa tcttcacggc tttaccactt ggtgagccca 420
tttatcagat gttggactta aaactagcca tgtacatgga tttcccctca cgcatgaagc 480
ctggagtttt ggtcacctgt gcagatgata ttgaactata cagcattggg gactctgagt 540
ccattgcatt tgagcagcct ggctttactg ccctagccca tccatctagt ctggctgtag 600
gcaccacaca tggagtattt gtattggact ctgccggttc tttgcaacat ggtgacctag 660
agtacaggca atgccaccgt ttcctccata agcccagcat tgaaaacatg caccacttta 720
atgccgtgca tagactagga agctttggtc aacaggactt gagtgggggt gacaccacct 780
gtcatccatt gcactctgag tatgtctaca cagatagcct attttacatg gatcataaat 840
cagccaaaaa gctacttgat ttctatgaaa gtgtaggccc actgaactgt gaaatagatg 900
cctatggtga ctttctgcag gcactgggac ctggagcaac tgcagagtac accaagaaca 960

cctcacacgt cactaaagag gaatcacact tgttggacat gaggcagaaa atattccacc 1020
tcctcaaggg aacacccctg aatgttgttg tccttaataa ctccaggttt tatcacattg 1080
gaacaacgga ggagtatctg ctacatttca cttccaatgg ttcgttacag gcagagctgg 1140
gcttgcaatc catagctttc agtgtctttc caaatgtgcc tgaagactcc catgagaaac 1200
cctgtgtcat tcacagcatc ctgaattcag gatgctgtgt ggcccctggc tcagtggtag 1260
aatattccag attaggacct gaggtgtcca tctcggaaaa ctgcattatc agcggttctg 1320
tcatagaaaa agctgttctg cccccatgtt ctttcgtgtg ctctttaagt gtggagataa 1380
atggacactt agaatattca actatggtgt ttggcatgga agacaacttg aagaacagtg 1440
ttaaaaccat atcagatata aagatgcttc agttctttgg agtctgtttc ctgacttgtt 1500
tagatatttg gaaccttaaa gctatggaag aactattttc aggaagtaag acgcagctga 1560
gcctgtggac tgctcgaatt ttccctgtct gttcttctct gagtgagtcg gttgcagcat 1620
cccttgggat gttaaatgcc attcgaaacc attcgccatt cagcctgagc aacttcaagc 1680
tgctgtccat ccaggaaatg cttctctgca aagatgtagg agacatgctt gcttacaggg 1740
agcaactctt tctagaaatc agttcaaaga gaaaacagtc tgattcggag aaatcttaaa 1800
tacaatggat tttgcctgga aacaggattg caaatgcagg catattctat agatctctgg 1860
gttcttcttt ctttctcccc tctctccttt cctttccctt tgatgtaatg acaaaggtaa 1920
aaatggccac ttctgatgga aaaaaaaaaa aaaaaaaaaa aaaaa 1965

<210> 22
<211> 27
<212> DNA
<213> Artificial Sequence

<220>
<223> Description of Artificial Sequence: Synthetic DNA

<400> 22
caggggtgtt cccttgagga ggtggaa 27

<210> 23
<211> 27
<212> DNA
<213> Artificial Sequence

<220>

<223> Description of Artificial Sequence: Synthetic DNA

<400> 23
cactgagcca ggggccacac agcatcc 27

<210> 24
<2 11> 23
<212> DNA
<213> Artificial Sequence

<220>
<223> Description of Artificial Sequence: Synthetic DNA

<400> 24
cccctcacgc atgaagcctg gag 23

<210> 25
<211> 27
<212> DNA
<213> Artificial Sequence

<220>
<223> Description of Artificial Sequence: Synthetic DNA

<400> 25
tgccaccgtt tcctccataa gcccagc 27

<210> 26
<211> 28
<212> DNA
<213> Artificial Sequence

<220>
<223> Description of Artificial Sequence: Synthetic DNA

<400> 26
atggctcaag ctcccgctaa gtgcccga 28

<210> 27
<211> 27
<212> DNA
<213> Artificial Sequence

<220>
<223> Description of Artificial Sequence: Synthetic DNA

<400> 27
tcaagcgttt gggttggtcc tcatgag 27

<210> 28
<211> 25
<212> DNA
<213> Artificial Sequence

<220>
<223> Description of Artificial Sequence: Synthetic DNA

<400> 28
tccggggatg gcgagatggg caagc 25

<210> 29
<211> 24
<212> DNA

<213> Artificial Sequence

<220>
<223> Description of Artificial Sequence: Synthetic DNA

<400> 29
cttgacatgg ctctgggctc caag 24

<210> 30
<211> 25
<212> DNA
<213> Artificial Sequence

<220>
<223> Description of Artificial Sequence: Synthetic DNA

<400> 30
ccacttcagt cggtcggtag tattt 25

<210> 31
<211> 24
<212> DNA
<213> Artificial Sequence

<220>
<223> Description of Artificial Sequence: Synthetic DNA

<400> 31
cgctcacccg cctgaggcga catg 24

<210> 32
<211> 32
<212> DNA
<213> Artificial Sequence

<220>
<223> Description of Artificial Sequence: Synthetic DNA

<400> 32
ggcaggtgct gtcggtgagg tcaccatagt gc 32

<210> 33
<211> 24
<212> DNA
<213> Artificial Sequence

<220>
<223> Description of Artificial Sequence: Synthetic DNA

<400> 33
ggggccatgc caaggactat gtcg 24

<210> 34
<211> 25
<212> DNA
<213> Artificial Sequence

<220>
<223> Description of Artificial Sequence: Synthetic DNA

<400> 34
atgtggctga tgttacaaaa tgatg 25

<210> 35
<211> 1504
<212> DNA
<213> Cricetulus griseus

<220>
<221> CDS
<222> (1)..(1119)

<400> 35

```
atg gct cac gct ccc gct agc tgc ccg agc tcc agg aac tct ggg gac      48
Met Ala His Ala Pro Ala Ser Cys Pro Ser Ser Arg Asn Ser Gly Asp
1               5                   10                  15
ggc gat aag ggc aag ccc agg aag gtg gcg ctc atc acg ggc atc acc      96
Gly Asp Lys Gly Lys Pro Arg Lys Val Ala Leu Ile Thr Gly Ile Thr
            20                  25                  30
ggc cag gat ggc tca tac ttg gca gaa ttc ctg ctg gag aaa gga tac      144
Gly Gln Asp Gly Ser Tyr Leu Ala Glu Phe Leu Leu Glu Lys Gly Tyr
        35                  40                  45
gag gtt cat gga att gta cgg cga tcc agt tca ttt aat aca ggt cga      192
Glu Val His Gly Ile Val Arg Arg Ser Ser Ser Phe Asn Thr Gly Arg
    50                  55                  60
att gaa cat tta tat aag aat cca cag gct cat att gaa gga aac atg      240
Ile Glu His Leu Tyr Lys Asn Pro Gln Ala His Ile Glu Gly Asn Met
65                  70                  75                  80
aag ttg cac tat ggt gac ctc acc gac agc acc tgc cta gta aaa atc      288
Lys Leu His Tyr Gly Asp Leu Thr Asp Ser Thr Cys Leu Val Lys Ile
85                  90                  95                  100
atc aat gaa gtc aaa cct aca gag atc tac aat ctt ggt gcc cag agc      336
Ile Asn Glu Val Lys Pro Thr Glu Ile Tyr Asn Leu Gly Ala Gln Ser
                105                 110                 115
cat gtc aag att tcc ttt gac tta gca gag tac act gca gat gtt gat      384
His Val Lys Ile Ser Phe Asp Leu Ala Glu Tyr Thr Ala Asp Val Asp
            120                 125                 130
gga gtt ggc acc ttg cgg ctt ctg gat gca att aag act tgt ggc ctt      432
Gly Val Gly Thr Leu Arg Leu Leu Asp Ala Ile Lys Thr Cys Gly Leu
        135                 140                 145
ata aat tct gtg aag ttc tac cag gcc tca act agt gaa ctg tat gga      480
Ile Asn Ser Val Lys Phe Tyr Gln Ala Ser Thr Ser Glu Leu Tyr Gly
    150                 155                 160
aaa gtg caa gaa ata ccc cag aaa gag acc acc cct ttc tat cca agg      528
Lys Val Gln Glu Ile Pro Gln Lys Glu Thr Thr Pro Phe Tyr Pro Arg
165                 170                 175                 180
tcg ccc tat gga gca gcc aaa ctt tat gcc tat tgg att gta gtg aac      576
Ser Pro Tyr Gly Ala Ala Lys Leu Tyr Ala Tyr Trp Ile Val Val Asn
```

```
            185                 190                 195
ttt cga gag gct tat aat ctc ttt gcg gtg aac ggc att ctc ttc aat     624
Phe Arg Glu Ala Tyr Asn Leu Phe Ala Val Asn Gly Ile Leu Phe Asn
        200                 205                 210
cat gag agt cct aga aga gga gct aat ttt gtt act cga aaa att agc     672
His Glu Ser Pro Arg Arg Gly Ala Asn Phe Val Thr Arg Lys Ile Ser
    215                 220                 225
cgg tca gta gct aag att tac ctt gga caa ctg gaa tgt ttc agt ttg     720
Arg Ser Val Ala Lys Ile Tyr Leu Gly Gln Leu Glu Cys Phe Ser Leu
230                 235                 240
gga aat ctg gac gcc aaa cga gac tgg ggc cat gcc aag gac tat gtc     768
Gly Asn Leu Asp Ala Lys Arg Asp Trp Gly His Ala Lys Asp Tyr Val
245                 250                 255                 260
gag gct atg tgg ctg atg tta caa aat gat gaa cca gag gac ttt gtc     816
Glu Ala Met Trp Leu Met Leu Gln Asn Asp Glu Pro Glu Asp Phe Val
            265                 270                 275
ata gct act ggg gaa gtt cat agt gtc cgt gaa ttt gtt gag aaa tca     864
Ile Ala Thr Gly Glu Val His Ser Val Arg Glu Phe Val Glu Lys Ser
        280                 285                 290
ttc atg cac att gga aag acc att gtg tgg gaa gga aag aat gaa aat     912
Phe Met His Ile Gly Lys Thr Ile Val Trp Glu Gly Lys Asn Glu Asn
    295                 300                 305
gaa gtg ggc aga tgt aaa gag acc ggc aaa att cat gtg act gtg gat     960
Glu Val Gly Arg Cys Lys Glu Thr Gly Lys Ile His Val Thr Val Asp
310                 315                 320
ctg aaa tac tac cga cca act gaa gtg gac ttc ctg cag gga gac tgc    1008
Leu Lys Tyr Tyr Arg Pro Thr Glu Val Asp Phe Leu Gln Gly Asp Cys
325                 330                 335                 340
tcc aag gcg cag cag aaa ctg aac tgg aag ccc cgc gtt gcc ttt gac    1056
Ser Lys Ala Gln Gln Lys Leu Asn Trp Lys Pro Arg Val Ala Phe Asp
            345                 350                 355
gag ctg gtg agg gag atg gtg caa gcc gat gtg gag ctc atg aga acc    1104
Glu Leu Val Arg Glu Met Val Gln Ala Asp Val Glu Leu Met Arg Thr
        360                 365                 370
aac ccc aac gcc tga gcacctctac aaaaaaattc gcgagacatg gactatggtg    1159
Asn Pro Asn Ala
    375
```

cagagccagc caaccagagt ccagccactc ctgagaccat cgaccataaa ccctcgactg 1219
cctgtgtcgt ccccacagct aagagctggg ccacaggttt gtgggcacca ggacggggac 1279
actccagagc taaggccact tcgcttttgt caaaggctcc tctcaatgat tttgggaaat 1339
caagaagttt aaaatcacat actcatttta cttgaaatta tgtcactaga caacttaaat 1399
ttttgagtct tgagattgtt tttctctttt cttattaaat gatctttcta tgacccagca 1459
aaaaaaaaaa aaaaaaggga tataaaaaaa aaaaaaaaaa aaaaa 1504

<210> 36
<211> 25
<212> DNA
<213> Artificial Sequence

<220>
<223> Description of Artificial Sequence: Synthetic DNA

<400> 36
atgaagttgc actatggtga cctca 25

<210> 37
<211> 59
<212> DNA
<213> Cricetulus griseus

<400> 37
ccgacagcac ctgcctagta aaaatcatca atgaagtcaa acctacagag atctacaat 59

<210> 38
<211> 25
<212> DNA
<213> Artificial Sequence

<220>
<223> Description of Artificial Sequence: Synthetic DNA

<400> 38
gacttagcag agtacactgc agatg 25

<210> 39
<211> 25
<212> DNA
<213> Artificial Sequence

<220>
<223> Description of Artificial Sequence: Synthetic DNA

<400> 39
accttggata gaaaggggtg gtctc 25

<210> 40
<211> 125
<212> DNA
<213> Cricetulus griseus

<400> 40
ttgatggagt tggcaccttg cggcttctgg atgcaattaa gacttgtggc cttataaatt 60
ctgtgaagtt ctaccaggcc tcaactagtg aactgtatgg aaaagtgcaa gaaatacccc 120
agaaa 125

<210> 41
<211> 372
<212> PRT
<213> Cricetulus griseus

<400> 41
Met Ala His Ala Pro Ala Ser Cys Pro Ser Ser Arg Asn Ser Gly Asp
1 5 10 15
Gly Asp Lys Gly Lys Pro Arg Lys Val Ala Leu Ile Thr Gly Ile Thr
20 25 30

Gly Gln Asp Gly Ser Tyr Leu Ala Glu Phe Leu Leu Glu Lys Gly Tyr
35 40 45
Glu Val His Gly Ile Val Arg Arg Ser Ser Ser Phe Asn Thr Gly Arg
50 55 60
Ile Glu His Leu Tyr Lys Asn Pro Gln Ala His Ile Glu Gly Asn Met
65 70 75 80
Lys Leu His Tyr Gly Asp Leu Thr Asp Ser Thr Cys Leu Val Lys Ile
85 90 95
Ile Asn Glu Val Lys Pro Thr Glu Ile Tyr Asn Leu Gly Ala Gln Ser
100 105 110
His Val Lys Ile Ser Phe Asp Leu Ala Glu Tyr Thr Ala Asp Val Asp
120 120 125
Gly Val Gly Thr Leu Arg Leu Leu Asp Ala Ile Lys Thr Cys Gly Leu
130 135 140
Ile Asn Ser Val Lys Phe Tyr Gln Ala Ser Thr Ser Glu Leu Tyr Gly
145 150 155 160
Lys Val Gln Glu Ile Pro Gln Lys Glu Thr Thr Pro Phe Tyr Pro Arg
165 170 175
Ser Pro Tyr Gly Ala Ala Lys Leu Tyr Ala Tyr Trp Ile Val Val Asn
180 185 190
Phe Arg Glu Ala Tyr Asn Leu Phe Ala Val Asn Gly Ile Leu Phe Asn
195 200 205
His Glu Ser Pro Arg Arg Gly Ala Asn Phe Val Thr Arg Lys Ile Ser
210 215 220
Arg Ser Val Ala Lys Ile Tyr Leu Gly Gln Leu Glu Cys Phe Ser Leu
225 230 235 240
Gly Asn Leu Asp Ala Lys Arg Asp Trp Gly His Ala Lys Asp Tyr Val
245 250 255
Glu Ala Met Trp Leu Met Leu Gln Asn Asp Glu Pro Glu Asp Phe Val
260 265 270
Ile Ala Thr Gly Glu Val His Ser Val Arg Glu Phe Val Glu Lys Ser
275 280 285
Phe Met His Ile Gly Lys Thr Ile Val Trp Glu Gly Lys Asn Glu Asn
290 295 300
Glu Val Gly Arg Cys Lys Glu Thr Gly Lys Ile His Val Thr Val Asp
305 310 315 320
Leu Lys Tyr Tyr Arg Pro Thr Glu Val Asp Phe Leu Gln Gly Asp Cys

325 330 335
Ser Lys Ala Gln Gln Lys Leu Asn Trp Lys Pro Arg Val Ala Phe Asp
340 345 350
Glu Leu Val Arg Glu Met Val Gln Ala Asp Val Glu Leu Met Arg Thr
355 360 365
Asn Pro Asn Ala
370

<210> 42
<211> 24
<212> DNA
<213> Artificial Sequence

<220>
<223> Description of Artificial Sequence: Synthetic DNA

<400> 42
gtccatggtg atcctgcagt gtgg 24

<210> 43
<211> 23
<212> DNA
<213> Artificial Sequence

<22 0>
<223> Description of Artificial Sequence: Synthetic DNA

<400> 43
caccaatgat atctccaggt tcc 23

<210> 44
<211> 24
<212> DNA
<213> Artificial Sequence

<220>
<223> Description of Artificial Sequence: Synthetic DNA

<400> 44
gatatcgctg cgctcgttgt cgac 24

<210> 45
<211> 24
<212> DNA
<213> Artificial Sequence

<220>
<223> Description of Artificial Sequence: Synthetic DNA

<400> 45
caggaaggaa ggctggaaaa gagc 24

<210> 46
<211> 384
<212> DNA
<213> Mus musculus

<400> 46
atg gat ttt cag gtg cag att atc agc ttc ctg cta atc agt gct tca 48
Met Asp Phe Gln Val Gln Ile Ile Ser Phe Leu Leu Ile Ser Ala Ser
1 5 10 15
gtc ata atg tcc aga gga caa att gtt ctc tcc cag tct cca gca atc 96
Val Ile Met Ser Arg Gly Gln Ile Val Leu Ser Gln Ser Pro Ala Ile
20 25 30
ctg tct gca tct cca ggg gag aag gtc aca atg act tgc agg gcc agc 144
Leu Ser Ala Ser Pro Gly Glu Lys Val Thr Met Thr Cys Arg Ala Ser
35 40 45
tca agt gta agt tac atc cac tgg ttc cag cag aag cca gga tcc tcc 192
Ser Ser Val Ser Tyr Ile His Trp Phe Gln Gln Lys Pro Gly Ser Ser

50 55 60

ccc aaa ccc tgg att tat gcc aca tcc aac ctg gct tct gga gtc cct 240
Pro Lys Pro Trp Ile Tyr Ala Thr Ser Asn Leu Ala Ser Gly Val Pro
65 70 75 80

gtt cgc ttc agt ggc agt ggg tct ggg act tct tac tct ctc acc atc 288
Val Arg Phe Ser Gly Ser Gly Ser Gly Thr Ser Tyr Ser Leu Thr Ile
85 90 95

agc aga gtg gag gct gaa gat gct gcc act tat tac tgc cag cag tgg 336
Ser Arg Val Glu Ala Glu Asp Ala Ala Thr Tyr Tyr Cys Gln Gln Trp
100 105 110

act agt aac cca ccc acg ttc gga ggg ggg acc aag ctg gaa atc aaa 384
Thr Ser Asn Pro Pro Thr Phe Gly Gly Gly Thr Lys Leu Glu Ile Lys
115 120 125

<210> 47
<211> 420
<212> DNA
<213> Mus musculus

<400> 47
atg ggt tgg agc ctc atc ttg ctc ttc ctt gtc gct gtt gct acg cgt 48
Met Gly Trp Ser Leu Ile Leu Leu Phe Leu Val Ala Val Ala Thr Arg
1 5 10 15

gtc ctg tcc cag gta caa ctg cag cag cct ggg gct gag ctg gtg aag 96
Val Leu Ser Gln Val Gln Leu Gln Gln Pro Gly Ala Glu Leu Val Lys
20 25 30

cct ggg gcc tca gtg aag atg tcc tgc aag gct tct ggc tac aca ttt 144
Pro Gly Ala Ser Val Lys Met Ser Cys Lys Ala Ser Gly Tyr Thr Phe
35 40 45

acc agt tac aat atg cac tgg gta aaa cag aca cct ggt cgg ggc ctg 192
Thr Ser Tyr Asn Met His Trp Val Lys Gln Thr Pro Gly Arg Gly Leu
50 55 60

gaa tgg att gga gct att tat ccc gga aat ggt gat act tcc tac aat 240
Glu Trp Ile Gly Ala Ile Tyr Pro Gly Asn Gly Asp Thr Ser Tyr Asn
65 70 75 80

cag aag ttc aaa ggc aag gcc aca ttg act gca gac aaa tcc tcc agc 288

Gln Lys Phe Lys Gly Lys Ala Thr Leu Thr Ala Asp Lys Ser Ser Ser
85 90 95

aca gcc tac atg cag ctc agc agc ctg aca tct gag gac tct gcg gtc 336
Thr Ala Tyr Met Gln Leu Ser Ser Leu Thr Ser Glu Asp Ser Ala Val
100 105 110

tat tac tgt gca aga tcg act tac tac ggc ggt gac tgg tac ttc aat 384
Tyr Tyr Cys Ala Arg Ser Thr Tyr Tyr Gly Gly Asp Trp Tyr Phe Asn
115 120 125

gtc tgg ggc gca ggg acc acg gtc acc gtc tct gca 420
Val Trp Gly Ala Gly Thr Thr Val Thr Val Ser Ala
130 135 140

<210> 48
<211> 91
<212> DNA
<213> Artificial Sequence

<220>
<223> Description of Artificial Sequence: Synthetic DNA

<400> 48
caggaaacag ctatgacgaa ttcgcctcct caaaatggat tttcaggtgc agattatcag 60
cttcctgcta atcagtgctt cagtcataat g 91

<210> 49
<211> 91
<212> DNA
<213> Artificial Sequence

<220>
<223> Description of Artificial Sequence: Synthetic DNA

<400> 49
gtgaccttct cccctggaga tgcagacagg attgctggag actgggagag aacaatttgt 60
cctctggaca ttatgactga agcactgatt a 91

<210> 50
<211> 90
<212> DNA
<213> Artificial Sequence

<220>
<223> Description of Artificial Sequence: Synthetic DNA

<400> 50
ctccagggga gaaggtcaca atgacttgca gggccagctc aagtgtaagt tacatccact 60
ggttccagca gaagccagga tcctccccca 90

<210> 51
<211> 89
<212> DNA
<213> Artificial Sequence

<220>
<223> Description of Artificial Sequence: Synthetic DNA

<400> 51
ccagacccac tgccactgaa gcgaacaggg actccagaag ccaggttgga tgtggcataa 60
atccagggtt tgggggagga tcctggctt 89

<210> 52
<211> 91
<212> DNA
<213> Artificial Sequence

<220>
<223> Description of Artificial Sequence: Synthetic DNA

<400> 52

tcagtggcag tgggtctggg acttcttact ctctcaccat cagcagagtg gaggctgaag 60
atgctgccac ttattactgc cagcagtgga c 91

<210> 53
<211> 90
<212> DNA
<213> Artificial Sequence

<220>
<22 3> Description of Artificial Sequence: Synthetic DNA

<400> 53
gttttcccag tcacgaccgt acgtttgatt tccagcttgg tcccccctcc gaacgtgggt 60
gggttactag tccactgctg gcagtaataa 90

<210> 54
<211> 99
<212> DNA
<213> Artificial Sequence

<220>
<223> Description of Artificial Sequence: Synthetic DNA

<400> 54
caggaaacag ctatgacgcg gccgcgaccc ctcaccatgg gttggagcct catcttgctc 60
ttccttgtcg ctgttgctac gcgtgtcctg tcccaggta 99

<210> 55
<211> 98
<212> DNA
<213> Artificial Sequence

<220>
<223> Description of Artificial Sequence: Synthetic DNA

<400> 55
atgtgtagcc agaagccttg caggacatct tcactgaggc cccagccttc accagctcag 60
ccccaggctg ctgcagttgt acctgggaca ggacacgc 98

<210> 56
<211> 97
<212> DNA
<213> Artificial Sequence

<220>
<223> Description of Artificial Sequence: Synthetic DNA

<400> 56
caaggcttct ggctacacat ttaccagtta caatatgcac tgggtaaaac agacacctgg 60
tcggggcctg gaatggattg gagctattta tcccgga 97

<210> 57
<211> 99
<212> DNA
<213> Artificial Sequence

<220>
<223> Description of Artificial Sequence: Synthetic DNA

<400> 57
gtaggctgtg ctggaggatt tgtctgcagt caatgtggcc ttgcctttga acttctgatt 60
gtaggaagta tcaccatttc cgggataaat agctccaat 99

<210> 58
<211> 99
<212> DNA
<213> Artificial Sequence

<220>

<223> Description of Artificial Sequence: Synthetic DNA

<400> 58
aatcctccag cacagcctac atgcagctca gcagcctgac atctgaggac tctgcggtct 60
attactgtgc aagatcgact tactacggcg gtgactggt 99

<210> 59
<211> 98
<212> DNA
<213> Artificial Sequence

<220>
<223> Description of Artificial Sequence: Synthetic DNA

<400> 59
gttttcccag tcacgacggg cccttggtgg aggctgcaga gacggtgacc gtggtccctg 60
cgccccagac attgaagtac cagtcaccgc cgtagtaa 98

<210> 60
<211> 25
<212> DNA
<213> Artificial Sequence

<220>
<223> Description of Artificial Sequence: Synthetic DNA

<400> 60
gagctggtga agcctggggc ctcag 25

<210> 61
<211> 24
<212> DNA
<213> Artificial Sequence

<220>
<223> Description of Artificial Sequence: Synthetic DNA

<400> 61
gtctgaagca ttatgtgttg aagc 24

<210> 62
<211> 23
<212> DNA
<213> Artificial Sequence

<220>
<223> Description of Artificial Sequence: Synthetic DNA

<400> 62
gtgagtacat tcattgtact gtg 23

<210> 63
<211> 19
<212> DNA
<213> Cricetulus griseus

<220>
<221> CDS

<400> 63
gctgagtctc tccgaatac 19

<210> 64
<211> 19
<212> DNA
<213> Cricetulus griseus

<220>

<221> CDS

<400> 64
gaacactcat cttggaatc 19

<210> 65
<211> 42
<212> DNA
<213> Artificial Sequence

<220>
<223> Description of Artificial Sequence: Synthetic DNA

<400> 65
gctctagaga attcaaggtc gggcaggaag agggcctatt tc 42

<210> 66
<211> 46
<212> DNA
<213> Artificial Sequence

<220>
<223> Description of Artificial Sequence: Synthetic DNA

<400> 66
cgggatcctt cacgtgtttc gtcctttcca caagatatat aaagcc 46

<210> 67
<211> 20
<212> DNA
<213> Artificial Sequence

<220>
<223> Description of Artificial Sequence: Synthetic DNA

<400> 67
cagatctgcg gccgcgagct 20

<210> 68
<211> 20
<212> DNA
<213> Artificial Sequence

<220>
<223> Description of Artificial Sequence: Synthetic DNA

<400> 68
cgcggccgca gatctgagct 20

<210> 69
<211> 37
<212> DNA
<213> Artificial Sequence

<220>
<223> Description of Artificial Sequence: Synthetic DNA

<400> 69
cgggatccaa ggtcgggcag gaagagggcc tatttcc 37

<210> 70
<211> 46
<212> DNA
<213> Artificial Sequence

<220>
<223> Description of Artificial Sequence: Synthetic DNA

<400> 70
cggaattctt cacgtgtttc gtcctttcca caagatatat aaagcc 46

<210> 71
<211> 26
<212> DNA
<213> Artificial Sequence

<220>
<223> Description of Artificial Sequence: Synthetic DNA

<400> 71
cgctgagtct ctccgaatac tttttg 26

<210> 72
<211> 30
<212> DNA
<213> Artificial Sequence

<220>
<223> Description of Artificial Sequence: Synthetic DNA

<400> 72
gatccaaaaa gtattcggag agactcagcg 30

<210> 73
<211> 26
<212> DNA
<213> Artificial Sequence

<220>
<223> Description of Artificial Sequence: Synthetic DNA

<400> 73

cgaacactca tcttggaatc tttttg 26

<210> 74
<211> 30
<212> DNA
<213> Artificial Sequence

<220>
<223> Description of Artificial Sequence: Synthetic DNA

<400> 74
gatccaaaaa gattccaaga tgagtgttcg 30

<210> 75
<211> 26
<212> DNA
<213> Artificial Sequence

<220>
<223> Description of Artificial Sequence: Synthetic DNA

<400> 75
cgtattcgga gagactcagc tttttt 26

<210> 76
<211> 30
<212> DNA
<213> Artificial Sequence

<220>
<223> Description of Artificial Sequence: Synthetic DNA

<400> 76
aattaaaaaa gctgagtctc tccgaatacg 30

<210> 77
<211> 26
<212> DNA
<213> Artificial Sequence

<220>
<223> Description of Artificial Sequence: Synthetic DNA

<400> 77
cgattccaag atgagtgttc tttttt 26

<210> 78
<211> 30
<212> DNA
<213> Artificial Sequence

<220>
<223> Description of Artificial Sequence: Synthetic DNA

<400> 78
aattaaaaaa gaacactcat cttggaatcg 30

<210> 79
<211> 2008
<212> DNA
<213> Cricetulus griseus

<220> 79
aacagaaact tattttcctg tgtggctaac tagaaccaga gtacaatgtt tccaattctt 60
tgagctccga gaagacagaa gggagttgaa actctgaaaa tgcgggcatg gactggttcc 120
tggcgttgga ttatgctcat tctttttgcc tgggggacct tattgtttta tataggtggt 180
catttggttc gagataatga ccaccctgac cattctagca gagaactctc caagattctt 240
gcaaagctgg agcgcttaaa acaacaaaat gaagacttga ggagaatggc tgagtctctc 300

cgaataccag aaggccctat tgatcagggg acagctacag gaagagtccg tgttttagaa 360
gaacagcttg ttaaggccaa agaacagatt gaaaattaca agaaacaagc taggaatgat 420
ctgggaaagg atcatgaaat cttaaggagg aggattgaaa atggagctaa agagctctgg 480
ttttttctac aaagtgaatt gaagaaatta aagaaattag aaggaaacga actccaaaga 540
catgcagatg aaattctttt ggatttagga catcatgaaa ggtctatcat gacagatcta 600
tactacctca gtcaaacaga tggagcaggt gagtggcggg aaaaagaagc caaagatctg 660
acagagctgg tccagcggag aataacatat ctgcagaatc ccaaggactg cagcaaagcc 720
agaaagctgg tatgtaatat caacaaaggc tgtggctatg gatgtcaact ccatcatgtg 780
gtttactgct tcatgattgc ttatggcacc cagcgaacac tcatcttgga atctcagaat 840
tggcgctatg ctactggagg atgggagact gtgtttagac ctgtaagtga gacatgcaca 900
gacaggtctg gcctctccac tggacactgg tcaggtgaag tgaaggacaa aaatgttcaa 960
gtggtcgagc tccccattgt agacagcctc catcctcgtc ctccttactt acccttggct 1020
gtaccagaag accttgcaga tcgactcctg agagtccatg gtgatcctgc agtgtggtgg 1080
gtatcccagt ttgtcaaata cttgatccgt ccacaacctt ggctggaaag ggaaatagaa 1140
gaaaccacca agaagcttgg cttcaaacat ccagttattg gagtccatgt cagacgcact 1200
gacaaagtgg gaacagaagc agccttccat cccattgagg aatacatggt acacgttgaa 1260
gaacattttc agcttctcga acgcagaatg aaagtggata aaaaaagagt gtatctggcc 1320
actgatgacc cttctttgtt aaaggaggca aagacaaagt actccaatta tgaatttatt 1380
agtgataact ctatttcttg gtcagctgga ctacacaacc gatacacaga aaattcactt 1440
cggggcgtga tcctggatat acactttctc tcccaggctg acttccttgt gtgtactttt 1500
tcatcccagg tctgtagggt tgcttatgaa atcatgcaaa cactgcatcc tgatgcctct 1560
gcaaacttcc attctttaga tgacatctac tattttggag gccaaaatgc ccacaaccag 1620
attgcagttt atcctcacca acctcgaact aaagaggaaa tccccatgga acctggagat 1680
atcattggtg tggctggaaa ccattggaat ggttactcta aaggtgtcaa cagaaaacta 1740
ggaaaaacag gcctgtaccc ttcctacaaa gtccgagaga agatagaaac agtcaaatac 1800
cctacatatc ctgaagctga aaaatagaga tggagtgtaa gagattaaca acagaattta 1860
gttcagacca tctcagccaa gcagaagacc cagactaaca tatggttcat tgacagacat 1920
gctccgcacc aagagcaagt gggaaccctc agatgctgca ctggtggaac gcctctttgt 1980
gaagggctgc tgtgccctca agcccatg 2008

<210> 80
<211> 1728
<212> DNA
<213> *Mus musculus*

<220> 80

```
atgcgggcat ggactggttc ctggcgttgg attatgctca ttctttttgc ctgggggacc 60
ttgttatttt atataggtgg tcatttggtt cgagataatg accaccctga tcactccagc 120
agagaactct ccaagattct tgcaaagctt gaacgcttaa aacagcaaaa tgaagacttg 180
aggcgaatgg ctgagtctct ccgaatacca gaaggcccca ttgaccaggg gacagctaca 240
ggaagagtcc gtgttttaga agaacagctt gttaaggcca aagaacagat tgaaaattac 300
aagaaacaag ctagaaatgg tctggggaag gatcatgaaa tcttaagaag gaggattgaa 360
aatggagcta aagagctctg gtttttctta caaagcgaac tgaagaaatt aaagcattta 420
gaaggaaatg aactccaaag acatgcagat gaaattcttt tggatttagg acaccatgaa 480
aggtctatca tgacagatct atactacctc agtcaaacag atggagcagg ggattggcgt 540
gaaaaagagg ccaaagatct gacagagctg gtccagcgga gaataacata tctccagaat 600
cctaaggact gcagcaaagc caggaagctg gtgtgtaaca tcaataaagg ctgtggctat 660
ggttgtcaac tccatcacgt ggtctactgt ttcatgattg cttatggcac ccagcgaaca 720
ctcatcttgg aatctcagaa ttggcgctat gctactggtg gatgggagac tgtgtttaga 780
cctgtaagtg agacatgtac agacagatct ggcctctcca ctggacactg gtcaggtgaa 840
gtaaatgaca aaaacattca agtggtcgag ctccccattg tagacagcct ccatcctcgg 900
cctccttact taccactggc tgttccagaa gaccttgcag accgactcct aagagtccat 960
ggtgaccctg cagtgtggtg ggtgtcccag tttgtcaaat acttgattcg tccacaacct 1020
tggctggaaa aggaaataga agaagccacc aagaagcttg gcttcaaaca tccagttatt 1080
ggagtccatg tcagacgcac agacaaagtg ggaacagaag cagccttcca ccccatcgag 1140
gagtacatgg tacacgttga agaacatttt cagcttctcg cacgcagaat gcaagtggat 1200
aaaaaaagag tatatctggc tactgatgat cctactttgt taaaggaggc aaagacaaag 1260
tactccaatt atgaatttat tagtgataac tctatttctt ggtcagctgg actacacaat 1320
cggtacacag aaaattcact tcggggtgtg atcctggata tacactttct ctcacaggct 1380
gactttctag tgtgtacttt ttcatcccag gtctgtcggg ttgcttatga aatcatgcaa 1440
accctgcatc ctgatgcctc tgcgaacttc cattctttgg atgacatcta ctattttgga 1500
ggccaaaatg cccacaatca gattgctgtt tatcctcaca aacctcgaac tgaagaggaa 1560
attccaatgg aacctggaga tatcattggt gtggctggaa accattggga tggttattct 1620
aaaggtatca acagaaaact tggaaaaaca ggcttatatc cctcctacaa agtccgagag 1680
aagatagaaa cagtcaagta tcccacatat cctgaagctg aaaaatag              1728
```

<210> 81
<211> 979
<212> DNA
<213> Rattus norvegicus

<400> 81

actcatcttg gaatctcaga attggcgcta tgctactggt ggatgggaga ctgtgtttag 60
acctgtaagt gagacatgca cagacagatc tggcctctcc actggacact ggtcaggtga 120
agtgaatgac aaaaatattc aagtggtgga gctccccatt gtagacagcc ttcatcctcg 180
gcctccttac ttaccactgg ctgttccaga agaccttgca gatcgactcg taagagtcca 240
tggtgatcct gcagtgtggt gggtgtccca gttcgtcaaa tatttgattc gtccacaacc 300
ttggctagaa aaggaaatag aagaagccac caagaagctt ggcttcaaac atccagtcat 360
tggagtccat gtcagacgca cagacaaagt gggaacagag gcagccttcc atcccatcga 420
agagtacatg gtacatgttg aagaacattt tcagcttctc gcacgcagaa tgcaagtgga 480
taaaaaaaga gtatatctgg ctaccgatga ccctgctttg ttaaaggagg caaagacaaa 540
gtactccaat tatgaattta ttagtgataa ctctatttct tggtcagctg gactacacaa 600
tcggtacaca gaaaattcac ttcggggcgt gatcctggat atacactttc tctctcaggc 660
tgacttccta gtgtgtactt tttcatccca ggtctgtcgg gttgcttatg aaatcatgca 720
aaccctgcat cctgatgcct ctgcaaactt ccactcttta gatgacatct actattttgg 780
aggccaaaat gcccacaacc agattgccgt ttatcctcac aaacctcgaa ctgatgagga 840
aattccaatg gaacctggag atatcattgg tgtggctgga aaccattggg atggttattc 900
taaaggtgtc aacagaaaac ttggaaaaac aggcttatat ccctcctaca aagtccgaga 960
gaagatagaa acggtcaag 979

<210> 82
<211> 1728
<212> DNA
<213> Human

<400> 82
atgcggccat ggactggttc ctggcgttgg attatgctca ttctttttgc ctgggggacc 60
ttgctgtttt atataggtgg tcacttggta cgagataatg accatcctga tcactctagc 120
cgagaactgt ccaagattct ggcaaagctt gaacgcttaa aacagcagaa tgaagacttg 180
aggcgaatgg ccgaatctct ccggatacca gaaggcccta ttgatcaggg gccagctata 240
ggaagagtac gcgttttaga agagcagctt gttaaggcca aagaacagat tgaaaattac 300
aagaaacaga ccagaaatgg tctggggaag gatcatgaaa tcctgaggag gaggattgaa 360
aatggagcta aagagctctg gtttttccta cagagtgaat tgaagaaatt aaagaactta 420
gaaggaaatg aactccaaag acatgcagat gaatttcttt tggatttagg acatcatgaa 480
aggtctataa tgacggatct atactacctc agtcagacag atggagcagg tgattggcgg 540
gaaaaagagg ccaaagatct gacagaactg gttcagcgga gaataacata tcttcagaat 600
cccaaggact gcagcaaagc caaaaagctg gtgtgtaata tcaacaaagg ctgtggctat 660
ggctgtcagc tccatcatgt ggtctactgc ttcatgattg catatggcac ccagcgaaca 720

ctcatcttgg aatctcagaa ttggcgctat gctactggtg gatgggagac tgtatttagg 780
cctgtaagtg agacatgcac agacagatct ggcatctcca ctggacactg gtcaggtgaa 840
gtgaaggaca aaaatgttca agtggtcgag cttcccattg tagacagtct tcatccccgt 900
cctccatatt tacccttggc tgtaccagaa gacctcgcag atcgacttgt acgagtgcat 960
ggtgaccctg cagtgtggtg ggtgtctcag tttgtcaaat acttgatccg cccacagcct 1020
tggctagaaa aagaaataga agaagccacc aagaagcttg gcttcaaaca tccagttatt 1080
ggagtccatg tcagacgcac agacaaagtg ggaacagaag ctgccttcca tcccattgaa 1140
gagtacatgg tgcatgttga agaacatttt cagcttcttg cacgcagaat gcaagtggac 1200
aaaaaaagag tgtatttggc cacagatgac ccttctttat taaaggaggc aaaaacaaag 1260
taccccaatt atgaatttat tagtgataac tctatttcct ggtcagctgg actgcacaat 1320
cgatacacag aaaattcact tcgtggagtg atcctggata tacattttct ctctcaggca 1380
gacttcctag tgtgtacttt ttcatcccag gtctgtcgag ttgcttatga aattatgcaa 1440
acactacatc ctgatgcctc tgcaaacttc cattctttag atgacatcta ctattttggg 1500
ggccagaatg cccacaatca aattgccatt tatgctcacc aaccccgaac tgcagatgaa 1560
attcccatgg aacctggaga tatcattggt gtggctggaa atcattggga tggctattct 1620
aaaggtgtca acaggaaatt gggaaggacg ggcctatatc cctcctacaa agttcgagag 1680
aagatagaaa cggtcaagta ccccacatat cctgaggctg agaaataa 1728

<210> 83
<211> 19
<212> RNA
<213> Artificial sequence

<220>
<223> Description of Artificial Sequence: Synthetic RNA

<400> 83
gcugagucuc uccgaauac 19

<210> 84
<211> 19
<212> RNA
<213> Artificial sequence

<220>

<223> Description of Artificial Sequence: Synthetic RNA

<400> 84
gaacacucau cuuggaauc 19

<210> 85
<211> 1504
<212> DNA
<213> Cricetulus griseus

<220>
<221> CDS
<222> (1)..(1119)

<400> 85
atg gct cac gct ccc gct agc tgc ccg agc tcc agg aac tct ggg gac 48
Met Ala His Ala Pro Ala Ser Cys Pro Ser Ser Arg Asn Ser Gly Asp
1 5 10 15
ggc gat aag ggc aag ccc agg aag gtg gcg ctc atc acg ggc atc acc 96
Gly Asp Lys Gly Lys Pro Arg Lys Val Ala Leu Ile Thr Gly Ile Thr
20 25 30
ggc cag gat ggc tca tac ttg gca gaa ttc ctg ctg gag aaa gga tac 144
Gly Gln Asp Gly Ser Tyr Leu Ala Glu Phe Leu Leu Glu Lys Gly Tyr
35 40 45
gag gtt cat gga att gta cgg cga tcc agt tca ttt aat aca ggt cga 192
Glu Val His Gly Ile Val Arg Arg Ser Ser Ser Phe Asn Thr Gly Arg
50 55 60
att gaa cat tta tat aag aat cca cag gct cat att gaa gga aac atg 240
Ile Glu His Leu Tyr Lys Asn Pro Gln Ala His Ile Glu Gly Asn Met
65 70 75 80
aag ttg cac tat ggt gac ctc acc gac agc acc tgc cta gta aaa atc 288
Lys Leu His Tyr Gly Asp Leu Thr Asp Ser Thr Cys Leu Val Lys Ile
85 90 95 100
atc aat gaa gtc aaa cct aca gag atc tac aat ctt ggt gcc cag agc 336
Ile Asn Glu Val Lys Pro Thr Glu Ile Tyr Asn Leu Gly Ala Gln Ser
105 110 115

cat gtc aag att tcc ttt gac tta gca gag tac act gca gat gtt gat 384
His Val Lys Ile Ser Phe Asp Leu Ala Glu Tyr Thr Ala Asp Val Asp
120 125 130

gga gtt ggc acc ttg cgg ctt ctg gat gca att aag act tgt ggc ctt 432
Gly Val Gly Thr Leu Arg Leu Leu Asp Ala Ile Lys Thr Cys Gly Leu
135 140 145

ata aat tct gtg aag ttc tac cag gcc tca act agt gaa ctg tat gga 480
Ile Asn Ser Val Lys Phe Tyr Gln Ala Ser Thr Ser Glu Leu Tyr Gly
150 155 160

aaa gtg caa gaa ata ccc cag aaa gag acc acc cct ttc tat cca agg 528
Lys Val Gln Glu Ile Pro Gln Lys Glu Thr Thr Pro Phe Tyr Pro Arg
165 170 175 180

tcg ccc tat gga gca gcc aaa ctt tat gcc tat tgg att gta gtg aac 576
Ser Pro Tyr Gly Ala Ala Lys Leu Tyr Ala Tyr Trp Ile Val Val Asn
185 190 195

ttt cga gag gct tat aat ctc ttt gcg gtg aac ggc att ctc ttc aat 624
Phe Arg Glu Ala Tyr Asn Leu Phe Ala Val Asn Gly Ile Leu Phe Asn
200 205 210

cat gag agt cct aga aga gga gct aat ttt gtt act cga aaa att agc 672
His Glu Ser Pro Arg Arg Gly Ala Asn Phe Val Thr Arg Lys Ile Ser
215 220 225

cgg tca gta gct aag att tac ctt gga caa ctg gaa tgt ttc agt ttg 720
Arg Ser Val Ala Lys Ile Tyr Leu Gly Gln Leu Glu Cys Phe Ser Leu
230 235 240

gga aat ctg gac gcc aaa cga gac tgg ggc cat gcc aag gac tat gtc 768
Gly Asn Leu Asp Ala Lys Arg Asp Trp Gly His Ala Lys Asp Tyr Val
245 250 255 260

gag gct atg tgg ctg atg tta caa aat gat gaa cca gag gac ttt gtc 816
Glu Ala Met Trp Leu Met Leu Gln Asn Asp Glu Pro Glu Asp Phe Val
265 270 275

ata gct act ggg gaa gtt cat agt gtc cgt gaa ttt gtt gag aaa tca 864
Ile Ala Thr Gly Glu Val His Ser Val Arg Glu Phe Val Glu Lys Ser
280 285 290

ttc atg cac att gga aag acc att gtg tgg gaa gga aag aat gaa aat 912
Phe Met His Ile Gly Lys Thr Ile Val Trp Glu Gly Lys Asn Glu Asn
295 300 305

gaa gtg ggc aga tgt aaa gag acc ggc aaa att cat gtg act gtg gat 960

Glu Val Gly Arg Cys Lys Glu Thr Gly Lys Ile His Val Thr Val Asp
310 315 320

ctg aaa tac tac cga cca act gaa gtg gac ttc ctg cag gga gac tgc 1008
Leu Lys Tyr Tyr Arg Pro Thr Glu Val Asp Phe Leu Gln Gly Asp Cys
325 330 335 340

tcc aag gcg cag cag aaa ctg aac tgg aag ccc cgc gtt gcc ttt gac 1056
Ser Lys Ala Gln Gln Lys Leu Asn Trp Lys Pro Arg Val Ala Phe Asp
345 350 355

gag ctg gtg agg gag atg gtg caa gcc gat gtg gag ctc atg aga acc 1104
Glu Leu Val Arg Glu Met Val Gln Ala Asp Val Glu Leu Met Arg Thr
360 365 370

aac ccc aac gcc tga gcacctctac aaaaaaattc gcgagacatg gactatggtg 1159
Asn Pro Asn Ala
375

cagagccagc caaccagagt ccagccactc ctgagaccat cgaccataaa ccctcgactg 1219
cctgtgtcgt ccccacagct aagagctggg ccacaggttt gtgggcacca ggacggggac 1279
actccagagc taaggccact tcgcttttgt caaaggctcc tctcaatgat tttgggaaat 1339
caagaagttt aaaatcacat actcatttta cttgaaatta tgtcactaga caacttaaat 1399
ttttgagtct tgagattgtt tttctctttt cttattaaat gatctttcta tgacccagca 1459
aaaaaaaaaa aaaaaaggga tataaaaaa aaaaaaaaaa aaaaa 1504

<210> 86
<211> 376
<212> PRT
<213> Cricetulus griseus

<400> 86
Met Ala His Ala Pro Ala Ser Cys Pro Ser Ser Arg Asn Ser Gly Asp
1 5 10 15
Gly Asp Lys Gly Lys Pro Arg Lys Val Ala Leu Ile Thr Gly Ile Thr
20 25 30
Gly Gln Asp Gly Ser Tyr Leu Ala Glu Phe Leu Leu Glu Lys Gly Tyr
35 40 45
Glu Val His Gly Ile Val Arg Arg Ser Ser Ser Phe Asn Thr Gly Arg
50 55 60
Ile Glu His Leu Tyr Lys Asn Pro Gln Ala His Ile Glu Gly Asn Met

65 70 75 80
Lys Leu His Tyr Gly Asp Leu Thr Asp Ser Thr Cys Leu Val Lys Ile
85 90 95 100
Ile Asn Glu Val Lys Pro Thr Glu Ile Tyr Asn Leu Gly Ala Gln Ser
105 110 115
His Val Lys Ile Ser Phe Asp Leu Ala Glu Tyr Thr Ala Asp Val Asp
120 125 130
Gly Val Gly Thr Leu Arg Leu Leu Asp Ala Ile Lys Thr Cys Gly Leu
135 140 145
Ile Asn Ser Val Lys Phe Tyr Gln Ala Ser Thr Ser Glu Leu Tyr Gly
150 155 160
Lys Val Gln Glu Ile Pro Gln Lys Glu Thr Thr Pro Phe Tyr Pro Arg
165 170 175 180
Ser Pro Tyr Gly Ala Ala Lys Leu Tyr Ala Tyr Trp Ile Val Val Asn
185 190 195
Phe Arg Glu Ala Tyr Asn Leu Phe Ala Val Asn Gly Ile Leu Phe Asn
200 205 210
His Glu Ser Pro Arg Arg Gly Ala Asn Phe Val Thr Arg Lys Ile Ser
215 220 225
Arg Ser Val Ala Lys Ile Tyr Leu Gly Gln Leu Glu Cys Phe Ser Leu
230 235 240
Gly Asn Leu Asp Ala Lys Arg Asp Trp Gly His Ala Lys Asp Tyr Val
245 250 255 260
Glu Ala Met Trp Leu Met Leu Gln Asn Asp Glu Pro Glu Asp Phe Val
265 270 275
Ile Ala Thr Gly Glu Val His Ser Val Arg Glu Phe Val Glu Lys Ser
280 285 290
Phe Met His Ile Gly Lys Thr Ile Val Trp Glu Gly Lys Asn Glu Asn
295 300 305
Glu Val Gly Arg Cys Lys Glu Thr Gly Lys Ile His Val Thr Val Asp
310 315 320
Leu Lys Tyr Tyr Arg Pro Thr Glu Val Asp Phe Leu Gln Gly Asp Cys
325 330 335 340
Ser Lys Ala Gln Gln Lys Leu Asn Trp Lys Pro Arg Val Ala Phe Asp
345 350 355
Glu Leu Val Arg Glu Met Val Gln Ala Asp Val Glu Leu Met Arg Thr
360 365 370

Asn Pro Asn Ala
        375

<210> 87
<211> 1316
<212> DNA
<213> Cricetulus griseus

<400> 87
gccccgcccc ctccacctgg accgagagta gctggagaat tgtgcaccgg aagtagctct 60
tggactggtg gaaccctgcg caggtgcagc aacaatgggt gagccccagg gatccaggag 120
gatcctagtg acaggggcct ctggactggt gggcagagct atccagaagg tggtcgcaga 180
tggcgctggc ttacccggag aggaatgggt gtttgtctcc tccaaagatg cagatctgac 240
ggatgcagca caaacccaag ccctgttcca gaaggtacag cccacccatg tcatccatct 300
tgctgcaatg gtaggaggcc ttttccggaa tatcaaatac aacttggatt tctggaggaa 360
gaatgtgcac atcaatgaca acgtcctgca ctcagctttc gaggtgggca ctcgcaaggt 420
ggtctcctgc ctgtccacct gtatcttccc tgacaagacc acctatccta ttgatgaaac 480
aatgatccac aatggtccac cccacagcag caattttggg tactcgtatg ccaagaggat 540
gattgacgtg cagaacaggg cctacttcca gcagcatggc tgcaccttca ctgctgtcat 600
ccctaccaat gtctttggac ctcatgacaa cttcaacatt gaagatggcc atgtgctgcc 660
tggcctcatc cataaggtgc atctggccaa gagtaatggt tcagccttga ctgtttgggg 720
tacagggaaa ccacggaggc agttcatcta ctcactggac ctagcccggc tcttcatctg 780
ggtcctgcgg gagtacaatg aagttgagcc catcatcctc tcagtgggcg aggaagatga 840
agtctccatt aaggaggcag ctgaggctgt agtggaggcc atggacttct gtggggaagt 900
cacttttgat tcaacaaagt cagatgggca gtataagaag acagccagca atggcaagct 960
tcgggcctac ttgcctgatt tccgtttcac acccttcaag caggctgtga aggagacctg 1020
tgcctggttc accgacaact atgagcaggc ccggaagtga agcatgggac aagcgggtgc 1080
tcagctggca atgcccagtc agtaggctgc agtctcatca tttgcttgtc aagaactgag 1140
gacagtatcc agcaacctga gccacatgct ggtctctctg ccagggggct tcatgcagcc 1200
atccagtagg gcccatgttt gtccatcctc gggggaaggc cagaccaaca ccttgtttgt 1260
ctgcttctgc cccaacctca gtgcatccat gctggtcctg ctgtcccttg tctaga 1316

<210> 88
<211> 321
<212> PRT

<213> Cricetulus griseus

<400> 88

Met Gly Glu Pro Gln Gly Ser Arg Arg Ile Leu Val Thr Gly Gly Ser
1               5                   10                  15
Gly Leu Val Gly Arg Ala Ile Gln Lys Val Val Ala Asp Gly Ala Gly
            20                  25                  30
Leu Pro Gly Glu Glu Trp Val Phe Val Ser Ser Lys Asp Ala Asp Leu
        35                  40                  45
Thr Asp Ala Ala Gln Thr Gln Ala Leu Phe Gln Lys Val Gln Pro Thr
    50                  55                  60
His Val Ile His Leu Ala Ala Met Val Gly Gly Leu Phe Arg Asn Ile
65                  70                  75                  80
Lys Tyr Asn Leu Asp Phe Trp Arg Lys Asn Val His Ile Asn Asp Asn
                85                  90                  95
Val Leu His Ser Ala Phe Glu Val Gly Thr Arg Lys Val Val Ser Cys
            100                 105                 110
Leu Ser Thr Cys Ile Phe Pro Asp Lys Thr Thr Tyr Pro Ile Asp Glu
        115                 120                 125
Thr Met Ile His Asn Gly Pro Pro His Ser Ser Asn Phe Gly Tyr Ser
    130                 135                 140
Tyr Ala Lys Arg Met Ile Asp Val Gln Asn Arg Ala Tyr Phe Gln Gln
145                 150                 155                 160
His Gly Cys Thr Phe Thr Ala Val Ile Pro Thr Asn Val Phe Gly Pro
                165                 170                 175
His Asp Asn Phe Asn Ile Glu Asp Gly His Val Leu Pro Gly Leu Ile
            180                 185                 190
His Lys Val His Leu Ala Lys Ser Asn Gly Ser Ala Leu Thr Val Trp
        195                 200                 205
Gly Thr Gly Lys Pro Arg Arg Gln Phe Ile Tyr Ser Leu Asp Leu Ala
    210                 215                 220
Arg Leu Phe Ile Trp Val Leu Arg Glu Tyr Asn Glu Val Glu Pro Ile
225                 230                 235                 240
Ile Leu Ser Val Gly Glu Glu Asp Glu Val Ser Ile Lys Glu Ala Ala
                245                 250                 255
Glu Ala Val Val Glu Ala Met Asp Phe Cys Gly Glu Val Thr Phe Asp
            260                 265                 270

Ser Thr Lys Ser Asp Gly Gln Tyr Lys Lys Thr Ala Ser Asn Gly Lys
275 280 285
Leu Arg Ala Tyr Leu Pro Asp Phe Arg Phe Thr Pro Phe Lys Gln Ala
290 295 300
Val Lys Glu Thr Cys Ala Trp Phe Thr Asp Asn Tyr Glu Gln Ala Arg
305 310 315 320
Lys

<210> 89
<211> 1965
<212> DNA
<213> Cricetulus griseus

<400> 89
acggggggct cccggaagcg gggaccatgg cgtctctgcg cgaagcgagc ctgcggaagc 60
tgcggcgctt ttccgagatg agaggcaaac ctgtggcaac tgggaaattc tgggatgtag 120
ttgtaataac agcagctgac gaaaagcagg agcttgctta caagcaacag ttgtcggaga 180
agctgaagag aaaggaattg ccccttggag ttaactacca tgttttcact gatcctcctg 240
gaaccaaaat tggaaatgga ggatcaacac tttgttctct tcagtgcctg gaaagcctct 300
atggagacaa gtggaattcc ttcacagtcc tgttaattca ctctggtggc tacagtcaac 360
gacttcccaa tgcaagcgct ttaggaaaaa tcttcacggc tttaccactt ggtgagccca 420
tttatcagat gttggactta aaactagcca tgtacatgga tttcccctca cgcatgaagc 480
ctggagtttt ggtcacctgt gcagatgata ttgaactata cagcattggg gactctgagt 540
ccattgcatt tgagcagcct ggctttactg ccctagccca tccatctagt ctggctgtag 600
gcaccacaca tggagtattt gtattggact ctgccggttc tttgcaacat ggtgacctag 660
agtacaggca atgccaccgt ttcctccata agcccagcat tgaaaacatg caccacttta 720
atgccgtgca tagactagga agctttggtc aacaggactt gagtgggggt gacaccacct 780
gtcatccatt gcactctgag tatgtctaca cagatagcct attttacatg gatcataaat 840
cagccaaaaa gctacttgat ttctatgaaa gtgtaggccc actgaactgt gaaatagatg 900
cctatggtga ctttctgcag gcactgggac ctggagcaac tgcagagtac accaagaaca 960
cctcacacgt cactaaagag gaatcacact tgttggacat gaggcagaaa atattccacc 1020
tcctcaaggg aacacccctg aatgttgttg tccttaataa ctccaggttt tatcacattg 1080
gaacaacgga ggagtatctg ctacatttca cttccaatgg ttcgttacag gcagagctgg 1140
gcttgcaatc catagctttc agtgtctttc caaatgtgcc tgaagactcc catgagaaac 1200
cctgtgtcat tcacagcatc ctgaattcag gatgctgtgt ggcccctggc tcagtggtag 1260
aatattccag attaggacct gaggtgtcca tctcggaaaa ctgcattatc agcggttctg 1320

tcatagaaaa agctgttctg cccccatgtt ctttcgtgtg ctctttaagt gtggagataa 1380
atggacactt agaatattca actatggtgt ttggcatgga agacaacttg aagaacagtg 1440
ttaaaaccat atcagatata aagatgcttc agttctttgg agtctgtttc ctgacttgtt 1500
tagatatttg gaaccttaaa gctatggaag aactattttc aggaagtaag acgcagctga 1560
gcctgtggac tgctcgaatt ttccctgtct gttcttctct gagtgagtcg gttgcagcat 1620
cccttgggat gttaaatgcc attcgaaacc attcgccatt cagcctgagc aacttcaagc 1680
tgctgtccat ccaggaaatg cttctctgca aagatgtagg agacatgctt gcttacaggg 1740
agcaactctt tctagaaatc agttcaaaga gaaaacagtc tgattcggag aaatcttaaa 1800
tacaatggat tttgcctgga aacaggattg caaatgcagg catattctat agatctctgg 1860
gttcttcttt ctttctcccc tctctccttt cctttccctt tgatgtaatg acaaaggtaa 1920
aaatggccac ttctgatgga aaaaaaaaaa aaaaaaaaaa aaaaa 1965

<210> 90
<211> 590
<212> PRT
<213> Cricetulus griseus

<400> 90
Met Ala Ser Leu Arg Glu Ala Ser Leu Arg Lys Leu Arg Arg Phe Ser
1 5 10 15
Glu Met Arg Gly Lys Pro Val Ala Thr Gly Lys Phe Trp Asp Val Val
20 25 30
Val Ile Thr Ala Ala Asp Glu Lys Gln Glu Leu Ala Tyr Lys Gln Gln
35 40 45
Leu Ser Glu Lys Leu Lys Arg Lys Glu Leu Pro Leu Gly Val Asn Tyr
50 55 60
His Val Phe Thr Asp Pro Pro Gly Thr Lys Ile Gly Asn Gly Gly Ser
65 70 75 80
Thr Leu Cys Ser Leu Gln Cys Leu Glu Ser Leu Tyr Gly Asp Lys Trp
85 90 95
Asn Ser Phe Thr Val Leu Leu Ile His Ser Gly Gly Tyr Ser Gln Arg
100 105 110
Leu Pro Asn Ala Ser Ala Leu Gly Lys Ile Phe Thr Ala Leu Pro Leu
115 120 125
Gly Glu Pro Ile Tyr Gln Met Leu Asp Leu Lys Leu Ala Met Tyr Met
130 135 140

Asp Phe Pro Ser Arg Met Lys Pro Gly Val Leu Val Thr Cys Ala Asp
145                 150                 155                 160
Asp Ile Glu Leu Tyr Ser Ile Gly Asp Ser Glu Ser Ile Ala Phe Glu
                165                 170                 175
Gln Pro Gly Phe Thr Ala Leu Ala His Pro Ser Ser Leu Ala Val Gly
            180                 185                 190
Thr Thr His Gly Val Phe Val Leu Asp Ser Ala Gly Ser Leu Gln His
        195                 200                 205
Gly Asp Leu Glu Tyr Arg Gln Cys His Arg Phe Leu His Lys Pro Ser
    210                 215                 220
Ile Glu Asn Met His His Phe Asn Ala Val His Arg Leu Gly Ser Phe
225                 230                 235                 240
Gly Gln Gln Asp Leu Ser Gly Gly Asp Thr Thr Cys His Pro Leu His
                245                 250                 255
Ser Glu Tyr Val Tyr Thr Asp Ser Leu Phe Tyr Met Asp His Lys Ser
            260                 265                 270
Ala Lys Lys Leu Leu Asp Phe Tyr Glu Ser Val Gly Pro Leu Asn Cys
        275                 280                 285
Glu Ile Asp Ala Tyr Gly Asp Phe Leu Gln Ala Leu Gly Pro Gly Ala
    290                 295                 300
Thr Ala Glu Tyr Thr Lys Asn Thr Ser His Val Thr Lys Glu Glu Ser
305                 310                 315                 320
His Leu Leu Asp Met Arg Gln Lys Ile Phe His Leu Leu Lys Gly Thr
                325                 330                 335
Pro Leu Asn Val Val Val Leu Asn Asn Ser Arg Phe Tyr His Ile Gly
            340                 345                 350
Thr Thr Glu Glu Tyr Leu Leu His Phe Thr Ser Asn Gly Ser Leu Gln
        355                 360                 365
Ala Glu Leu Gly Leu Gln Ser Ile Ala Phe Ser Val Phe Pro Asn Val
    370                 375                 380
Pro Glu Asp Ser His Glu Lys Pro Cys Val Ile His Ser Ile Leu Asn
385                 390                 395                 400
Ser Gly Cys Cys Val Ala Pro Gly Ser Val Val Glu Tyr Ser Arg Leu
                405                 410                 415
Gly Pro Glu Val Ser Ile Ser Glu Asn Cys Ile Ile Ser Gly Ser Val
            420                 425                 430
Ile Glu Lys Ala Val Leu Pro Pro Cys Ser Phe Val Cys Ser Leu Ser

435 440 445
Val Glu Ile Asn Gly His Leu Glu Tyr Ser Thr Met Val Phe Gly Met
450 455 460
Glu Asp Asn Leu Lys Asn Ser Val Lys Thr Ile Ser Asp Ile Lys Met
465 470 475 480
Leu Gln Phe Phe Gly Val Cys Phe Leu Thr Cys Leu Asp Ile Trp Asn
485 490 495
Leu Lys Ala Met Glu Glu Leu Phe Ser Gly Ser Lys Thr Gln Leu Ser
500 505 510
Leu Trp Thr Ala Arg Ile Phe Pro Val Cys Ser Ser Leu Ser Glu Ser
515 520 525
Val Ala Ala Ser Leu Gly Met Leu Asn Ala Ile Arg Asn His Ser Pro
530 535 540
Phe Ser Leu Ser Asn Phe Lys Leu Leu Ser Ile Gln Glu Met Leu Leu
545 550 555 560
Cys Lys Asp Val Gly Asp Met Leu Ala Tyr Arg Glu Gln Leu Phe Leu
565 570 575
Glu Ile Ser Ser Lys Arg Lys Gln Ser Asp Ser Glu Lys Ser
580 585 590

<210> 91
<211> 575
<212> PRT
<213> Cricetulus griseus

<400> 91
Met Arg Ala Trp Thr Gly Ser Trp Arg Trp Ile Met Leu Ile Leu Phe
1 5 10 15
Ala Trp Gly Thr Leu Leu Phe Tyr Ile Gly Gly His Leu Val Arg Asp
20 25 30
Asn Asp His Pro Asp His Ser Ser Arg Glu Leu Ser Lys Ile Leu Ala
35 40 45
Lys Leu Glu Arg Leu Lys Gln Gln Asn Glu Asp Leu Arg Arg Met Ala
50 55 60
Glu Ser Leu Arg Ile Pro Glu Gly Pro Ile Asp Gln Gly Thr Ala Thr
65 70 75 80

Gly Arg Val Arg Val Leu Glu Glu Gln Leu Val Lys Ala Lys Glu Gln
85 90 95

Ile Glu Asn Tyr Lys Lys Gln Ala Arg Asn Asp Leu Gly Lys Asp His
100 105 110

Glu Ile Leu Arg Arg Arg Ile Glu Asn Gly Ala Lys Glu Leu Trp Phe
115 120 125

Phe Leu Gln Ser Glu Leu Lys Lys Leu Lys Lys Leu Glu Gly Asn Glu
130 135 140

Leu Gln Arg His Ala Asp Glu Ile Leu Leu Asp Leu Gly His His Glu
145 150 155 160

Arg Ser Ile Met Thr Asp Leu Tyr Tyr Leu Ser Gln Thr Asp Gly Ala
165 170 175

Gly Glu Trp Arg Glu Lys Glu Ala Lys Asp Leu Thr Glu Leu Val Gln
180 185 190

Arg Arg Ile Thr Tyr Leu Gln Asn Pro Lys Asp Cys Ser Lys Ala Arg
195 200 205

Lys Leu Val Cys Asn Ile Asn Lys Gly Cys Gly Tyr Gly Cys Gln Leu
210 215 220

His His Val Val Tyr Cys Phe Met Ile Ala Tyr Gly Thr Gln Arg Thr
225 230 235 240

Leu Ile Leu Glu Ser Gln Asn Trp Arg Tyr Ala Thr Gly Gly Trp Glu
245 250 255

Thr Val Phe Arg Pro Val Ser Glu Thr Cys Thr Asp Arg Ser Gly Leu
260 265 270

Ser Thr Gly His Trp Ser Gly Glu Val Lys Asp Lys Asn Val Gln Val
275 280 285

Val Glu Leu Pro Ile Val Asp Ser Leu His Pro Arg Pro Pro Tyr Leu
290 295 300

Pro Leu Ala Val Pro Glu Asp Leu Ala Asp Arg Leu Leu Arg Val His
305 310 315 320

Gly Asp Pro Ala Val Trp Trp Val Ser Gln Phe Val Lys Tyr Leu Ile
325 330 335

Arg Pro Gln Pro Trp Leu Glu Arg Glu Ile Glu Glu Thr Thr Lys Lys
340 345 350

Leu Gly Phe Lys His Pro Val Ile Gly Val His Val Arg Arg Thr Asp
355 360 365

Lys Val Gly Thr Glu Ala Ala Phe His Pro Ile Glu Glu Tyr Met Val

370 375 380

His Val Glu Glu His Phe Gln Leu Leu Glu Arg Arg Met Lys Val Asp

385 390 395 400

Lys Lys Arg Val Tyr Leu Ala Thr Asp Asp Pro Ser Leu Leu Lys Glu

405 410 415

Ala Lys Thr Lys Tyr Ser Asn Tyr Glu Phe Ile Ser Asp Asn Ser Ile

420 425 430

Ser Trp Ser Ala Gly Leu His Asn Arg Tyr Thr Glu Asn Ser Leu Arg

435 440 445

Gly Val Ile Leu Asp Ile His Phe Leu Ser Gln Ala Asp Phe Leu Val

450 455 460

Cys Thr Phe Ser Ser Gln Val Cys Arg Val Ala Tyr Glu Ile Met Gln

465 470 475 480

Thr Leu His Pro Asp Ala Ser Ala Asn Phe His Ser Leu Asp Asp Ile

485 490 495

Tyr Tyr Phe Gly Gly Gln Asn Ala His Asn Gln Ile Ala Val Tyr Pro

500 505 510

His Gln Pro Arg Thr Lys Glu Glu Ile Pro Met Glu Pro Gly Asp Ile

515 520 525

Ile Gly Val Ala Gly Asn His Trp Asn Gly Tyr Ser Lys Gly Val Asn

530 535 540

Arg Lys Leu Gly Lys Thr Gly Leu Tyr Pro Ser Tyr Lys Val Arg Glu

545 550 555 560

Lys Ile Glu Thr Val Lys Tyr Pro Thr Tyr Pro Glu Ala Glu Lys

565 570 575

<210> 92

<211> 575

<212> PRT

<213> Mus musculus

<400> 92

Met Arg Ala Trp Thr Gly Ser Trp Arg Trp Ile Met Leu Ile Leu Phe

1 5 10 15

Ala Trp Gly Thr Leu Leu Phe Tyr Ile Gly Gly His Leu Val Arg Asp

20 25 30

Asn Asp His Pro Asp His Ser Ser Arg Glu Leu Ser Lys Ile Leu Ala
35 40 45
Lys Leu Glu Arg Leu Lys Gln Gln Asn Glu Asp Leu Arg Arg Met Ala
50 55 60
Glu Ser Leu Arg Ile Pro Glu Gly Pro Ile Asp Gln Gly Thr Ala Thr
65 70 75 80
Gly Arg Val Arg Val Leu Glu Glu Gln Leu Val Lys Ala Lys Glu Gln
85 90 95
Ile Glu Asn Tyr Lys Lys Gln Ala Arg Asn Gly Leu Gly Lys Asp His
100 105 110
Glu Ile Leu Arg Arg Arg Ile Glu Asn Gly Ala Lys Glu Leu Trp Phe
115 120 125
Phe Leu Gln Ser Glu Leu Lys Lys Leu Lys His Leu Glu Gly Asn Glu
130 135 140
Leu Gln Arg His Ala Asp Glu Ile Leu Leu Asp Leu Gly His His Glu
145 150 155 160
Arg Ser Ile Met Thr Asp Leu Tyr Tyr Leu Ser Gln Thr Asp Gly Ala
165 170 175
Gly Asp Trp Arg Glu Lys Glu Ala Lys Asp Leu Thr Glu Leu Val Gln
180 185 190
Arg Arg Ile Thr Tyr Leu Gln Asn Pro Lys Asp Cys Ser Lys Ala Arg
195 200 205
Lys Leu Val Cys Asn Ile Asn Lys Gly Cys Gly Tyr Gly Cys Gln Leu
210 215 220
His His Val Val Tyr Cys Phe Met Ile Ala Tyr Gly Thr Gln Arg Thr
225 230 235 240
Leu Ile Leu Glu Ser Gln Asn Trp Arg Tyr Ala Thr Gly Gly Trp Glu
245 250 255
Thr Val Phe Arg Pro Val Ser Glu Thr Cys Thr Asp Arg Ser Gly Leu
260 265 270
Ser Thr Gly His Trp Ser Gly Glu Val Asn Asp Lys Asn Ile Gln Val
275 280 285
Val Glu Leu Pro Ile Val Asp Ser Leu His Pro Arg Pro Pro Tyr Leu
290 295 300
Pro Leu Ala Val Pro Glu Asp Leu Ala Asp Arg Leu Leu Arg Val His
305 310 315 320
Gly Asp Pro Ala Val Trp Trp Val Ser Gln Phe Val Lys Tyr Leu Ile

325 330 335

Arg Pro Gln Pro Trp Leu Glu Lys Glu Ile Glu Glu Ala Thr Lys Lys

340 345 350

Leu Gly Phe Lys His Pro Val Ile Gly Val His Val Arg Arg Thr Asp

355 360 365

Lys Val Gly Thr Glu Ala Ala Phe His Pro Ile Glu Glu Tyr Met Val

370 375 380

His Val Glu Glu His Phe Gln Leu Leu Ala Arg Arg Met Gln Val Asp

385 390 395 400

Lys Lys Arg Val Tyr Leu Ala Thr Asp Asp Pro Thr Leu Leu Lys Glu

405 410 415

Ala Lys Thr Lys Tyr Ser Asn Tyr Glu Phe Ile Ser Asp Asn Ser Ile

420 425 430

Ser Trp Ser Ala Gly Leu His Asn Arg Tyr Thr Glu Asn Ser Leu Arg

435 440 445

Gly Val Ile Leu Asp Ile His Phe Leu Ser Gln Ala Asp Phe Leu Val

450 455 460

Cys Thr Phe Ser Ser Gln Val Cys Arg Val Ala Tyr Glu Ile Met Gln

465 470 475 480

Thr Leu His Pro Asp Ala Ser Ala Asn Phe His Ser Leu Asp Asp Ile

485 490 495

Tyr Tyr Phe Gly Gly Gln Asn Ala His Asn Gln Ile Ala Val Tyr Pro

500 505 510

His Lys Pro Arg Thr Glu Glu Glu Ile Pro Met Glu Pro Gly Asp Ile

515 520 525

Ile Gly Val Ala Gly Asn His Trp Asp Gly Tyr Ser Lys Gly Ile Asn

530 535 540

Arg Lys Leu Gly Lys Thr Gly Leu Tyr Pro Ser Tyr Lys Val Arg Glu

545 550 555 560

Lys Ile Glu Thr Val Lys Tyr Pro Thr Tyr Pro Glu Ala Glu Lys

565 570 575

<210> 93

<211> 1245

<212> DNA

<213> Cricetulus griseus

<400> 93

```
gaacttcacc caagccatgt gacaattgaa ggctgtaccc ccagacccta acatcttgga    60
gccctgtaga ccagggagtg cttctggccg tggggtgacc tagctcttct accaccatga   120
acagggcccc tctgaagcgg tccaggatcc tgcgcatggc gctgactgga ggctccactg   180
cctctgagga ggcagatgaa gacagcagga acaagccgtt tctgctgcgg gcgctgcaga   240
tcgcgctggt cgtctctctc tactgggtca cctccatctc catggtattc ctcaacaagt   300
acctgctgga cagcccctcc ctgcagctgg ataccccctat cttcgtcact ttctaccaat   360
gcctggtgac ctctctgctg tgcaagggcc tcagcactct ggccacctgc tgccctggca   420
ccgttgactt ccccaccctg aacctggacc ttaaggtggc ccgcagcgtg ctgccactgt   480
cggtagtctt cattggcatg ataagtttca ataacctctg cctcaagtac gtaggggtgg   540
ccttctacaa cgtggggcgc tcgctcacca ccgtgttcaa tgtgcttctg tcctacctgc   600
tgctcaaaca gaccacttcc ttctatgccc tgctcacatg tggcatcatc attggtggtt   660
tctggctggg tatagaccaa gagggagctg agggcaccct gtccctcata ggcaccatct   720
tcggggtgct ggccagcctc tgcgtctccc tcaatgccat ctataccaag aaggtgctcc   780
cagcagtgga caacagcatc tggcgcctaa ccttctataa caatgtcaat gcctgtgtgc   840
tcttcttgcc cctgatggtt ctgctgggtg agctccgtgc cctccttgac tttgctcatc   900
tgtacagtgc ccacttctgg ctcatgatga cgctgggtgg cctcttcggc tttgccattg   960
gctatgtgac aggactgcag atcaaattca ccagtcccct gacccacaat gtatcaggca  1020
cagccaaggc ctgtgcgcag acagtgctgg ccgtgctcta ctatgaagag actaagagct  1080
tcctgtggtg gacaagcaac ctgatggtgc tgggtggctc ctcagcctat acctgggtca  1140
ggggctggga gatgcagaag acccaagagg accccagctc caaagagggt gagaagagtg  1200
ctattggggt gtgagcttct tcaggacct gggactgaac ccaag                    1245
```

<210> 94

<211> 365

<212> PRT

<213> Cricetulus griseus

<400> 94

```
Met Asn Arg Ala Pro Leu Lys Arg Ser Arg Ile Leu Arg Met Ala Leu
1               5                   10                  15
Thr Gly Gly Ser Thr Ala Ser Glu Glu Ala Asp Glu Asp Ser Arg Asn
            20                  25                  30
Lys Pro Phe Leu Leu Arg Ala Leu Gln Ile Ala Leu Val Val Ser Leu
        35                  40                  45
```

Tyr Trp Val Thr Ser Ile Ser Met Val Phe Leu Asn Lys Tyr Leu Leu
50 55 60

Asp Ser Pro Ser Leu Gln Leu Asp Thr Pro Ile Phe Val Thr Phe Tyr
65 70 75 80

Gln Cys Leu Val Thr Ser Leu Leu Cys Lys Gly Leu Ser Thr Leu Ala
85 90 95

Thr Cys Cys Pro Gly Thr Val Asp Phe Pro Thr Leu Asn Leu Asp Leu
100 105 110

Lys Val Ala Arg Ser Val Leu Pro Leu Ser Val Val Phe Ile Gly Met
115 120 125

Ile Ser Phe Asn Asn Leu Cys Leu Lys Tyr Val Gly Val Ala Phe Tyr
130 135 140

Asn Val Gly Arg Ser Leu Thr Thr Val Phe Asn Val Leu Leu Ser Tyr
145 150 155 160

Leu Leu Leu Lys Gln Thr Thr Ser Phe Tyr Ala Leu Leu Thr Cys Gly
165 170 175

Ile Ile Ile Gly Gly Phe Trp Leu Gly Ile Asp Gln Glu Gly Ala Glu
180 185 190

Gly Thr Leu Ser Leu Ile Gly Thr Ile Phe Gly Val Leu Ala Ser Leu
195 200 205

Cys Val Ser Leu Asn Ala Ile Tyr Thr Lys Lys Val Leu Pro Ala Val
210 215 220

Asp Asn Ser Ile Trp Arg Leu Thr Phe Tyr Asn Asn Val Asn Ala Cys
225 230 235 240

Val Leu Phe Leu Pro Leu Met Val Leu Leu Gly Glu Leu Arg Ala Leu
245 250 255

Leu Asp Phe Ala His Leu Tyr Ser Ala His Phe Trp Leu Met Met Thr
260 265 270

Leu Gly Gly Leu Phe Gly Phe Ala Ile Gly Tyr Val Thr Gly Leu Gln
275 280 285

Ile Lys Phe Thr Ser Pro Leu Thr His Asn Val Ser Gly Thr Ala Lys
290 295 300

Ala Cys Ala Gln Thr Val Leu Ala Val Leu Tyr Tyr Glu Glu Thr Lys
305 310 315 320

Ser Phe Leu Trp Trp Thr Ser Asn Leu Met Val Leu Gly Gly Ser Ser
325 330 335

Ala Tyr Thr Trp Val Arg Gly Trp Glu Met Gln Lys Thr Gln Glu Asp

340 345 350

Pro Ser Ser Lys Glu Gly Glu Lys Ser Ala Ile Gly Val

355 360 365

<210> 95

<211> 1095

<212> DNA

<213> Homo sapiens

<400> 95

atgaataggg cccctctgaa gcggtccagg atcctgcaca tggcgctgac cggggcctca 60
gacccctctg cagaggcaga ggccaacggg gagaagccct ttctgctgcg ggcattgcag 120
atcgcgctgg tggtctccct ctactgggtc acctccatct ccatggtgtt ccttaataag 180
tacctgctgg acagcccctc cctgcggctg gacacccccca tcttcgtcac cttctaccag 240
tgcctggtga ccacgctgct gtgcaaaggc ctcagcgctc tggccgcctg ctgccctggt 300
gccgtggact tccccagctt gcgcctggac ctcagggtgg cccgcagcgt cctgcccctg 360
tcggtggtct tcatcggcat gatcaccttc aataacctct gcctcaagta cgtcggtgtg 420
gccttctaca atgtgggccg ctcactcacc accgtcttca acgtgctgct ctcctacctg 480
ctgctcaagc agaccacctc cttctatgcc ctgctcacct gcggtatcat catcgggggc 540
ttctggcttg gtgtggacca ggagggggca gaaggcaccc tgtcgtggct gggcaccgtc 600
ttcggcgtgc tggctagcct ctgtgtctcg ctcaacgcca tctacaccac gaaggtgctc 660
ccggcggtgg acggcagcat ctggcgcctg actttctaca acaacgtcaa cgcctgcatc 720
ctcttcctgc ccctgctcct gctgctcggg gagcttcagg ccctgcgtga ctttgcccag 780
ctgggcagtg cccacttctg gggggatgatg acgctgggcg gcctgtttgg ctttgccatc 840
ggctacgtga caggactgca gatcaagttc accagtccgc tgacccacaa tgtgtcgggc 900
acggccaagg cctgtgccca gacagtgctg gccgtgctct actacgagga gaccaagagc 960
ttcctctggt ggacgagcaa catgatggtg ctgggcggct cctccgccta cacctgggtc 1020
aggggctggg agatgaagaa gactccggag gagcccagcc ccaaagacag cgagaagagc 1080
gccatggggg tgtga 1095

<210> 96

<211> 364

<212> PRT

<213> Homo sapiens

<400> 96

Met Asn Arg Ala Pro Leu Lys Arg Ser Arg Ile Leu His Met Ala Leu
1 5 10 15

Thr Gly Ala Ser Asp Pro Ser Ala Glu Ala Glu Ala Asn Gly Glu Lys
20 25 30

Pro Phe Leu Leu Arg Ala Leu Gln Ile Ala Leu Val Val Ser Leu Tyr
35 40 45

Trp Val Thr Ser Ile Ser Met Val Phe Leu Asn Lys Tyr Leu Leu Asp
50 55 60

Ser Pro Ser Leu Arg Leu Asp Thr Pro Ile Phe Val Thr Phe Tyr Gln
65 70 75 80

Cys Leu Val Thr Thr Leu Leu Cys Lys Gly Leu Ser Ala Leu Ala Ala
85 90 95

Cys Cys Pro Gly Ala Val Asp Phe Pro Ser Leu Arg Leu Asp Leu Arg
100 105 110

Val Ala Arg Ser Val Leu Pro Leu Ser Val Val Phe Ile Gly Met Ile
115 120 125

Thr Phe Asn Asn Leu Cys Leu Lys Tyr Val Gly Val Ala Phe Tyr Asn
130 135 140

Val Gly Arg Ser Leu Thr Thr Val Phe Asn Val Leu Leu Ser Tyr Leu
145 150 155 160

Leu Leu Lys Gln Thr Thr Ser Phe Tyr Ala Leu Leu Thr Cys Gly Ile
165 170 175

Ile Ile Gly Gly Phe Trp Leu Gly Val Asp Gln Glu Gly Ala Glu Gly
180 185 190

Thr Leu Ser Trp Leu Gly Thr Val Phe Gly Val Leu Ala Ser Leu Cys
195 200 205

Val Ser Leu Asn Ala Ile Tyr Thr Thr Lys Val Leu Pro Ala Val Asp
210 215 220

Gly Ser Ile Trp Arg Leu Thr Phe Tyr Asn Asn Val Asn Ala Cys Ile
225 230 235 240

Leu Phe Leu Pro Leu Leu Leu Leu Leu Gly Glu Leu Gln Ala Leu Arg
245 250 255

Asp Phe Ala Gln Leu Gly Ser Ala His Phe Trp Gly Met Met Thr Leu
260 265 270

Gly Gly Leu Phe Gly Phe Ala Ile Gly Tyr Val Thr Gly Leu Gln Ile
275 280 285

Lys Phe Thr Ser Pro Leu Thr His Asn Val Ser Gly Thr Ala Lys Ala
290 295 300

Cys Ala Gln Thr Val Leu Ala Val Leu Tyr Tyr Glu Glu Thr Lys Ser
305 310 315 320

Phe Leu Trp Trp Thr Ser Asn Met Met Val Leu Gly Gly Ser Ser Ala
325 330 335

Tyr Thr Trp Val Arg Gly Trp Glu Met Lys Lys Thr Pro Glu Glu Pro
340 345 350

Ser Pro Lys Asp Ser Glu Lys Ser Ala Met Gly Val
355 360 364

<210> 97
<211> 2609
<212> DNA
<213> Mus musculus

<400> 97
gagccgaggg tggtgctgca ggtgcacccg agggcaccgc cgagggtgag caccaggtcc 60
ctgcatcagc caggacacca gagcccagtc gggtggacgg acgggcgcct ctgaagcggt 120
ccaggatcct gcgcatggcg ctgactggag tctctgctgt ctccgaggag tcagagagcg 180
ggaacaagcc atttctgctc cgggctctgc agatcgcgct ggtggtctct ctctactggg 240
tcacctccat ttccatggta ttcctcaaca agtacctgct ggacagcccc tccctgcagc 300
tggataccca catttttgtc accttctacc aatgcctggt gacctcactg ctgtgcaagg 360
gcctcagcac tctggccacc tgctgccccg gcatggtaga cttccccacc ctaaacctgg 420
acctcaaggt ggcccgaagt gtgctgccgc tgtcagtggt ctttatcggc atgataacct 480
tcaataacct ctgcctcaag tacgtagggg tgcccttcta caacgtggga cgctcgctca 540
ccaccgtgtt caacgttctt ctctcctacc tgctgctcaa acagaccact tccttctatg 600
ccctgctcac ctgcggcgtc atcattggtg gtttctggct gggtatagac caagaaggag 660
ctgagggaac cttgtccctg acgggcacca tcttcggggt gctggccagc ctctgcgtct 720
ccctcaatgc catctatacc aagaaggtgc tccctgcagt agaccacagt atctggcgcc 780
taaccttcta taacaatgtc aatgcctgcg tgctcttctt gcccctgatg atagtgctgg 840
gcgagctccg tgccctcctg gccttcactc atctgagcag tgcccacttc tggctcatga 900
tgacgctggg tggcctgttt ggctttgcca tcggctatgt gacaggactg cagatcaaat 960
tcaccagtcc cctgacccat aacgtgtcag gcacggccaa ggcctgtgca cagacagtgc 1020
tggccgtgct ctactacgaa gagattaaga gcttcctgtg gtggacaagc aacctgatgg 1080
tgctgggtgg ctcctccgcc tacacctggg tcaggggctg ggagatgcag aagacccagg 1140

aggaccccag ctccaaagat ggtgagaaga gtgctatcag ggtgtgagct ccttcaggga 1200
gccagggctg agctcgggtg gggcctgccc agcacggaag gcttcccata gagcctactg 1260
ggtatggccc tgagcaataa tgtttacatc cttctcagaa gaccatctaa gaagagccag 1320
gttctttcct gataatgtca gaaagctgcc aaatctcctg cctgccccat cttctagtct 1380
tgggaaagcc ctaccaggag tggcaccctt cctgcctcct cctggggcct gtctacctcc 1440
atatggtctc tggggttggg gccagctgca ctctttgggc actggactga tgaagtgatg 1500
tcttactttc tacacaaggg agatgggttg tgaccctact atagctagtt gaagggagct 1560
gtgtaacccc acatctctgg ggccctgggc aggtagcata atagctaggt gctattaaca 1620
tcaataacac ttcagactac ctttggaggc agttgggagc tgagccgaga gagagagatg 1680
gccattctgc cctcttctgt gtggatgggt atgacagacc aactgtccat ggggtgactg 1740
acacctccac acttcatatt ttcaacttta gaaaaggggg agccacacgt tttacagatt 1800
aagtggagtg atgaatgcct ctacagcccc taaccccact ttccctgcct ggcttctctt 1860
ggcccagaag ggccaccatc ctgttctcca acacctgacc cagctatctg gctatactct 1920
ctttctgtac tcccttcccc ttcccccccc cattagcctc ctccccaaca cctccatctt 1980
caggcaggaa gtggggtcca ctcagcctct gttcccatct gcttggaccc ctgagcctct 2040
catgaaggta ggcttatgtt ctctgaggct ggggccggag gagcgcactg attctcggag 2100
ttatcccatc aggctcctgt cacaaaatag cctaggccgt gtgtctaaga acagtggagg 2160
ttggcttata actgttctgg gggcagcgaa gcccacatca aggtactcat agacccagta 2220
tttctgagga aacccctgtc cacatcctca cttggtaaag gcacagataa tctccctcag 2280
gcctcttgta taggagcact agccctggga gggctccgcc ccatgacctg atcaccccaa 2340
agccttcaac agaaggattc caacatgaat ttggggacag aagcactcag accacgatgc 2400
ccagcaccac accctcctat cctcagggta gctgtcactg tcctagtccc ttctgtttgg 2460
ccttttgtac cctcatttcc ttggcgtcat gtttgatgtc tttgtctctt tcgtgagaag 2520
atggggaaac catgtcagcc tctgcttccg acttcccatg ggttctaatg aagttggtgg 2580
ggcctgatgc cctgagttgt atgtgattt 2609

<210> 98
<211> 1053
<212> DNA
<213> Rattus norvegiucus

<400> 98
atggcgctga ctggagcctc tgctgtctct gaggaggcag acagcgagaa caagccattt 60
ctgctacggg ctctgcagat cgcgctggtg gtttctctct actgggtcac ctccatctcc 120
atggtattcc tcaacaagta cctgctggac agcccctccc tgcagctgga tacccccatc 180
ttcgtcacct tctaccaatg cctggtgacc tcactgctgt gcaagggcct cagcactctg 240

gccacctgct gccctggcat ggtagacttc cccaccctaa acctggacct caaggtggcc 300
cgaagtgtgc tgccgctgtc cgtggtcttt atcggcatga taaccttcaa taacctctgc 360
ctcaagtacg tgggggtggc cttctacaac gtgggacgct cgctcactac cgtgttcaat 420
gtgcttctct cctacctgct gcttaaacag accacttcct tttatgccct gctcacctgt 480
gccatcatca ttggtggttt ctggctggga atagatcaag agggagctga gggcaccctg 540
tccctgacgg gcaccatctt cggggtgctg gccagcctct gtgtctcact caatgccatc 600
tacaccaaga aggtgctccc tgccgtagac cacagtatct ggcgcctaac cttctataac 660
aacgtcaacg cctgtgtgct cttcttgccc ctgatggtag tgctgggcga gctccatgct 720
ctcctggcct tcgctcatct gaacagcgcc cacttctggg tcatgatgac gctgggtgga 780
ctcttcggct ttgccattgg ctatgtgaca ggactgcaga tcaaattcac cagtcccctg 840
acccataatg tgtcgggcac agccaaggcc tgtgcacaga cagtgctggc tgtgctctac 900
tatgaagaga ttaagagctt cctgtggtgg acaagcaact tgatggtgct gggtggctcc 960
tctgcctaca cctgggtcag ggctgggag atgcagaaga cccaggagga ccccagctcc 1020
aaagagggtg agaagagtgc tatcggggtg tga 1053

<210> 99
<211> 360
<212> PRT
<213> Mus musculus

<400> 99

Met Ala Leu Thr Gly Val Ser Ala Val Ser Glu Glu Ser Glu Ser Gly
1 5 10 15
Asn Lys Pro Phe Leu Leu Arg Ala Leu Gln Ile Ala Leu Val Val Ser
20 25 30
Leu Tyr Trp Val Thr Ser Ile Ser Met Val Phe Leu Asn Lys Tyr Leu
35 40 45
Leu Asp Ser Pro Ser Leu Gln Leu Asp Thr Pro Ile Phe Val Thr Phe
50 55 60
Tyr Gln Cys Leu Val Thr Ser Leu Leu Cys Lys Gly Leu Ser Thr Leu
65 70 75 80
Ala Thr Cys Cys Pro Gly Met Val Asp Phe Pro Thr Leu Asn Leu Asp
85 90 95
Leu Lys Val Ala Arg Ser Val Leu Pro Leu Ser Val Val Phe Ile Gly
100 105 110
Met Ile Thr Phe Asn Asn Leu Cys Leu Lys Tyr Val Gly Val Pro Phe

115 120 125

Tyr Asn Val Gly Arg Ser Leu Thr Thr Val Phe Asn Val Leu Leu Ser

130 135 140

Tyr Leu Leu Leu Lys Gln Thr Thr Ser Phe Tyr Ala Leu Leu Thr Cys

145 150 155 160

Gly Val Ile Ile Gly Gly Phe Trp Leu Gly Ile Asp Gln Glu Gly Ala

165 170 175

Glu Gly Thr Leu Ser Leu Thr Gly Thr Ile Phe Gly Val Leu Ala Ser

180 185 190

Leu Cys Val Ser Leu Asn Ala Ile Tyr Thr Lys Lys Val Leu Pro Ala

195 200 205

Val Asp His Ser Ile Trp Arg Leu Thr Phe Tyr Asn Asn Val Asn Ala

210 215 220

Cys Val Leu Phe Leu Pro Leu Met Ile Val Leu Gly Glu Leu Arg Ala

225 230 235 240

Leu Leu Ala Phe Thr His Leu Ser Ser Ala His Phe Trp Leu Met Met

245 250 255

Thr Leu Gly Gly Leu Phe Gly Phe Ala Ile Gly Tyr Val Thr Gly Leu

260 265 270

Gln Ile Lys Phe Thr Ser Pro Leu Thr His Asn Val Ser Gly Thr Ala

275 280 285

Lys Ala Cys Ala Gln Thr Val Leu Ala Val Leu Tyr Tyr Glu Glu Ile

290 295 300

Lys Ser Phe Leu Trp Trp Thr Ser Asn Leu Met Val Leu Gly Gly Ser

305 310 315 320

Ser Ala Tyr Thr Trp Val Arg Gly Trp Glu Met Gln Lys Thr Gln Glu

325 330 335

Asp Pro Ser Ser Lys Asp Gly Glu Lys Ser Ala Ile Arg Val

340 345 350

<210> 100

<211> 350

<212> PRT

<213> Rattus norvegiucus

<400> 100

Met Ala Leu Thr Gly Ala Ser Ala Val Ser Glu Glu Ala Asp Ser Glu
1 5 10 15
Asn Lys Pro Phe Leu Leu Arg Ala Leu Gln Ile Ala Leu Val Val Ser
20 25 30
Leu Tyr Trp Val Thr Ser Ile Ser Met Val Phe Leu Asn Lys Tyr Leu
35 40 45
Leu Asp Ser Pro Ser Leu Gln Leu Asp Thr Pro Ile Phe Val Thr Phe
50 55 60
Tyr Gln Cys Leu Val Thr Ser Leu Leu Cys Lys Gly Leu Ser Thr Leu
65 70 75 80
Ala Thr Cys Cys Pro Gly Met Val Asp Phe Pro Thr Leu Asn Leu Asp
85 90 95
Leu Lys Val Ala Arg Ser Val Leu Pro Leu Ser Val Val Phe Ile Gly
100 105 110
Met Ile Thr Phe Asn Asn Leu Cys Leu Lys Tyr Val Gly Val Ala Phe
115 120 125
Tyr Asn Val Gly Arg Ser Leu Thr Thr Val Phe Asn Val Leu Leu Ser
130 135 140
Tyr Leu Leu Leu Lys Gln Thr Thr Ser Phe Tyr Ala Leu Leu Thr Cys
145 150 155 160
Ala Ile Ile Ile Gly Gly Phe Trp Leu Gly Ile Asp Gln Glu Gly Ala
165 170 175
Glu Gly Thr Leu Ser Leu Thr Gly Thr Ile Phe Gly Val Leu Ala Ser
180 185 190
Leu Cys Val Ser Leu Asn Ala Ile Tyr Thr Lys Lys Val Leu Pro Ala
195 200 205
Val Asp His Ser Ile Trp Arg Leu Thr Phe Tyr Asn Asn Val Asn Ala
210 215 220
Cys Val Leu Phe Leu Pro Leu Met Val Val Leu Gly Glu Leu His Ala
225 230 235 240
Leu Leu Ala Phe Ala His Leu Asn Ser Ala His Phe Trp Val Met Met
245 250 255
Thr Leu Gly Gly Leu Phe Gly Phe Ala Ile Gly Tyr Val Thr Gly Leu
260 265 270
Gln Ile Lys Phe Thr Ser Pro Leu Thr His Asn Val Ser Gly Thr Ala
275 280 285
Lys Ala Cys Ala Gln Thr Val Leu Ala Val Leu Tyr Tyr Glu Glu Ile

```
    290                 295                 300
Lys Ser Phe Leu Trp Trp Thr Ser Asn Leu Met Val Leu Gly Gly Ser
305                 310                 315                 320
Ser Ala Tyr Thr Trp Val Arg Gly Trp Glu Met Gln Lys Thr Gln Glu
                325                 330                 335
Asp Pro Ser Ser Lys Glu Gly Glu Lys Ser Ala Ile Gly Val
            340                 345                 350
```